# EUROPEAN PATENT SPECIFICATION

(11) **EP 4 421 076 B1**
(45) Date of publication and mention of the grant of the patent: **18.11.2026**
(21) Application number: 22882976.8
(22) Date of filing: 21.10.2022
(51) Int. Cl.: C07D 471/12, C07D 471/14, C07D 487/12, C07D 487/14, A61K 31/4985, A61P 35/00, C07D 487/16

(54) **NITROGEN-CONTAINING TETRACYCLIC COMPOUND, PREPARATION METHOD THEREFOR, AND MEDICAL USE THEREOF**
STICKSTOFFHALTIGE TETRACYCLISCHE VERBINDUNG, HERSTELLUNGSVERFAHREN DAFÜR UND MEDIZINISCHE VERWENDUNG DAVON
COMPOSÉ TÉTRACYCLIQUE CONTENANT DE L'AZOTE, SON PROCÉDÉ DE PRÉPARATION ET SON UTILISATION MÉDICALE

(30) Priority: 22.10.2021 CN 202111231664; 15.12.2021 CN 202111536255; 23.02.2022 CN 202210166912; 12.04.2022 CN 202210382949
(43) Date of publication of application: 28.08.2024
(73) Proprietor: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); CAI, Guodong, Shanghai 200245 (CN); CHEN, Yang, Shanghai 200245 (CN); HE, Feng, Shanghai 200245 (CN); TAO, Weikang, Shanghai 200245 (CN)
(74) Representative: Dragotti & Associati S.P.A.
(86) International application number: PCT/CN2022/126650
(87) International publication number: WO 2023/066371

(56) References cited:
- WO-A1-2020/221239
- WO-A1-2021/000885
- WO-A1-2021/023154
- CN-A- 110 603 258
- CN-A- 112 074 520
- CN-A- 112 390 818
- CN-A- 112 745 335

## Description

### TECHNICAL FIELD

The present disclosure belongs to the field of pharmaceutics and relates to a nitrogen-containing tetracyclic compound, a preparation method therefor, and pharmaceutical use thereof. Specifically, the present disclosure relates to a nitrogen-containing tetracyclic compound represented by general formula (IM), a preparation method therefor, a pharmaceutical composition comprising the compound, use thereof as a therapeutic agent, in particular as a KRAS G12C inhibitor, and use thereof in the preparation of a medicament for treating and/or preventing tumors.

### BACKGROUND

The RAS (rat sarcoma viral oncogene homolog) family belongs to the small GTPase superfamily and is widely expressed in various eukaryotes. There are three RAS genes (HRAS, KRAS, and NARS) in humans, which are expressed as four highly related RAS small GTPases (HRAS, KRAS4A, KARS4B, and NRAS). They act as binary switches for GDP-GTP regulation. They generally exist in two forms: a GDP (guanosine diphosphate)-bound form when in an inactive state and a GTP (guanosine triphosphate)-bound form when in an activated state. RAS proteins regulate multiple downstream pathways including RAP-MEK-ERK and PI3K/Akt/mTOR by switching between the two active states, thereby affecting cell growth, proliferation, and differentiation (Nat Rev Cancer, 2007, 7, 295-308). The RAS genes exhibit high mutation rates in various tumors such as pancreatic cancer, colorectal cancer, and non-small cell lung cancer. Activated mutant RAS proteins promote abnormal signal transduction, thereby causing cancer development and progression, as well as resistance to targeted drugs. The KRAS mutation is the gene with the highest mutation rate among human oncogenes and accounts for 20-30% of all tumors.

In recent years, molecular biology has made significant progress in the study of the mutated forms of the KRAS protein and its signaling pathway. However, the development of related targeted drugs continues to be a formidable challenge. In terms of chemical drug development, the high affinity between KRAS and GTP, which is up to 60 pM, combined with the mM-level concentrations of GTP inside cells, necessitates that directly competing molecules must have exceptionally high compound affinity. To date, there has been no success in this area. In terms of biological drug development, antibody drugs penetrate cell membranes to target the KRAS protein, resulting in relatively low drug delivery efficiency. Therefore, many researchers have sought alternative routes, aiming to inhibit the activity of kinases such as RAF, MEK, and ERK in the downstream signaling pathways of KRAS, in hopes of inhibiting the KRAS pathway. While such compounds have shown some therapeutic effect, their inability to completely block the KRAS signal, along with significant target-related toxic and side effects, results in their poor efficacy against tumors with KRAS mutations. Therefore, the development of KRAS inhibitors with new mechanisms of action holds great clinical application value.

KRAS mutations are mainly point mutations, including mutations at amino acid positions 12, 13, and 61. The mutation of glycine at position 12 into cysteine (G12C) is the most common of them. This mutation is found in a significant proportion of lung cancers, especially non-small cell lung cancer (14%). In addition, it is expressed in some patients with colorectal cancer (4%) and pancreatic cancer (2%). In the U.S. cancer population, the incidence of this gene mutation is even higher than the combined incidence of ALK, RET, and TRK gene mutations.

Confronted by the challenges of KRAS protein druggability, Professor Kevan M. Shokat from the University of California, San Francisco, was the first to verify that certain special compounds can bind covalently to the KRAS G12C mutant protein. Further research revealed that these covalent compounds can bind to the cysteine at position 12 of the KRAS mutant protein and occupy a hydrophobic allosteric regulatory pocket in the switch-II regions. The bound KRAS G12C mutant is irreversibly locked in an inactive state, thereby disrupting the signaling pathways depending on that protein and the survival of cancer cells (Nature 2013, 503, 548-551). The KRAS G12C small-molecule inhibitor ARS-1620 has been shown to effectively inhibit tumor growth in various KRAS G12C mutant tumor models, and even lead to complete tumor regression. Since KRAS G12C is a mutant protein in tumor cells, and the wild-type KRAS does not have this mutation site, it provides a perfect tumor-selective target (Cell, 2018, 572, 578-589).

KRAS G12C has drawn numerous renowned pharmaceutical companies around the world to the research and development of related new drugs. Although the fastest progressing small-molecule KRAS G12C inhibitor, Sotorasib (AMG510) by Amgen, was approved by the FDA on May 28, 2021, for patients with non-small cell lung cancer carrying the KRAS G12C mutation who have previously undergone at least one systemic treatment, Eli Lilly and Company's next-generation KRAS G12C inhibitor LY3537982 has been attracting even more interest. Eli Lilly and Company presented preclinical data on LY3537982 at the American Association for Cancer Research (AACR) Annual Meeting in April 2021. The data revealed that LY3537982 is over 10 times more effective than Sotorasib in inhibiting cellular activity, and it entered phase one clinical trials in July 2021. Thus, there is still a clinical need for KRAS G12C inhibitors that are highly selective, safe, and effective.

Patent applications that disclose KRAS G12C inhibitors include WO2014152588A1, WO2015054572A1, WO2016164675A1, WO2017087528A1, WO2017201161A1, WO2018119183A2, WO2018206539A1, WO2018217651A1, WO2019099524A1, WO2019215203A1, WO2020081282A1, WO2020178282A1, WO2021118877A1, and WO2021000885.

**SUMMARY** Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present disclosure aims to provide a compound represented by general formula (IM) or a pharmaceutically acceptable salt thereof: 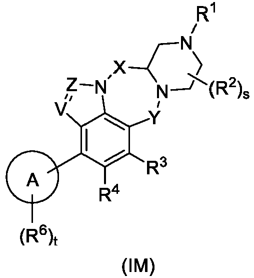 wherein:
X is C(R^{a}R^{b}) or C(R^{a}Rb)-C(R^{c}R^{d});
Y is C(O) or CH₂;
Z is CR^{5a} or N;
V is CR⁵ or N;
ring A is aryl or heteroaryl;
R^{a}, R^{b}, R^{c}, and R^{d} are identical or different at each occurrence and are each independently selected from the group consisting of a hydrogen atom, halogen, alkyl, haloalkyl, hydroxyalkyl, alkoxy, haloalkoxy, alkenyl, alkynyl, hydroxy, and cyano;
R¹ is selected from the group consisting of cyano, 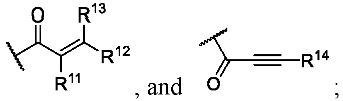
each R² is identical or different and is independently selected from the group consisting of a hydrogen atom, halogen, cyano, alkyl, alkoxy, hydroxy, and amino, wherein the alkyl and alkoxy are each independently optionally substituted with one or more substituents selected from the group consisting of halogen, cyano, amino, and hydroxy;
R³, R⁴, R⁵, and R^{5a} are identical or different and are each independently selected from the group consisting of a hydrogen atom, halogen, cyano, alkyl, alkenyl, alkynyl, -NR^{7a}R^{7b}, -C(O)R⁸, -OR⁸, -S(O)ₚR⁸, cycloalkyl, heterocyclyl, aryl and heteroaryl, wherein the alkyl, cycloalkyl, heterocyclyl, aryl, and heteroaryl are each independently optionally substituted with one or more substituents selected from the group consisting of halogen, alkyl, haloalkyl, cyano, -NR^{7c}R^{7d}, -OR^{8a}, cycloalkyl, heterocyclyl, aryl, and heteroaryl;
each R⁶ is identical or different and is independently selected from the group consisting of a hydrogen atom, halogen, cyano, alkyl, alkenyl, alkynyl, -NR^{9a}R^{9b}, -C(O)NR^{9a}R^{9b}, -C(O)R¹⁰, -C(O)OR¹⁰, -OC(O)R¹⁰, -OR¹⁰, -S(O)ₚR¹⁰, -S(O)ₚNR^{9a}R^{9b}, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, haloalkyl, cyano, -NR^{9c}R^{9d}, -OR^{10a}, cycloalkyl, heterocyclyl, aryl, and heteroaryl;
R¹¹, R¹², R¹³, and R¹⁴ are identical or different and are each independently selected from the group consisting of a hydrogen atom, halogen, alkyl, -NR^{15a}R^{15b}, -OR¹⁶, cyano, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, oxo, alkyl, haloalkyl, alkoxy, haloalkoxy, cyano, -NR^{15c}R^{15d}, hydroxy, hydroxyalkyl, cycloalkyl, heterocyclyl, aryl, and heteroaryl;
R⁸, R^{8a}, R¹⁰, R^{10a}, and R¹⁶ are identical or different at each occurrence and are each independently selected from the group consisting of a hydrogen atom, alkyl, hydroxyalkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkenyl, alkynyl, oxo, alkoxy, haloalkyl, haloalkoxy, cyano, -NR^{17a}R^{17b}, hydroxy, cycloalkyl, heterocyclyl, aryl, and heteroaryl;
R^{7a}, R^{7b}, R^{7c}, R^{7d}, R^{9a}, R^{9b}, R^{9c}, R^{9d}, R^{15a}, R^{15b}, R^{15c}, R^{15d}, R^{17a}, and R^{17b} are identical or different at each occurrence and are each independently selected from the group consisting of a hydrogen atom, alkyl, hydroxyalkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, oxo, hydroxy, cyano, alkyl, alkoxy, haloalkyl, and haloalkoxy;
or R^{7a} and R^{7b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{7c} and R^{7d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{9a} and R^{9b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{9c} and R^{9d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{15a} and R^{15b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{15c} and R^{15d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{17a} and R^{17b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
s is 0, 1, 2, 3, 4, 5, or 6;
t is 0, 1, 2, 3, 4, or 5; and
p is 0, 1, or 2.

In some embodiments of the present disclosure, in the compound represented by general formula (IM) or the pharmaceutically acceptable salt thereof, V is CR⁵, and R⁵ is as defined in general formula (IM).

In some embodiments of the present disclosure, in the compound represented by general formula (IM) or the pharmaceutically acceptable salt thereof, V is N.

In some embodiments of the present disclosure, the compound represented by general formula (IM) or the pharmaceutically acceptable salt thereof is a compound represented by general formula (I) or a pharmaceutically acceptable salt thereof: 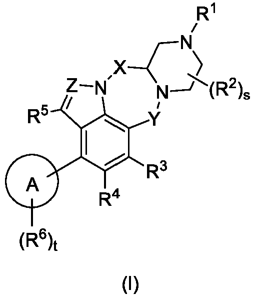 wherein:
X is C(R^{a}R^{b}) or C(R^{a}Rb)-C(R^{c}R^{d});
Y is C(O) or CH₂;
Z is CR^{5a} or N;
ring A is aryl or heteroaryl;
R^{a}, R^{b}, R^{c}, and R^{d} are identical or different at each occurrence and are each independently selected from the group consisting of a hydrogen atom, halogen, alkyl, haloalkyl, hydroxyalkyl, alkoxy, haloalkoxy, alkenyl, alkynyl, hydroxy, and cyano;
R¹ is selected from the group consisting of cyano, 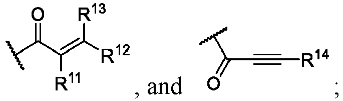
each R² is identical or different and is independently selected from the group consisting of a hydrogen atom, halogen, cyano, alkyl, alkoxy, hydroxy, and amino, wherein the alkyl and alkoxy are each independently optionally substituted with one or more substituents selected from the group consisting of halogen, cyano, amino, and hydroxy;
R³, R⁴, R⁵, and R^{5a} are identical or different and are each independently selected from the group consisting of a hydrogen atom, halogen, cyano, alkyl, alkenyl, alkynyl, -NR^{7a}R^{7b}, -C(O)R⁸, -OR⁸, -S(O)ₚR⁸, cycloalkyl, heterocyclyl, aryl and heteroaryl, wherein the alkyl, cycloalkyl, heterocyclyl, aryl, and heteroaryl are each independently optionally substituted with one or more substituents selected from the group consisting of halogen, alkyl, haloalkyl, cyano, -NR^{7c}R^{7d}, -OR^{8a}, cycloalkyl, heterocyclyl, aryl, and heteroaryl;
each R⁶ is identical or different and is independently selected from the group consisting of a hydrogen atom, halogen, cyano, alkyl, alkenyl, alkynyl, -NR^{9a}R^{9b}, -C(O)NR^{9a}R^{9b}, -C(O)R¹⁰, -C(O)OR¹⁰, -OC(O)R¹⁰, -OR¹⁰, -S(O)ₚR¹⁰, -S(O)ₚNR^{9a}R^{9b}, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, haloalkyl, cyano, -NR^{9c}R^{9d}, -OR^{10a}, cycloalkyl, heterocyclyl, aryl, and heteroaryl;
R¹¹, R¹², R¹³, and R¹⁴ are identical or different and are each independently selected from the group consisting of a hydrogen atom, halogen, alkyl, -NR^{15a}R^{15b}, -OR¹⁶, cyano, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, oxo, alkyl, haloalkyl, alkoxy, haloalkoxy, cyano, -NR^{15c}R^{15d}, hydroxy, hydroxyalkyl, cycloalkyl, heterocyclyl, aryl, and heteroaryl;
R⁸, R^{8a}, R¹⁰, R^{10a}, and R¹⁶ are identical or different at each occurrence and are each independently selected from the group consisting of a hydrogen atom, alkyl, hydroxyalkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkenyl, alkynyl, oxo, alkoxy, haloalkyl, haloalkoxy, cyano, -NR^{17a}R^{17b}, hydroxy, cycloalkyl, heterocyclyl, aryl, and heteroaryl;
R^{7a}, R^{7b}, R^{7c}, R^{7d}, R^{9a}, R^{9b}, R^{9c}, R^{9d}, R^{15a}, R^{15b}, R^{15c}, R^{15d}, R^{17a}, and R^{17b} are identical or different at each occurrence and are each independently selected from the group consisting of a hydrogen atom, alkyl, hydroxyalkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, oxo, hydroxy, cyano, alkyl, alkoxy, haloalkyl, and haloalkoxy;
or R^{7a} and R^{7b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{7c} and R^{7d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{9a} and R^{9b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{9c} and R^{9d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{15a} and R^{15b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{15c} and R^{15d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{17a} and R^{17b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
s is 0, 1, 2, 3, 4, 5, or 6;
t is 0, 1, 2, 3, 4, or 5; and
p is 0, 1, or 2.

In some embodiments of the present disclosure, in the compound represented by general formula (IM) or general formula (I) or the pharmaceutically acceptable salt thereof, Y is C(O).

In some embodiments of the present disclosure, in the compound represented by general formula (IM) or general formula (I) or the pharmaceutically acceptable salt thereof, each R² is identical or different and is independently selected from the group consisting of a hydrogen atom, halogen, cyano, C₁₋₆ alkyl, C₁₋₆ alkoxy, hydroxy, and amino, wherein the C₁₋₆ alkyl is optionally substituted with one or more substituents selected from the group consisting of halogen and cyano; preferably, each R² is identical or different and is independently a hydrogen atom or C₁₋₆ alkyl; more preferably, each R² is identical or different and is independently a hydrogen atom or methyl; most preferably, R² is a hydrogen atom.

In some embodiments of the present disclosure, in the compound represented by general formula (IM) or general formula (I) or the pharmaceutically acceptable salt thereof, s is 0, 1, or 2; preferably, s is 0 or 1; more preferably, s is 0.

In some embodiments of the present disclosure, in the compound represented by general formula (IM) or general formula (I) or the pharmaceutically acceptable salt thereof, each R² is identical or different and is independently selected from the group consisting of halogen, cyano, C₁₋₆ alkyl, C₁₋₆ alkoxy, hydroxy, and amino, wherein the C₁₋₆ alkyl is optionally substituted with one or more substituents selected from the group consisting of halogen and cyano, and s is 0, 1, or 2; preferably, R² is C₁₋₆ alkyl, and s is 0 or 1; more preferably, R² is methyl, and s is 0 or 1.

In some embodiments of the present disclosure, the compound represented by general formula (IM) or general formula (I) or the pharmaceutically acceptable salt thereof is a compound represented by general formula (II) or a pharmaceutically acceptable salt thereof: 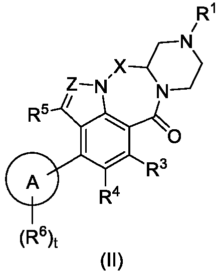 wherein:
ring A, X, Z, R¹, R³, R⁴, R⁵, R⁶, and t are as defined in general formula (I).

In some embodiments of the present disclosure, in the compound represented by general formula (IM), general formula (I), or general formula (II) or the pharmaceutically acceptable salt thereof, ring A is 6- to 10-membered aryl or 5- to 10-membered heteroaryl; preferably, ring A is 5- to 10-membered heteroaryl; more preferably, ring A is 8- to 10-membered bicyclic heteroaryl containing in the ring 1, 2, or 3 heteroatoms selected from the group consisting of nitrogen, oxygen, and sulfur; most preferably, ring A is benzothienyl or benzothiazolyl.

In some embodiments of the present disclosure, in the compound represented by general formula (IM), general formula (I), or general formula (II) or the pharmaceutically acceptable salt thereof, 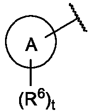 is 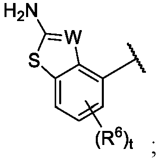 W is C(CN) or N; t is 0, 1, 2, or 3; and R⁶ is as defined in general formula (I).

In some embodiments of the present disclosure, in the compound represented by general formula (IM), general formula (I), or general formula (II) or the pharmaceutically acceptable salt thereof, R¹ is 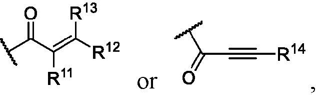 wherein R¹¹, R¹², R¹³, and R¹⁴ are as defined in general formula (I); preferably, R¹ is 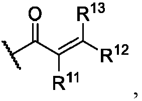 wherein R¹¹, R¹², and R¹³ are as defined in general formula (I).

In some embodiments of the present disclosure, in the compound represented by general formula (IM), general formula (I), or general formula (II) or the pharmaceutically acceptable salt thereof, X is C(R^{a}R^{b})-C(R^{c}R^{d}), wherein R^{a}, R^{b}, R^{c}, and R^{d} are as defined in general formula (I).

In some embodiments of the present disclosure, in the compound represented by general formula (IM), general formula (I), or general formula (II) or the pharmaceutically acceptable salt thereof, R^{a}, R^{b}, R^{c}, and R^{d} are identical or different and are each independently selected from the group consisting of a hydrogen atom, halogen, C₁₋₆ alkyl, and C₁₋₆ haloalkyl; preferably, R^{a}, R^{b}, R^{c}, and R^{d} are all hydrogen atoms.

In some embodiments of the present disclosure, in the compound represented by general formula (IM), general formula (I), or general formula (II) or the pharmaceutically acceptable salt thereof, X is CH₂ or CH₂-CH₂; preferably, X is CH₂-CH₂.

In some embodiments of the present disclosure, the compound represented by general formula (IM), general formula (I), or general formula (II) or the pharmaceutically acceptable salt thereof is a compound represented by general formula (III) or a pharmaceutically acceptable salt thereof: 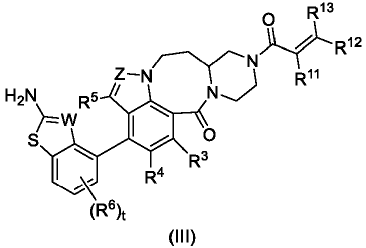 wherein:
W is C(CN) or N;
t is 0, 1, 2, or 3;
Z, R³, R⁴, R⁵, R⁶, R¹¹, R¹², and R¹³ are as defined in general formula (I).

In some embodiments of the present disclosure, the compound represented by general formula (IM), general formula (I), general formula (II), or general formula (III) or the pharmaceutically acceptable salt thereof is a compound represented by general formula (III-1) or a pharmaceutically acceptable salt thereof: 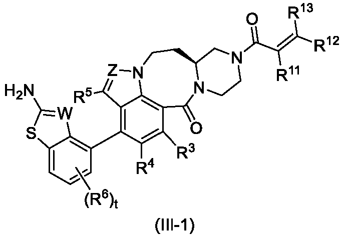 wherein:
W is C(CN) or N;
t is 0, 1, 2, or 3;
Z, R³, R⁴, R⁵, R⁶, R¹¹, R¹², and R¹³ are as defined in general formula (I).

In some embodiments of the present disclosure, the compound represented by general formula (IM), general formula (I), general formula (II), or general formula (III) or the pharmaceutically acceptable salt thereof is a compound represented by general formula (III-2) or a pharmaceutically acceptable salt thereof: 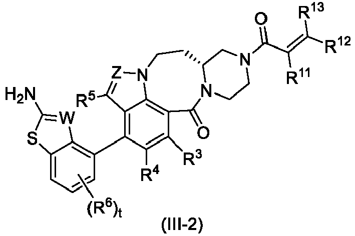 wherein:
W is C(CN) or N;
t is 0, 1, 2, or 3;
Z, R³, R⁴, R⁵, R⁶, R¹¹, R¹², and R¹³ are as defined in general formula (I).

In some embodiments of the present disclosure, the compound represented by general formula (IM), general formula (I), general formula (II), general formula (III), or general formula (III-1) or the pharmaceutically acceptable salt thereof is a compound represented by general formula (III-1-A) or a pharmaceutically acceptable salt thereof: 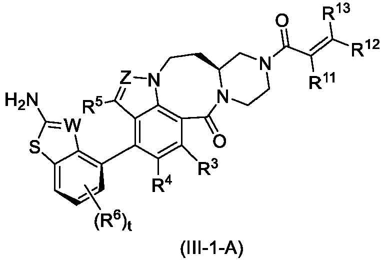 wherein:
W is C(CN) or N;
t is 0, 1, 2, or 3;
Z, R³, R⁴, R⁵, R⁶, R¹¹, R¹², and R¹³ are as defined in general formula (I).

In some embodiments of the present disclosure, the compound represented by general formula (IM), general formula (I), general formula (II), general formula (III), or general formula (III-1) or the pharmaceutically acceptable salt thereof is a compound represented by general formula (III-1-B) or a pharmaceutically acceptable salt thereof: 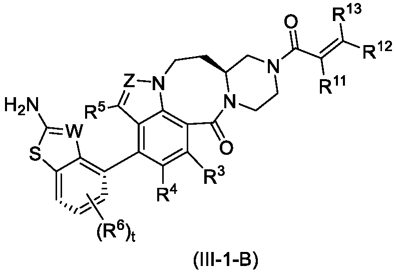 wherein:
W is C(CN) or N;
t is 0, 1, 2, or 3;
Z, R³, R⁴, R⁵, R⁶, R¹¹, R¹², and R¹³ are as defined in general formula (I).

In some embodiments of the present disclosure, the compound represented by general formula (IM), general formula (I), general formula (II), general formula (III), or general formula (III-2) or the pharmaceutically acceptable salt thereof is a compound represented by general formula (III-2-A) or a pharmaceutically acceptable salt thereof: 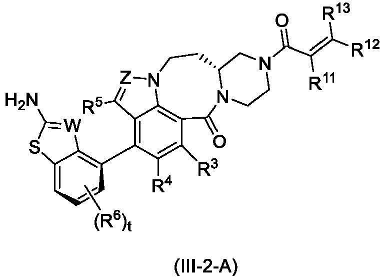 wherein:
W is C(CN) or N;
t is 0, 1, 2, or 3;
Z, R³, R⁴, R⁵, R⁶, R¹¹, R¹², and R¹³ are as defined in general formula (I).

In some embodiments of the present disclosure, the compound represented by general formula (IM), general formula (I), general formula (II), general formula (III), or general formula (III-2) or the pharmaceutically acceptable salt thereof is a compound represented by general formula (III-2-B) or a pharmaceutically acceptable salt thereof: 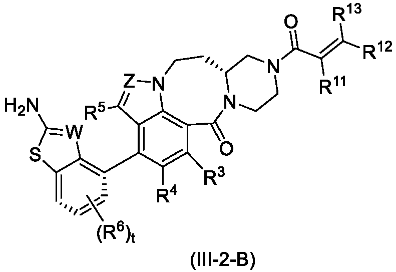 wherein:
W is C(CN) or N;
t is 0, 1, 2, or 3;
Z, R³, R⁴, R⁵, R⁶, R¹¹, R¹², and R¹³ are as defined in general formula (I).

In some embodiments of the present disclosure, in the compound represented by general formula (III), general formula (III-1), general formula (III-2), general formula (III-1-A), general formula (III-1-B), general formula (III-2-A), or general formula (III-2-B) or the pharmaceutically acceptable salt thereof, W is C(CN).

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R³, R⁴, R⁵, and R^{5a} are identical or different and are each independently selected from the group consisting of a hydrogen atom, halogen, C₁₋₆ alkyl, and C₁₋₆ haloalkyl.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R³ is selected from the group consisting of a hydrogen atom, halogen, C₁₋₆ alkyl, and C₁₋₆ haloalkyl; preferably, R³ is a hydrogen atom or halogen; more preferably, R³ is a hydrogen atom or F; most preferably, R³ is a hydrogen atom.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R⁴ is selected from the group consisting of a hydrogen atom, halogen, C₁₋₆ alkyl, and C₁₋₆ haloalkyl; preferably, R⁴ is halogen; more preferably, R⁴ is F.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R⁵ is selected from the group consisting of a hydrogen atom, halogen, C₁₋₆ alkyl, and C₁₋₆ haloalkyl; preferably, R⁵ is selected from the group consisting of a hydrogen atom, halogen, and C₁₋₆ alkyl; more preferably, R⁵ is halogen or C₁₋₆ alkyl; most preferably, R⁵ is Cl or methyl.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R⁵ is selected from the group consisting of a hydrogen atom, F, Cl, and methyl.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R^{5a} is selected from the group consisting of a hydrogen atom, halogen, C₁₋₆ alkyl, and C₁₋₆ haloalkyl; preferably, R^{5a} is a hydrogen atom or C₁₋₆ alkyl; more preferably, R^{5a} is a hydrogen atom or methyl.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, Z is CR^{5a} or N, and R^{5a} is selected from the group consisting of a hydrogen atom, halogen, C₁₋₆ alkyl, and C₁₋₆ haloalkyl; preferably, Z is CR^{5a} or N, and R^{5a} is a hydrogen atom or C₁₋₆ alkyl; more preferably, Z is N.

In some embodiments of the present disclosure, in the compound represented by general formula (IM), general formula (I), or general formula (II) or the pharmaceutically acceptable salt thereof, each R⁶ is identical or different and is independently selected from the group consisting of a hydrogen atom, halogen, cyano, -NH₂, C₁₋₆ alkyl, and C₁₋₆ haloalkyl; preferably, each R⁶ is identical or different and is independently selected from the group consisting of a hydrogen atom, halogen, cyano, and -NH₂.

In some embodiments of the present disclosure, in the compound represented by general formula (IM), general formula (III), general formula (III-1), general formula (III-2), general formula (III-1-A), general formula (III-1-B), general formula (III-2-A), or general formula (III-2-B) or the pharmaceutically acceptable salt thereof, each R⁶ is identical or different and is independently selected from the group consisting of a hydrogen atom, halogen, cyano, -NH₂, C₁₋₆ alkyl, and C₁₋₆ haloalkyl; preferably, each R⁶ is identical or different and is independently a hydrogen atom or halogen; more preferably, each R⁶ is identical or different and is independently a hydrogen atom or F.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R¹¹ is selected from the group consisting of a hydrogen atom, halogen, and C₁₋₆ alkyl; preferably, R¹¹ is a hydrogen atom or halogen; more preferably, R¹¹ is a hydrogen atom or F.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R¹² is selected from the group consisting of a hydrogen atom, halogen, and C₁₋₆ alkyl; preferably, R¹² is a hydrogen atom.

In some embodiments of the present disclosure, in the compound represented by general formula (IM), general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R¹³ is selected from the group consisting of a hydrogen atom, halogen, and C₁₋₆ alkyl; preferably, R¹³ is a hydrogen atom.

In some embodiments of the present disclosure, in the compound represented by general formula (I), general formula (II), general formula (III), general formula (III-1), general formula (III-2), general formula (III-1-A), general formula (III-1-B), general formula (III-2-A), or general formula (III-2-B) or the pharmaceutically acceptable salt thereof, R¹⁴ is selected from the group consisting of a hydrogen atom, halogen, and C₁₋₆ alkyl; preferably, R¹⁴ is a hydrogen atom.

In some embodiments of the present disclosure, in the compound represented by general formula (IM), general formula (I), or general formula (II) or the pharmaceutically acceptable salt thereof, t is 0, 1, 2, or 3.

In some embodiments of the present disclosure, in the compound represented by general formula (IM), general formula (I), or general formula (II) or the pharmaceutically acceptable salt thereof, each R⁶ is identical or different and is independently selected from the group consisting of halogen, cyano, -NH₂, C₁₋₆ alkyl, and C₁₋₆ haloalkyl, and t is 0, 1, 2, or 3.

In some embodiments of the present disclosure, in the compound represented by general formula (III), general formula (III-1), general formula (III-2), general formula (III-1-A), general formula (III-1-B), general formula (III-2-A), or general formula (III-2-B) or the pharmaceutically acceptable salt thereof, t is 0 or 1.

In some embodiments of the present disclosure, in the compound represented by general formula (III), general formula (III-1), general formula (III-2), general formula (III-1-A), general formula (III-1-B), general formula (III-2-A), or general formula (III-2-B) or the pharmaceutically acceptable salt thereof, R⁶ is halogen, and t is 0 or 1.

In some embodiments of the present disclosure, in the compound represented by general formula (III), general formula (III-1), general formula (III-2), general formula (III-1-A), general formula (III-1-B), general formula (III-2-A), or general formula (III-2-B) or the pharmaceutically acceptable salt thereof, R⁶ is F, and t is 0 or 1.

In some embodiments of the present disclosure, in the compound represented by general formula (IM) or the pharmaceutically acceptable salt thereof, V is CR⁵ or N; X is C(R^{a}R^{b}) or C(R^{a}Rb)-C(R^{c}R^{d}); R^{a}, R^{b}, R^{c}, and R^{d} are identical or different and are each independently selected from the group consisting of a hydrogen atom, halogen, C₁₋₆ alkyl, and C₁₋₆ haloalkyl; Y is C(O); Z is CR^{5a} or N; ring A is 5- to 10-membered heteroaryl; R¹ is 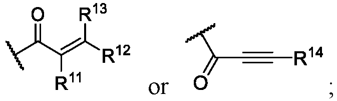 R³, R⁴, R⁵, and R^{5a} are identical or different and are each independently selected from the group consisting of a hydrogen atom, halogen, C₁₋₆ alkyl, and C₁₋₆ haloalkyl; R² is C₁₋₆ alkyl, and s is 0 or 1; each R⁶ is identical or different and is independently selected from the group consisting of halogen, cyano, -NH₂, C₁₋₆ alkyl, and C₁₋₆ haloalkyl, and t is 0, 1, 2, or 3; R¹¹ is selected from the group consisting of a hydrogen atom, halogen, and C₁₋₆ alkyl; R¹² is selected from the group consisting of a hydrogen atom, halogen, and C₁₋₆ alkyl; R¹³ is selected from the group consisting of a hydrogen atom, halogen, and C₁₋₆ alkyl; R¹⁴ is selected from the group consisting of a hydrogen atom, halogen, and C₁₋₆ alkyl.

In some embodiments of the present disclosure, in the compound represented by general formula (IM) or the pharmaceutically acceptable salt thereof, V is CR⁵ or N; X is CH₂ or CH₂-CH₂; Y is C(O); Z is CR^{5a} or N; R^{5a} is a hydrogen atom or C₁₋₆ alkyl; R¹ is 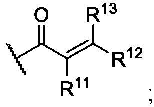 R³ is a hydrogen atom or halogen; R⁴ is halogen; R⁵ is selected from the group consisting of a hydrogen atom, halogen, and C₁₋₆ alkyl; R² is C₁₋₆ alkyl, and s is 0 or 1; 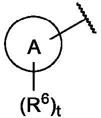 is 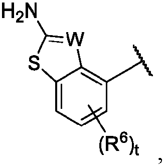 and W is C(CN) or N; each R⁶ is identical or different and is independently selected from the group consisting of halogen, cyano, -NH₂, C₁₋₆ alkyl, and C₁₋₆ haloalkyl, and t is 0, 1, 2, or 3; R¹¹ is selected from the group consisting of a hydrogen atom, halogen, and C₁₋₆ alkyl; R¹² is selected from the group consisting of a hydrogen atom, halogen, and C₁₋₆ alkyl; and R¹³ is selected from the group consisting of a hydrogen atom, halogen, and C₁₋₆ alkyl.

In some embodiments of the present disclosure, in the compound represented by general formula (I) or the pharmaceutically acceptable salt thereof, X is C(R^{a}R^{b}) or C(R^{a}Rb)-C(R^{c}R^{d}); R^{a}, R^{b}, R^{c}, and R^{d} are identical or different and are each independently selected from the group consisting of a hydrogen atom, halogen, C₁₋₆ alkyl, and C₁₋₆ haloalkyl; Y is C(O); Z is CR^{5a} or N; ring A is 5- to 10-membered heteroaryl; R¹ is 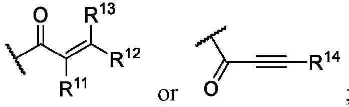 R³, R⁴, R⁵, and R^{5a} are identical or different and are each independently selected from the group consisting of a hydrogen atom, halogen, C₁₋₆ alkyl, and C₁₋₆ haloalkyl; R² is C₁₋₆ alkyl, and s is 0 or 1; each R⁶ is identical or different and is independently selected from the group consisting of halogen, cyano, -NH₂, C₁₋₆ alkyl, and C₁₋₆ haloalkyl, and t is 0, 1, 2, or 3; R¹¹ is selected from the group consisting of a hydrogen atom, halogen, and C₁₋₆ alkyl; R¹² is selected from the group consisting of a hydrogen atom, halogen, and C₁₋₆ alkyl; R¹³ is selected from the group consisting of a hydrogen atom, halogen, and C₁₋₆ alkyl; R¹⁴ is selected from the group consisting of a hydrogen atom, halogen, and C₁₋₆ alkyl.

In some embodiments of the present disclosure, in the compound represented by general formula (I) or the pharmaceutically acceptable salt thereof, X is C(R^{a}R^{b}) or C(R^{a}Rb)-C(R^{c}R^{d}); R^{a}, R^{b}, R^{c}, and R^{d} are identical or different and are each independently selected from the group consisting of a hydrogen atom, halogen, C₁₋₆ alkyl, and C₁₋₆ haloalkyl; Y is C(O); Z is CR^{5a} or N; ring A is 5- to 10-membered heteroaryl; R¹ is 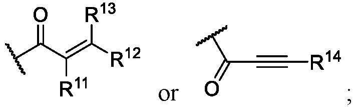 R³, R⁴, R⁵, and R^{5a} are identical or different and are each independently selected from the group consisting of a hydrogen atom, halogen, C₁₋₆ alkyl, and C₁₋₆ haloalkyl; s is 0; each R⁶ is identical or different and is independently selected from the group consisting of halogen, cyano, -NH₂, C₁₋₆ alkyl, and C₁₋₆ haloalkyl, and t is 0, 1, 2, or 3; R¹¹ is selected from the group consisting of a hydrogen atom, halogen, and C₁₋₆ alkyl; R¹² is selected from the group consisting of a hydrogen atom, halogen, and C₁₋₆ alkyl; R¹³ is selected from the group consisting of a hydrogen atom, halogen, and C₁₋₆ alkyl; R¹⁴ is selected from the group consisting of a hydrogen atom, halogen, and C₁₋₆ alkyl.

In some embodiments of the present disclosure, in the compound represented by general formula (II) or the pharmaceutically acceptable salt thereof, ring A is 8- to 10-membered bicyclic heteroaryl containing in the ring 1, 2, or 3 heteroatoms selected from the group consisting of nitrogen, oxygen, and sulfur; X is CH₂ or CH₂-CH₂; Z is CR^{5a} or N; R¹ is 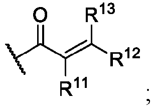 R³, R⁴, R⁵, and R^{5a} are identical or different and are each independently selected from the group consisting of a hydrogen atom, halogen, C₁₋₆ alkyl, and C₁₋₆ haloalkyl; each R⁶ is identical or different and is independently selected from the group consisting of halogen, cyano, -NH₂, C₁₋₆ alkyl, and C₁₋₆ haloalkyl, and t is 0, 1, 2, or 3; R¹¹ is a hydrogen atom or halogen; R¹² is a hydrogen atom; R¹³ is a hydrogen atom.

In some embodiments of the present disclosure, in the compound represented by general formula (III), general formula (III-1), general formula (III-2), general formula (III-1-A), general formula (III-1-B), general formula (III-2-A), or general formula (III-2-B) or the pharmaceutically acceptable salt thereof, W is C(CN) or N; Z is CR^{5a} or N; R³, R⁴, R⁵, and R^{5a} are identical or different and are each independently selected from the group consisting of a hydrogen atom, halogen, C₁₋₆ alkyl, and C₁₋₆ haloalkyl; R¹¹ is a hydrogen atom or halogen; R¹² is a hydrogen atom; R¹³ is a hydrogen atom; R⁶ is halogen, and t is 0 or 1.

In some embodiments of the present disclosure, in the compound represented by general formula (III), general formula (III-1), general formula (III-2), general formula (III-1-A), general formula (III-1-B), general formula (III-2-A), or general formula (III-2-B) or the pharmaceutically acceptable salt thereof, W is C(CN) or N; Z is CR^{5a} or N, and R^{5a} is a hydrogen atom or C₁₋₆ alkyl; R³ is a hydrogen atom or halogen; R⁴ is halogen; R⁵ is selected from the group consisting of a hydrogen atom, halogen, and C₁₋₆ alkyl; R¹¹ is a hydrogen atom or halogen; R¹² is a hydrogen atom; R¹³ is a hydrogen atom; R⁶ is halogen, and t is 0 or 1.

In some embodiments of the present disclosure, in the compound represented by general formula (III), general formula (III-1), general formula (III-2), general formula (III-1-A), general formula (III-1-B), general formula (III-2-A), or general formula (III-2-B) or the pharmaceutically acceptable salt thereof, W is C(CN); Z is N; R³ is a hydrogen atom; R⁴ is halogen; R⁵ is selected from the group consisting of a hydrogen atom, halogen, and C₁₋₆ alkyl; R¹¹ is a hydrogen atom or halogen; R¹² is a hydrogen atom; R¹³ is a hydrogen atom; R⁶ is halogen, and t is 0 or 1.

**Table A. Typical compounds of the present disclosure include, but are not limited to:**

| Example No. | Compound structure | Name |
|---|---|---|
| | 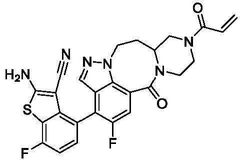 | 4-(10-Acryloyl-2-fluoro-14-oxo-8,8a,9,10,11,12-h exahydro-7*H*,14*H*-pyrazino[1',2':5,6][1,5]diazocin o[3,2,1-*hi*]indazol-3-yl)-2-amino-7-fluorobenzo[*b* ]thiophene-3-carbonitrile |
| **1** | 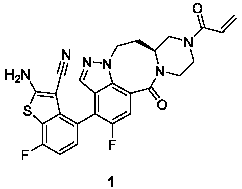 | 4-((*S*)-10-Acryloyl-2-fluoro-14-oxo-8,8*a*,9,10,11, 12-hexahydro-7*H*,14*H*-pyrazino[1',2':5,6][1,5]dia zocino[3,2,1-*hi*]indazol-3-yl)-2-amino-7-fluorobe nzo[*b*]thiophene-3-carbonitrile **1** |
| **1-P1** | 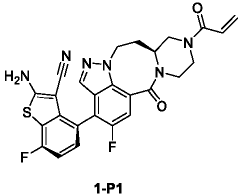 | (*R*)-4-((*S*)-10-Acryloyl-2-fluoro-14-oxo-8,8*a*,9,10, 11,12-hexahydro-7*H*,14*H*-pyrazino[1',2':5,6][1,5] diazocino[3,2,1-*hi*]indazol-3-yl)-2-amino-7-fluoro benzo[*b*]thiophene-3-carbonitrile **1-P1** |
| **1-P2** | 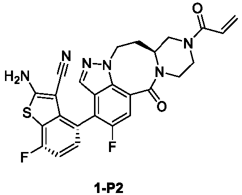 | (*S*)-4-((*S*)-10-Acryloyl-2-fluoro-14-oxo-8,8*a*,9,10, 11,12-hexahydro-7*H*,14*H*-pyrazino[1',2':5,6][1,5] diazocino[3,2,1-*hi*]indazol-3-yl)-2-amino-7-fluoro benzo[*b*]thiophene-3-carbonitrile **1-P2** |
| **12** | 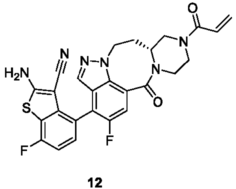 | 4-((*R*)-10-Acryloyl-2-fluoro-14-oxo-8,8*a*,9,10,11, 12-hexahydro-7*H*,14*H*-pyrazino[1',2':5,6][1,5]dia zocino[3,2,1-*hi*]indazol-3-yl)-2-amino-7-fluorobe nzo[*b*]thiophene-3-carbonitrile **12** |
| **12-P1** | 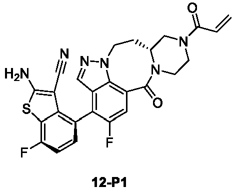 | (*R*)-4-((*R*)-10-Acryloyl-2-fluoro-14-oxo-8,8*a*,9,10 ,11,12-hexahydro-7*H*,14*H*-pyrazino[1',2':5,6][1,5] diazocino[3,2,1-*hi*]indazol-3-yl)-2-amino-7-fluoro benzo[*b*]thiophene-3-carbonitrile **12-P1** |
| **12-P2** | 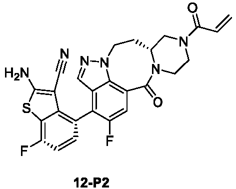 | (*S*)-4-((*R*)-10-Acryloyl-2-fluoro-14-oxo-8,8*a*,9,10, 11,12-hexahydro-7*H*,14*H*-pyrazino[1',2':5,6][1,5] diazocino[3,2,1-*hi*]indazol-3-yl)-2-amino-7-fluoro benzo[*b*]thiophene-3-carbonitrile **12-P2** |
| | 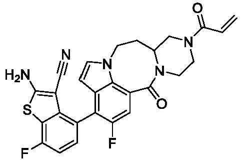 | 4-(10-Acryloyl-2-fluoro-14-oxo-8,8*a*,9,10,11,12-h exahydro-7*H*,14*H*-pyrazino[1',2':5,6][1,5]diazocin o[3,2,1-*hi*]indol-3-yl)-2-amino-7-fluorobenzo[*b*]th iophene-3-carbonitrile |
| **2** | 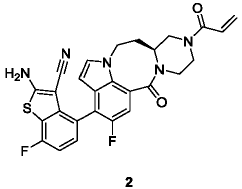 | 4-((*S*)-10-Acryloyl-2-fluoro-14-oxo-8,8*a*,9,10,11, 12-hexahydro-7*H*,14*H*-pyrazino[1',2':5,6][1,5]dia zocino[3,2,1-*hi*]indol-3-yl)-2-amino-7-fluorobenz o[*b*]thiophene-3-carbonitrile **2** |
| | 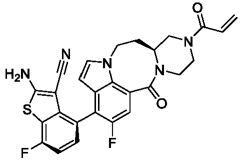 | (*R*)-4-((*S*)-10-Acryloyl-2-fluoro-14-oxo-8,8*a*,9,10, 11,12-hexahydro-7*H*,14*H*-pyrazino[1',2':5,6][1,5] diazocino[3,2,1-*hi*]indol-3-yl)-2-amino-7-fluorobe nzo[*b*]thiophene-3-carbonitrile |
| | 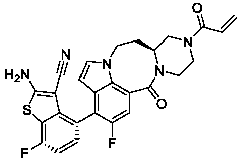 | (*S*)-4-((*S*)-10-Acryloyl-2-fluoro-14-oxo-8,8*a*,9,10, 11, 12-hexahydro-7*H*, 14*H*-pyrazino[1',2':5,6][1,5] diazocino[3,2,1-*hi*]indol-3-yl)-2-amino-7-fluorobe nzo[*b*]thiophene-3-carbonitrile |
| | 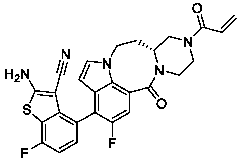 | 4-((*R*)-10-Acryloyl-2-fluoro-14-oxo-8,8*a*,9,10,11, 12-hexahydro-7*H*,14*H*-pyrazino[1',2':5,6][1,5]dia zocino[3,2,1-*hi*]indol-3-yl)-2-amino-7-fluorobenz o[*b*]thiophene-3-carbonitrile |
| | 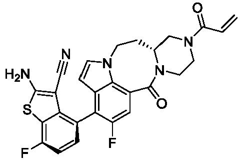 | (*R*)-4-((*R*)-10-Acryloyl-2-fluoro-14-oxo-8,8*a*,9,10 ,11,12-hexahydro-7*H*,14*H*-pyrazino[1',2':5,6][1,5] diazocino[3,2,1-*hi*]indol-3-yl)-2-amino-7-fluorobe nzo[*b*]thiophene-3-carbonitrile |
| | 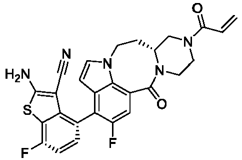 | (*S*)-4-((*R*)-10-Acryloyl-2-fluoro-14-oxo-8,8*a*,9,10, 11,12-hexahydro-7*H*,14*H*-pyrazino[1',2':5,6][1,5] diazocino[3,2,1-*hi*]indol-3-yl)-2-amino-7-fluorobe nzo[*b*]thiophene-3-carbonitrile |
| | 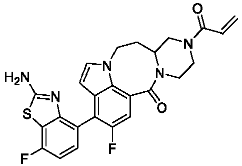 | 10-Acryloyl-3-(2-amino-7-fluorobenzo[*d*]thiazol-4-yl)-2-fluoro-8,8*a*,9,10,11,12-hexahydro-7*H*,14*H* -pyrazino[1',2':5,6][1,5]diazocino[3,2,1-*hi*]indol-1 4-one |
| **3** | 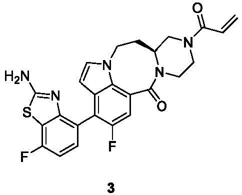 | (8*aS*)-10-Acryloyl-3-(-2-amino-7-fluorobenzo[*d*]t hiazol-4-yl)-2-fluoro-8,8*a*,9,10,11,12-hexahydro-7 *H,*14*H*-pyrazino[1',2':5,6][1,5]diazocino[3,2,1-*hi*]i ndol-14-one **3** |
| | 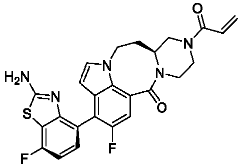 | (*S*)-10-Acryloyl-3-((*R*)-2-amino-7-fluorobenzo[*d*]t hiazol-4-yl)-2-fluoro-8,8*a*,9,10,11,12-hexahydro-7 *H,*14*H*-pyrazino[1',2':5,6][1,5]diazocino[3,2,1-*hi*]i ndol-14-one |
| | 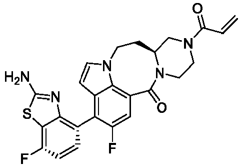 | (*S*)-10-Acryloyl-3-((*S*)-2-amino-7-fluorobenzo[*d*]t hiazol-4-yl)-2-fluoro-8,8*a*,9,10,11,12-hexahydro-7 *H,*14*H*-pyrazino[1',2':5,6][1,5]diazocino[3,2,1-*hi*]i ndol-14-one |
| | 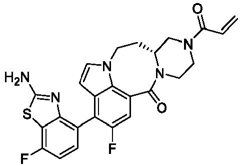 | (8*aR*)-10-Acryloyl-3-(-2-amino-7-fluorobenzo[*d*]t hiazol-4-yl)-2-fluoro-8,8*a*,9,10,11,12-hexahydro-7 *H,*14*H*-pyrazino[1',2':5,6][1,5]diazocino[3,2,1-*hi*]i ndol-14-one |
| | 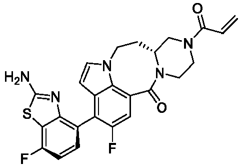 | (*R*)-10-Acryloyl-3-((*R*)-2-amino-7-fluorobenzo[*d*] thiazol-4-yl)-2-fluoro-8,8*a*,9,10,11,12-hexahydro-7*H,*14*H*-pyrazino[1',2':5,6][1,5]diazocino[3,2,1*-hi* ]indol-14-one |
| | 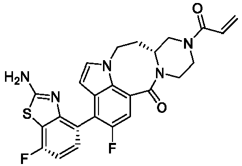 | (*R*)-10-Acryloyl-3-((*S*)-2-amino-7-fluorobenzo[*d*]t hiazol-4-yl)-2-fluoro-8,8*a*,9,10,11,12-hexahydro-7 *H,*14*H*-pyrazino[1',2':5,6][1,5]diazocino[3,2,1-*hi*]i ndol-14-one |
| | 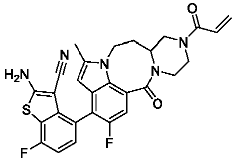 | 4-(10-Acryloyl-2-fluoro-5-methyl-14-oxo-8,8*a*,9, 10,11,12-hexahydro-7*H*,14*H*-pyrazino[1',2':5,6][1, 5]diazocino[3,2,1-*hi*]indol-3-yl)-2-amino-7-fluoro benzo[b]thiophene-3-carbonitrile |
| **4** | 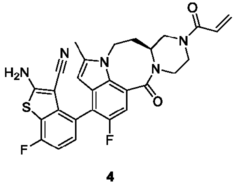 | 4-((*S*)-10-Acryloyl-2-fluoro-5-methyl-14-oxo-8,8 *a*,9,10,11,12-hexahydro-7*H*,14*H*-pyrazino[1',2':5, 6][1,5]diazocino[3,2,1-*hi*]indol-3-yl)-2-amino-7-fl uorobenzo[*b*]thiophene-3-carbonitrile **4** |
| | 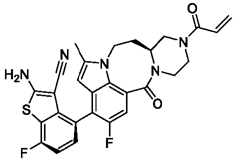 | (*R*)-4-((*S*)-10-Acryloyl-2-fluoro-5-methyl-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile |
| | 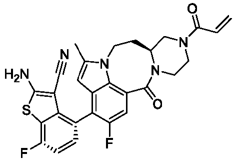 | (*S*)-4-((*S*)-10-Acryloyl-2-fluoro-5-methyl-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile |
| | 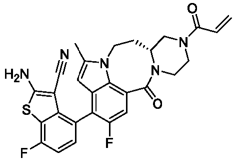 | 4-((*R*)-10-Acryloyl-2-fluoro-5-methyl-14-oxo-8,8 *a*,9,10,11,12-hexahydro-7*H*,14*H*-pyrazino[1',2':5, 6][1,5]diazocino[3,2,1-*hi*]indol-3-yl)-2-amino-7-fl uorobenzo[*b*]thiophene-3-carbonitrile |
| | 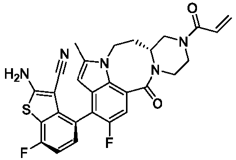 | (*R*)-4-((*R*)-10-Acryloyl-2-fluoro-5-methyl-14-oxo -8,8*a*,9,10,11,12-hexahydro-7*H*,14*H*-pyrazino[1',2 ':5,6][1,5]diazocino[3,2,1-*hi*]indol-3-yl)-2-amino-7-fluorobenzo[b]thiophene-3-carbonitrile |
| | 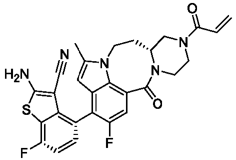 | (*S*)-4-((*R*)-10-Acryloyl-2-fluoro-5-methyl-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile |
| | 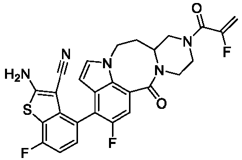 | 2-Amino-7-fluoro-4-(2-fluoro-10-(2-fluoroacryloy 1)-14-oxo-8,8*a*,9,10,11,12-hexahydro-7*H*,14*H*-pyr azino[1',2':5,6][1,5]diazocino[3,2,1-*hi*]indol-3-yl) benzo[*b*]thiophene-3-carbonitrile |
| **5** | 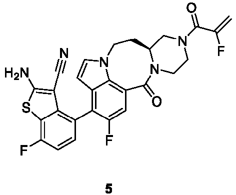 | 2-Amino-7-fluoro-4-((*S*)-2-fluoro-10-(2-fluoroacr yloyl)-14-oxo-8,8*a,*9,10,11,12-hexahydro-7*H,*14*H* -pyrazino[1',2':5,6][1,5]diazocino[3,2,1-*hi*]indol-3 -yl)benzo[*b*]thiophene-3-carbonitrile **5** |
| | 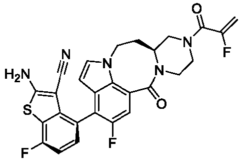 | (*R*)-2-Amino-7-fluoro-4-((*S*)-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind ol-3-yl)benzo[*b*]thiophene-3-carbonitrile |
| | 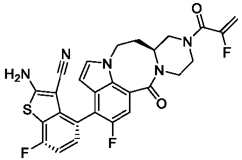 | (*S*)-2-Amino-7-fluoro-4-((*S*)-2-fluoro-10-(2-fluoro acryloyl)-14-oxo-8,8*a*,9,10,11,12-hexahydro-7*H*,1 4*H*-pyrazino[1',2':5,6][1,5]diazocino[3,2,1-*hi*]indo 1-3-yl)benzo[*b*]thiophene-3-carbonitrile |
| | 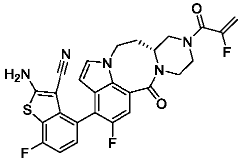 | 2-Amino-7-fluoro-4-((*R*)-2-fluoro-10-(2-fluoroacr yloyl)-14-oxo-8,8*a,*9,10,11,12-hexahydro-7*H,*14*H* -pyrazino[1',2':5,6][1,5]diazocino[3,2,1-*hi*]indol-3 -yl)benzo[*b*]thiophene-3-carbonitrile |
| | 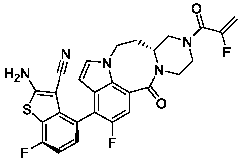 | (*R*)-2-Amino-7-fluoro-4-((*R*)-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind ol-3-yl)benzo[b]thiophene-3-carbonitrile |
| | 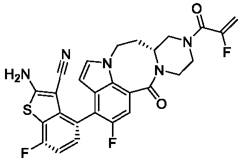 | (*S*)-2-Amino-7-fluoro-4-((*R*)-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind ol-3-yl)benzo[*b*]thiophene-3-carbonitrile |
| | 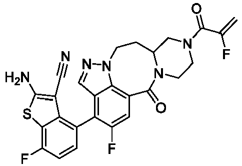 | 2-Amino-7-fluoro-4-(2-fluoro-10-(2-fluoroacryloy 1)-14-oxo-8,8*a*,9,10,11,12-hexahydro-7*H*,14*H*-pyr azino[1',2':5,6][1,5]diazocino[3,2,1-*hi*]indazol-3-y 1)benzo[*b*]thiophene-3-carbonitrile |
| **6** | 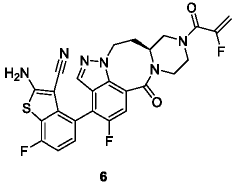 | 2-Amino-7-fluoro-4-((*S*)-2-fluoro-10-(2-fluoroacr yloyl)-14-oxo-8,8*a,*9,10,11,12-hexahydro-7*H,*14*H* -pyrazino[1',2':5,6][1,5]diazocino[3,2,1-*hi*]indazol -3-yl)benzo[*b*]thiophene-3-carbonitrile **6** |
| **6-P1** | 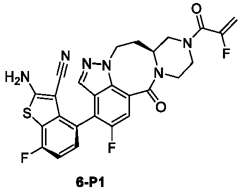 | (*R*)-2-Amino-7-fluoro-4-((*S*)-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind azol-3-yl)benzo[*b*]thiophene-3-carbonitrile **6-P1** |
| **6-P2** | 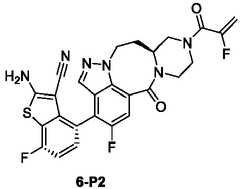 | (*S*)-2-Amino-7-fluoro-4-((*S*)-2-fluoro-10-(2-fluoro acryloyl)-14-oxo-8,8*a*,9,10,11,12-hexahydro-7*H*,1 4*H*-pyrazino[1',2':5,6][1,5]diazocino[3,2,1-*hi*]inda zol-3-yl)benzo[*b*]thiophene-3-carbonitrile **6-P2** |
| | 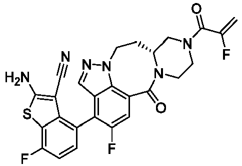 | 2-Amino-7-fluoro-4-((*R*)-2-fluoro-10-(2-fluoroacr yloyl)-14-oxo-8,8*a,*9,10,11,12-hexahydro-7*H,*14*H* -pyrazino[1',2':5,6][1,5]diazocino[3,2,1-*hi*]indazol -3-yl)benzo[*b*]thiophene-3-carbonitrile |
| | 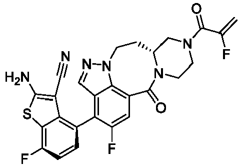 | (*R*)-2-Amino-7-fluoro-4-((*R*)-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind azol-3-yl)benzo[*b*]thiophene-3-carbonitrile |
| | 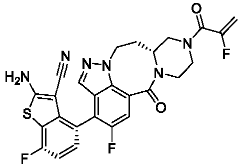 | (*S*)-2-Amino-7-fluoro-4-((*R*)-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind azol-3-yl)benzo[*b*]thiophene-3-carbonitrile |
| | 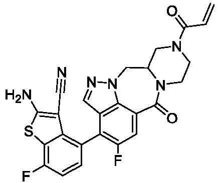 | 4-(10-Acryloyl-4-fluoro-6-oxo-8,9,10,11,11*a,*12-h exahydro-6*H*-pyrazino[2',1':3,4][1,4]diazepino[6, 7,1-*hi*]indazol-3-yl)-2-amino-7-fluorobenzo[*b*]thi ophene-3-carbonitrile |
| **14** | 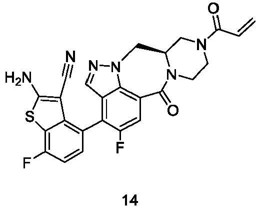 | 4-((R)-10-Acryloyl-4-fluoro-6-oxo-8,9,10,11,11*a,* 12-hexahydro-6*H*-pyrazino[2',1':3,4][1,4]diazepin o[6,7,1-*hi*]indazol-3-yl)-2-amino-7-fluorobenzo[*b* ]thiophene-3-carbonitrile **14** |
| | 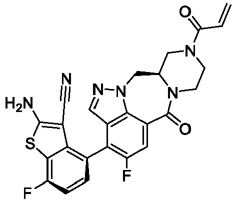 | 4-((3*R,*11a*R*)-10-Acryloyl-4-fluoro-6-oxo-8,9,10,1 1,11*a*,12-hexahydro-6*H*-pyrazino[2',1':3,4][1,4]di azepino[6,7,1-*hi*]indazol-3-yl)-2-amino-7-fluorob enzo[*b*]thiophene-3-carbonitrile |
| | 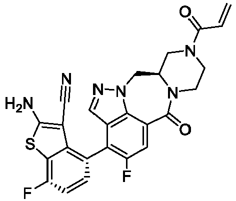 | 4-((3*S,*11*aR*)-10-Acryloyl-4-fluoro-6-oxo-8,9,10,1 1,11*a*,12-hexahydro-6*H*-pyrazino[2',1':3,4][1,4]di azepino[6,7,1-*hi*]indazol-3-yl)-2-amino-7-fluorob enzo[*b*]thiophene-3-carbonitrile |
| | 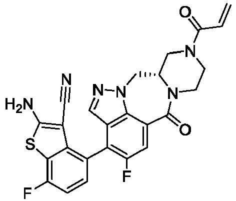 | 4-((11a*S*)-10-Acryloyl-4-fluoro-6-oxo-8,9,10,11,1 1*a*,12-hexahydro-6*H*-pyrazino[2',1':3,4][1,4]diaze pino[6,7,1-*hi*]indazol-3-yl)-2-amino-7-fluorobenz o[*b*]thiophene-3-carbonitrile |
| | 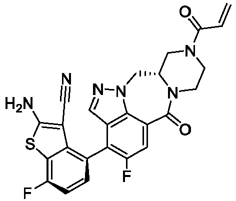 | 4-((3*R,*11a*S*)-10-Acryloyl-4-fluoro-6-oxo-8,9,10,1 1,11*a*,12-hexahydro-6*H*-pyrazino[2',1':3,4][1,4]di azepino[6,7,1-*hi*]indazol-3-yl)-2-amino-7-fluorob enzo[*b*]thiophene-3-carbonitrile |
| | 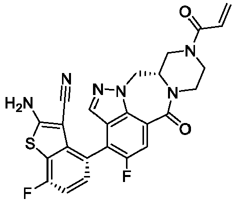 | 4-((3*S*,11a*S*)-10-Acryloyl-4-fluoro-6-oxo-8,9,10,1 1,11*a*,12-hexahydro-6*H*-pyrazino[2',1':3,4][1,4]di azepino[6,7,1-*hi*]indazol-3-yl)-2-amino-7-fluorob enzo[*b*]thiophene-3-carbonitrile |
| | 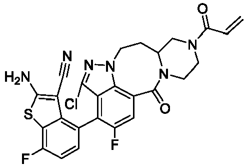 | 4-(10-Acryloyl-4-chloro-2-fluoro-14-oxo-8,8a,9,1 0, 11, 12-hexahydro-7*H*, 14*H*-pyrazino[1',2':5][1,5] diazocino[3,2,1-*hi*]indazol-3-yl)-2-amino-7-fluoro benzo[*b*]thiophene-3-carbonitrile |
| **7** | 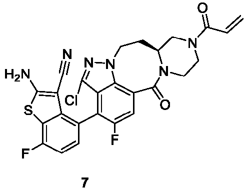 | 4-((*S*)-10-Acryloyl-4-chloro-2-fluoro-14-oxo-8,8*a* ,9,10,11,12-hexahydro-7*H*,14*H*-pyrazino[1',2':5][1 ,5]diazocino[3,2,1-*hi*]indazol-3-yl)-2-amino-7-flu orobenzo[*b*]thiophene-3-carbonitrile **7** |
| **7-P1** | 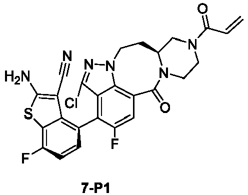 | (*R*)-4-((*S*)-10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indazol-3-yl)-2-amin o-7-fluorobenzo[*b*]thiophene-3-carbonitrile **7-P1** |
| **7-P2** | 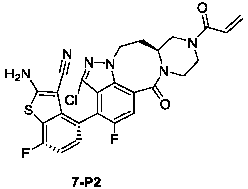 | (*S*)-4-((*S*)-10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indazol-3-yl)-2-amin o-7-fluorobenzo[*b*]thiophene-3-carbonitrile **7-P2** |
| | 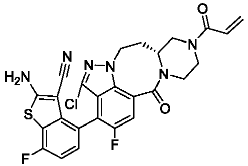 | 4-((*R*)-10-Acryloyl-4-chloro-2-fluoro-14-oxo*-*8,8*a* ,9,10,11,12-hexahydro-7*H*,14*H*-pyrazino[1',2':5][1 ,5]diazocino[3,2,1-*hi*]indazol-3-yl)-2-amino-7-flu orobenzo[*b*]thiophene-3-carbonitrile |
| | 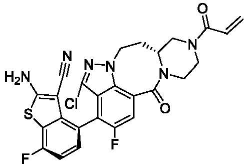 | (*R*)-4-((*R*)-10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indazol-3-yl)-2-amin o-7-fluorobenzo[*b*]thiophene-3-carbonitrile |
| | 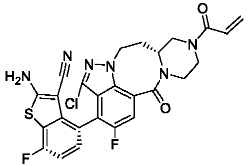 | (*S*)-4-((*R*)-10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indazol-3-yl)-2-amin o-7-fluorobenzo[*b*]thiophene-3-carbonitrile |
| | 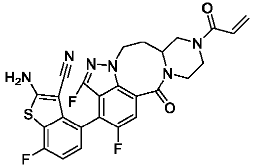 | 4-(10-Acryloyl-2,4-difluoro-14-oxo-8,8*a*,9,10,11, 12-hexahydro-7*H*,14*H*-pyrazino[1',2':5.6][1,5]dia zocino[3,2,1-*hi*]indazol-3-yl)-2-amino-7-fluorobe nzo[*b*]thiophene-3-carbonitrile |
| **8** | 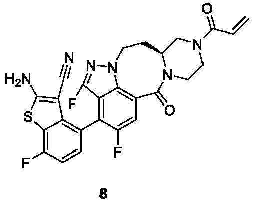 | 4-((*S*)-10-Acryloyl-2,4-difluoro-14-oxo-8,8*a*,9,10, 11, 12-hexahydro-7H, 14H-pyrazino[1',2':5.6][1,5] diazocino[3,2,1-*hi*]indazol-3-yl)-2-amino-7-fluoro benzo[*b*]thiophene-3-carbonitrile **8** |
| **8-P1** | 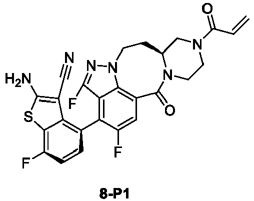 | (*R*)-4-((*S*)-10-Acryloyl-2,4-difluoro-14-oxo-8,8*a*, 9,10,11,12-hexahydro-7*H*,14*H*-pyrazino[1',2':5][1, 5]diazocino[3,2,1-*hi*]indazol-3-yl)-2-amino-7-fluo robenzo[*b*]thiophene-3-carbonitrile **8-P1** |
| **8-P2** | 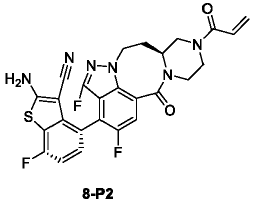 | (*S*)-4-((*S*)-10*-*Acryloyl*-*2,4-difluoro*-*14-oxo-8,8*a,*9 ,10,11,12-hexahydro-7*H*,14*H*-pyrazino[1',2':5][1,5 ]diazocino[3,2,1-*hi*]indazol-3-yl)-2-amino-7-fluor obenzo[*b*]thiophene-3-carbonitrile **8-P2** |
| | 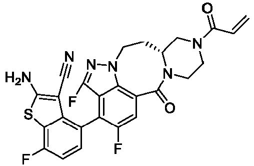 | 4-((*R*)-10-Acryloyl-2,4-difluoro-14-oxo-8,8*a*,9,10 ,11,12-hexahydro-7*H*,14*H-*pyrazino[1',2':5.6][1,5] diazocino[3,2,1-*hi*]indazol-3-yl)-2-amino-7-fluoro benzo[*b*]thiophene-3-carbonitrile |
| | 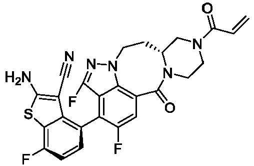 | (*R*)-4-((*R*)-10-Acryloyl-2,4-difluoro-14-oxo*-*8,8*a,* 9,10,11,12-hexahydro-7*H*,14*H*-pyrazino[1',2':5][1, 5]diazocino[3,2,1-*hi*]indazol-3-yl)-2-amino-7-fluo robenzo[*b*]thiophene-3-carbonitrile |
| | 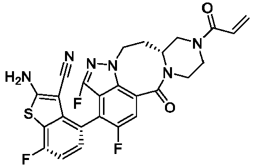 | (*S*)-4-((*R*)-10-Acryloyl-2,4-difluoro-14-oxo-8,8*a*, 9,10,11,12-hexahydro-7*H*,14*H*-pyrazino[1',2':5][1, 5]diazocino[3,2,1-*hi*]indazol-3-yl)-2-amino-7-fluo robenzo[b]thiophene-3-carbonitrile |
| | 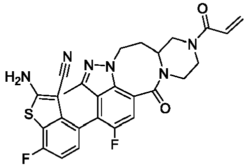 | 4-(10-Acryloyl-2-fluoro-4-methyl-14-oxo-8,8*a*,9, 10,11,12-hexahydro-7*H*,14*H*-pyrazino[1',2':5,6][1, 5]diazocino[3,2,1-*hi*]indazol-3-yl)-2-amino-7-fluo robenzo[*b*]thiophene-3-carbonitrile |
| **9** | 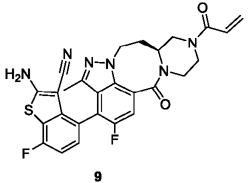 | 4-((*S*)-10-Acryloyl-2-fluoro-4-methyl-14-oxo-8,8 *a*,9,10,11,12-hexahydro-7*H*,14*H*-pyrazino[1',2':5, 6][1,5]diazocino[3,2,1-*hi*]indazol-3-yl)-2-amino-7 -fluorobenzo[*b*]thiophene-3-carbonitrile **9** |
| **9-P1** | 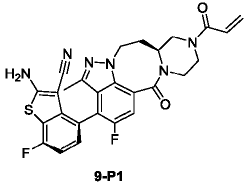 | (*R*)-4-((*S*)-10-Acryloyl-2-fluoro-4-methyl-14-oxo-8,8*a*,9,10,11,12-hexahydro-7*H*,14*H*-pyrazino[1',2' :5,6][1,5]diazocino[3,2,1-*hi*]indazol-3-yl)-2-amin o-7-fluorobenzo[*b*]thiophene-3-carbonitrile **9-P1** |
| **9-P2** | 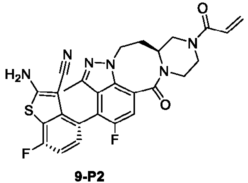 | (*S*)-4-((*S*)-10-Acryloyl-2-fluoro-4-methyl-14-oxo-8,8*a*,9,10,11,12-hexahydro-7*H*,14*H*-pyrazino[1',2' :5,6][1,5]diazocino[3,2,1-*hi*]indazol-3-yl)-2-amin o-7-fluorobenzo[*b*]thiophene-3-carbonitrile **9-P2** |
| | 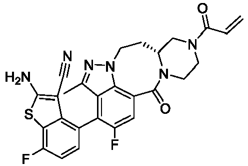 | 4-((*R*)-10-Acryloyl-2-fluoro-4-methyl-14-oxo-8,8 *a*,9,10,11,12-hexahydro-7*H*,14*H*-pyrazino[1',2':5, 6][1,5]diazocino[3,2,1-*hi*]indazol-3-yl)-2-amino-7 -fluorobenzo[*b*]thiophene-3-carbonitrile |
| | 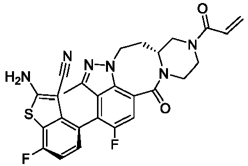 | (*R*)-4-((*R*)-10-Acryloyl-2-fluoro-4-methyl-14-oxo -8,8*a*,9,10,11,12-hexahydro-7*H*,14*H*-pyrazino[1',2 ':5,6][1,5]diazocino[3,2,1-*hi*]indazol-3-yl)-2-amin o-7-fluorobenzo[*b*]thiophene-3-carbonitrile |
| | 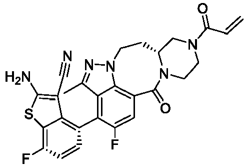 | (*S*)-4-((R)-10-Acryloyl-2-fluoro-4-methyl-14-oxo-8,8*a*,9,10,11,12-hexahydro-7*H*,14*H*-pyrazino[1',2' :5,6][1,5]diazocino[3,2,1-*hi*]indazol-3-yl)-2-amin o-7-fluorobenzo[*b*]thiophene-3-carbonitrile |
| | 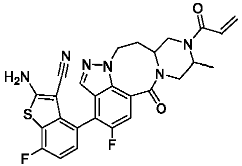 | 4-(10-Acryloyl-2-fluoro-11-methyl-14-oxo-8,8*a*,9 ,10,11,12-hexahydro-7*H*,14*H*-pyrazino[1',2': 5,6][1 ,5]diazocino[3,2,1-*hi*]indazol-3-yl)-2-amino-7-flu orobenzo[*b*]thiophene-3-carbonitrile |
| **10** | 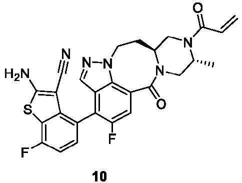 | 4-((8*aS*,11*R*)-10-Acryloyl-2-fluoro-11-methyl-14-oxo-8,8*a*,9,10,11,12-hexahydro-7*H*,14*H*-pyrazino [1',2':5,6][1,5]diazocino[3,2,1-*hi*]indazol-3-yl)-2-a mino-7-fluorobenzo[*b*]thiophene-3-carbonitrile **10** |
| | 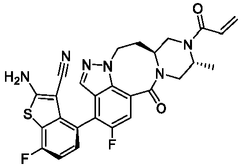 | (*R*)-4-((8*aS,*11*R*)-10-Acryloyl-2-fluoro-11-methyl -14-oxo-8,8*a*,9,10,11,12-hexahydro-7*H*,14*H*-pyra zino[1',2':5,6][1,5]diazocino[3,2,1-*hi*]indazol-3-yl )-2-amino-7-fluorobenzo[*b*]thiophene-3-carbonitri le |
| | 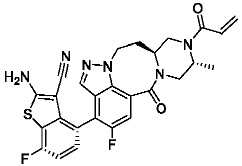 | (*S*)-4-((8*aS,*11*R*)*-*10*-*Acryloyl-2-fluoro-11*-*methyl-14-oxo-8,8*a*,9,10,11,12-hexahydro-7*H*,14*H*-pyraz ino[1',2':5,6][1,5]diazocino[3,2,1-*hi*]indazol-3-yl)-2-amino-7-fluorobenzo[*b*]thiophene-3-carbonitrile |
| | 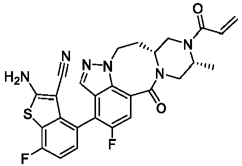 | 4-((8*aR*,11*R*)-10-Acryloyl-2-fluoro-11-methyl-14-oxo-8,8*a*,9,10,11,12-hexahydro-7*H*,14*H*-pyrazino [1',2':5,6][1,5]diazocino[3,2,1-*hi*]indazol-3-yl)-2-a mino-7-fluorobenzo[*b*]thiophene-3-carbonitrile |
| | 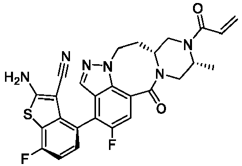 | (*R*)-4-((8*aR,*11*R*)-10-Acryloyl-2-fluoro-11-methyl -14-oxo-8,8*a*,9,10,11,12-hexahydro-7*H*,14*H*-pyra zino[1',2':5,6][1,5]diazocino[3,2,1-*hi*]indazol-3-yl )-2-amino-7-fluorobenzo[*b*]thiophene-3-carbonitri le |
| | 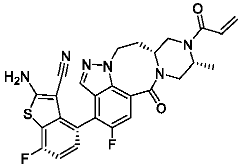 | (*S*)-4-((8*aR*,11*R*)-10-Acryloyl-2-fluoro-11-methyl -14-oxo-8,8*a*,9,10,11,12-hexahydro-7*H*,14*H*-pyra zino[1',2':5,6][1,5]diazocino[3,2,1-*hi*]indazol-3-yl )-2-amino-7-fluorobenzo[*b*]thiophene-3-carbonitri le |
| | 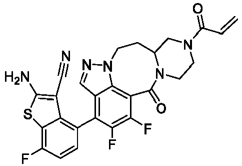 | 4-(10-Acryloyl-1,2-difluoro-14-oxo-8,8*a*,9,10,11, 12-hexahydro-7*H,*14*H*-pyrazino[1',2':5.6][1,5]dia zocino[3,2,1-*hi*]indazol-3-yl)-2-amino-7-fluorobe nzo[*b*]thiophene-3-carbonitrile |
| **11** | 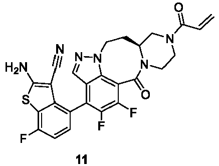 | 4-((*S*)-10-Acryloyl-1,2-difluoro-14-oxo-8,8*a*,9,10, 11, 12-hexahydro-7*H*,14*H*-pyrazino[1',2':5.6][1,5] diazocino[3,2,1-*hi*]indazol-3-yl)-2-amino-7-fluoro benzo[*b*]thiophene-3-carbonitrile **11** |
| **11-P1** | 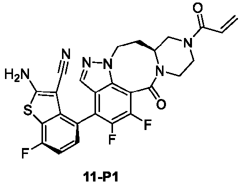 | (*R*)-4-((*S*)-10-Acryloyl-1,2-difluoro-14-oxo-8,8*a,* 9,10,11,12-hexahydro-7*H*,14*H*-pyrazino[1',2':5.6][ 1,5]diazocino[3,2,1-*hi*]indazol-3-yl)-2-amino-7-fl uorobenzo[*b*]thiophene-3-carbonitrile **11-P1** |
| **11-P2** | 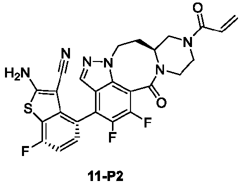 | (*S*)-4-((*S*)-10*-*Acryloyl*-*1,2-difluoro-14-oxo-8,8*a,*9 ,10,11,12-hexahydro-7*H*,14*H*-pyrazino[1',2':5.6][1 ,5]diazocino[3,2,1-*hi*]indazol-3-yl)-2-amino-7-flu orobenzo[*b*]thiophene-3-carbonitrile **11-P2** |
| | 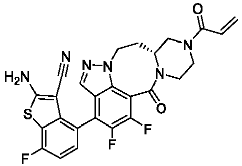 | 4-((*R*)-10-Acryloyl-1,2-difluoro-14-oxo-8,8a,9,10 ,11,12-hexahydro-7*H*,14*H*-pyrazino[1',2':5.6][1,5] diazocino[3,2,1-*hi*]indazol-3-yl)-2-amino-7-fluoro benzo[*b*]thiophene-3-carbonitrile |
| | 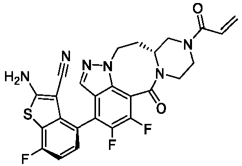 | (*R*)-4-((*R*)-10-Acryloyl-1,2-difluoro-14-oxo*-*8,8*a,* 9,10,11,12-hexahydro-7*H*,14*H*-pyrazino[1',2':5.6][ 1,5]diazocino[3,2,1-*hi*]indazol-3-yl)-2-amino-7-fl uorobenzo[*b*]thiophene-3-carbonitrile |
| | 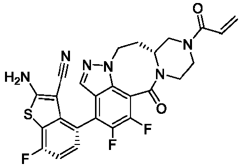 | (*S*)-4-((*R*)-10-Acryloyl-1,2-difluoro-14-oxo-8,8*a*, 9,10,11,12-hexahydro-7*H*,14*H*-pyrazino[1',2':5.6][ 1,5]diazocino[3,2,1-*hi*]indazol-3-yl)-2-amino-7-fl uorobenzo[*b*]thiophene-3-carbonitrile |
| | 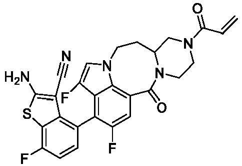 | 4-(10-Acryloyl-2,4-difluoro-14-oxo-8,8*a*,9,10,11, 12-hexahydro-7*H*,14*H*-pyrazino[1',2':5.6][1,5]dia zocino[3,2,1-*hi*]indol-3-yl)-2-amino-7-fluorobenz o[*b*]thiophene-3-carbonitrile |
| **13** | 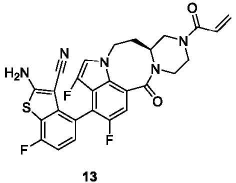 | 4-((*S*)-10-Acryloyl-2,4-difluoro-14-oxo-8,8*a*,9,10, 11, 12-hexahydro-7H, 14H-pyrazino[1',2':5.6][1,5] diazocino[3,2,1-*hi*]indol-3-yl)-2-amino-7-fluorobe nzo[*b*]thiophene-3-carbonitrile **13** |
| **13-P1** | 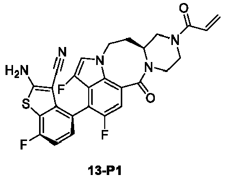 | (*R*)-4-((*S*)-10-Acryloyl-2,4-difluoro-14-oxo-8,8*a*, 9,10,11,12-hexahydro-7*H*,14*H*-pyrazino[1',2':5.6][ 1,5]diazocino[3,2,1-*hi*]indol-3-yl)-2-amino-7-fluo robenzo[*b*]thiophene-3-carbonitrile **13-P1** |
| **13-P2** | 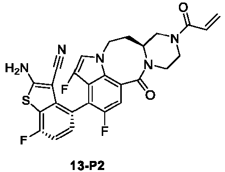 | (*S*)-4-((*S*)-10*-*Acryloyl*-*2*,*4-difluoro-14-oxo-8,8*a,*9 ,10,11,12-hexahydro-7*H*,14*H*-pyrazino[1',2':5.6][1 ,5]diazocino[3,2,1-*hi*]indol-3-yl)-2-amino-7-fluor obenzo[*b*]thiophene-3-carbonitrile **13-P2** |
| | 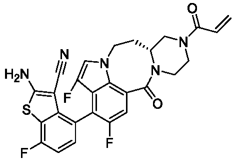 | 4-((*R*)-10-Acryloyl-2,4-difluoro-14-oxo-8,8*a*,9,10 ,11,12-hexahydro-7*H*,14*H*-pyrazino[1',2':5.6][1,5] diazocino[3,2,1-*hi*]indol-3-yl)-2-amino-7-fluorobe nzo[*b*]thiophene-3-carbonitrile |
| | 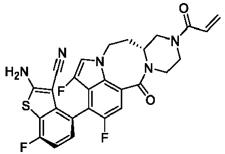 | (*R*)-4-((*R*)-10-Acryloyl-2,4-difluoro-14-oxo*-*8,8*a,* 9,10,11,12-hexahydro-7*H*,14*H*-pyrazino[1',2':5.6][ 1,5]diazocino[3,2,1-*hi*]indol-3-yl)-2-amino-7-fluo robenzo[*b*]thiophene-3-carbonitrile |
| | 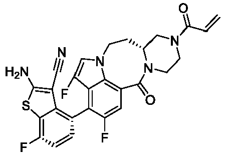 | (*S*)-4-((*R*)-10-Acryloyl-2,4-difluoro-14-oxo-8,8*a*, 9,10,11,12-hexahydro-7*H*,14*H*-pyrazino[1',2':5.6][ 1,5]diazocino[3,2,1-*hi*]indol-3-yl)-2-amino-7-fluo robenzo[*b*]thiophene-3-carbonitrile |
| | 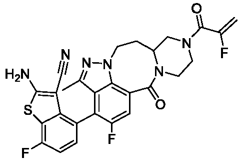 | 2-Amino-7-fluoro-4-(2-fluoro-10-(2-fluoroacryloy 1)-4-methyl-14-oxo-8,8*a*,9,10,11,12-hexahydro-7 *H,*14*H*-pyrazino[1',2':5,6][1,5]diazocino[3,2,1-*hi*]i ndazol-3-yl)benzo[*b*]thiophene-3-carbonitrile |
| **15** | 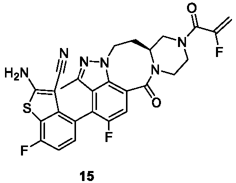 | 2-Amino-7-fluoro-4-((*S*)-2-fluoro-10-(2-fluoroacr yloyl)-4-methyl-14-oxo-8,8*a*,9,10,11,12-hexahydr o-7*H*,14*H*-pyrazino[1',2':5,6][1,5]diazocino[3,2,1-*hi*]indazol-3-yl)benzo[*b*]thiophene-3-carbonitrile **15** |
| **15-P1** | 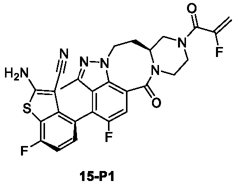 | (*R*)-2-Amino-7-fluoro-4-((*S*)-2-fluoro-10-(2-fluor oacryloyl)-4-methyl-14-oxo-8,8*a*,9,10,11,12-hexa hydro-7*H*,14*H*-pyrazino[1',2':5,6][1,5]diazocino[3 ,2,1-*hi*]indazol-3-yl)benzo[*b*]thiophene-3-carbonit rile **15-P1** |
| **15-P2** | 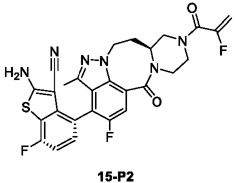 | (*S*)-2-Amino-7-fluoro-4-((*S*)-2-fluoro-10-(2-fluoro acryloyl)-4-methyl-14-oxo-8,8*a*,9,10,11,12-hexah ydro-7*H*,14*H*-pyrazino[1',2':5,6][1,5]diazocino[3, 2,1-*hi*]indazol-3-yl)benzo[*b*]thiophene-3-carbonitr ile **15-P2** |
| | 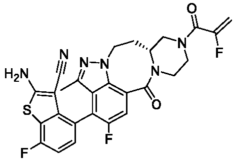 | 2-Amino-7-fluoro-4-((*R*)-2-fluoro-10-(2-fluoroacr yloyl)-4-methyl-14-oxo-8,8*a*,9,10,11,12-hexahydr o-7*H*,14*H*-pyrazino[1',2':5,6][1,5]diazocino[3,2,1-*hi*]indazol-3-yl)benzo[b]thiophene-3-carbonitrile |
| | 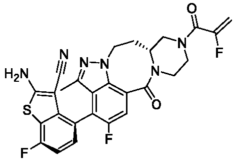 | (*R*)-2-Amino-7-fluoro-4-((*R*)-2-fluoro-10-(2-fluor oacryloyl)-4-methyl-14-oxo-8,8*a*,9,10,11,12-hexa hydro-7*H*,14*H*-pyrazino[1',2':5,6][1,5]diazocino[3 ,2,1-*hi*]indazol-3-yl)benzo[*b*]thiophene-3-carbonit rile |
| | 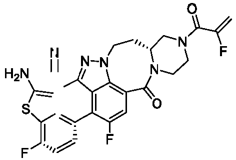 | (*S*)-2-Amino-7-fluoro-4-((*R*)-2-fluoro-10-(2-fluor oacryloyl)-4-methyl-14-oxo-8,8*a*,9,10,11,12-hexa hydro-7*H*,14*H*-pyrazino[1',2':5,6][1,5]diazocino[3 ,2,1-*hi*]indazol-3-yl)benzo[*b*]thiophene-3-carbonit rile |
| | 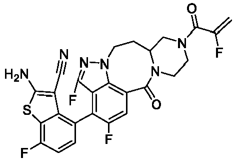 | 2-Amino-4-(2,4-difluoro-10-(2-fluoroacryloyl)-14 -oxo-8,8*a*,9,10,11,12-hexahydro-7*H*,14*H*-pyrazin o[1',2':5,6][1,5]diazocino[3,2,1-*hi*]indazol-3-yl)-7-fluorobenzo[*b*]thiophene-3-carbonitrile |
| **16** | 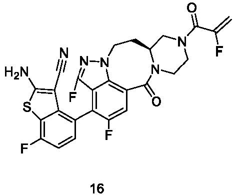 | 2-Amino-4-((*S*)-2,4-difluoro-10-(2-fluoroacryloyl )-14-oxo-8,8*a*,9,10,11,12-hexahydro-7*H*,14*H*-pyra zino[1',2':5,6][1,5]diazocino[3,2,1-*hi*]indazol-3-yl )-7-fluorobenzo[*b*]thiophene-3-carbonitrile **16** |
| **16-P1** | 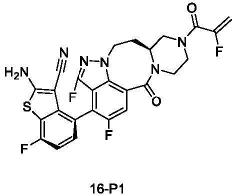 | (*R*)-2-Amino-4-((*S*)-2,4-difluoro-10-(2-fluoroacry loyl)-14-oxo-8,8*a,*9,10,11,12-hexahydro-7*H,*14*H-*pyrazino[1',2':5,6][1,5]diazocino[3,2,1-*hi*]indazol-3-yl)-7-fluorobenzo[*b*]thiophene-3-carbonitrile **16-P1** |
| **16-P2** | 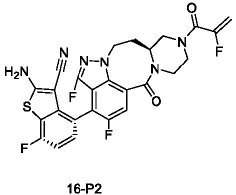 | (*S*)-2-Amino-4-((*S*)-2,4-difluoro-10-(2-fluoroacryl oyl)-14-oxo-8,8*a,*9,10,11,12-hexahydro-7*H,*14*H*-p yrazino[1',2':5,6][1,5]diazocino[3,2,1-*hi*]indazol-3 -yl)-7-fluorobenzo[*b*]thiophene-3-carbonitrile **16-P2** |
| | 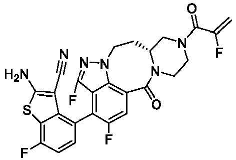 | 2-Amino-4-((*R*)-2,4-difluoro-10-(2-fluoroacryloyl )-14-oxo-8,8*a*,9,10,11,12-hexahydro-7*H*,14*H*-pyra zino[1',2':5,6][1,5]diazocino[3,2,1-*hi*]indazol-3-yl )-7-fluorobenzo[*b*]thiophene-3-carbonitrile |
| | 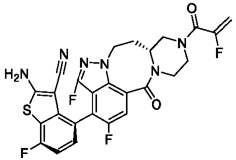 | (*R*)-2-Amino-4-((*R*)-2,4-difluoro-10-(2-fluoroacry loyl)-14-oxo-8,8*a,*9,10,11,12-hexahydro-7*H,*14*H-*pyrazino[1',2':5,6][1,5]diazocino[3,2,1-*hi*]indazol-3-yl)-7-fluorobenzo[*b*]thiophene-3-carbonitrile |
| | 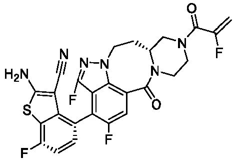 | (*S*)-2-Amino-4-((*R*)-2,4-difluoro-10-(2-fluoroacry loyl)-14-oxo-8,8*a*,9,10,11,12-hexahydro-7*H,*14*H-*pyrazino[1',2':5,6][1,5]diazocino[3,2,1-*hi*]indazol-3-yl)-7-fluorobenzo[*b*]thiophene-3-carbonitrile |
| | 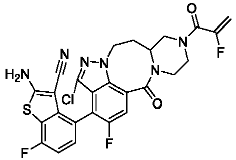 | 2-Amino-4-(4-chloro-2-fluoro-10-(2-fluoroacrylo yl)-14-oxo-8,8*a*,9,10,11,12-hexahydro-7*H*,14*H*-py razino[1',2':5,6][1,5]diazocino[3,2,1-*hi*]indazol-3-yl)-7-fluorobenzo[*b*]thiophene-3-carbonitrile |
| **17** | 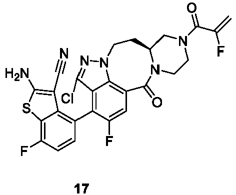 | 2-Amino-4-((*S*)-4-chloro-2-fluoro-10-(2-fluoroacr yloyl)-14-oxo-8,8*a,*9,10,11,12-hexahydro-7*H,*14*H* -pyrazino[1',2':5,6][1,5]diazocino[3,2,1-*hi*]indazol -3-yl)-7-fluorobenzo[*b*]thiophene-3-carbonitrile **17** |
| **17-P1** | 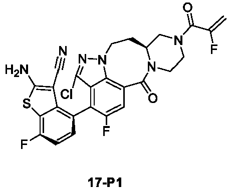 | (*R*)-2-Amino-4-((*S*)-4-chloro-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind azol-3-yl)-7-fluorobenzo[*b*]thiophene-3-carbonitri le **17-P1** |
| **17-P2** | 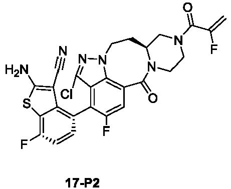 | (*S*)-2-Amino-4-((*S*)-4-chloro-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind azol-3-yl)-7-fluorobenzo[*b*]thiophene-3-carbonitri le **17-P2** |
| | 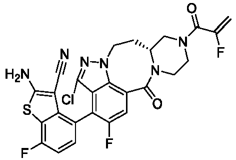 | 2-Amino-4-((*R*)-4-chloro-2-fluoro-10-(2-fluoroacr yloyl)-14-oxo-8,8*a,*9,10,11,12-hexahydro-7*H,*14*H* -pyrazino[1',2':5,6][1,5]diazocino[3,2,1-*hi*]indazol -3-yl)-7-fluorobenzo[*b*]thiophene-3-carbonitrile |
| | 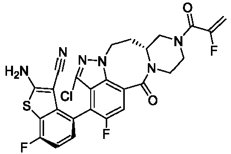 | (*R*)-2-Amino-4-((*R*)-4-chloro-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind azol-3-yl)-7-fluorobenzo[*b*]thiophene-3-carbonitri le |
| | 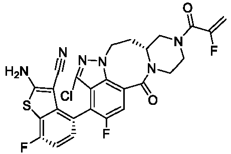 | (*S*)-2-Amino-4-((*R*)-4-chloro-2-fluoro-10-(2-fluor oacryloyl)-14-oxo-8,8*a*,9,10,11,12-hexahydro-7*H*, 14*H*-pyrazino[1',2':5,6][1,5]diazocino[3,2,1-*hi*]ind azol-3-yl)-7-fluorobenzo[*b*]thiophene-3-carbonitri le |
| | 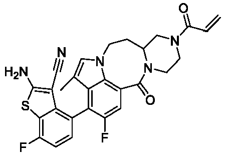 | 4-(10-Acryloyl-2-fluoro-4-methyl-14-oxo-8,8*a*,9, 10,11,12-hexahydro-7*H*,14*H*-pyrazino[1',2':5,6][1, 5]diazocino[3,2,1-*hi*]indol-3-yl)-2-amino-7-fluoro benzo[*b*]thiophene-3-carbonitrile |
| **18** | 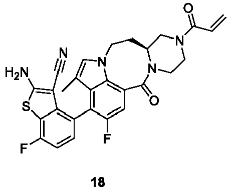 | 4-((*S*)-10-Acryloyl-2-fluoro-4-methyl-14-oxo-8,8 *a*,9,10,11,12-hexahydro-7*H*,14*H*-pyrazino[1',2':5, 6][1,5]diazocino[3,2,1-*hi*]indol-3-yl)-2-amino-7-fl uorobenzo[*b*]thiophene-3-carbonitrile **18** |
| **18-P1** | 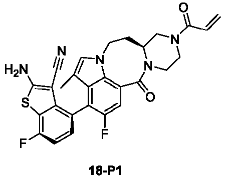 | (*R*)-4-((*S*)-10-Acryloyl-2-fluoro-4-methyl-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile **18-P1** |
| **18-P2** | 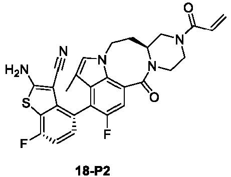 | (*S*)-4-((*S*)-10-Acryloyl-2-fluoro-4-methyl-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile **18-P2** |
| | 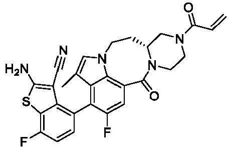 | 4-((*R*)-10-Acryloyl-2-fluoro-4-methyl-14-oxo-8,8 *a*,9,10,11,12-hexahydro-7*H*,14*H*-pyrazino[1',2':5, 6][1,5]diazocino[3,2,1-*hi*]indol-3-yl)-2-amino-7-fl uorobenzo[*b*]thiophene-3-carbonitrile |
| | 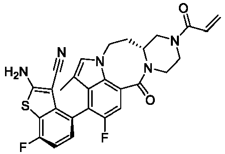 | (*R*)-4-((*R*)-10-Acryloyl-2-fluoro-4-methyl-14-oxo -8,8*a*,9,10,11,12-hexahydro-7*H*,14*H*-pyrazino[1',2 ':5,6][1,5]diazocino[3,2,1-*hi*]indol-3-yl)-2-amino-7-fluorobenzo[*b*]thiophene-3-carbonitrile |
| | 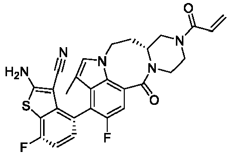 | (*S*)-4-((*R*)-10-Acryloyl-2-fluoro-4-methyl-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile |
| | 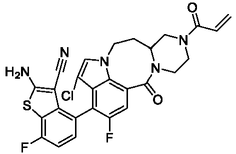 | 4-(10-Acryloyl-4-chloro-2-fluoro-14-oxo-8,8*a*,9,1 0,11,12-hexahydro-7*H*,14*H*-pyrazino[1',2':5.6][1,5 ]diazocino[3,2,1-*hi*]indol-3-yl)-2-amino-7-fluorob enzo[*b*]thiophene-3-carbonitrile |
| **19** | 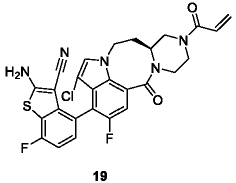 | 4-((*S*)-10-Acryloyl-4-chloro-2-fluoro-14-oxo-8,8*a* ,9,10,11,12-hexahydro-7*H*,14*H*-pyrazino[1',2':5.6] [1,5]diazocino[3,2,1-*hi*]indol-3-yl)-2-amino-7-flu orobenzo[*b*]thiophene-3-carbonitrile **19** |
| **19-P1** | 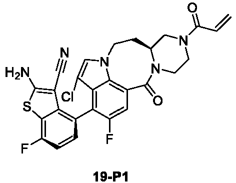 | (*R*)-4-((*S*)-10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile **19-P1** |
| **19-P2** | 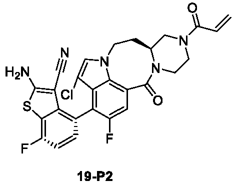 | (*S*)-4-((*S*)-10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile **19-P2** |
| | 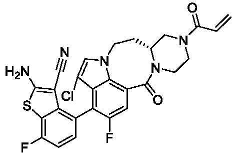 | 4-((*R*)-10-Acryloyl-4-chloro-2-fluoro-14-oxo*-*8,8*a* ,9,10,11,12-hexahydro-7*H*,14*H*-pyrazino[1',2':5.6] [1,5]diazocino[3,2,1-*hi*]indol-3-yl)-2-amino-7-flu orobenzo[*b*]thiophene-3-carbonitrile |
| | 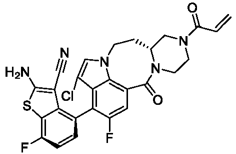 | (*R*)-4-((*R*)*-*10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile |
| | 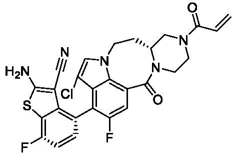 | (*S*)-4-((*R*)-10-Acryloyl-4-chloro-2-fluoro-14-oxo-8,8*a*,9,10,11,12-hexahydro-7*H*,14*H*-pyrazino[1',2' :5,6][1,5]diazocino[3,2,1-*hi*]indol-3-yl)-2-amino-7 -fluorobenzo[*b*]thiophene-3-carbonitrile |
| | 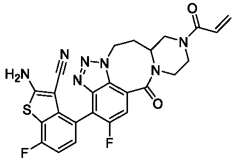 | 4-(9-Acryloyl-2-fluoro-12-oxo-7,7a,8,9,10,11-hex ahydro-6*H*,12*H*-4,5,5*a*,9,11*a*-pentaazabenzo[5,6]c ycloocta[1,2,3-*cd*]inden-3-yl)-2-amino-7-fluorobe nzo[*b*]thiophene-3-carbonitrile |
| **20** | 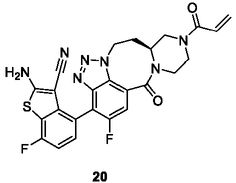 | 4-((*S*)-9-Acryloyl-2-fluoro-12-oxo-7,7*a*,8,9,10,11-hexahydro-6*H*,12*H-*4,5,5*a,*9,11*a*-pentaazabenzo[5 ,6]cycloocta[1,2,3-*cd*]inden-3-yl)-2-amino-7-fluor obenzo[*b*]thiophene-3-carbonitrile **20** |
| **20-P1** | 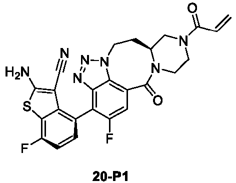 | (*R*)-4-((*S*)-9-Acryloyl-2-fluoro-12-oxo-7,7*a*,8,9,10 *,*11-hexahydro-6*H,*12*H-*4,5,5*a,*9,11*a*-pentaazaben zo[5,6]cycloocta[1,2,3-*cd*]inden-3-yl)-2-amino-7-fluorobenzo[b]thiophene-3-carbonitrile **20-P1** |
| **20-P2** | 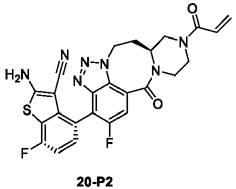 | (*S*)-4-((*S*)-9-Acryloyl-2-fluoro-12-oxo-7,7*a*,8,9,10 *,*11-hexahydro-6*H,*12*H-*4,5,5*a,*9,11*a*-pentaazaben zo[5,6]cycloocta[1,2,3-*cd*]inden-3-yl)-2-amino-7-fluorobenzo[*b*]thiophene-3-carbonitrile **20-P2** |
| | 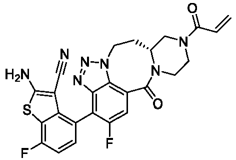 | 4-((*R*)-9-Acryloyl-2-fluoro-12-oxo-7,7*a*,8,9,10,11 -hexahydro-6*H*,12*H*-4,5,5*a*,9,11*a*-pentaazabenzo[ 5,6]cycloocta[1,2,3-*cd*]inden-3-yl)-2-amino-7-flu orobenzo[*b*]thiophene-3-carbonitrile |
| | 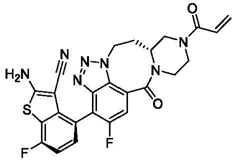 | (*R*)-4-((*R*)-9-Acryloyl-2-fluoro-12-oxo-7,7*a*,8,9,1 0*,*11-hexahydro-6*H*,12*H-*4,5,5*a,*9,11*a*-pentaazabe nzo[5,6]cycloocta[1,2,3-*cd*]inden-3-yl)-2-amino-7 -fluorobenzo[*b*]thiophene-3-carbonitrile |
| | 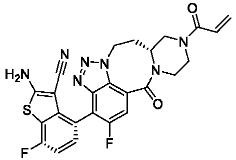 | (*S*)-4-((*R*)-9-Acryloyl-2-fluoro-12-oxo-7,7*a*,8,9,10 *,*11-hexahydro-6*H,*12*H-*4,5,5*a,*9,11*a*-pentaazaben zo[5,6]cycloocta[1,2,3-*cd*]inden-3-yl)-2-amino-7-fluorobenzo[*b*]thiophene-3-carbonitrile |
| | 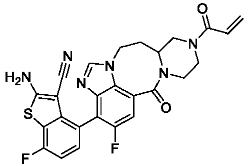 | 4-(9-Acryloyl-2-fluoro-12-oxo-7,7*a*,8,9,10,11-hex ahydro-6*H*,12*H*-4,5*a*,9,11*a*-tetraazabenzo[5,6]cycl oocta[1,2,3-*cd*]inden-3-yl)-2-amino-7-fluorobenzo [*b*]thiophene-3-carbonitrile |
| **21** | 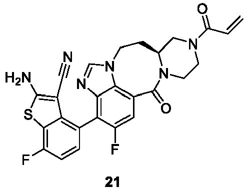 | 4-((*S*)-9-Acryloyl-2-fluoro-12-oxo-7,7*a*,8,9,10,11-hexahydro-6*H*,12*H*-4,5*a*,9,11*a*-tetraazabenzo[5,6] cycloocta[1,2,3-*cd*]inden-3-yl)-2-amino-7-fluorob enzo[*b*]thiophene-3-carbonitrile **21** |
| **21-P1** | 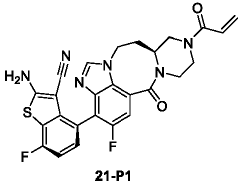 | (*R*)-4-((*S*)-9-Acryloyl-2-fluoro-12-oxo-7,7*a*,8,9,10 *,*11-hexahydro-6*H,*12*H-*4,5*a,*9,11*a*-tetraazabenzo[ 5,6]cycloocta[1,2,3-*cd*]inden-3-yl)-2-amino-7-flu orobenzo[*b*]thiophene-3-carbonitrile **21-P1** |
| **21-P2** | 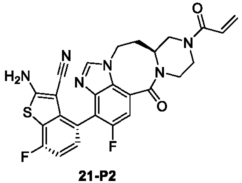 | (*S*)-4-((*S*)-9-Acryloyl-2-fluoro-12-oxo-7,7*a*,8,9,10 *,*11-hexahydro-6*H,*12*H-*4,5*a,*9,11*a*-tetraazabenzo[ 5,6]cycloocta[1,2,3-*cd*]inden-3-yl)-2-amino-7-flu orobenzo[*b*]thiophene-3-carbonitrile **21-P2** |
| | 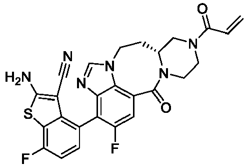 | 4-((*R*)-9-Acryloyl-2-fluoro-12-oxo-7,7*a*,8,9,10,11 -hexahydro-6*H*,12*H*-4,5*a*,9,11*a*-tetraazabenzo[5,6 ]cycloocta[1,2,3-*cd*]inden-3-yl)-2-amino-7-fluoro benzo[*b*]thiophene-3-carbonitrile |
| | 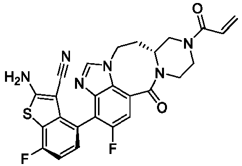 | (*R*)-4-((*R*)-9-Acryloyl-2-fluoro-12-oxo-7,7*a*,8,9,1 0,11-hexahydro-6*H,*12*H-*4,5*a,*9,11*a*-tetraazabenzo [5,6]cycloocta[1,2,3-*cd*]inden-3-yl)-2-amino-7-flu orobenzo[*b*]thiophene-3-carbonitrile |
| | 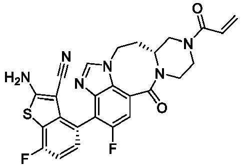 | (*S*)-4-((*R*)-9-Acryloyl-2-fluoro-12-oxo-7,7*a*,8,9,10 *,*11-hexahydro-6*H,*12*H-*4,5*a,*9,11*a*-tetraazabenzo[ 5,6]cycloocta[1,2,3-*cd*]inden-3-yl)-2-amino-7-flu orobenzo[*b*]thiophene-3-carbonitrile |

Another aspect of the present disclosure relates to a compound represented by general formula (IMa) or a salt thereof: 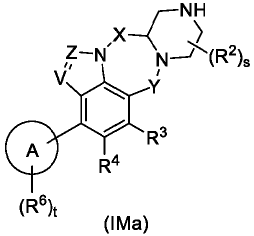 wherein:
ring A, V, X, Y, Z, R², R³, R⁴, R⁶, s, and t are as defined in general formula (IM).

Another aspect of the present disclosure relates to a compound represented by general formula (Ia) or a salt thereof: 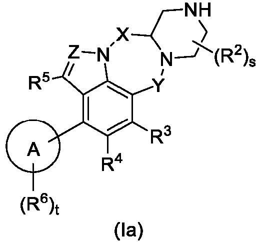 wherein:
ring A, X, Y, Z, R², R³, R⁴, R⁵, R⁶, s, and t are as defined in general formula (I).

Another aspect of the present disclosure relates to a compound represented by general formula (IIa) or a salt thereof: 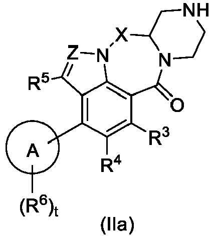 wherein:
ring A, X, Z, R³, R⁴, R⁵, R⁶, and t are as defined in general formula (II).

Another aspect of the present disclosure relates to a compound represented by general formula (IIIa) or a salt thereof: 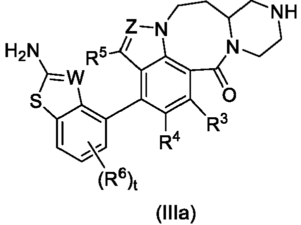 wherein:
W, Z, R³, R⁴, R⁵, R⁶, and t are as defined in general formula (III).

Another aspect of the present disclosure relates to a compound represented by general formula (III-1a) or a salt thereof: 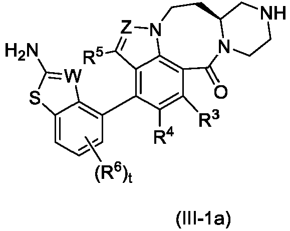 wherein:
W, Z, R³, R⁴, R⁵, R⁶, and t are as defined in general formula (III-1).

Another aspect of the present disclosure relates to a compound represented by general formula (III-2a) or a salt thereof: 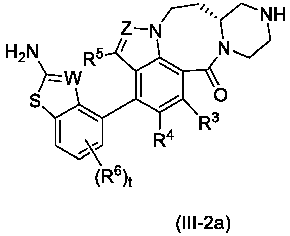 wherein:
W, Z, R³, R⁴, R⁵, R⁶, and t are as defined in general formula (III-2).

Another aspect of the present disclosure relates to a compound represented by general formula (III-1-Aa) or a salt thereof: 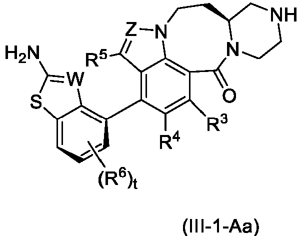 wherein:
W, Z, R³, R⁴, R⁵, R⁶, and t are as defined in general formula (III-1-A).

Another aspect of the present disclosure relates to a compound represented by general formula (III-1-Ba) or a salt thereof: 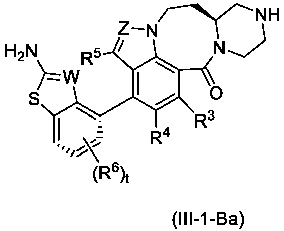 wherein:
W, Z, R³, R⁴, R⁵, R⁶, and t are as defined in general formula (III-1-B).

Another aspect of the present disclosure relates to a compound represented by general formula (III-2-Aa) or a salt thereof: 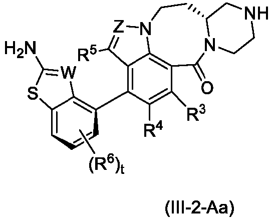 wherein:
W, Z, R³, R⁴, R⁵, R⁶, and t are as defined in general formula (III-2-A).

Another aspect of the present disclosure relates to a compound represented by general formula (III-2-Ba) or a salt thereof: 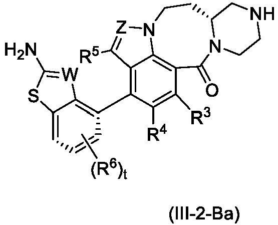 wherein:
W, Z, R³, R⁴, R⁵, R⁶, and t are as defined in general formula (III-2-B).

**Table B. Typical intermediate compounds of the present disclosure include, but are not limited to:**

| Compound No. | Compound structure | Name |
|---|---|---|
| | 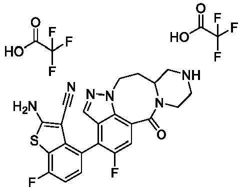 | 2-Amino-7-fluoro-4-(2-fluoro-14-oxo-8,8*a*, 9,10,11,12-hexahydro-7*H*,14*H*-pyrazino[1', 2':5,6][1,5]diazocino[3,2,1-*hi*]indazol-3-yl) benzo[*b*]thiophene-3-carbonitrile bis(2,2,2-trifluoroacetate) |
| | 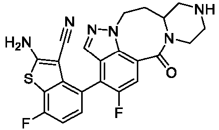 | 2-Amino-7-fluoro-4-(2-fluoro-14-oxo-8,8*a*, 9,10,11,12-hexahydro-7*H*,14*H*-pyrazino[1', 2':5,6][1,5]diazocino[3,2,1-*hi*]indazol-3-yl) benzo[*b*]thiophene-3-carbonitrile |
| **1j** | 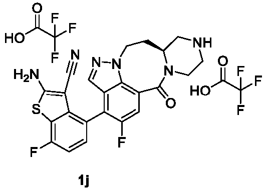 | 2-Amino-7-fluoro-4-((*S*)-2-fluoro-14-oxo-8 ,8*a*,9,10,11,12-hexahydro-7*H*,14*H*-pyrazin o[1',2':5,6][1,5]diazocino[3,2,1-*hi*]indazol-3-yl)benzo[*b*]thiophene-3-carbonitrile bis(2,2,2-trifluoroacetate) **1j** |
| | 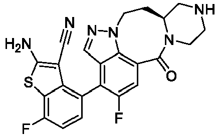 | 2-Amino-7-fluoro-4-((*S*)-2-fluoro-14-oxo-8 ,8*a*,9,10,11,12-hexahydro-7*H*,14*H*-pyrazin o[1',2':5,6][1,5]diazocino[3,2,1-*hi*]indazol-3-yl)benzo[*b*]thiophene-3-carbonitrile |
| | 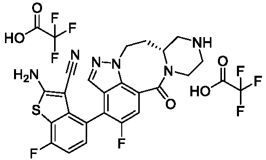 | 2-Amino-7-fluoro-4-((*R*)-2-fluoro-14-oxo-8,8*a*,9,10,11,12-hexahydro-7*H*,14*H*-pyrazi no[1',2':5,6][1,5]diazocino[3,2,1-*hi*]indazol -3-yl)benzo[*b*]thiophene-3-carbonitrile bis(2,2,2-trifluoroacetate) |
| | 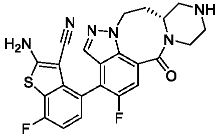 | 2-Amino-7-fluoro-4-((*R*)-2-fluoro-14-oxo-8,8*a*,9,10,11,12-hexahydro-7*H*,14*H*-pyrazi no[1',2':5,6][1,5]diazocino[3,2,1-*hi*]indazol -3-yl)benzo[*b*]thiophene-3-carbonitrile |
| | 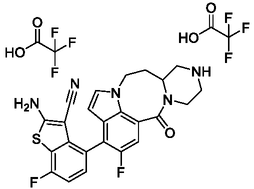 | 2-Amino-7-fluoro-4-(2-fluoro-14-oxo-8,8*a*, 9,10,11,12-hexahydro-7*H*,14*H*-pyrazino[1', 2':5,6][1,5]diazocino[3,2,1-*hi*]indol-3-yl)be nzo[*b*]thiophene-3-carbonitrile bis(2,2,2-trifluoroacetate) |
| | 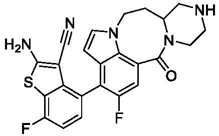 | 2-Amino-7-fluoro-4-(2-fluoro-14-oxo-8,8*a*, 9,10,11,12-hexahydro-7*H*,14*H*-pyrazino[1', 2':5,6][1,5]diazocino[3,2,1-*hi*]indol-3-yl)be nzo[*b*]thiophene-3-carbonitrile |
| **2f** | 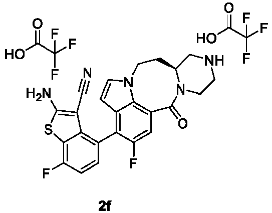 | 2-Amino-7-fluoro-4-((*S*)-2-fluoro-14-oxo-8 ,8*a*,9,10,11,12-hexahydro-7*H*,14*H*-pyrazin o[1',2':5,6][1,5]diazocino[3,2,1-*hi*]indol-3-yl)benzo[*b*]thiophene-3-carbonitrile bis(2,2,2-trifluoroacetate) **2f** |
| | 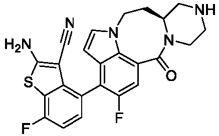 | 2-Amino-7-fluoro-4-((*S*)-2-fluoro-14-oxo-8 ,8*a*,9,10,11,12-hexahydro-7*H*,14*H*-pyrazin o[1',2':5,6][1,5]diazocino[3,2,1-*hi*]indol-3-yl)benzo[*b*]thiophene-3-carbonitrile |
| | 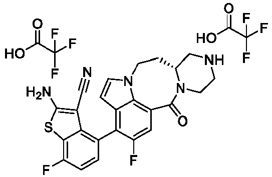 | 2-Amino-7-fluoro-4-((*R*)-2-fluoro-14-oxo-8,8*a*,9,10,11,12-hexahydro-7*H*,14*H*-pyrazi no[1',2':5,6][1,5]diazocino[3,2,1-*hi*]indol-3 -yl)benzo[*b*]thiophene-3-carbonitrile bis(2,2,2-trifluoroacetate) |
| | 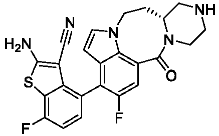 | 2-Amino-7-fluoro-4-((*R*)-2-fluoro-14-oxo-8,8*a*,9,10,11,12-hexahydro-7*H*,14*H*-pyrazi no[1',2':5,6][1,5]diazocino[3,2,1-*hi*]indol-3 -yl)benzo[*b*]thiophene-3-carbonitrile |
| | 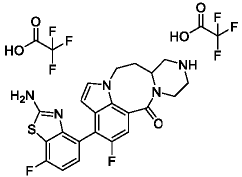 | 3-(2-Amino-7-fluorobenzo[*d*]thiazol-4-yl)-2-fluoro-8,8a,9,10,11,12-hexahydro-7*H*,14 *H*-pyrazino[1',2':5,6][1,5]diazocino[3,2,1-*h i*]indol-14-onebis(2,2,2-trifluoroacetate) |
| | 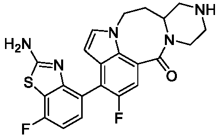 | 3-(2-Amino-7-fluorobenzo[*d*]thiazol-4-yl)-2-fluoro-8,8*a*,9,10,11,12-hexahydro-7*H*,14 *H*-pyrazino[1',2':5,6][1,5]diazocino[3,2,1-*h i*]indol-14-one |
| **3c** | 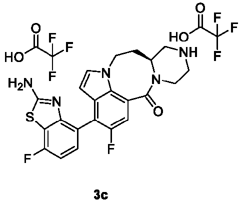 | (8*aS*)-3-(2-Amino-7-fluorobenzo[*d*]thiazol-4-yl)-2-fluoro-8,8*a*,9,10,11,12-hexahydro-7 *H*,14*H*-pyrazino[1',2':5,6][1,5]diazocino[3, 2,1-*hi*]indol-14-one bis(2,2,2-trifluoroacetate) **3c** |
| | 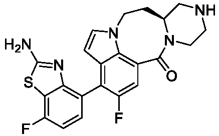 | (8*aS*)-3-(2-Amino-7-fluorobenzo[d]thiazol-4-yl)-2-fluoro-8,8*a*,9,10,11,12-hexahydro-7 *H*,14*H*-pyrazino[1',2':5,6][1,5]diazocino[3, 2,1-*hi*]indol-14-one |
| | 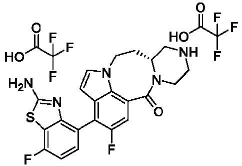 | (8*aR*)-3-(2-Amino-7-fluorobenzo[*d*]thiazol -4-yl)-2-fluoro-8,8*a*,9,10,11,12-hexahydro-7*H*,14*H*-pyrazino[1',2':5,6][1,5]diazocino[3 ,2,1-*hi*]indol-14-one bis(2,2,2-trifluoroacetate) |
| | 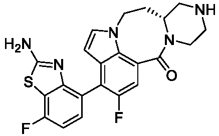 | (8*aR*)-3-(2-Amino-7-fluorobenzo[*d*]thiazol -4-yl)-2-fluoro-8,8*a*,9,10,11,12-hexahydro-7*H*,14*H*-pyrazino[1',2':5,6][1,5]diazocino[3 ,2,1-*hi*]indol-14-one |
| | 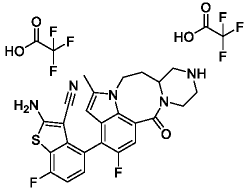 | 2-Amino-7-fluoro-4-(2-fluoro-5-methyl-14 -oxo-8,8*a*,9,10,11,12-hexahydro-7*H*,14*H*-p yrazino[1',2':5,6][1,5]diazocino[3,2,1-*hi*]in dol-3-yl)benzo[*b*]thiophene-3-carbonitrile bis(2,2,2-trifluoroacetate) |
| | 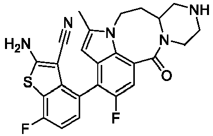 | 2-Amino-7-fluoro-4-(2-fluoro-5-methyl-14 -oxo-8,8*a*,9,10,11,12-hexahydro-7*H*,14*H*-p yrazino[1',2':5,6][1,5]diazocino[3,2,1-*hi*]in dol-3-yl)benzo[*b*]thiophene-3-carbonitrile |
| **4f** | 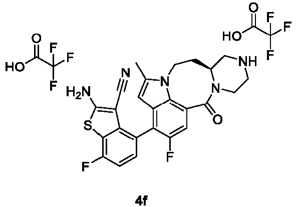 | 2-Amino-7-fluoro-4-((*S*)-2-fluoro-5-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le bis(2,2,2-trifluoroacetate) **4f** |
| | 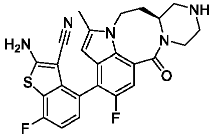 | 2-Amino-7-fluoro-4-((*S*)-2-fluoro-5-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le |
| | 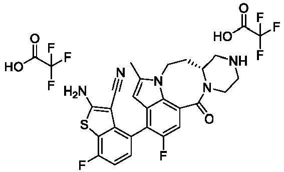 | 2-Amino-7-fluoro-4-((*R*)-2-fluoro-5-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le bis(2,2,2-trifluoroacetate) |
| | 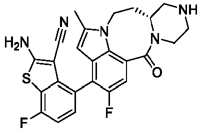 | 2-Amino-7-fluoro-4-((*R*)-2-fluoro-5-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le |
| | 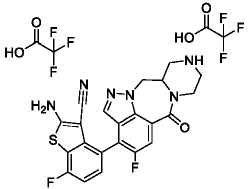 | 2-Amino-7-fluoro-4-(4-fluoro-6-oxo-8,9,10 ,11,11*a*,12-hexahydro-6*H*-pyrazino[2',1':3, 4][1,4]diazepino[6,7,1-*hi*]indazol-3-yl)benz o[*b*]thiophene-3-carbonitrile bis(2,2,2-trifluoroacetate) |
| | 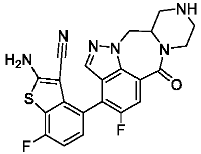 | 2-Amino-7-fluoro-4-(4-fluoro-6-oxo-8,9,10 ,11,11a,12-hexahydro-6H-pyrazino[2',1':3,4 ][1,4]diazepino[6,7,1-*hi*]indazol-3-yl)benzo [b]thiophene-3-carbonitrile |
| | 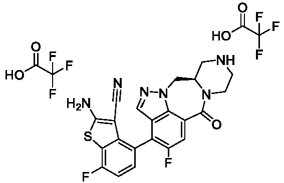 | 2-Amino-7-fluoro-4-((11a*R*)-4-fluoro-6-ox o-8,9,10,11,11a,12-hexahydro-6*H*-pyrazino [2',1':3,4][1,4]diazepino[6,7,1-*hi*]indazol-3-yl)benzo[*b*]thiophene-3-carbonitrile bis(2,2,2-trifluoroacetate) |
| | 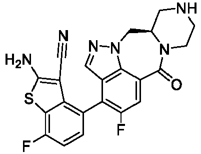 | 2-Amino-7-fluoro-4-((11aR)-4-fluoro-6-ox o-8,9,10,11,11a,12-hexahydro-6H-pyrazino [2',1':3,4][1,4]diazepino[6,7,1-*hi*]indazol-3-yl)benzo[b]thiophene-3-carbonitrile |
| | 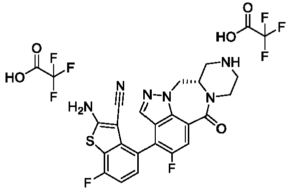 | 2-Amino-7-fluoro-4-((11*aS*)-4-fluoro-6-oxo -8,9,10,11,11*a*,12-hexahydro-6*H*-pyrazino[ 2',1':3,4][1,4]diazepino[6,7,1-*hi*]indazol-3-yl)benzo[*b*]thiophene-3-carbonitrile bis(2,2,2-trifluoroacetate) |
| | 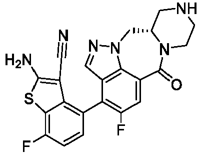 | 2-Amino-7-fluoro-4-((11aS)-4-fluoro-6-ox o-8,9,10,11,11a,12-hexahydro-6H-pyrazino [2',1':3,4][1,4]diazepino[6,7,1-*hi*]indazol-3-yl)benzo[b]thiophene-3-carbonitrile |
| | 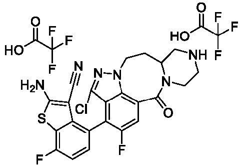 | 2-Amino-4-(4-chloro-2-fluoro-14-oxo-8,8*a* ,9,10,11,12-hexahydro-7*H*,14*H*-pyrazino[1' ,2':5,6][1,5]diazocino[3,2,1-*hi*]indazol-3-yl )-7-fluorobenzo[*b*]thiophene-3-carbonitrile bis(2,2,2-trifluoroacetate) |
| | 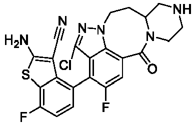 | 2-Amino-4-(4-chloro-2-fluoro-14-oxo-8,8*a* ,9,10,11,12-hexahydro-7*H*,14*H*-pyrazino[1' ,2':5,6][1,5]diazocino[3,2,1-*hi*]indazol-3-yl )-7-fluorobenzo[*b*]thiophene-3-carbonitrile |
| **7c** | 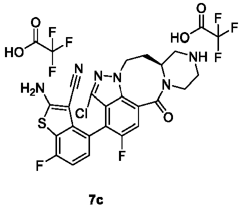 | 2-Amino-4-((*S*)-4-chloro-2-fluoro-14-oxo-8,8*a*,9,10,11,12-hexahydro-7*H*,14*H*-pyrazi no[1',2':5,6][1,5]diazocino[3,2,1-*hi*]indazol -3-yl)-7-fluorobenzo[*b*]thiophene-3-carboni trile bis(2,2,2-trifluoroacetate) **7c** |
| | 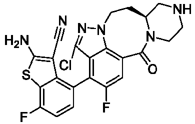 | 2-Amino-4-((*S*)-4-chloro-2-fluoro-14-oxo-8,8*a*,9,10,11,12-hexahydro-7*H*,14*H*-pyrazi no[1',2':5,6][1,5]diazocino[3,2,1-*hi*]indazol -3-yl)-7-fluorobenzo[*b*]thiophene-3-carboni trile |
| | 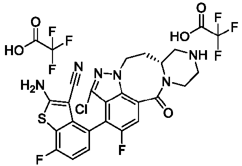 | 2-Amino-4-((*R*)-4-chloro-2-fluoro-14-oxo-8,8*a*,9,10,11,12-hexahydro-7*H*,14*H*-pyrazi no[1',2':5,6][1,5]diazocino[3,2,1-*hi*]indazol -3-yl)-7-fluorobenzo[*b*]thiophene-3-carboni trile bis(2,2,2-trifluoroacetate) |
| | 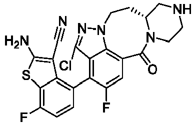 | 2-Amino-4-((*R*)-4-chloro-2-fluoro-14-oxo-8,8*a*,9,10,11,12-hexahydro-7*H*,14*H*-pyrazi no[1',2':5,6][1,5]diazocino[3,2,1-*hi*]indazol -3-yl)-7-fluorobenzo[*b*]thiophene-3-carboni trile |
| | 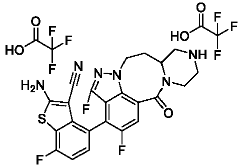 | 2-Amino-4-(2,4-difluoro-14-oxo-8,8a,9,10, 11,12-hexahydro-7*H*,14*H*-pyrazino[1',2':5, 6][1,5]diazocino[3,2,1-*hi*]indazol-3-yl)-7-fl uorobenzo[*b*]thiophene-3-carbonitrile bis(2,2,2-trifluoroacetate) |
| | 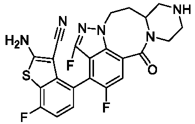 | 2-Amino-4-(2,4-difluoro-14-oxo-8,8*a*,9,10, 11,12-hexahydro-7*H*,14*H*-pyrazino[1',2'-5, 6][1,5]diazocino[3,2,1-*hi*]indazol-3-yl)-7-fl uorobenzo[*b*]thiophene-3-carbonitrile |
| **8e** | 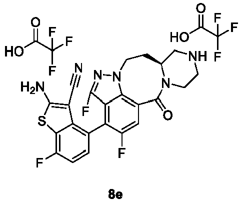 | 2-Amino-4-((*S*)-2,4-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile bis(2,2,2-trifluoroacetate) **8e** |
| | 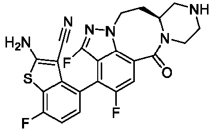 | 2-Amino-4-((*S*)-2,4-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile |
| | 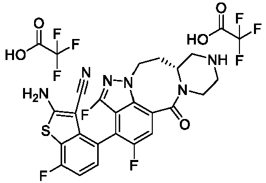 | 2-Amino-4-((*R*)-2,4-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile bis(2,2,2-trifluoroacetate) |
| | 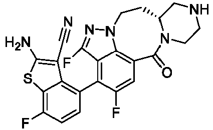 | 2-Amino-4-((*R*)-2,4-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile |
| | 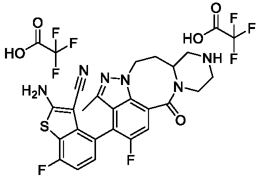 | 2-Amino-7-fluoro-4-(2-fluoro-4-methyl-14 -oxo-8,8*a*,9,10,11,12-hexahydro-7*H*,14*H*-p yrazino[1',2':5,6][1,5]diazocino[3,2,1-*hi*]in dazol-3-yl)benzo[*b*]thiophene-3-carbonitril e bis(2,2,2-trifluoroacetate) |
| | 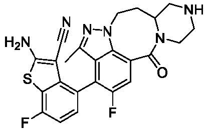 | 2-Amino-7-fluoro-4-(2-fluoro-4-methyl-14 -oxo-8,8*a*,9,10,11,12-hexahydro-7*H*,14*H*-p yrazino[1',2':5,6][1,5]diazocino[3,2,1-*hi*]in dazol-3-yl)benzo[*b*]thiophene-3-carbonitril e |
| **9h** | 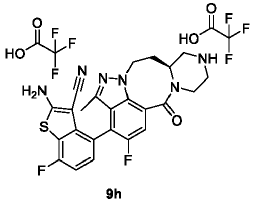 | 2-Amino-7-fluoro-4-((*S*)-2-fluoro-4-methyl -14-oxo-8,8*a*,9,10,11,12-hexahydro-7*H*,14 *H*-pyrazino[1',2':5,6][1,5]diazocino[3,2,1-*h i*]indazol-3-yl)benzo[*b*]thiophene-3-carboni trile bis(2,2,2-trifluoroacetate) **9h** |
| | 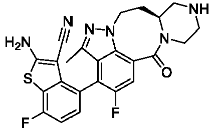 | 2-Amino-7-fluoro-4-((*S*)-2-fluoro-4-methyl -14-oxo-8,8*a*,9,10,11,12-hexahydro-7*H*,14 *H*-pyrazino[1',2':5,6][1,5]diazocino[3,2,1-*h i*]indazol-3-yl)benzo[*b*]thiophene-3-carboni trile |
| | 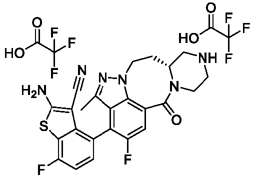 | 2-Amino-7-fluoro-4-((*R*)-2-fluoro-4-methyl -14-oxo-8,8*a*,9,10,11,12-hexahydro-7*H*,14 *H*-pyrazino[1',2':5,6][1,5]diazocino[3,2,1-*h i*]indazol-3-yl)benzo[*b*]thiophene-3-carboni trilebis(2,2,2-trifluoroacetate) |
| | 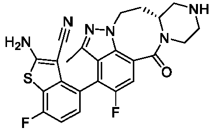 | 2-Amino-7-fluoro-4-((*R*)-2-fluoro-4-methyl -14-oxo-8,8*a*,9,10,11,12-hexahydro-7*H*,14 *H*-pyrazino[1',2':5,6][1,5]diazocino[3,2,1-*h i*]indazol-3-yl)benzo[*b*]thiophene-3-carboni trile |
| | 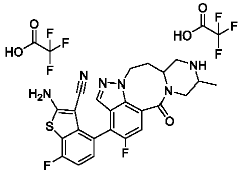 | 2-Amino-7-fluoro-4-(2-fluoro-11-methyl-1 4-oxo-8,8*a*,9,10,11,12-hexahydro-7*H*,14*H-*pyrazino[1',2':5,6][1,5]diazocino[3,2,1-*hi*]i ndazol-3-yl)-benzo[*b*]thiophene-3-carbonitr ile bis(2,2,2-trifluoroacetate) |
| | 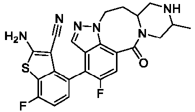 | 2-Amino-7-fluoro-4-(2-fluoro-11-methyl-1 4-oxo-8,8a,9,10,11,12-hexahydro-7*H*,14*H-*pyrazino[1',2':5,6][1,5]diazocino[3,2,1-*hi*]i ndazol-3-yl)-benzo[*b*]thiophene-3-carbonitr ile |
| | 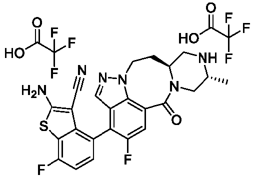 | 2-Amino-7-fluoro-4-((8*aS*,11*R*)-2-fluoro-1 1-methyl-14-oxo-8,8*a*,9,10,11,12-hexahydr o-7*H*,14*H*-pyrazino[1',2':5,6][1,5]diazocino [3,2,1-*hi*]indazol-3-yl)-benzo[*b*]thiophene-3-carbonitrile bis(2,2,2-trifluoroacetate) |
| | 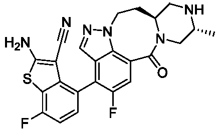 | 2-Amino-7-fluoro-4-((8*aS*,11*R*)-2-fluoro-1 1-methyl-14-oxo-8,8*a*,9,10,11,12-hexahydr o-7*H*,14*H*-pyrazino[1',2':5,6][1,5]diazocino [3,2,1-*hi*]indazol-3-yl)-benzo[*b*]thiophene-3-carbonitrile |
| | 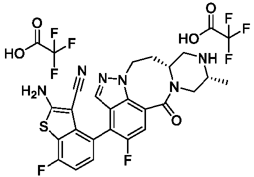 | 2-Amino-7-fluoro-4-((8*aR*,11*R*)-2-fluoro-1 1-methyl-14-oxo-8,8*a*,9,10,11,12-hexahydr o-7*H*,14*H*-pyrazino[1',2':5,6][1,5]diazocino [3,2,1-*hi*]indazol-3-yl)-benzo[*b*]thiophene-3-carbonitrile bis(2,2,2-trifluoroacetate) |
| | 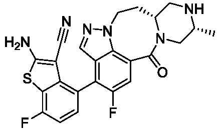 | 2-Amino-7-fluoro-4-((8*aR*,11*R*)-2-fluoro-1 1-methyl-14-oxo-8,8*a*,9,10,11,12-hexahydr o-7*H*,14*H*-pyrazino[1',2':5,6][1,5]diazocino [3,2,1-*hi*]indazol-3-yl)-benzo[*b*]thiophene-3-carbonitrile |
| | 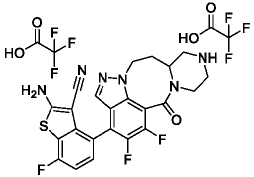 | 2-Amino-4-(1,2-difluoro-14-oxo-8,8*a*,9,10, 11,12-hexahydro-7*H*,14*H*-pyrazino[1',2':5, 6][1,5]diazocino[3,2,1-*hi*]indazol-3-yl)-7-fl uorobenzo[*b*]thiophene-3-carbonitrile bis(2,2,2-trifluoroacetate) |
| | 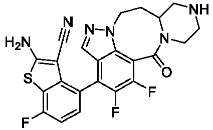 | 2-Amino-4-(1,2-difluoro-14-oxo-8,8*a*,9,10, 11,12-hexahydro-7*H*,14*H*-pyrazino[1',2':5, 6][1,5]diazocino[3,2,1-*hi*]indazol-3-yl)-7-fl uorobenzo[*b*]thiophene-3-carbonitrile |
| | 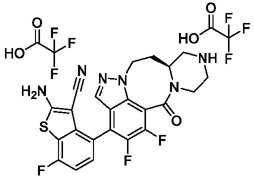 | 2-Amino-4-((*S*)-1,2-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile bis(2,2,2-trifluoroacetate) |
| | 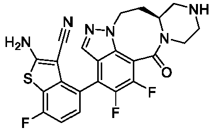 | 2-Amino-4-((*S*)-1,2-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile |
| | 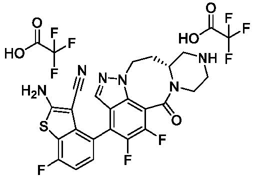 | 2-Amino-4-((*R*)-1,2-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile bis(2,2,2-trifluoroacetate) |
| | 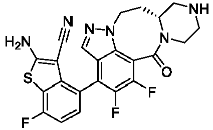 | 2-Amino-4-((*R*)-1,2-difluoro-14-oxo-8,8*a*,9 ,10,11,12-hexahydro-7*H*,14*H*-pyrazino[1',2' :5,6][1,5]diazocino[3,2,1-*hi*]indazol-3-yl)-7 -fluorobenzo[*b*]thiophene-3-carbonitrile |
| | 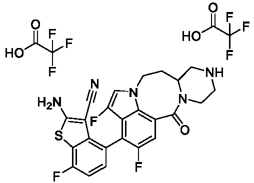 | 2-Amino-4-(2,4-difluoro-14-oxo-8,8*a*,9,10, 11,12-hexahydro-7*H*,14*H*-pyrazino[1',2':5, 6][1,5]diazocino[3,2,1-*hi*]indol-3-yl)-7-fluo robenzo[*b*]thiophene-3-carbonitrile bis(2,2,2-trifluoroacetate) |
| | 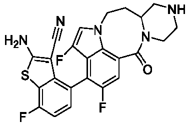 | 2-Amino-4-(2,4-difluoro-14-oxo-8,8*a*,9,10, 11,12-hexahydro-7*H*,14*H*-pyrazino[1',2'-5, 6][1,5]diazocino[3,2,1-*hi*]indol-3-yl)-7-fluo robenzo[*b*]thiophene-3-carbonitrile |
| | 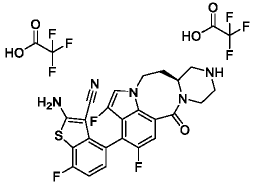 | 2-Amino-4-((*S*)-2,4-difluoro-14-oxo-8,8*a*,9 ,10,11,12-hexahydro-7*H*,14*H*-pyrazino[1',2' :5,6][1,5]diazocino[3,2,1-*hi*]indol-3-yl)-7-fl uorobenzo[*b*]thiophene-3-carbonitrile bis(2,2,2-trifluoroacetate) |
| | 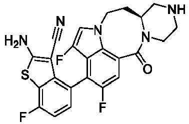 | 2-Amino-4-((*S*)-2,4-difluoro-14-oxo-8,8*a*,9 ,10,11,12-hexahydro-7*H*,14*H*-pyrazino[1',2' :5,6][1,5]diazocino[3,2,1-*hi*]indol-3-yl)-7-fl uorobenzo[*b*]thiophene-3-carbonitrile |
| | 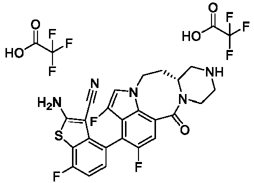 | 2-Amino-4-((*R*)-2,4-difluoro-14-oxo-8,8*a*,9 ,10,11,12-hexahydro-7*H*,14*H*-pyrazino[1',2' :5,6][1,5]diazocino[3,2,1-*hi*]indol-3-yl)-7-fl uorobenzo[*b*]thiophene-3-carbonitrile bis(2,2,2-trifluoroacetate) |
| | 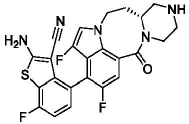 | 2-Amino-4-((*R*)-2,4-difluoro-14-oxo-8,8*a*,9 ,10,11,12-hexahydro-7*H*,14*H*-pyrazino[1',2' :5,6][1,5]diazocino[3,2,1-*hi*]indol-3-yl)-7-fl uorobenzo[*b*]thiophene-3-carbonitrile |
| | 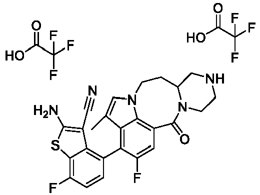 | 2-Amino-7-fluoro-4-(2-fluoro-4-methyl-14 -oxo-8,8*a*,9,10,11,12-hexahydro-7*H*,14*H*-p yrazino[1',2':5,6][1,5]diazocino[3,2,1-*hi*]in dol-3-yl)benzo[*b*]thiophene-3-carbonitrile bis(2,2,2-trifluoroacetate) |
| | 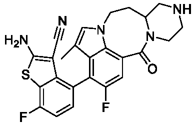 | 2-Amino-7-fluoro-4-(2-fluoro-4-methyl-14 -oxo-8,8*a*,9,10,11,12-hexahydro-7*H*,14*H*-p yrazino[1',2':5,6][1,5]diazocino[3,2,1-*hi*]in dol-3-yl)benzo[*b*]thiophene-3-carbonitrile |
| | 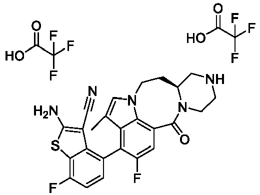 | 2-Amino-7-fluoro-4-((*S*)-2-fluoro-4-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le bis(2,2,2-trifluoroacetate) |
| | 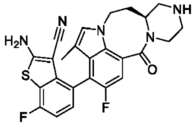 | 2-Amino-7-fluoro-4-((*S*)-2-fluoro-4-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le |
| | 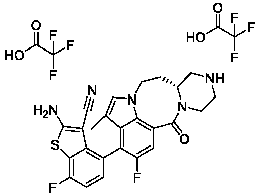 | 2-Amino-7-fluoro-4-((*R*)-2-fluoro-4-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le bis(2,2,2-trifluoroacetate) |
| | 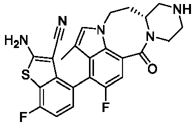 | 2-Amino-7-fluoro-4-((*R*)-2-fluoro-4-methyl -14-oxo-8,8*a*,9,10,11,12-hexahydro-7*H*,14 *H*-pyrazino[1',2':5,6][1,5]diazocino[3,2,1-*h i*]indol-3-yl)benzo[*b*]thiophene-3-carbonitri le |
| | 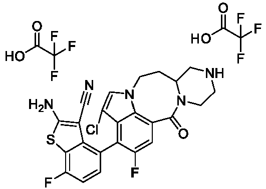 | 2-Amino-4-(4-chloro-2-fluoro-14-oxo-8,8*a* ,9,10,11,12-hexahydro-7*H*,14*H*-pyrazino[1' ,2':5,6][1,5]diazocino[3,2,1-*hi*]indol-3-yl)-7 -fluorobenzo[*b*]thiophene-3-carbonitrile bis(2,2,2-trifluoroacetate) |
| | 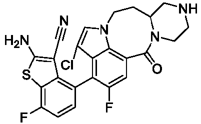 | 2-Amino-4-(4-chloro-2-fluoro-14-oxo-8,8*a* ,9,10,11,12-hexahydro-7*H*,14*H*-pyrazino[1' ,2':5,6][1,5]diazocino[3,2,1-*hi*]indol-3-yl)-7 -fluorobenzo[*b*]thiophene-3-carbonitrile |
| | 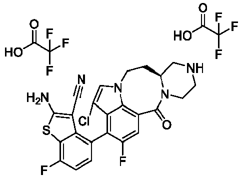 | 2-Amino-4-((*S*)-4-chloro-2-fluoro-14-oxo-8,8*a*,9,10,11,12-hexahydro-7*H*,14*H*-pyrazi no[1',2':5,6][1,5]diazocino[3,2,1-*hi*]indol-3 -yl)-7-fluorobenzo[*b*]thiophene-3-carbonitr ile bis(2,2,2-trifluoroacetate) |
| | 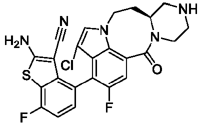 | 2-Amino-4-((*S*)-4-chloro-2-fluoro-14-oxo-8,8*a*,9,10,11,12-hexahydro-7*H*,14*H*-pyrazi no[1',2':5,6][1,5]diazocino[3,2,1-*hi*]indol-3 -yl)-7-fluorobenzo[*b*]thiophene-3-carbonitr ile |
| | 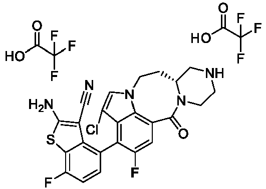 | 2-Amino-4-((*R*)-4-chloro-2-fluoro-14-oxo-8,8*a*,9,10,11,12-hexahydro-7*H*,14*H*-pyrazi no[1',2':5,6][1,5]diazocino[3,2,1-*hi*]indol-3 -yl)-7-fluorobenzo[*b*]thiophene-3-carbonitr ile bis(2,2,2-trifluoroacetate) |
| | 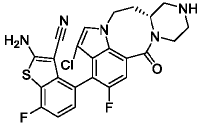 | 2-Amino-4-((*R*)-4-chloro-2-fluoro-14-oxo-8,8*a*,9,10,11,12-hexahydro-7*H*,14*H*-pyrazi no[1',2':5,6][1,5]diazocino[3,2,1-*hi*]indol-3 -yl)-7-fluorobenzo[*b*]thiophene-3-carbonitr ile |
| | 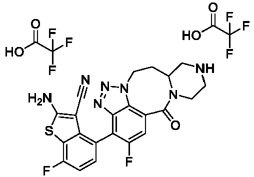 | 2-Amino-7-fluoro-4-(2-fluoro-12-oxo-7,7*a*, 8,9,10,11-hexahydro-6*H*,12*H*-4,5,5*a*,9,11*a-*pentaazabenzo[5,6]cycloocta[1,2,3-*cd*]inde n-3-yl)benzo[*b*]thiophene-3-carbonitrile bis(2,2,2-trifluoroacetate) |
| | 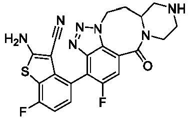 | 2-Amino-7-fluoro-4-(2-fluoro-12-oxo-7,7*a*, 8,9,10,11-hexahydro-6*H*,12*H*-4,5,5*a*,9,11*a-*pentaazabenzo[5,6]cycloocta[1,2,3-*cd*]inde n-3-yl)benzo[*b*]thiophene-3-carbonitrile |
| | 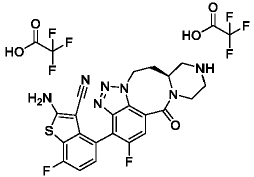 | 2-Amino-7-fluoro-4-((*S*)-2-fluoro-12-oxo-7 ,7*a*,8,9,10,11-hexahydro-6*H*,12*H*-4,5,5*a*,9, 11*a*-pentaazabenzo[5,6]cycloocta[1,2,3-*cd*] inden-3-yl)benzo[*b*]thiophene-3-carbonitril e bis(2,2,2-trifluoroacetate) |
| | 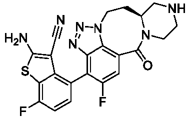 | 2-Amino-7-fluoro-4-((*S*)-2-fluoro-12-oxo-7 ,7*a*,8,9,10,11-hexahydro-6*H*,12*H*-4,5,5*a*,9, 11*a*-pentaazabenzo[5,6]cycloocta[1,2,3-*cd*] inden-3-yl)benzo[*b*]thiophene-3-carbonitril e |
| | 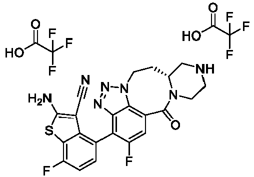 | 2-Amino-7-fluoro-4-((*R*)-2-fluoro-12-oxo-7,7*a*,8,9,10,11-hexahydro-6*H*,12*H*-4,5,5*a*,9 ,11*a*-pentaazabenzo[5,6]cycloocta[1,2,3-*cd* ]inden-3-yl)benzo[*b*]thiophene-3-carbonitri le bis(2,2,2-trifluoroacetate) |
| | 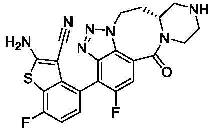 | 2-Amino-7-fluoro-4-((*R*)-2-fluoro-12-oxo-7,7*a*,8,9,10,11-hexahydro-6*H*,12*H*-4,5,5*a*,9 ,11*a*-pentaazabenzo[5,6]cycloocta[1,2,3-*cd* ]inden-3-yl)benzo[*b*]thiophene-3-carbonitri le |
| | 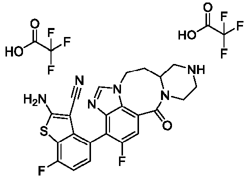 | 2-Amino-7-4-(2-fluoro-12-oxo-7,7*a*,8,9,10, 11-hexahydro-6*H*,12*H*-4,5*a*,9,11*a*-tetraazab enzo[5,6]cycloocta[1,2,3-*cd*]inden-3-yl)-be nzo[*b*]thiophene-3-carbonitrile bis(2,2,2-trifluoroacetate) |
| | 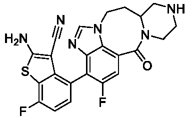 | 2-Amino-7-4-(2-fluoro-12-oxo-7,7*a*,8,9,10, 11-hexahydro-6*H*,12*H*-4,5*a*,9,11*a*-tetraazab enzo[5,6]cycloocta[1,2,3-*cd*]inden-3-yl)-be nzo[*b*]thiophene-3-carbonitrile |
| | 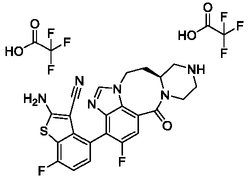 | 2-Amino-7-4-((*S*)-2-fluoro-12-oxo-7,7*a*,8,9 ,10,11-hexahydro-6*H*,12*H*-4,5*a*,9,11*a*-tetra azabenzo[5,6]cycloocta[1,2,3-*cd*]inden-3-yl )-benzo[*b*]thiophene-3-carbonitrile bis(2,2,2-trifluoroacetate) |
| | 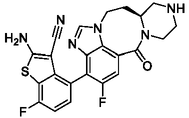 | 2-Amino-7-4-((*S*)-2-fluoro-12-oxo-7,7*a*,8,9 ,10,11-hexahydro-6*H*,12*H*-4,5*a*,9,11*a*-tetra azabenzo[5,6]cycloocta[1,2,3-*cd*]inden-3-yl )-benzo[*b*]thiophene-3-carbonitrile |
| | 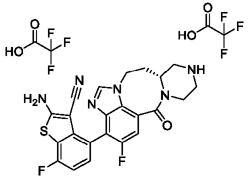 | 2-Amino-7-4-((*R*)-2-fluoro-12-oxo-7,7*a*,8, 9,10,11-hexahydro-6*H*,12H-4,5*a*,9,11*a*-tetr aazabenzo[5,6]cycloocta[1,2,3-*cd*]inden-3-yl)-benzo[*b*]thiophene-3-carbonitrile bis(2,2,2-trifluoroacetate) |
| | 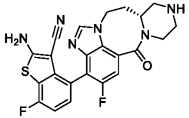 | 2-Amino-7-4-((*R*)-2-fluoro-12-oxo-7,7*a*,8, 9,10,11-hexahydro-6*H*,12*H*-4,5*a*,9,11*a*-tetr aazabenzo[5,6]cycloocta[1,2,3-*cd*]inden-3-yl)-benzo[*b*]thiophene-3-carbonitrile |

The compound represented by general formula (IMa), general formula (Ia), general formula (IIa), general formula (IIIa), general formula (III-1a), general formula (III-2a), general formula (III-1-Aa), general formula (III-1-Ba), general formula (III-2-Aa), or general formula (III-2-Ba) or the salt thereof is disclosed herein, wherein the salt is preferably a bis(2,2,2-trifluoroacetate).

Another aspect of the present disclosure relates to a compound represented by general formula (IMaa) or a salt thereof: 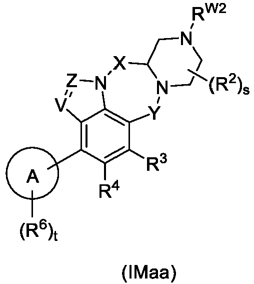 wherein:
R^{W2} is an amino protecting group; preferably, R^{W2} is tert-butyloxycarbonyl;
ring A, V, X, Y, Z, R², R³, R⁴, R⁶, s, and t are as defined in general formula (IMa).

Another aspect of the present disclosure relates to a compound represented by general formula (IMaa) or a salt thereof: 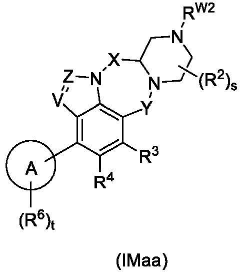 wherein: 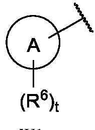
is 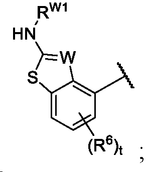
R^{W1} and R^{W2} are identical or different and are each independently an amino protecting group; preferably, R^{W1} and R^{W2} are both tert-butyloxycarbonyl;
t is 0, 1, 2, or 3;
V, X, Y, Z, R², R³, R⁴, R⁶, and s are as defined in general formula (IMa).

Another aspect of the present disclosure relates to a compound represented by general formula (Iaa) or a salt thereof: 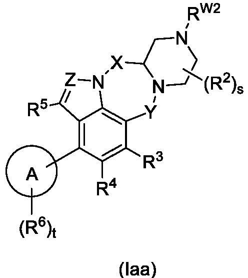 wherein:
R^{W2} is an amino protecting group; preferably, R^{W2} is tert-butyloxycarbonyl;
ring A, X, Y, Z, R², R³, R⁴, R⁵, R⁶, s, and t are as defined in general formula (Ia).

Another aspect of the present disclosure relates to a compound represented by general formula (Iaa) or a salt thereof: 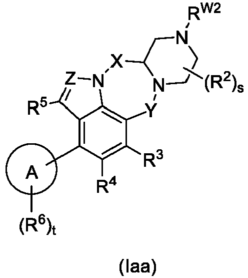 wherein: 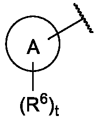
is 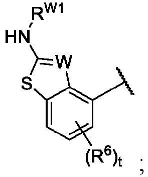
R^{W1} and R^{W2} are identical or different and are each independently an amino protecting group; preferably, R^{W1} and R^{W2} are both tert-butyloxycarbonyl;
t is 0, 1, 2, or 3;
X, Y, Z, R², R³, R⁴, R⁵, R⁶, and s are as defined in general formula (Ia).

Another aspect of the present disclosure relates to a compound represented by general formula (IIaa) or a salt thereof: 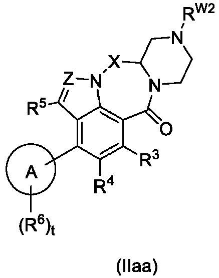 wherein:
R^{W2} is an amino protecting group; preferably, R^{W2} is tert-butyloxycarbonyl;
ring A, X, Z, R³, R⁴, R⁵, R⁶, and t are as defined in general formula (IIa).

Another aspect of the present disclosure relates to a compound represented by general formula (IIaa) or a salt thereof: 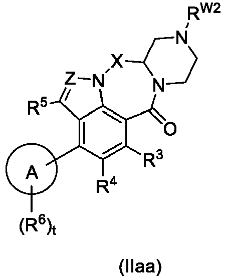 wherein: 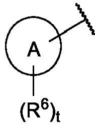
is 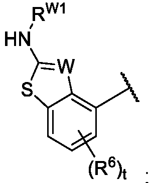
R^{W1} and R^{W2} are identical or different and are each independently an amino protecting group; preferably, R^{W1} and R^{W2} are both tert-butyloxycarbonyl;
t is 0, 1, 2, or 3;
X, Z, R³, R⁴, R⁵, and R⁶ are as defined in general formula (IIa).

Another aspect of the present disclosure relates to a compound represented by general formula (IIIaa) or a salt thereof: 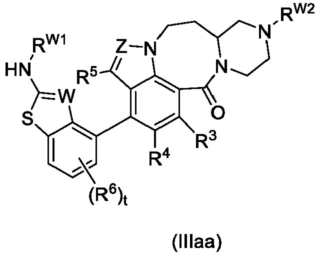 wherein:
R^{W1} and R^{W2} are identical or different and are each independently an amino protecting group; preferably, R^{W1} and R^{W2} are both tert-butyloxycarbonyl;
W, Z, R³, R⁴, R⁵, R⁶, and t are as defined in general formula (IIIa).

Another aspect of the present disclosure relates to a compound represented by general formula (III-1aa) or a salt thereof: 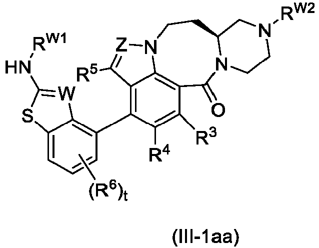 wherein:
R^{W1} and R^{W2} are identical or different and are each independently an amino protecting group; preferably, R^{W1} and R^{W2} are both tert-butyloxycarbonyl;
W, Z, R³, R⁴, R⁵, R⁶, and t are as defined in general formula (III-1a).

Another aspect of the present disclosure relates to a compound represented by general formula (III-2aa) or a salt thereof: 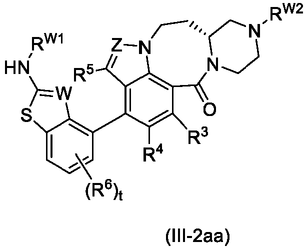 wherein:
R^{W1} and R^{W2} are identical or different and are each independently an amino protecting group; preferably, R^{W1} and R^{W2} are both tert-butyloxycarbonyl;
W, Z, R³, R⁴, R⁵, R⁶, and t are as defined in general formula (III-2a).

**Table C. Typical intermediate compounds of the present disclosure include, but are not limited to:**

| Compound No. | Compound structure | Name |
|---|---|---|
| | 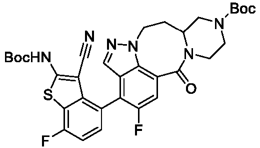 | tert-Butyl 3-(2-((tert-butoxycarbonyl)amino)-3-cya no-7-fluorobenzo[*b*]thiophen-4-yl)-2-flu oro-14-oxo-7,8,8*a*,9,11,12-hexahydro-10 *H*,14*H*-pyrazino[1',2':5,6][1,5]diazocino[ 3,2,1-*hi*]indazole-10-carboxylate |
| **1i** | 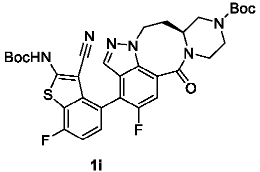 | tert-Butyl (8*aS*)-3-(2-((tert-butoxycarbonyl)amino)-3-cyano-7-fluorobenzo[b]thiophen-4-yl)-2-fluoro-14-oxo-7,8,8*a*,9,11,12-hexahydr o-10*H*,14*H*-pyrazino[1',2':5,6][1,5]diazo cino[3,2,1-*hi*]indazole-10-carboxylate **1i** |
| | 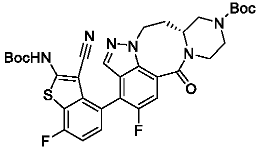 | tert-Butyl (8*aR*)-3-(2-((tert-butoxycarbonyl)amino) -3-cyano-7-fluorobenzo[*b*]thiophen-4-yl) -2-fluoro-14-oxo-7,8,8*a*,9,11,12-hexahyd ro-10*H*,14*H*-pyrazino[1',2':5,6][1,5]diazo cino[3,2,1-*hi*]indazole-10-carboxylate |
| | 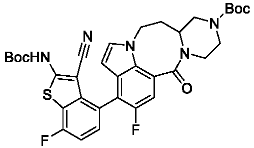 | tert-Butyl 3-(2-((tert-butoxycarbonyl)amino)-3-cya no-7-fluorobenzo[*b*]thiophen-4-yl)-2-flu oro-14-oxo-7,8,8*a*,9,11,12-hexahydro-10 *H*,14*H*-pyrazino[1',2':5,6][1,5]diazocino[ 3,2,1-*hi*]indole-10-carboxylate |
| 2e | 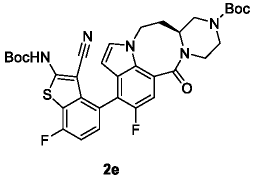 | tert-Butyl (8*aS*)-3-(2-((tert-butoxycarbonyl)amino)-3-cyano-7-fluorobenzo[*b*]thiophen-4-yl)-2-fluoro-14-oxo-7,8,8*a*,9,11,12-hexahydr o-10*H*,14*H*-pyrazino[1',2':5,6][1,5]diazo cino[3,2,1-*hi*]indole-10-carboxylate **2e** |
| | 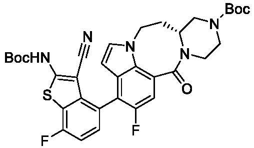 | tert-Butyl (8*aR*)-3-(2-((tert-butoxycarbonyl)amino) -3-cyano-7-fluorobenzo[*b*]thiophen-4-yl) -2-fluoro-14-oxo-7,8,8*a*,9,11,12-hexahyd ro-10*H*,14*H*-pyrazino[1',2':5,6][1,5]diazo cino[3,2,1-*hi*]indole-10-carboxylate |
| | 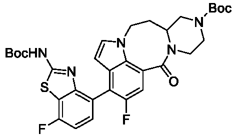 | tert-Butyl 3-(2-((tert-butoxycarbonyl)amino)-7-fluo robenzo[*d*]thiazol-4-yl)-2-fluoro-14-oxo-7,8,8*a*,9,11,12-hexahydro-10*H*,14*H*-pyra zino[1',2':5,6][1,5]diazocino[3,2,1-*hi*]ind ole-10-carboxylate |
| **3b** | 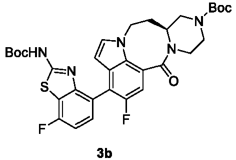 | tert-Butyl (8*aS*)-3-(2-((tert-butoxycarbonyl)amino)-7-fluorobenzo[*d*]thiazol-4-yl)-2-fluoro-1 4-oxo-7,8,8*a*,9,11,12-hexahydro-10*H*,14 *H*-pyrazino[1',2':5,6][1,5]diazocino[3,2,1 -*hi*]indole-10-carboxylate **3b** |
| | 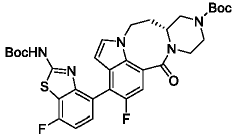 | tert-Butyl (8*aR*)-3-(2-((tert-butoxycarbonyl)amino) -7-fluorobenzo[*d*]thiazol-4-yl)-2-fluoro-1 4-oxo-7,8,8ia,9,11,12-hexahydro-10*H*,14 *H*-pyrazino[1',2':5,6][1,5]diazocino[3,2,1 -*hi*]indole-10-carboxylate |
| | 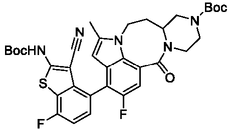 | tert-Butyl 3-(2-((tert-butoxycarbonyl)amino)-3-cya no-7-fluorobenzo[*b*]thiophen-4-yl)-2-flu oro-5-methyl-14-oxo-7,8,8*a*,9,11,12-hex ahydro-10*H*,14*H*-pyrazino[1',2':5,6][1,5] diazocino[3,2,1-*hi*]indole-10-carboxylate |
| **4e** | 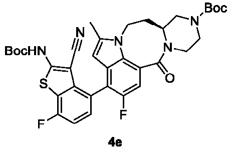 | tert-Butyl (8*aS*)-3-(2-((tert-butoxycarbonyl)amino)-3-cyano-7-fluorobenzo[*b*]thiophen-4-yl)-2-fluoro-5-methyl-14-oxo-7,8,8*a*,9,11,12 -hexahydro-10*H*,14*H*-pyrazino[1',2':5,6][ 1,5]diazocino[3,2,1-*hi*]indole-10-carboxy late **4e** |
| | 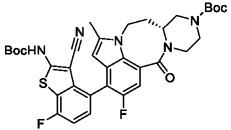 | tert-Butyl (8*aR*)-3-(2-((tert-butoxycarbonyl)amino) -3-cyano-7-fluorobenzo[*b*]thiophen-4-yl) -2-fluoro-5-methyl-14-oxo-7,8,8*a*,9,11,1 2-hexahydro-10*H*,14*H*-pyrazino[1',2':5,6 ][1,5]diazocino[3,2,1-*hi*]indole-10-carbo xylate |
| | 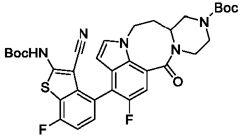 | tert-Butyl 3-(2-((tert-butoxycarbonyl)amino)-3-cya no-7-fluorobenzo[*b*]thiophen-4-yl)-2-flu oro-14-oxo-7,8,8*a*,9,11,12-hexahydro-10 *H*,14*H*-pyrazino[1',2':5,6][1,5]diazocino[ 3,2,1-*hi*]indole-10-carboxylate |
| | 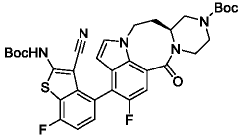 | tert-Butyl (8*aS*)-3-(2-((tert-butoxycarbonyl)amino)-3-cyano-7-fluorobenzo[*b*]thiophen-4-yl)-2-fluoro-14-oxo-7,8,8*a*,9,11,12-hexahydr o-10*H*,14*H*-pyrazino[1',2':5,6][1,5]diazo cino[3,2,1-*hi*]indole-10-carboxylate |
| | 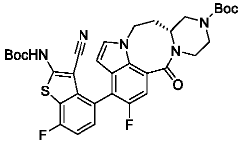 | tert-Butyl (8*aR*)-3-(2-((tert-butoxycarbonyl)amino) -3-cyano-7-fluorobenzo[*b*]thiophen-4-yl) -2-fluoro-14-oxo-7,8,8a,9,11,12-hexahyd ro-10*H*,14*H*-pyrazino[1',2':5,6][1,5]diazo cino[3,2,1-*hi*]indole-10-carboxylate |
| | 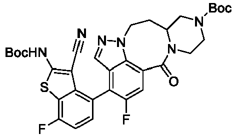 | tert-Butyl 3-(2-((tert-butoxycarbonyl)amino)-3-cya no-7-fluorobenzo[*b*]thiophen-4-yl)-2-flu oro-14-oxo-7,8,8*a*,9,11,12-hexahydro-10 *H*,14*H*-pyrazino[1',2':5,6][1,5]diazocino[ 3,2,1-*hi*]indazole-10-carboxylate |
| | 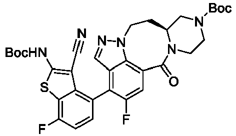 | tert-Butyl (8*aS*)-3-(2-((tert-butoxycarbonyl)amino)-3-cyano-7-fluorobenzo[*b*]thiophen-4-yl)-2-fluoro-14-oxo-7,8,8*a*,9,11,12-hexahydr o-10*H*,14*H*-pyrazino[1',2':5,6][1,5]diazo cino[3,2,1-*hi*]indazole-10-carboxylate |
| | 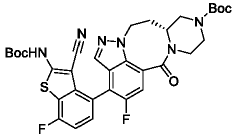 | tert-Butyl (8*aR*)-3-(2-((tert-butoxycarbonyl)amino) -3-cyano-7-fluorobenzo[*b*]thiophen-4-yl) -2-fluoro-14-oxo-7,8,8a,9,11,12-hexahyd ro-10*H*,14*H*-pyrazino[1',2':5,6][1,5]diazo cino[3,2,1-*hi*]indazole-10-carboxylate |
| | 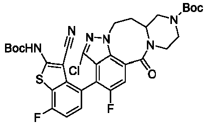 | tert-Butyl 3-(2-((tert-butoxycarbonyl)amino)-3-cya no-7-fluorobenzo[*b*]thiophen-4-yl)-4-chl oro-2-fluoro-14-oxo-7,8,8*a*,9,11,12-hexahydro-10*H*,14*H*-pyrazino[1',2':5,6][1,5]d iazocino[3,2,1-*hi*]indazole-10-carboxylat e |
| | 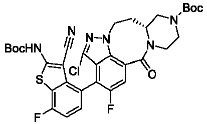 | tert-Butyl (8*aR*)-3-(2-((tert-butoxycarbonyl)amino) -3-cyano-7-fluorobenzo[*b*]thiophen-4-yl) -4-chloro-2-fluoro-14-oxo-7,8,8*a*,9,11,12 -hexahydro-10*H*,14*H*-pyrazino[1',2':5,6][ 1,5]diazocino[3,2,1-*hi*]indazole-10-carbo xylate |
| **7b** | 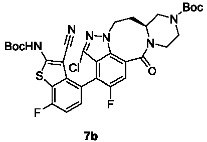 | tert-Butyl (8*aS*)-3-(2-((tert-butoxycarbonyl)amino)-3-cyano-7-fluorobenzo[*b*]thiophen-4-yl)-4-chloro-2-fluoro-14-oxo-7,8,8*a*,9,11,12-hexahydro-10*H*,14*H*-pyrazino[1',2':5,6][ 1,5]diazocino[3,2,1-*hi*]indazole-10-carbo xylate **7b** |
| | 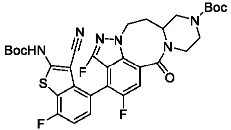 | tert-Butyl 3-(2-((tert-butoxycarbonyl)amino)-3-cya no-7-fluorobenzo[*b*]thiophen-4-yl)-2,4-di fluoro-14-oxo-7,8,8*a*,9,11,12-hexahydro-10*H*,14*H*-pyrazino[1,2:5,6][1,5]diazocin o[3,2,1-*hi*]indazole-10-carboxylate |
| | 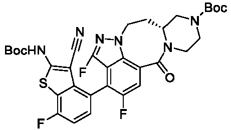 | tert-Butyl (8*aR*)-3-(2-((tert-butoxycarbonyl)amino) -3-cyano-7-fluorobenzo[*b*]thiophen-4-yl) -2,4-difluoro-14-oxo-7,8,8*a*,9,11,12-hexa hydro-10*H*,14*H*-pyrazino[1,2:5,6][1,5]di azocino[3,2,1-*hi*]indazole-10-carboxylate |
| **8d** | 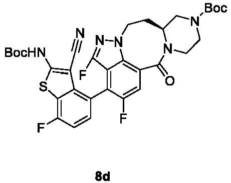 | tert-Butyl (8*aS*)-3-(2-((tert-butoxycarbonyl)amino)-3-cyano-7-fluorobenzo[*b*]thiophen-4-yl)-2,4-difluoro-14-oxo-7,8,8*a*,9,11,12-hexa hydro-10*H*,14*H*-pyrazino[1,2:5,6][1,5]di azocino[3,2,1-*hi*]indazole-10-carboxylate **8d** |
| | 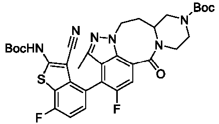 | tert-Butyl 3-(2-((tert-butoxycarbonyl)amino)-3-cya no-7-fluorobenzo[*b*]thiophen-4-yl)-2-flu oro-4-methyl-14-oxo-7,8,8*a*,9,11,12-hexahydro-10*H*,14*H*-pyrazino[1',2':5,6][1,5] diazocino[3,2,1-*hi*]indazole-10-carboxyla te |
| | 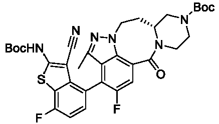 | tert-Butyl (8*aR*)-3-(2-((tert-butoxycarbonyl)amino) -3-cyano-7-fluorobenzo[*b*]thiophen-4-yl) -2-fluoro-4-methyl-14-oxo-7,8,8*a*,9,11,1 2-hexahydro-10*H*,14*H*-pyrazino[1',2':5,6 ][1,5]diazocino[3,2,1-*hi*]indazole-10-car boxylate |
| **9g** | 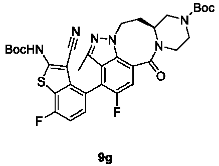 | tert-Butyl (8*aS*)-3-(2-((tert-butoxycarbonyl)amino)-3-cyano-7-fluorobenzo[*b*]thiophen-4-yl)-2-fluoro-4-methyl-14-oxo-7,8,8*a*,9,11,12 -hexahydro-10*H*,14*H*-pyrazino[1',2':5,6][ 1,5]diazocino[3,2,1-*hi*]indazole-10-carbo xylate **9g** |
| | 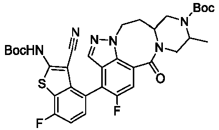 | tert-Butyl 3-(2-((tert-butoxycarbonyl)amino)-3-cya no-7-fluorobenzo[*b*]thiophen-4-yl)-2-flu oro-11-methyl-14-oxo-7,8,8*a*,9,11,12-he xahydro-10*H*,14*H*-pyrazino[1',2':5,6][1,5 ]diazocino[3,2,1-*hi*]indazole-10-carboxyl ate |
| | 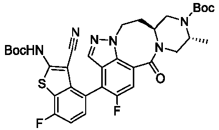 | tert-Butyl (8*aS*,11*R*)-3-(2-((tert-butoxycarbonyl)am ino)-3-cyano-7-fluorobenzo[*b*]thiophen-4 -yl)-2-fluoro-11-methyl-14-oxo-7,8,8*a*,9, 11,12-hexahydro-10*H*,14*H*-pyrazino[1',2' :5,6][1,5]diazocino[3,2,1-*hi*]indazole-10-carboxylate |
| | 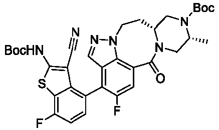 | tert-Butyl (8*aR*,11*R*)-3-(2-((tert-butoxycarbonyl)am ino)-3-cyano-7-fluorobenzo[*b*]thiophen-4 -yl)-2-fluoro-11-methyl-14-oxo-7,8,8*a*,9, 11,12-hexahydro-10*H*,14*H*-pyrazino[1',2' :5,6][1,5]diazocino[3,2,1-*hi*]indazole-10-carboxylate |
| | 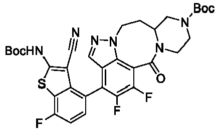 | tert-Butyl 3-(2-((tert-butoxycarbonyl)amino)-3-cya no-7-fluorobenzo[*b*]thiophen-4-yl)-1,2-di fluoro-14-oxo-7,8,8*a*,9,11,12-hexahydro-10*H*,14*H*-pyrazino[1',2':5,6][1,5]diazocin o[3,2,1-*hi*]indazole-10-carboxylate |
| | 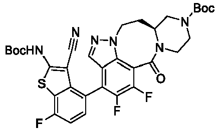 | tert-Butyl (8*aS*)-3-(2-((tert-butoxycarbonyl)amino)-3-cyano-7-fluorobenzo[*b*]thiophen-4-yl)-1,2-difluoro-14-oxo-7,8,8*a*,9,11,12-hexa hydro-10*H*,14*H*-pyrazino[1',2':5,6][1,5]d iazocino[3,2,1-*hi*]indazole-10-carboxylat e |
| | 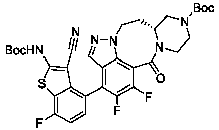 | tert-Butyl (8*aR*)-3-(2-((tert-butoxycarbonyl)amino) -3-cyano-7-fluorobenzo[*b*]thiophen-4-yl) -1,2-difluoro-14-oxo-7,8,8*a*,9,11,12-hexa hydro-10*H*,14*H*-pyrazino[1',2':5,6][1,5]d iazocino[3,2,1-*hi*]indazole-10-carboxylat e |
| | 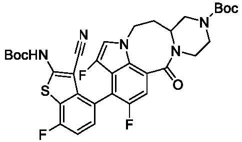 | tert-Butyl 3-(2-((tert-butoxycarbonyl)amino)-3-cya no-7-fluorobenzo[*b*]thiophen-4-yl)-2,4-di fluoro-14-oxo-7,8,8*a*,9,11,12-hexahydro-10*H*,14*H*-pyrazino[1',2':5,6][1,5]diazocin o[3,2,1-*hi*]indole-10-carboxylate |
| | 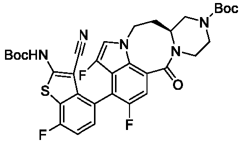 | tert-Butyl (8*aS*)-3-(2-((tert-butoxycarbonyl)amino)-3-cyano-7-fluorobenzo[*b*]thiophen-4-yl)-2,4-difluoro-14-oxo-7,8,8*a*,9,11,12-hexa hydro-10*H*,14*H*-pyrazino[1',2':5,6][1,5]d iazocino[3,2,1-*hi*]indole-10-carboxylate |
| | 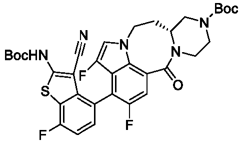 | tert-Butyl (8*aR*)-3-(2-((tert-butoxycarbonyl)amino) -3-cyano-7-fluorobenzo[*b*]thiophen-4-yl) -2,4-difluoro-14-oxo-7,8,8*a*,9,11,12-hexa hydro-10*H*,14*H*-pyrazino[1',2':5,6][1,5]d iazocino[3,2,1-*hi*]indole-10-carboxylate |
| | 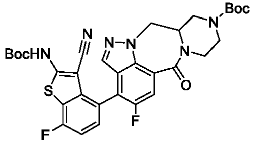 | tert-Butyl 3-(2-((tert-butoxycarbonyl)amino)-3-cya no-7-fluorobenzo[*b*]thiophen-4-yl)-4-flu oro-6-oxo-8,9,11*a*,12-tetrahydro-6*H*-pyra zino[2',1':3,4][1,4]diazepino[6,7,1-*hi*]ind azole-10(11*H*)-carboxylate |
| | 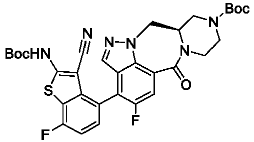 | tert-Butyl (11*aR*)-3-(2-((tert-butoxycarbonyl)amino )-3-cyano-7-fluorobenzo[*b*]thiophen-4-yl )-4-fluoro-6-oxo-8,9,11*a*,12-tetrahydro-6 *H*-pyrazino[2',1':3,4][1,4]diazepino[6,7,1 -hi]indazole-10(11*H*)-carboxylate |
| | 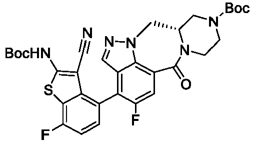 | tert-Butyl (11*aS*)-3-(2-((tert-butoxycarbonyl)amino )-3-cyano-7-fluorobenzo[*b*]thiophen-4-yl )-4-fluoro-6-oxo-8,9,11*a*,12-tetrahydro-6 *H*-pyrazino[2',1':3,4][1,4]diazepino[6,7,1 -hi]indazole-10(11*H*)-carboxylate |
| | 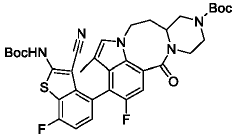 | tert-Butyl 3-(2-((tert-butoxycarbonyl)amino)-3-cya no-7-fluorobenzo[*b*]thiophen-4-yl)-2-flu oro-4-methyl-14-oxo-7,8,8*a*,9,11,12-hex ahydro-10*H*,14*H*-pyrazino[1',2':5,6][1,5] diazocino[3,2,1-*hi*]indole-10-carboxylate |
| | 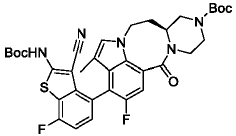 | tert-Butyl (8*aS*)-3-(2-((tert-butoxycarbonyl)amino)-3-cyano-7-fluorobenzo[*b*]thiophen-4-yl)-2-fluoro-4-methyl-14-oxo-7,8,8*a*,9,11,12 -hexahydro-10*H*,14*H*-pyrazino[1',2':5,6][ 1,5]diazocino[3,2,1-*hi*]indole-10-carboxy late |
| | 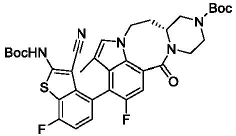 | tert-Butyl (8*aR*)-3-(2-((tert-butoxycarbonyl)amino) -3-cyano-7-fluorobenzo[*b*]thiophen-4-yl) -2-fluoro-4-methyl-14-oxo-7,8,8*a*,9, 11, 1 2-hexahydro-10*H*,14*H*-pyrazino[1',2':5,6 ][1,5]diazocino[3,2,1-*hi*]indole-10-carbo xylate |
| | 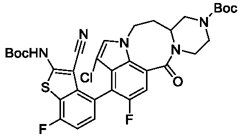 | tert-Butyl 3-(2-((tert-butoxycarbonyl)amino)-3-cya no-7-fluorobenzo[*b*]thiophen-4-yl)-4-chl oro-2-fluoro-14-oxo-7,8,8*a*,9,11,12-hexa hydro-10*H*,14*H*-pyrazino[1',2':5,6][1,5]d iazocino[3,2,1-*hi*]indole-10-carboxylate |
| | 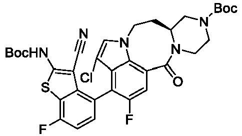 | tert-Butyl (8*aS*)-3-(2-((tert-butoxycarbonyl)amino)-3-cyano-7-fluorobenzo[*b*]thiophen-4-yl)-4-chloro-2-fluoro-14-oxo-7,8,8*a*,9,11,12-hexahydro-10*H*,14*H*-pyrazino[1',2':5,6][ 1,5]diazocino[3,2,1-*hi*]indole-10-carboxy late |
| | 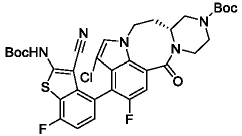 | tert-Butyl (8*aR*)-3-(2-((tert-butoxycarbonyl)amino) -3-cyano-7-fluorobenzo[*b*]thiophen-4-yl) -4-chloro-2-fluoro-14-oxo-7,8,8*a*,9,11,12 -hexahydro-10*H*,14*H*-pyrazino[1',2':5,6][ 1,5]diazocino[3,2,1-*hi*]indole-10-carboxy late |
| | 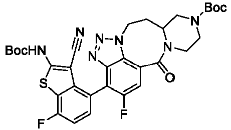 | tert-Butyl 3-(2-((tert-butoxycarbonyl)amino)-3-cya no-7-fluorobenzo[*b*]thiophen-4-yl)-2-flu oro-12-oxo-6,7,7*a*,8,10,11-hexahydro-9*H* ,12*H*-4,5,6*a*,9,11*a*-pentaazabenzo[5,6]cy cloocta[1,2,3-*cd*]indene-9-carboxylate |
| | 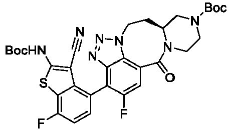 | tert-Butyl (7*aS*)-3-(2-((tert-butoxycarbonyl)amino)-3-cyano-7-fluorobenzo[*b*]thiophen-4-yl)-2-fluoro-12-oxo-6,7,7*a*,8,10,11-hexahydr o-9*H*,12*H*-4,5,5*a*,9,11*a*-pentaazabenzo[5, 6]cycloocta[1,2,3-*cd*]indene-9-carboxylat e |
| | 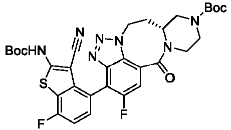 | tert-Butyl (7*aR*)-3-(2-((tert-butoxycarbonyl)amino) -3-cyano-7-fluorobenzo[*b*]thiophen-4-yl) -2-fluoro-12-oxo-6,7,7*a*,8,10,11-hexahyd ro-9*H*,12*H*-4,5,5*a*,9,11*a*-pentaazabenzo[ 5,6]cycloocta[1,2,3-*cd*]indene-9-carboxy late |
| | 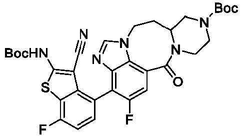 | tert-Butyl 3-(2-((tert-butoxycarbonyl)amino)-3-cya no-7-fluorobenzo[*b*]thiophen-4-yl)-2-flu oro-12-oxo-6,7,7a,8,10,11-hexahydro-9*H* ,12*H*-4,5*a*,9,11*a*-tetraazabenzo[5,6]cyclo octa[1,2,3-*cd*]indene-9-carboxylate |
| | 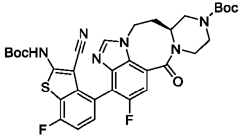 | tert-Butyl (7*aS*)-3-(2-((tert-butoxycarbonyl)amino)-3-cyano-7-fluorobenzo[*b*]thiophen-4-yl)-2-fluoro-12-oxo-6,7,7*a*,8,10,11-hexahydr o-9*H*,12*H*-4,5*a*,9,11*a*-tetraazabenzo[5,6] cycloocta[1,2,3-*cd*]indene-9-carboxylate |
| | 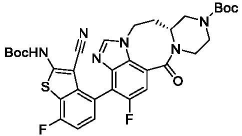 | tert-Butyl (7*aR*)-3-(2-((tert-butoxycarbonyl)amino) -3-cyano-7-fluorobenzo[*b*]thiophen-4-yl) -2-fluoro-12-oxo-6,7,7*a*,8,10,11-hexahyd ro-9*H*,12*H*-4,5*a*,9,11*a*-tetraazabenzo[5,6] cycloocta[1,2,3-*cd*]indene-9-carboxylate |

Another aspect of the present disclosure relates to a method for preparing a compound represented by general formula (IM) or a pharmaceutically acceptable salt thereof, comprising: 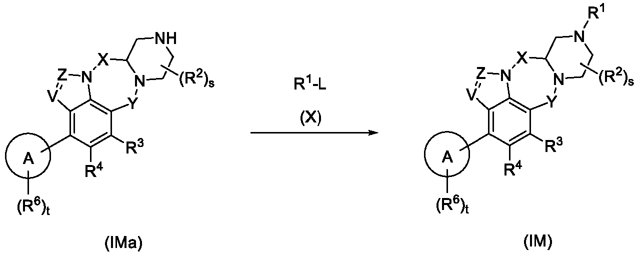 conducting a reaction of a compound represented by general formula (IMa) or a salt thereof with a compound of general formula (X) or a salt thereof to give the compound represented by general formula (IM) or the pharmaceutically acceptable salt thereof; wherein:
L is halogen; preferably, L is Cl;
R¹ is 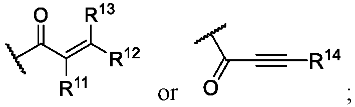
ring A, V, X, Y, Z, R², R³, R⁴, R⁶, R¹¹, R¹², R¹³, R¹⁴, s, and t are as defined in general formula (IM).

Another aspect of the present disclosure relates to a method for preparing a compound represented by general formula (I) or a pharmaceutically acceptable salt thereof, comprising: 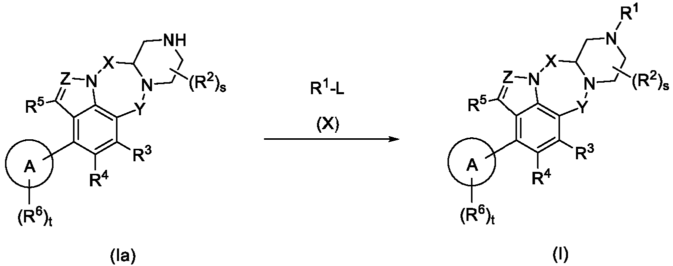 conducting a reaction of a compound represented by general formula (Ia) or a salt thereof with a compound of general formula (X) or a salt thereof to give the compound represented by general formula (I) or the pharmaceutically acceptable salt thereof;
wherein:
L is halogen; preferably, L is Cl;
R¹ is 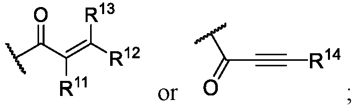
ring A, X, Y, Z, R², R³, R ⁴ , R⁵, R⁶, R¹¹, R¹², R¹³, R¹⁴, s, and t are as defined in general formula (I).

Another aspect of the present disclosure relates to a method for preparing a compound represented by general formula (II) or a pharmaceutically acceptable salt thereof, comprising: 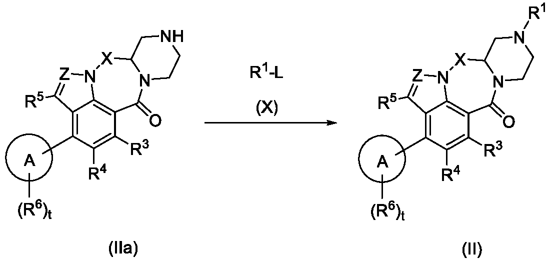 conducting a reaction of a compound represented by general formula (IIa) or a salt thereof with a compound of general formula (X) or a salt thereof to give the compound represented by general formula (II) or the pharmaceutically acceptable salt thereof; wherein:
L is halogen; preferably, L is Cl;
R¹ is 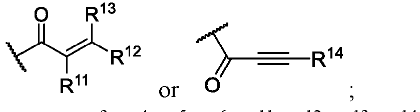
ring A, X, Z, R⁵, R⁴, R⁵, R⁶, R¹¹, R¹², R¹³, R¹⁴, and t are as defined in general formula (II).

Another aspect of the present disclosure relates to a method for preparing a compound represented by general formula (III) or a pharmaceutically acceptable salt thereof, comprising: 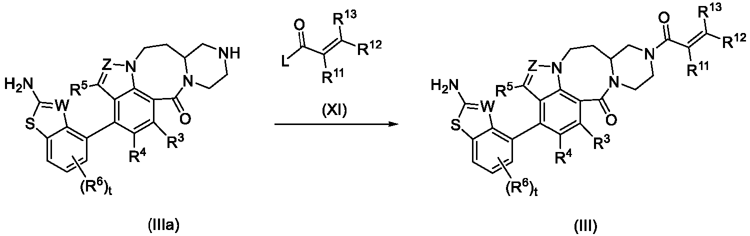 conducting a reaction of a compound represented by general formula (IIIa) or a salt thereof with a compound of general formula (XI) or a salt thereof to give the compound represented by general formula (III) or the pharmaceutically acceptable salt thereof; wherein:
L is halogen; preferably, L is Cl;
W, Z, R³, R⁴, R⁵, R⁶, R¹¹, R¹², R¹³, and t are as defined in general formula (III).

Another aspect of the present disclosure relates to a method for preparing a compound represented by general formula (III-1) or a pharmaceutically acceptable salt thereof, comprising: 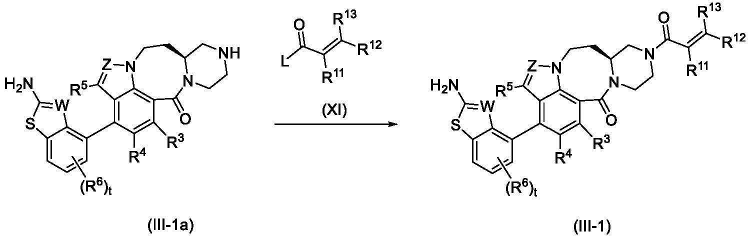 conducting a reaction of a compound represented by general formula (III-1a) or a salt thereof with a compound of general formula (XI) or a salt thereof to give the compound represented by general formula (III-1) or the pharmaceutically acceptable salt thereof; wherein:
L is halogen; preferably, L is Cl;
W, Z, R³, R⁴, R⁵, R⁶, R¹¹, R¹², R¹³, and t are as defined in general formula (III-1).

Another aspect of the present disclosure relates to a method for preparing a compound represented by general formula (III-2) or a pharmaceutically acceptable salt thereof, comprising: 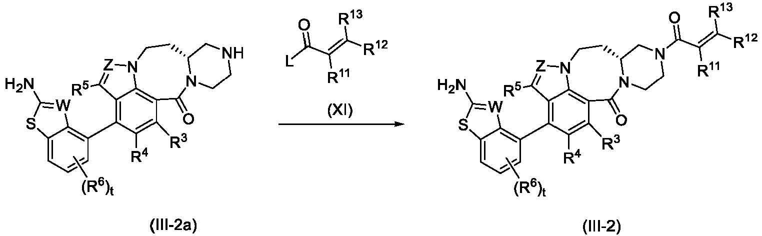 conducting a reaction of a compound represented by general formula (III-2a) or a salt thereof with a compound of general formula (XI) or a salt thereof to give the compound represented by general formula (III-2) or the pharmaceutically acceptable salt thereof; wherein:
L is halogen; preferably, L is Cl;
W, Z, R³, R⁴, R⁵, R⁶, R¹¹, R¹², R¹³, and t are as defined in general formula (III-2).

Another aspect of the present disclosure relates to a method for preparing compounds represented by general formula (III-1-A) and general formula (III-1-B) or pharmaceutically acceptable salts thereof, comprising: 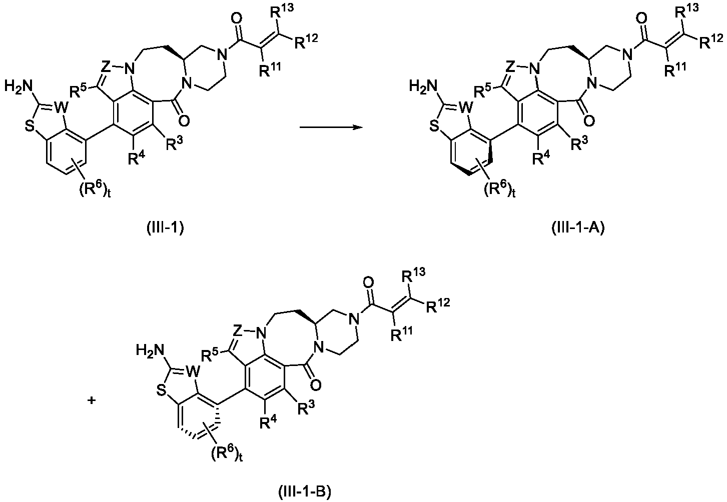 resolving a compound represented by general formula (III-1) or a pharmaceutically acceptable salt thereof to give the compounds represented by general formula (III-1-A) and general formula (III-1-B) or the pharmaceutically acceptable salts thereof; wherein:
W, Z, R³, R⁴, R⁵, R⁶, R¹¹, R¹², R¹³, and t are as defined in general formula (III-1).

Another aspect of the present disclosure relates to a method for preparing compounds represented by general formula (III-2-A) and general formula (III-2-B) or pharmaceutically acceptable salts thereof, comprising: 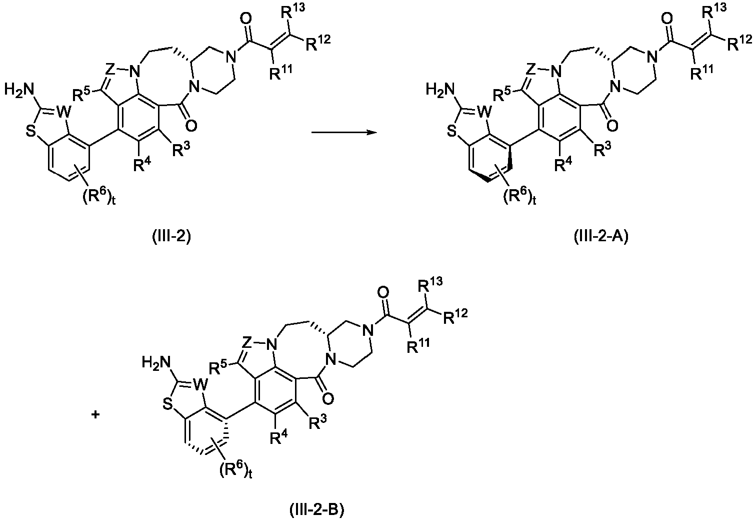 resolving a compound represented by general formula (III-2) or a pharmaceutically acceptable salt thereof to give the compounds represented by general formula (III-2-A) and general formula (III-2-B) or the pharmaceutically acceptable salts thereof;
wherein:
W, Z, R³, R⁴, R⁵, R⁶, R¹¹, R¹², R¹³, and t are as defined in general formula (III-2).

Another aspect of the present disclosure relates to a method for preparing a compound represented by general formula (IMa) or a pharmaceutically acceptable salt thereof, comprising: 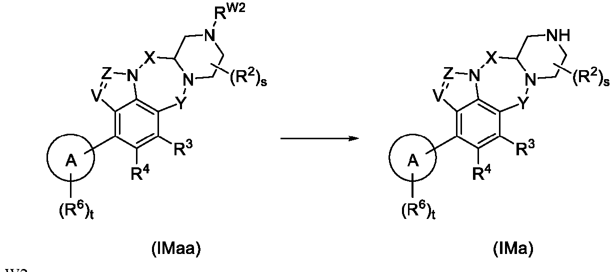 removing R^{W2} from a compound represented by general formula (IMaa) or a salt thereof to give the compound represented by general formula (IMa) or the pharmaceutically acceptable salt thereof;
wherein:
R^{W2} is an amino protecting group; preferably, R^{W2} is tert-butyloxycarbonyl;
ring A, V, X, Y, Z, R², R³, R⁴, R⁶, s, and t are as defined in general formula (IMa).

Another aspect of the present disclosure relates to a method for preparing a compound represented by general formula (IMa) or a salt thereof, comprising: 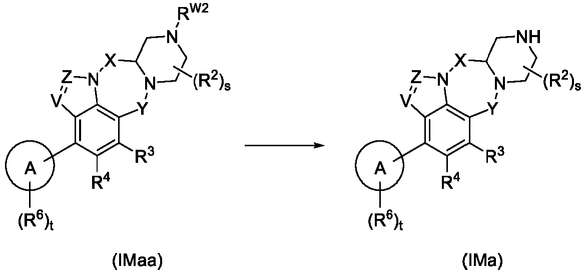 removing R^{W2} from a compound represented by general formula (IMaa) or a salt thereof to give the compound represented by general formula (IMa) or the salt thereof, wherein optionally, when a protecting group is present on the R⁶ group, a step of removing the protecting group from the R⁶ group is also included before, during, or after the deprotection reaction;
wherein:
R^{W2} is an amino protecting group; preferably, R^{W2} is tert-butyloxycarbonyl;
ring A, V, X, Y, Z, R², R³, R⁴, R⁶, s, and t are as defined in general formula (IMa).

Another aspect of the present disclosure relates to a method for preparing a compound represented by general formula (Ia) or a pharmaceutically acceptable salt thereof, comprising: 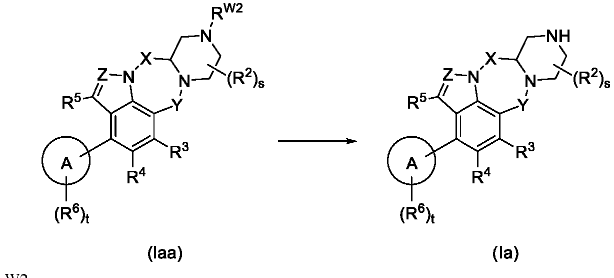 removing R^{W2} from a compound represented by general formula (Iaa) or a salt thereof to give the compound represented by general formula (Ia) or the pharmaceutically acceptable salt thereof;
wherein:
R^{W2} is an amino protecting group; preferably, R^{W2} is tert-butyloxycarbonyl;
ring A, X, Y, Z, R², R³, R⁴, R⁵, R⁶, s, and t are as defined in general formula (Ia).

Another aspect of the present disclosure relates to a method for preparing a compound represented by general formula (Ia) or a salt thereof, comprising: 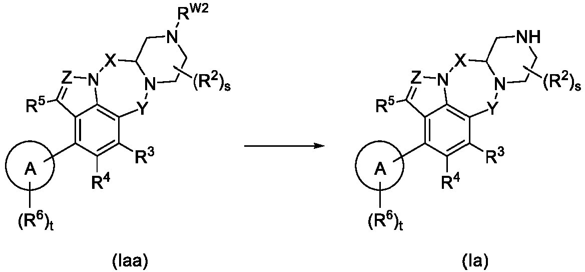 removing R^{W2} from a compound represented by general formula (Iaa) or a salt thereof to give the compound represented by general formula (Ia) or the salt thereof, wherein optionally, when a protecting group is present on the R⁶ group, a step of removing the protecting group from the R⁶ group is also included before, during, or after the deprotection reaction;
wherein:
R^{W2} is an amino protecting group; preferably, R^{W2} is tert-butyloxycarbonyl;
ring A, X, Y, Z, R², R³, R⁴, R⁵, R⁶, s, and t are as defined in general formula (Ia).

Another aspect of the present disclosure relates to a method for preparing a compound represented by general formula (IIa) or a pharmaceutically acceptable salt thereof, comprising: 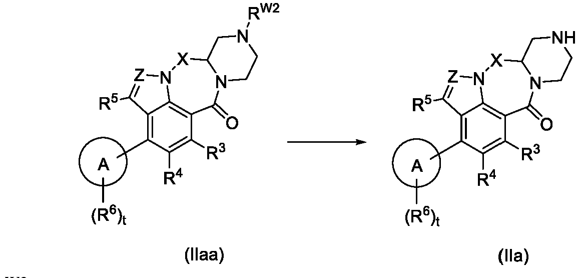 removing R^{W2} from a compound represented by general formula (IIaa) or a salt thereof to give the compound represented by general formula (IIa) or the pharmaceutically acceptable salt thereof;
wherein:
R^{W2} is an amino protecting group; preferably, R^{W2} is tert-butyloxycarbonyl;
ring A, X, Z, R³, R⁴, R⁵, R⁶, and t are as defined in general formula (IIa).

Another aspect of the present disclosure relates to a method for preparing a compound represented by general formula (IIa) or a salt thereof, comprising: 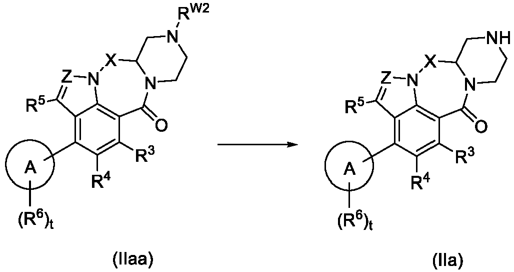 removing R^{W2} from a compound represented by general formula (IIaa) or a salt thereof to give the compound represented by general formula (IIa) or the salt thereof, wherein optionally, when a protecting group is present on the R⁶ group, a step of removing the protecting group from the R⁶ group is also included before, during, or after the deprotection reaction;
wherein:
R^{W2} is an amino protecting group; preferably, R^{W2} is tert-butyloxycarbonyl;
ring A, X, Z, R³, R⁴, R⁵, R⁶, and t are as defined in general formula (IIa).

Another aspect of the present disclosure relates to a method for preparing a compound represented by general formula (IIIa) or a pharmaceutically acceptable salt thereof, comprising: 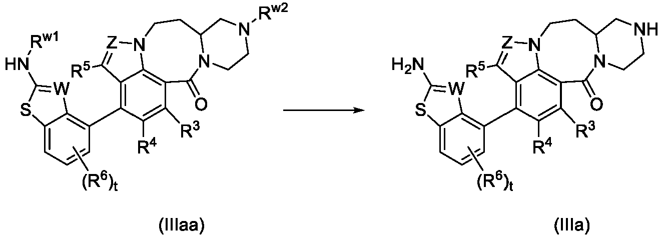 removing R^{W1} and R^{W2} from a compound represented by general formula (IIIaa) or a salt thereof to give the compound represented by general formula (IIIa) or the pharmaceutically acceptable salt thereof;
wherein:
R^{W1} and R^{W2} are both amino protecting groups; preferably, R^{W1} and R^{W2} are both tert-butyloxycarbonyl;
W, Z, R³, R⁴, R⁵, R⁶, and t are as defined in general formula (IIIa).

Another aspect of the present disclosure relates to a method for preparing a compound represented by general formula (III-1a) or a pharmaceutically acceptable salt thereof, comprising: 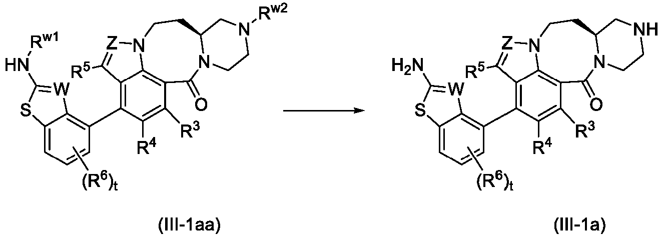 removing R^{W1} and R^{W2} from a compound represented by general formula (III-1aa) or a salt thereof to give the compound represented by general formula (III-1a) or the pharmaceutically acceptable salt thereof;
wherein:
R^{W1} and R^{W2} are both amino protecting groups; preferably, R^{W1} and R^{W2} are both tert-butyloxycarbonyl;
W, Z, R³, R⁴, R⁵, R⁶, and t are as defined in general formula (III-1a).

Another aspect of the present disclosure relates to a method for preparing a compound represented by general formula (III-2a) or a pharmaceutically acceptable salt thereof, comprising: 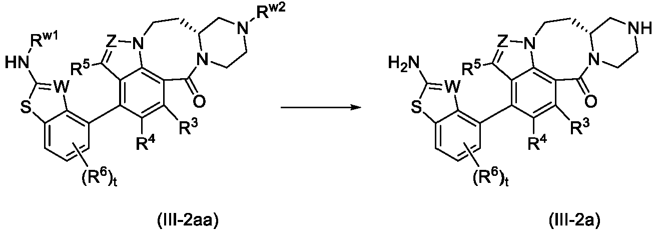 removing R^{W1} and R^{W2} from a compound represented by general formula (III-2aa) or a salt thereof to give the compound represented by general formula (III-2a) or the pharmaceutically acceptable salt thereof;
wherein:
R^{W1} and R^{W2} are both amino protecting groups; preferably, R^{W1} and R^{W2} are both tert-butyloxycarbonyl;
W, Z, R³, R⁴, R⁵, R⁶, and t are as defined in general formula (III-2a).

Another aspect of the present disclosure relates to a pharmaceutical composition comprising the compound represented by general formula (IM), general formula (I), general formula (II), general formula (III), general formula (III-1), general formula (III-2), general formula (III-1-A), general formula (III-1-B), general formula (III-2-A), or general formula (III-2-B) of the present disclosure and a compound from Table A or a pharmaceutically acceptable salt thereof, and one or more pharmaceutically acceptable carriers, diluents, or excipients.

The present disclosure further relates to use of the compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof or a pharmaceutical composition comprising same in the preparation of a medicament for inhibiting KRAS G12C.

The present disclosure further relates to use of the compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof or a pharmaceutical composition comprising same in the preparation of a medicament for treating and/or preventing a KRAS G12C-mediated disease or disorder.

The present disclosure further relates to use of the compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof or a pharmaceutical composition comprising same in the preparation of a medicament for treating and/or preventing a tumor.

The present disclosure also relates to a method for inhibiting KRAS G12C, comprising administering to a patient in need thereof a therapeutically effective amount of the compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof, or a pharmaceutical composition comprising same.

The present disclosure also relates to a method for treating and/or preventing a KRAS G12C-mediated disease or disorder, comprising administering to a patient in need thereof a therapeutically effective amount of the compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof, or a pharmaceutical composition comprising same.

The present disclosure also relates to a method for treating and/or preventing a tumor, comprising administering to a patient in need thereof a therapeutically effective amount of the compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof, or a pharmaceutical composition comprising same.

The present disclosure further relates to a compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof, or a pharmaceutical composition comprising same, for use as a medicament.

The present disclosure further relates to a compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof, or a pharmaceutical composition comprising same, for use as a KRAS G12C inhibitor.

The present disclosure further relates to a compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof, or a pharmaceutical composition comprising same, for use in treating and/or preventing a KRAS G12C-mediated disease or disorder. The present disclosure further relates to a compound represented by general formula (IM), general formula (I), general formula (II), general formula (III), general formula (III-1), general formula (III-2), general formula (III-1-A), general formula (III-1-B), general formula (III-2-A), or general formula (III-2-B) and a compound from Table A or a pharmaceutically acceptable salt thereof, or a pharmaceutical composition comprising same, for use in treating and/or preventing a tumor.

The tumor described above in the present disclosure is preferably a cancer; further preferably, the cancer is selected from the group consisting of lung cancer (such as non-small cell lung cancer and small cell lung cancer), pancreatic cancer, cervical cancer, esophageal cancer (also known as esophageal carcinoma), endometrial cancer, ovarian cancer, cholangiocarcinoma, colorectal cancer (such as colon cancer and rectal cancer), liver cancer, breast cancer, prostate cancer, thyroid cancer, stomach cancer, urothelial cancer, testicular cancer, leukemia, skin cancer, squamous cell cancer, basal cell cancer, bladder cancer, head and neck cancer, kidney cancer, nasopharyngeal cancer, bone cancer, lymphoma, melanoma, sarcoma, peripheral neuroepithelioma, glioma (such as astrocytoma and glioblastoma), brain tumor, and myeloma; more preferably, the cancer is selected from the group consisting of lung cancer, pancreatic cancer, cervical cancer, esophageal cancer, endometrial cancer, ovarian cancer, cholangiocarcinoma, and colorectal cancer.

The KRAS G12C-mediated disease or disorder described above in the present disclosure is preferably a tumor; further preferably, the tumor is a cancer; more preferably, the cancer is selected from the group consisting of lung cancer (such as non-small cell lung cancer and small cell lung cancer), pancreatic cancer, cervical cancer, esophageal cancer (also known as esophageal carcinoma), endometrial cancer, ovarian cancer, cholangiocarcinoma, colorectal cancer (such as colon cancer and rectal cancer), liver cancer, breast cancer, prostate cancer, thyroid cancer, stomach cancer, urothelial cancer, testicular cancer, leukemia, skin cancer, squamous cell cancer, basal cell cancer, bladder cancer, head and neck cancer, kidney cancer, nasopharyngeal cancer, bone cancer, lymphoma, melanoma, sarcoma, peripheral neuroepithelioma, glioma (such as astrocytoma and glioblastoma), brain tumor, and myeloma; most preferably, the cancer is selected from the group consisting of lung cancer, pancreatic cancer, cervical cancer, esophageal cancer, endometrial cancer, ovarian cancer, cholangiocarcinoma, and colorectal cancer.

As a general guide, the active compound of the present disclosure is preferably in the form of a unit dose, or in the form of a single dose that can be self-administered by a patient. The unit dose of the compound or composition of the present disclosure may be expressed in the form of a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials; the auxiliary materials may be selected from the group consisting of a filler (diluent), a binder, a wetting agent, a disintegrant, an excipient, and the like. Depending on the method of administration, the composition may comprise 0.1 to 99 wt.% of the active compound.

The pharmaceutical composition comprising the active ingredient may be in a form suitable for oral administration, for example, in the form of a tablet, dragee, lozenge, aqueous or oil suspension, dispersible powder or granule, emulsion, hard or soft capsule, or syrup or elixir. An oral composition may be prepared by following any method known in the art for preparing pharmaceutical compositions, and such a composition may comprise one or more ingredients selected from the group consisting of a sweetener, a flavoring agent, a colorant, and a preservative, so as to provide a pharmaceutical formulation that is pleasing to the eye and palatable. The tablet comprises the active ingredient, and non-toxic pharmaceutically acceptable excipients that are used for mixing and are suitable for the preparation of the tablet. These excipients may be an inert excipient, a granulating agent, a disintegrant, a binder, and a lubricant. These tablets may be uncoated or coated using known techniques that mask the taste of the drug or delay disintegration and absorption in the gastrointestinal tract, thereby providing a sustained-release effect over an extended period of time.

An oral formulation may also be provided in the form of a soft gelatin capsule in which the active ingredient is mixed with an inert solid diluent or with a water-soluble carrier or oil vehicle.

An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant.

The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using suitable dispersants or wetting agents and suspending agents. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

As is well known to those skilled in the art, the dosage of the drug depends on a variety of factors, including, but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

### Description of the terms

Unless otherwise stated, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). The alkyl is preferably an alkyl group having 1 to 12 carbon atoms (i.e., C₁₋₁₂ alkyl), and more preferably an alkyl group having 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of a deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkenyl). The alkenyl is preferably an alkenyl group having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of a deuterium atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkynyl). The alkynyl is preferably an alkynyl group having 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted, and when it is substituted, it may be substituted at any accessible point of attachment, and the substituent is preferably selected from the group consisting of one or more of a deuterium atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of a deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e.,3- to 12-membered cycloalkyl), more preferably a cycloalkyl group having 3 to 8 ring atoms (i.e., 3- to 8-membered cycloalkyl), and most preferably a cycloalkyl group having 3 to 6 ring atoms (i.e., 3- to 6-membered cycloalkyl).

Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings, which may contain in the rings one or more double bonds or may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one all-carbon ring is contained and the point of attachment is on the all-carbon ring; and which has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). The spirocycloalkyl is preferably a spirocycloalkyl group having 6 to 14 ring atoms (i.e., 6- to 14-membered spirocycloalkyl), and more preferably a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl), preferably monospirocycloalkyl or bispirocycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples include: 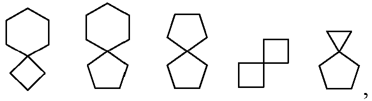 wherein the point of attachment may be at any position; 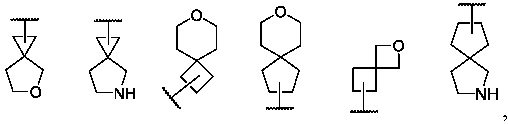 and the like.

The term "fused cycloalkyl" refers to a polycyclic system in which two adjacent carbon atoms are shared between rings, which is formed by fusing a monocyclic cycloalkyl group with one or more monocyclic cycloalkyl groups, or fusing a monocyclic cycloalkyl group with one or more of a heterocyclyl group, an aryl group, or a heteroaryl group, wherein the point of attachment is on a monocyclic cycloalkyl group; and which may contain in the rings one or more double bonds and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). The fused cycloalkyl is preferably a fused cycloalkyl group having 6 to 14 ring atoms (i.e., 6- to 14-membered fused cycloalkyl), and more preferably a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl), preferably bicyclic fused cycloalkyl or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl. Non-limiting examples include: 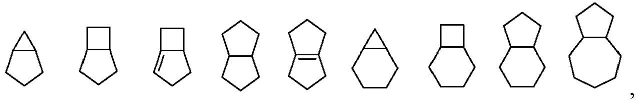 wherein the point of attachment may be at any position; 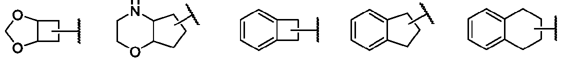 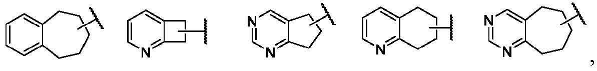 and the like.

The term "bridged cycloalkyl" refers to an all-carbon polycyclic system in which two carbon atoms that are not directly connected are shared between rings, which may contain in the rings one or more double bonds and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). The bridged cycloalkyl is preferably a bridged cycloalkyl group having 6 to 14 carbon atoms (i.e., 6- to 14-membered bridged cycloalkyl), and more preferably a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl and tetracyclic bridged cycloalkyl), preferably bicyclic bridged cycloalkyl or tricyclic bridged cycloalkyl. Non-limiting examples include: 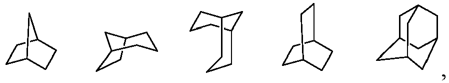 wherein the point of attachment may be at any position.

Cycloalkyl may be substituted or unsubstituted, and when it is substituted, it may be substituted at any accessible point of attachment, and the substituent is preferably selected from the group consisting of one or more of a deuterium atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocycle (i.e., monocyclic heterocyclyl) or polycyclic heterocyclic system (i.e., polycyclic heterocyclyl), which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). The heterocyclyl is preferably a heterocyclyl group having 3 to 12 ring atoms (i.e., 3- to 12-membered heterocyclyl); further preferably a heterocyclyl group having 3 to 8 ring atoms (i.e., 3- to 8-membered heterocyclyl); more preferably a heterocyclyl group having 3 to 6 ring atoms (i.e., 3- to 6-membered heterocyclyl); and most preferably a heterocyclyl group having 5 or 6 ring atoms (i.e., 5- or 6-membered heterocyclyl).

Non-limiting examples of the monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, which may contain in the rings one or more double bonds and contains in the rings at least one (e.g., 1, 2, 3, or 4) heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one monocyclic heterocyclyl group is contained and the point of attachment is on the monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). The spiroheterocyclyl is preferably a spiroheterocyclyl group having 6 to 14 ring atoms (i.e., 6- to 14-membered spiroheterocyclyl), and more preferably a spiroheterocyclyl group having 7 to 10 ring atoms (i.e., 7- to 10-membered spiroheterocyclyl). The spiroheterocyclyl includes monospiroheterocyclyl and polyspiroheterocyclyl (e.g., bispiroheterocyclyl), preferably monospiroheterocyclyl or bispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples include: 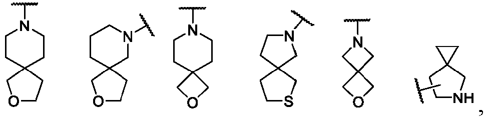 and the like.

The term "fused heterocyclyl" refers to a polycyclic heterocyclic system in which two adjacent atoms are shared between rings, which may contain in the rings one or more double bonds and contains in the rings at least one (e.g., 1, 2, 3, or 4) heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); which is formed by fusing a monocyclic heterocyclyl group with one or more monocyclic heterocyclyl groups, or fusing a monocyclic heterocyclyl group with one or more of a cycloalkyl group, an aryl group, or a heteroaryl group, wherein the point of attachment is on a monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). The fused heterocyclyl is preferably a fused heterocyclyl group having 6 to 14 ring atoms (i.e., 6- to 14-membered fused heterocyclyl), and more preferably a fused heterocyclyl group having 7 to 10 ring atoms (i.e., 7- to 10-membered fused heterocyclyl). The fused heterocyclyl includes bicyclic and polycyclic fused heterocyclyl (e.g., tricyclic fused heterocyclyl and tetracyclic fused heterocyclyl), preferably bicyclic fused heterocyclyl or tricyclic fused heterocyclyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples include: 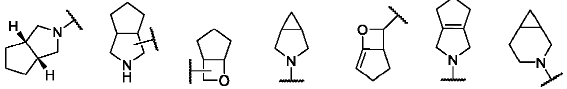 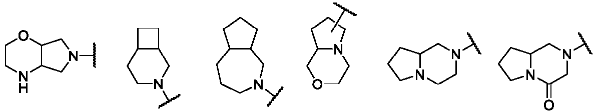 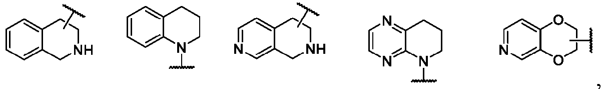 and the like.

The term "bridged heterocyclyl" refers to a polycyclic heterocyclic system in which two atoms that are not directly connected are shared between rings, which may contain in the rings one or more double bonds and contains in the rings at least one (e.g., 1, 2, 3, or 4) heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); and which has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). The bridged heterocyclyl is preferably a bridged heterocyclyl group having 6 to 14 ring atoms (i.e., 6- to 14-membered bridged heterocyclyl), and more preferably a bridged heterocyclyl group having 7 to 10 ring atoms (i.e., 7- to 10-membered bridged heterocyclyl). According to the number of constituent rings, bridged heterocyclyl can be divided into bicyclic bridged heterocyclyl and polycyclic bridged heterocyclyl (e.g., tricyclic bridged heterocyclyl and tetracyclic bridged heterocyclyl), preferably bicyclic bridged heterocyclyl or tricyclic bridged heterocyclyl. Non-limiting examples include: 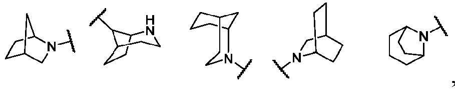 and the like.

Heterocyclyl may be substituted or unsubstituted, and when it is substituted, it may be substituted at any accessible point of attachment, and the substituent is preferably selected from the group consisting of one or more of a deuterium atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system, which has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl). An example of the monocyclic aryl is phenyl. Non-limiting examples of the polycyclic aryl include: naphthyl, anthryl, phenanthryl, and the like. The polycyclic aryl also includes those formed by fusing a phenyl group with one or more of a heterocyclyl group or a cycloalkyl group, or fusing a naphthyl group with one or more of a heterocyclyl group or a cycloalkyl group, wherein the point of attachment is on the phenyl group or the naphthyl group, and in such cases, the number of ring atoms continues to represent the number of ring atoms in the polycyclic aromatic ring system; non-limiting examples include: 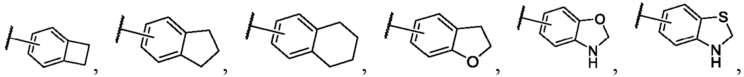 and the like.

Aryl may be substituted or unsubstituted, and when it is substituted, it may be substituted at any accessible point of attachment, and the substituent is preferably selected from the group consisting of one or more of a deuterium atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 5 to 14 (e.g., 5, 6, 7, 8, 9, 10, 11, 12, 13, or 14) ring atoms (i.e., 5- to 14-membered heteroaryl). The heteroaryl is preferably a heteroaryl group having 5 to 10 ring atoms (i.e., 5- to 10-membered heteroaryl), more preferably a monocyclic heteroaryl group having 5 or 6 ring atoms (i.e., 5- or 6-membered monocyclic heteroaryl) or a bicyclic heteroaryl group having 8 to 10 ring atoms (i.e., 8- to 10-membered bicyclic heteroaryl), and most preferably a 5- or 6-membered monocyclic heteroaryl group containing in the ring 1, 2, or 3 heteroatoms selected from the group consisting of nitrogen, oxygen, and sulfur or an 8- to 10-membered bicyclic heteroaryl group containing in the ring 1, 2, or 3 heteroatoms selected from the group consisting of nitrogen, oxygen, and sulfur.

Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridonyl, N-alkylpyridinone (e.g., 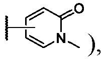 pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, quinazolinyl, benzothiazolyl, carbazolyl, and the like. The polycyclic heteroaryl also includes those formed by fusing a monocyclic heteroaryl group with one or more aryl groups, wherein the point of attachment is on an aromatic ring, and in such cases, the number of ring atoms continues to represent the number of ring atoms in the polycyclic heteroaromatic ring system. The polycyclic heteroaryl also includes those formed by fusing a monocyclic heteroaryl group with one or more of a cycloalkyl group or a heterocyclyl group, wherein the point of attachment is on the monocyclic heteroaromatic ring, and in such cases, the number of ring atoms continues to represent the number of ring atoms in the polycyclic heteroaromatic ring system. Non-limiting examples include: 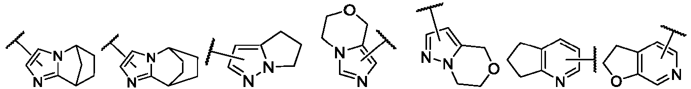 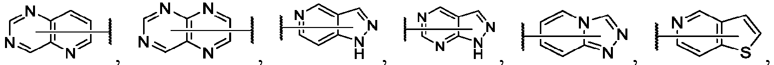 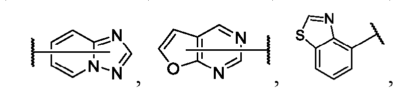 and the like.

Heteroaryl may be substituted or unsubstituted, and when it is substituted, it may be substituted at any accessible point of attachment, and the substituent is preferably selected from the group consisting of one or more of a deuterium atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "amino protecting group" refers to an easily removable group that is introduced onto an amino group in order for the amino group to remain unchanged when other parts of the molecule are involved in reactions. Non-limiting examples include: (trimethylsilyl)ethoxymethyl, tetrahydropyranyl, tert-butyloxycarbonyl (Boc), benzyloxycarbonyl (Cbz), fluorenylmethoxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulfonyl (Tos), trifluoroacetyl (Tfa), trityl (Trt), 2,4-dimethoxybenzyl (DMB), acetyl, benzyl, allyl, p-methoxybenzyl, and the like.

The term "hydroxy protecting group" refers to an easily removable group that is introduced onto a hydroxy group to block or protect the hydroxy group so that reactions take place on other functional groups of the compound. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to an alkyl group substituted with one or more halogens, wherein the alkyl group is as defined above.

The term "haloalkoxy" refers to an alkoxy group substituted with one or more halogens, wherein the alkoxy group is as defined above.

The term "deuterated alkyl" refers to an alkyl group substituted with one or more deuterium atoms, wherein the alkyl is as defined above.

The term "hydroxyalkyl" refers to an alkyl group substituted with one or more hydroxy groups, wherein the alkyl group is as defined above.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

The compounds of the present disclosure may exist in specific stereoisomeric forms. The term "stereoisomer" refers to isomers that are structurally identical but differ in the arrangement of the atoms in space. It includes cis and trans (or Z and E) isomers, (-)- and (+)-isomers, (R)- and (S)-enantiomers, diastereomers, (D)- and (L)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is generally accomplished by chromatography.

In the chemical structures of the compounds of the present disclosure, a bond " 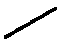 " represents an unspecified configuration; that is, if chiral isomers exist in the chemical structures, the bond " 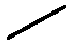 " may be " 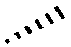 " or " 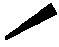 ", or both the configurations of " 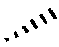 ", and " 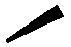 " are included simultaneously. For all carbon-carbon double bonds, both Z- and E-forms are included, even if only one configuration is named.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers; that is, it is present in the form of a single isomer or in the form of a mixture of the tautomers in any ratio. Non-limiting examples include: keto-enol, imine-enamine, lactam-lactim, and the like. An example of lactam-lactim in equilibrium is shown below:

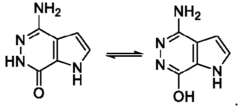

For example, reference to pyrazolyl is understood to include any of the following two structures or a mixture of the two tautomers:

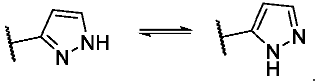

All tautomeric forms fall within the scope of the present disclosure, and the nomenclature of the compounds does not exclude any tautomer.

The compound of the present disclosure may include atropisomers. The term "atropisomers" refers to conformational stereoisomers that result from hindered or greatly slowed rotation about a single bond in a molecule (as a result of the steric interactions with other parts of the molecule and the asymmetry of the substituents at both ends of the single bond), which interconvert sufficiently slowly to allow separation and isolation under predetermined conditions. For example, certain compounds of the present disclosure may exist in the form of a mixture of atropisomers (e.g., an equal ratio mixture, a mixture enriched in one atropisomer) or a purified atropisomer.

Non-limiting examples include: 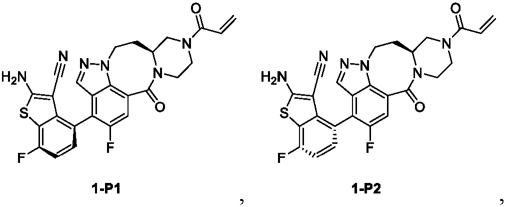 and the like.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (deuterium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸² Br, ¹²³I, ¹²⁴I, ¹²⁵I, ¹²⁹I, and ¹³¹I, deuterium is preferred.

Compared to non-deuterated drugs, deuterated drugs have the advantages of reduced toxic and side effects, increased drug stability, enhanced curative effect, prolonged biological half-lives, and the like. All isotopic variations of the compounds of the present disclosure, whether radioactive or not, are intended to be included within the scope of the present disclosure. Each available hydrogen atom linked to a carbon atom may be independently replaced with a deuterium atom, wherein replacement of deuterium may be partial or complete, and replacement of partial deuterium refers to replacement of at least one hydrogen atom with at least one deuterium atom.

When a position is specifically assigned deuterium (D), the position should be understood as deuterium with an abundance that is at least 1000 times greater than the natural abundance of deuterium (which is 0.015%) (i.e., at least 15% deuterium incorporation). The deuterium of the compounds in the examples with an abundance greater than the natural abundance of deuterium may be deuterium with an abundance that is at least 1000 times greater (i.e., at least 15% deuterium incorporation), at least 2000 times greater (i.e., at least 30% deuterium incorporation), at least 3000 times greater (i.e., at least 45% deuterium incorporation), at least 3340 times greater (i.e., at least 50.1% deuterium incorporation), at least 3500 times greater (i.e., at least 52.5% deuterium incorporation), at least 4000 times greater (i.e., at least 60% deuterium incorporation), at least 4500 times greater (i.e., at least 67.5% deuterium incorporation), at least 5000 times greater (i.e., at least 75% deuterium incorporation), at least 5500 times greater (i.e., at least 82.5% deuterium incorporation), at least 6000 times greater (i.e., at least 90% deuterium incorporation), at least 6333.3 times greater (i.e., at least 95% deuterium incorporation), at least 6466.7 times greater (i.e., at least 97% deuterium incorporation), at least 6600 times greater (i.e., at least 99% deuterium incorporation), or at least 6633.3 times greater (i.e., at least 99.5% deuterium incorporation), or deuterium with a higher abundance.

"Optionally" or "optional" means that the subsequently described event or circumstance may, but does not necessarily, occur; it includes both the instance where the event or circumstance occurs and the instance where it does not. For example, "C₁₋₆ alkyl optionally substituted with halogen or cyano" includes the instance where the alkyl is substituted with the halogen or cyano and the instance where it is not.

"Substitution" or "substituted" means that one or more, preferably 1-6, and more preferably 1-3, hydrogen atoms in the group are independently substituted with a corresponding number of substituents. Those skilled in the art can determine (experimentally or theoretically) possible or impossible substitutions without undue effort. For example, it may be unstable when an amino or hydroxy group having free hydrogen binds to a carbon atom having an unsaturated bond (e.g., an olefin).

"Pharmaceutical composition" refers to a mixture containing one or more of the compounds or the pharmaceutically acceptable salts thereof described herein, and other chemical components, and other components, for example, pharmaceutically acceptable carriers and excipients. The pharmaceutical composition is intended to promote the administration to an organism, so as to facilitate the absorption of the active ingredient, thereby exerting biological activity.

"Pharmaceutically acceptable salt" refers to a salt of a compound of the present disclosure, which may be selected from the group consisting of inorganic and organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the therapeutically effective amount varies from person to person. It depends on the age and general condition of the subject, as well as the specific active substance used. The appropriate therapeutically effective amount in an individual case can be determined by those skilled in the art through a conventional experiment.

The term "pharmaceutically acceptable" as used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be understood by those skilled in the art, when the parameters are not critical, the numbers are generally given for illustrative purposes only and are not intended to be limiting.

### Synthesis Methods for Compounds of the Present Disclosure

To achieve the purposes of the present disclosure, the following technical solutions are adopted in the present disclosure:

### Solution 1

A method for preparing the compound represented by general formula (IM) or the pharmaceutically acceptable salt thereof of the present disclosure, which comprises the following step: 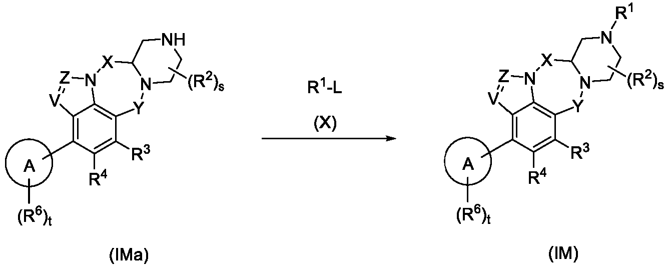 conducting a reaction of a compound represented by general formula (IMa) or a salt thereof with a compound represented by general formula (X) or a salt thereof under alkaline conditions to give the compound represented by general formula (IM) or the pharmaceutically acceptable salt thereof;
wherein:
L is halogen; preferably, L is Cl;
R¹ is 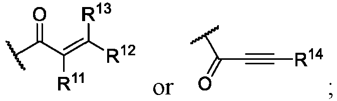
ring A, V, X, Y, Z, R², R³, R⁴, R⁶, R¹¹, R¹², R¹³, R¹⁴, s, and t are as defined in general formula (IM).

### Solution 2

A method for preparing the compound represented by general formula (I) or the pharmaceutically acceptable salt thereof of the present disclosure, which comprises the following step: 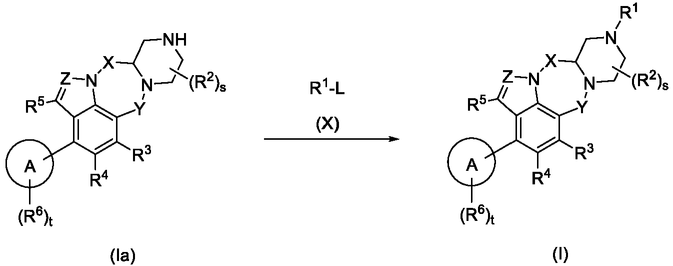 conducting a reaction of a compound represented by general formula (Ia) or a salt thereof with a compound represented by general formula (X) or a salt thereof under alkaline conditions to give the compound represented by general formula (I) or the pharmaceutically acceptable salt thereof;
wherein:
L is halogen; preferably, L is Cl;
R¹ is 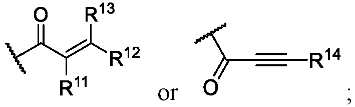
ring A, X, Y, Z, R², R³, R⁴, R⁵, R⁶, R¹¹, R¹², R¹³, R¹⁴, s, and t are as defined in general formula (I).

### Solution 3

A method for preparing the compound represented by general formula (II) or the pharmaceutically acceptable salt thereof of the present disclosure, which comprises the following step: 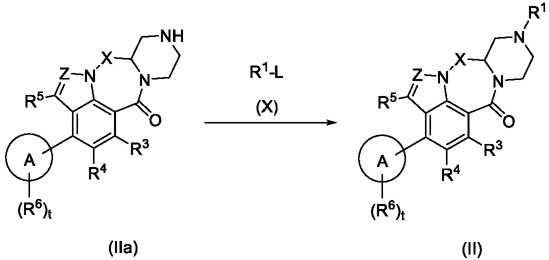 conducting a reaction of a compound represented by general formula (IIa) or a salt thereof with a compound represented by general formula (X) or a salt thereof under alkaline conditions to give the compound represented by general formula (II) or the pharmaceutically acceptable salt thereof;
wherein:
L is halogen; preferably, L is Cl;
R¹ is 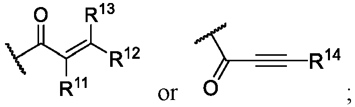
ring A, X, Z, R³, R⁴, R⁵, R⁶, R¹¹, R¹², R¹³, R¹⁴, and t are as defined in general formula (II).

### Solution 4

A method for preparing the compound represented by general formula (III) or the pharmaceutically acceptable salt thereof of the present disclosure, which comprises the following step: 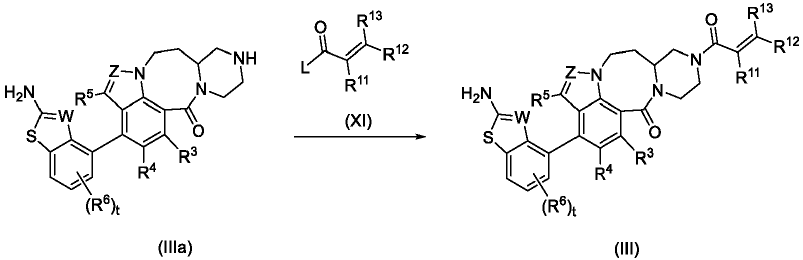 conducting a reaction of a compound represented by general formula (IIIa) or a salt thereof with a compound represented by general formula (XI) or a salt thereof under alkaline conditions to give the compound represented by general formula (III) or the pharmaceutically acceptable salt thereof;
wherein:
L is halogen; preferably, L is Cl;
W, Z, R³, R⁴, R⁵, R⁶, R¹¹, R¹², R¹³, and t are as defined in general formula (III).

### Solution 5

A method for preparing the compound represented by general formula (III-1) or the pharmaceutically acceptable salt thereof of the present disclosure, which comprises the following step: 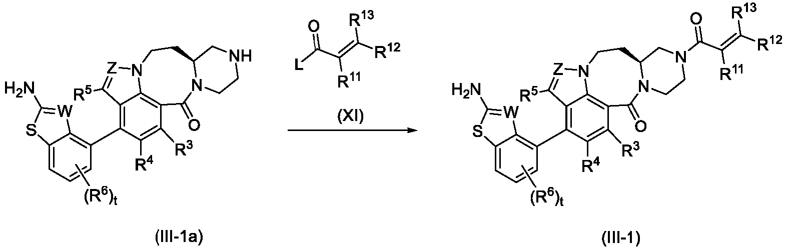 conducting a reaction of a compound represented by general formula (III-1a) or a salt thereof with a compound represented by general formula (XI) or a salt thereof under alkaline conditions to give the compound represented by general formula (III-1) or the pharmaceutically acceptable salt thereof;
wherein:
L is halogen; preferably, L is Cl;
W, Z, R³, R⁴, R⁵, R⁶, R¹¹, R¹², R¹³, and t are as defined in general formula (III-1).

### Solution 6

A method for preparing the compound represented by general formula (III-2) or the pharmaceutically acceptable salt thereof of the present disclosure, which comprises the following step: 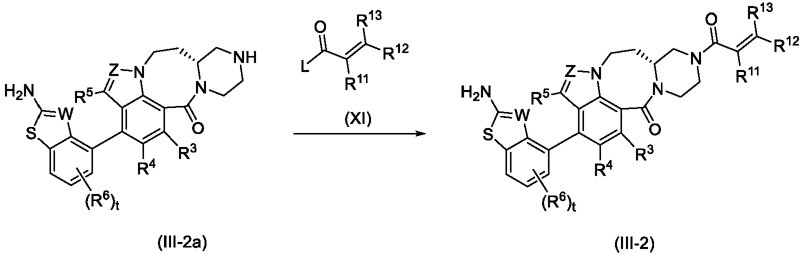 conducting a reaction of a compound represented by general formula (III-2a) or a salt thereof with a compound represented by general formula (XI) or a salt thereof under alkaline conditions to give the compound represented by general formula (III-2) or the pharmaceutically acceptable salt thereof;
wherein:
L is halogen; preferably, L is Cl;
W, Z, R³, R⁴, R⁵, R⁶, R¹¹, R¹², R¹³, and t are as defined in general formula (III-2).

### Solution 7

A method for preparing the compounds represented by general formula (III-1-A) and general formula (III-1-B) or the pharmaceutically acceptable salts thereof of the present disclosure, which comprises the following step: 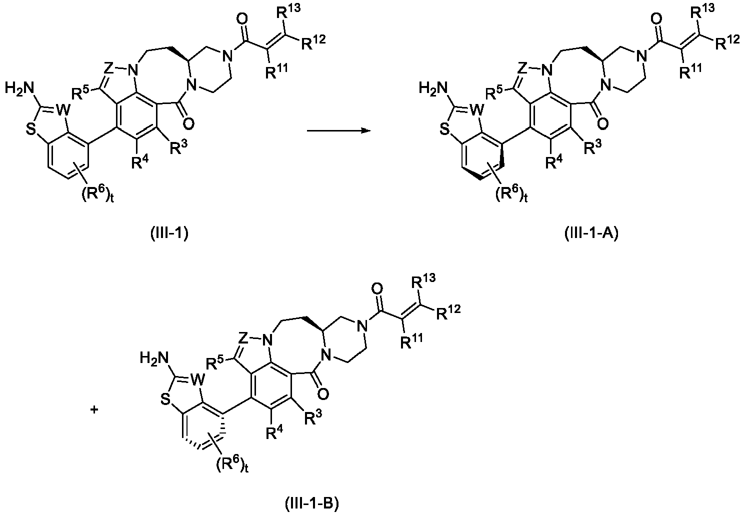 resolving a compound represented by general formula (III-1) or a pharmaceutically acceptable salt thereof by preparative high performance liquid chromatography to give the compounds represented by general formula (III-1-A) and general formula (III-1-B) or the pharmaceutically acceptable salts thereof;
wherein:
W, Z, R³, R⁴, R⁵, R⁶, R¹¹, R¹², R¹³, and t are as defined in general formula (III-1).

### Solution 8

A method for preparing the compounds represented by general formula (III-2-A) and general formula (III-2-B) or the pharmaceutically acceptable salts thereof of the present disclosure, which comprises the following step: 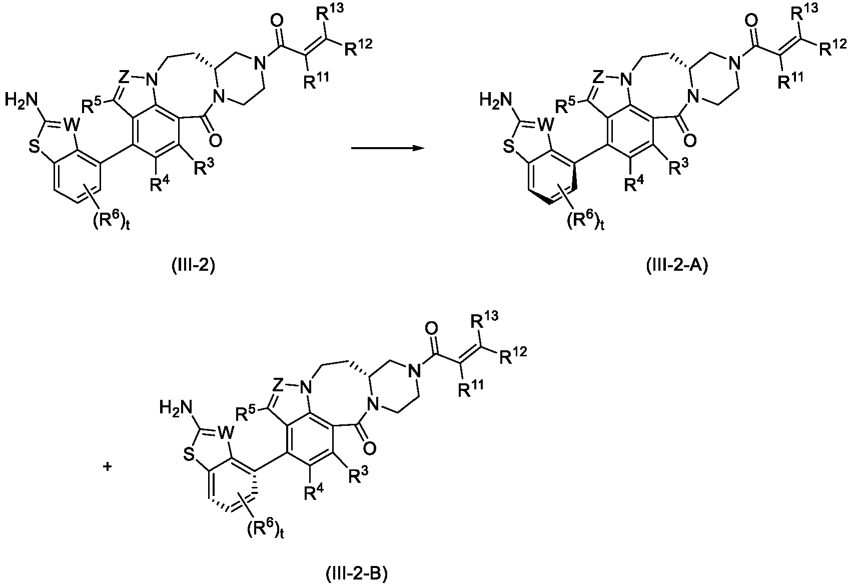 resolving a compound represented by general formula (III-2) or a pharmaceutically acceptable salt thereof by preparative high performance liquid chromatography to give the compounds represented by general formula (III-2-A) and general formula (III-2-B) or the pharmaceutically acceptable salts thereof;
wherein:
W, Z, R³, R⁴, R⁵, R⁶, R¹¹, R¹², R¹³, and t are as defined in general formula (III-2).

### Solution 9-1

A method for preparing the compound represented by general formula (IMa) or the pharmaceutically acceptable salt thereof of the present disclosure, which comprises the following steps: 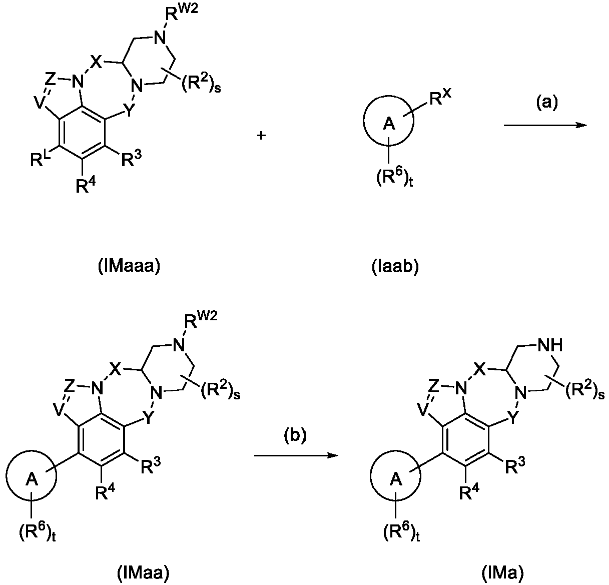
(a) conducting a Suzuki coupling reaction of a compound represented by general formula (IMaaa) or a salt thereof with a compound represented by general formula (Iaab) or a salt thereof to give a compound represented by general formula (IMaa) or a salt thereof; and
(b) removing R^{W2} from the compound represented by general formula (IMaa) or the salt thereof under acidic conditions to give the compound represented by general formula (IMa) or the pharmaceutically acceptable salt thereof;
   wherein:
   R^{L} is halogen; preferably, R^{L} is Br;
   R^{W2} is an amino protecting group; preferably, R^{W2} is tert-butyloxycarbonyl;
   R^{X} is selected from the group consisting of 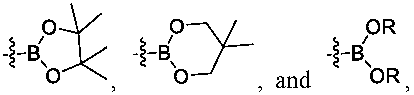 and R is a hydrogen atom or C₁₋₆ alkyl;
   ring A, V, X, Y, Z, R², R³, R⁴, R⁶, s, and t are as defined in general formula (IMa).

### Solution 9-2

A method for preparing the compound represented by general formula (IMa) or the salt thereof of the present disclosure, which comprises the following steps: 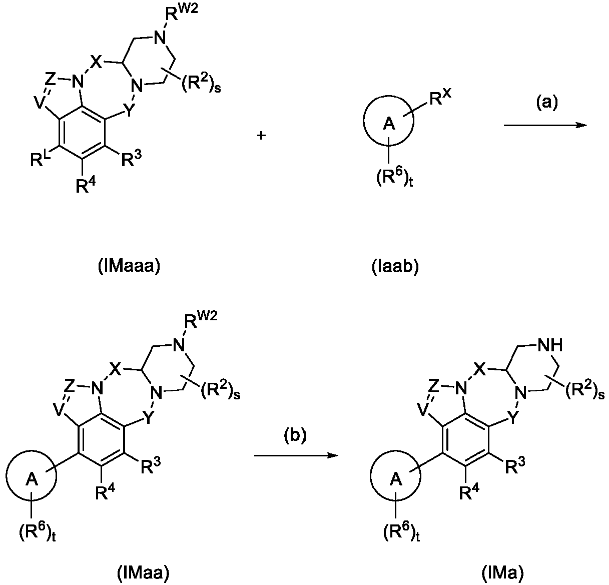
(a) conducting a Suzuki coupling reaction of a compound represented by general formula (IMaaa) or a salt thereof with a compound represented by general formula (Iaab) or a salt thereof to give a compound represented by general formula (IMaa) or a salt thereof; and
(b) removing R^{W2} from the compound represented by general formula (IMaa) or the salt thereof under acidic conditions to give the compound represented by general formula (IMa) or the salt thereof, wherein optionally, when a protecting group is present on the R⁶ group, a step of removing the protecting group from the R⁶ group under acidic conditions is also included before, during, or after the deprotection reaction;
   wherein:
   R^{L} is halogen; preferably, R^{L} is Br;
   R^{W2} is an amino protecting group; preferably, R^{W2} is tert-butyloxycarbonyl;
   R^{X} is selected from the group consisting of 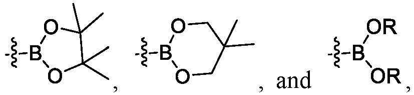 and R is a hydrogen atom or C₁₋₆ alkyl;
   ring A, V, X, Y, Z, R², R³, R⁴, R⁶, s, and t are as defined in general formula (IMa).

### Solution 10-1

A method for preparing the compound represented by general formula (Ia) or the pharmaceutically acceptable salt thereof of the present disclosure, which comprises the following steps: 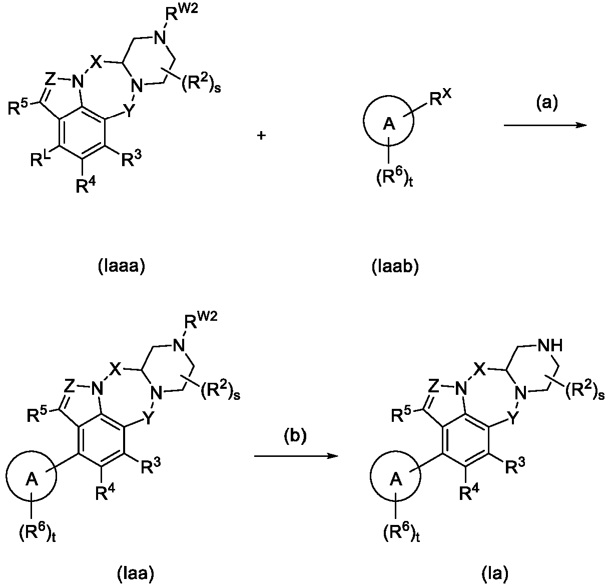
(a) conducting a Suzuki coupling reaction of a compound represented by general formula (Iaaa) or a salt thereof with a compound represented by general formula (Iaab) or a salt thereof to give a compound represented by general formula (Iaa) or a pharmaceutically acceptable salt thereof; and
(b) removing R^{W2} from the compound represented by general formula (Iaa) or the salt thereof under acidic conditions to give the compound represented by general formula (Ia) or the pharmaceutically acceptable salt thereof;
   wherein:
   R^{L} is halogen; preferably, R^{L} is Br;
   R^{W2} is an amino protecting group; preferably, R^{W2} is tert-butyloxycarbonyl;
   R^{X} is selected from the group consisting of 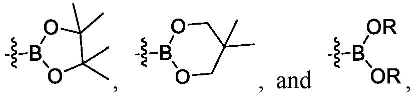 and R is a hydrogen atom or C₁₋₆ alkyl;
   ring A, X, Y, Z, R², R³, R⁴, R⁵, R⁶, s, and t are as defined in general formula (Ia).

### Solution 10-2

A method for preparing the compound represented by general formula (Ia) or the salt thereof of the present disclosure, which comprises the following steps: 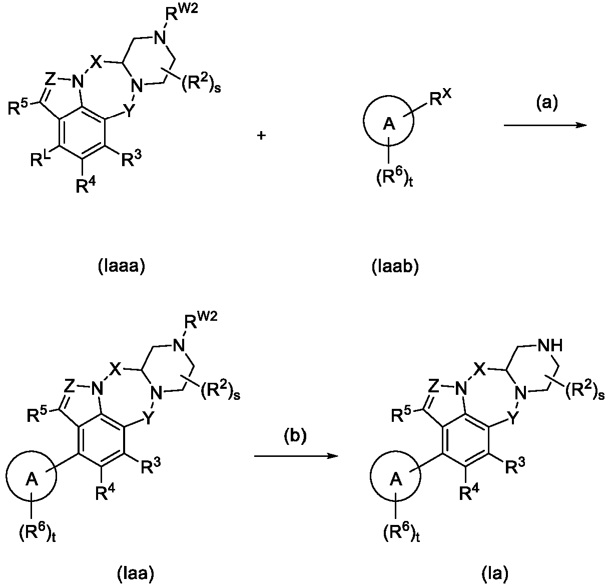
(a) conducting a Suzuki coupling reaction of a compound represented by general formula (Iaaa) or a salt thereof with a compound represented by general formula (Iaab) or a salt thereof to give a compound represented by general formula (Iaa) or a salt thereof; and
(b) removing R^{W2} from the compound represented by general formula (Iaa) or the salt thereof under acidic conditions to give the compound represented by general formula (Ia) or the salt thereof, wherein optionally, when a protecting group is present on the R⁶ group, a step of removing the protecting group from the R⁶ group under acidic conditions is also included before, during, or after the deprotection reaction;
   wherein:
   R^{L} is halogen; preferably, R^{L} is Br;
   R^{W2} is an amino protecting group; preferably, R^{W2} is tert-butyloxycarbonyl;
   R^{X} is selected from the group consisting of 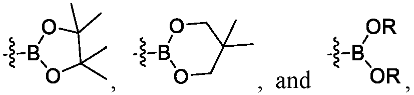 and R is a hydrogen atom or C₁₋₆ alkyl;
   ring A, X, Y, Z, R², R³, R⁴, R⁵, R⁶, s, and t are as defined in general formula (Ia).

### Solution 11-1

A method for preparing the compound represented by general formula (IIa) or the pharmaceutically acceptable salt thereof of the present disclosure, which comprises the following steps: 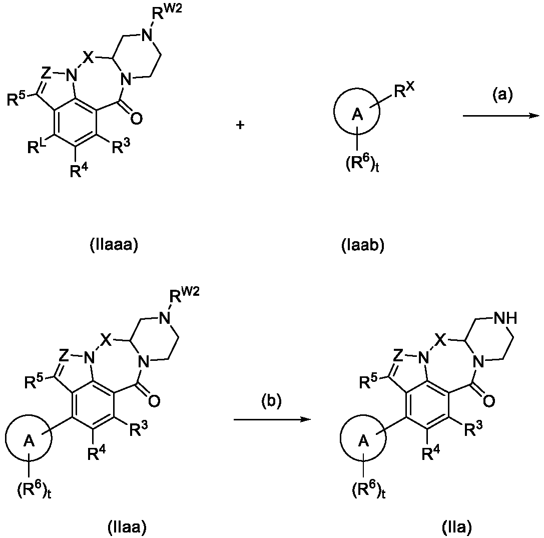
(a) conducting a Suzuki coupling reaction of a compound represented by general formula (IIaaa) or a salt thereof with a compound represented by general formula (Iaab) or a salt thereof to give a compound represented by general formula (IIaa) or a pharmaceutically acceptable salt thereof; and
(b) removing R^{W2} from the compound represented by general formula (IIaa) or the salt thereof under acidic conditions to give the compound represented by general formula (IIa) or the pharmaceutically acceptable salt thereof;
   wherein:
   R^{L} is halogen; preferably, R^{L} is Br;
   R^{W2} is an amino protecting group; preferably, R^{W2} is tert-butyloxycarbonyl;
   R^{X} is selected from the group consisting of 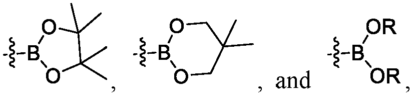 and R is a hydrogen atom or C₁₋₆ alkyl;
   ring A, X, Z, R³, R⁴, R⁵, R⁶, and t are as defined in general formula (IIa).

### Solution 11-2

A method for preparing the compound represented by general formula (IIa) or the salt thereof of the present disclosure, which comprises the following steps: 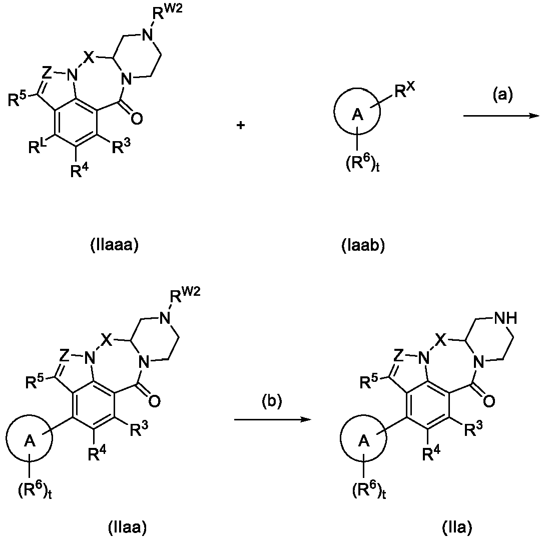
(a) conducting a Suzuki coupling reaction of a compound represented by general formula (IIaaa) or a salt thereof with a compound represented by general formula (Iaab) or a salt thereof to give a compound represented by general formula (IIaa) or a salt thereof; and
(b) removing R^{W2} from the compound represented by general formula (IIaa) or the salt thereof under acidic conditions to give the compound represented by general formula (IIa) or the salt thereof, wherein optionally, when a protecting group is present on the R⁶ group, a step of removing the protecting group from the R⁶ group under acidic conditions is also included before, during, or after the deprotection reaction;
   wherein:
   R^{L} is halogen; preferably, R^{L} is Br;
   R^{W2} is an amino protecting group; preferably, R^{W2} is tert-butyloxycarbonyl;
   R^{X} is selected from the group consisting of 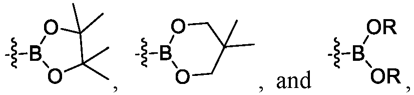 and R is a hydrogen atom or C₁₋₆ alkyl;
   ring A, X, Z, R³, R⁴, R⁵, R⁶, and t are as defined in general formula (IIa).

### Solution 12

A method for preparing the compound represented by general formula (IIIa) or the pharmaceutically acceptable salt thereof of the present disclosure, which comprises the following steps: 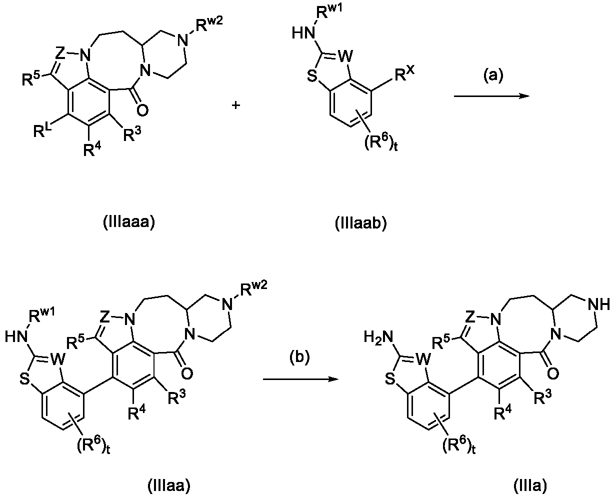
(a) conducting a Suzuki coupling reaction of a compound represented by general formula (IIIaaa) or a salt thereof with a compound represented by general formula (IIIaab) or a salt thereof to give a compound represented by general formula (IIIaa) or a pharmaceutically acceptable salt thereof; and
(b) removing R^{w1} and R^{w2} from the compound represented by general formula (IIIaa) or the salt thereof under acidic conditions to give the compound represented by general formula (IIIa) or the pharmaceutically acceptable salt thereof;
   wherein:
   R^{L} is halogen; preferably, R^{L} is Br;
   R^{W1} and R^{W2} are both amino protecting groups; preferably, R^{W1} and R^{W2} are both tert-butyloxycarbonyl;
   R^{X} is selected from the group consisting of 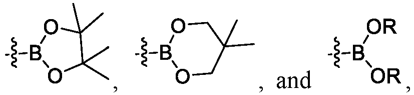 and R is a hydrogen atom or C₁₋₆ alkyl;
   W, Z, R³, R⁴, R⁵, R⁶, and t are as defined in general formula (IIIa).

### Solution 13

A method for preparing the compound represented by general formula (III-1a) or the pharmaceutically acceptable salt thereof of the present disclosure, which comprises the following steps: 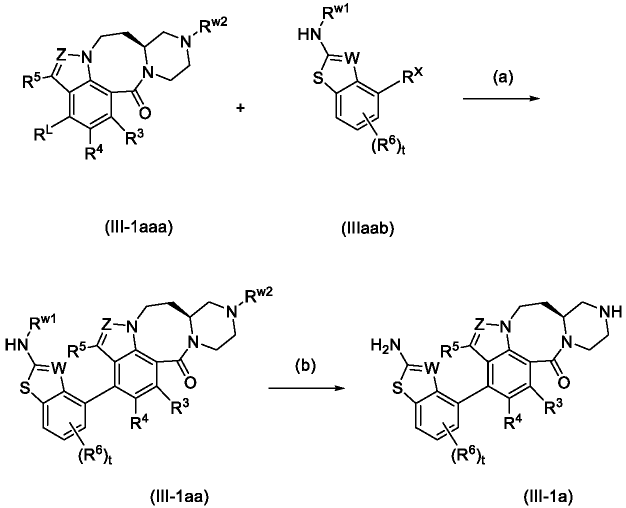
(a) conducting a Suzuki coupling reaction of a compound represented by general formula (III-1aaa) or a salt thereof with a compound represented by general formula (IIIaab) or a salt thereof to give a compound represented by general formula (III-1aa) or a pharmaceutically acceptable salt thereof; and
(b) removing R^{W1} and R^{W2} from the compound represented by general formula (III-1aa) or the salt thereof under acidic conditions to give the compound represented by general formula (III-Ia) or the pharmaceutically acceptable salt thereof;
   wherein:
   R^{L} is halogen; preferably, R^{L} is Br;
   R^{W1} and R^{W2} are both amino protecting groups; preferably, R^{W1} and R^{W2} are both tert-butyloxycarbonyl;
   R^{X} is selected from the group consisting of 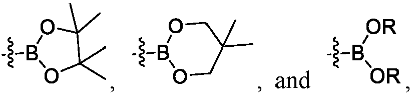 and R is a hydrogen atom or C₁₋₆ alkyl;
   W, Z, R³, R⁴, R⁵, R⁶, and t are as defined in general formula (III-1a).

### Solution 14

A method for preparing the compound represented by general formula (III-2a) or the pharmaceutically acceptable salt thereof of the present disclosure, which comprises the following steps: 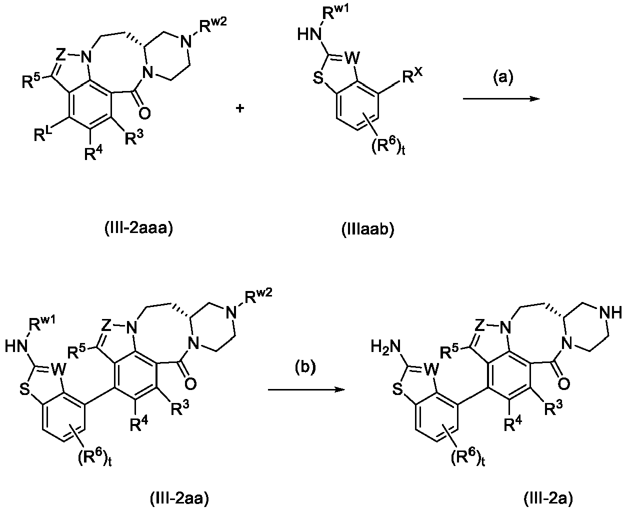
(a) conducting a Suzuki coupling reaction of a compound represented by general formula (III-2aaa) or a salt thereof with a compound represented by general formula (IIIaab) or a salt thereof to give a compound represented by general formula (III-2aa) or a pharmaceutically acceptable salt thereof; and
(b) removing R^{W1} and R^{W2} from the compound represented by general formula (III-2aa) or the salt thereof under acidic conditions to give the compound represented by general formula (III-2a) or the pharmaceutically acceptable salt thereof;
   wherein:
   R^{L} is halogen; preferably, R^{L} is Br;
   R^{W1} and R^{W2} are both amino protecting groups; preferably, R^{W1} and R^{W2} are both tert-butyloxycarbonyl;
   R^{X} is selected from the group consisting of 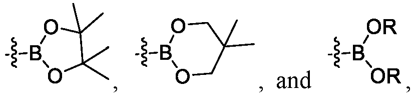 and R is a hydrogen atom or C₁₋₆ alkyl;
   W, Z, R³, R⁴, R⁵, R⁶, and t are as defined in general formula (III-2a).

In the above synthesis solutions, the Suzuki coupling reactions are preferably carried out in the presence of a base (e.g., potassium carbonate or cesium carbonate) and a metal catalyst
(e.g., dichloro[bis(diphenylphosphinophenyl)ether]palladium(II) or dichloro[1,1'-bis(di-tert-butylphosphino)ferrocene]palladium(II)).

In the above synthesis solutions, bases that provide the alkaline conditions include organic bases and inorganic bases. The organic bases include,
but are not limited to, triethylamine, N,N-diisopropylethylamine, n-butyllithium, lithium diisopropylamide, potassium acetate, sodium acetate, sodium ethoxide, sodium tert-butoxide, or potassium tert-butoxide; triethylamine is preferred. The inorganic bases include, but are not limited to, sodium hydride, potassium phosphate, sodium carbonate, potassium carbonate, anhydrous potassium carbonate, cesium carbonate, sodium hydroxide, lithium hydroxide monohydrate, lithium hydroxide, and potassium hydroxide; potassium carbonate or anhydrous potassium carbonate is preferred.

In the above synthesis solutions, acids that provide the acidic conditions include organic acids and inorganic acids. The organic acids include,
but are not limited to, trifluoroacetic acid, formic acid, acetic acid, methanesulfonic acid, p-toluenesulfonic acid, Me₃SiCl, and TMSOTf; trifluoroacetic acid is preferred. The inorganic acids include, but are not limited to, hydrogen chloride, a solution of hydrogen chloride in 1,4-dioxane, hydrochloric acid, sulfuric acid, nitric acid, and phosphoric acid.

The above synthesis solutions are preferably carried out in solvents, including but not limited to: ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylacetamide, N,N-dimethylformamide, and mixtures thereof.

### DETAILED DESCRIPTION

The present disclosure is further described below with reference to examples; however, these examples are not intended to limit the scope of the present disclosure.

### Examples

The structures of the compounds were determined by nuclear magnetic resonance (NMR) spectroscopy or/and mass spectrometry (MS). The NMR shifts (δ) are given in 10⁻⁶ (ppm). The NMR analyses were performed on a Bruker AVANCE-400 nuclear magnetic resonance instrument or Bruker AVANCE NEO 500M, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed on an Agilent 1200/1290 DAD-6110/6120 Quadrupole MS liquid chromatography-mass spectrometry system (manufacturer: Agilent; MS model: 6110/6120 Quadrupole MS); Waters ACQuity UPLC-QD/SQD (manufacturer: Waters; MS model: Waters ACQuity Qda Detector/Waters SQ Detector); and THERMO Ultimate 3000-Q Exactive (manufacturer: THERMO; MS model: THERMO Q Exactive).

The high performance liquid chromatography (HPLC) analyses were performed on Agilent HPLC 1200DAD, Agilent HPLC 1200VWD, and Waters HPLC e2695-2489 high performance liquid chromatographs.

The chiral HPLC analyses were performed on an Agilent 1260 DAD high performance liquid chromatograph.

The preparative high performance liquid chromatography steps were performed on Waters 2545-2767, Waters 2767-SQ Detecor2, Shimadzu LC-20AP, and Gilson GX-281 preparative chromatographs.

The preparative chiral chromatography steps were performed on a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) analyses had a layer thickness of 0.15 mm-0.2 mm, and those used in the thin-layer chromatography separations and purifications had a layer thickness of 0.4 mm-0.5 mm.

In the silica gel column chromatography steps, a 200-300 mesh silica gel (Huanghai, Yantai) was generally used as a carrier.

The kinase mean inhibition rates and IC₅₀ values were measured using a NovoStar microplate reader (BMG, Germany).

The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions refer to aqueous solutions unless otherwise specified.

In the examples, the reaction temperature was room temperature, i.e., 20 °C-30 °C, unless otherwise specified.

The monitoring of reaction progress in the examples was performed using thin-layer chromatography (TLC). The developing solvents used in the reactions, the eluent systems used in the column chromatography purifications and the developing solvent systems used in the thin-layer chromatography analyses include: A: a petroleum ether/ethyl acetate system, and B: a dichloromethane/methanol system. The volume ratio of the solvents was adjusted depending on the polarity of a compound, or by adding a small amount of a basic or acidic reagent such as triethylamine and acetic acid.

### Example 1-P1, 1-P2

### (R)-4-((S)-10-Acryloyl-2-fluoro-14-oxo-8,8a,9,10,11,12-hexahydro-7H,14H-pyrazino[1' ,2':5,6][1,5]diazocino[3,2,1-hi]indazol-3-yl)-2-amino-7-fluorobenzo[b]thiophene-3-carb onitrile 1-P1

### (S)-4-((S)-10-Acryloyl-2-fluoro-14-oxo-8,8a,9,10,11,12-hexahydro-7H,14H-pyrazino[1' ,2':5,6][1,5]diazocino[3,2,1-hi]indazol-3-yl)-2-amino-7-fluorobenzo[b]thiophene-3-carb onitrile 1-P2

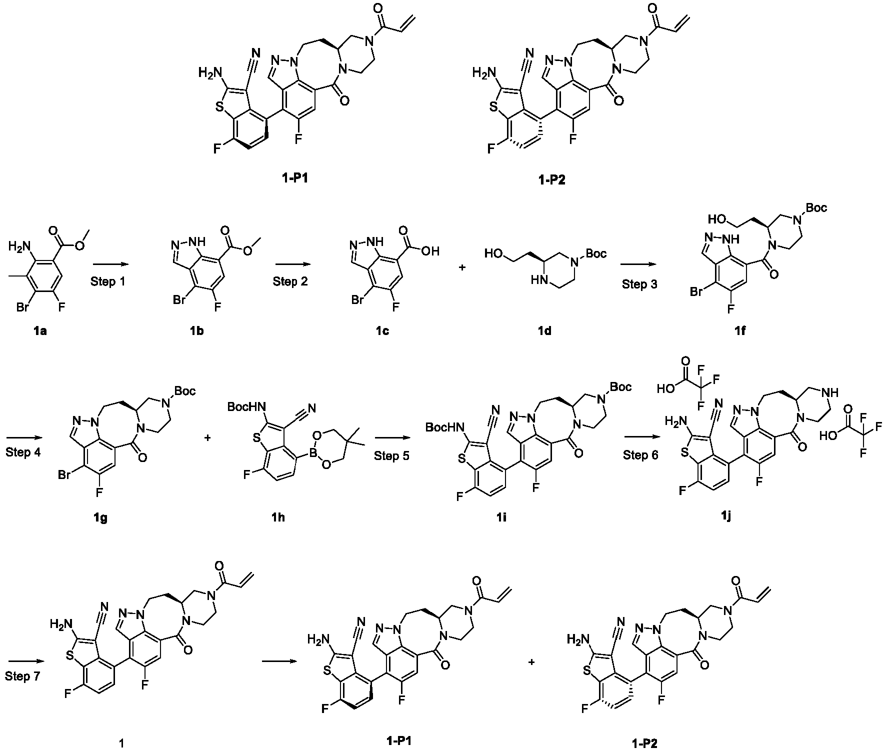

### Step 1

### Methyl 4-bromo-5-fluoro-1H-indazole-7-carboxylate 1b

Methyl 2-amino-4-bromo-5-fluoro-3-methylbenzoate **1a** (2.7 g, 10.3 mmol, prepared using the method disclosed in step (iv) on page 103 of the specification of the patent application "WO2016020836A1") was dissolved in chloroform (50 mL), and acetic anhydride (3.16 g, 30.9 mmol) was added. After the mixture was stirred for 90 min with the temperature maintained at below 40 °C, potassium acetate (302 mg, 3.08 mmol) and tert-butyl nitrite (2.12 g, 20.6 mmol) were added. The mixture was refluxed for 14 h. The reaction mixture was cooled to room temperature, diluted with dichloromethane (50 mL), and washed with water (30 mL), saturated sodium carbonate solution (10 mL), and saturated sodium chloride solution (10 mL) in sequence. The organic phase was dried over anhydrous sodium sulfate and filtered. The filtrate was concentrated under reduced pressure to give a crude product of the title compound **1b** (2.8 g). The product was directly used in the next step without purification.

MS m/z (ESI): 272.9 [M+1].

### Step 2

### 4-Bromo-5-fluoro-1H-indazole-7-carboxylic acid 1c

The crude product of compound **1b** (2.8 g, 10.25 mmol) was dissolved in a mixed solvent of tetrahydrofuran (20 mL), methanol (10 mL), and water (10 mL), and lithium hydroxide (2.15 g, 51.26 mmol) was added. The mixture was stirred at 40 °C for 1 h. The reaction mixture was concentrated under reduced pressure to remove most of the solvent. Water was added, and extraction was performed with ethyl acetate (150 mL × 6). The organic phases were combined, washed with dilute hydrochloric acid and saturated sodium chloride solution in sequence, and dried over anhydrous sodium sulfate, and the drying agent was removed by filtration. The filtrate was concentrated under reduced pressure to give a crude product of compound **1c** (1.4 g). The product was directly used in the next step without purification.

MS m/z (ESI): 259.0 [M+1].

### Step 3

### tert-Butyl (S)-4-(4-bromo-5-fluoro-1H-indazole-7-carbonyl)-3-(2-hydroxyethyl)piperazine-1-carb oxylate 1f

The crude product of compound **1c** (600 mg, 2.32 mmol) and tert-butyl (*S*)-3-(2-hydroxyethyl)piperazine-1-carboxylate **1d** (586 mg, 2.54 mmol, prepared using the method disclosed in preparation 65 on page 80 of the specification of the patent application "WO2021118877A1") were dissolved in 30 mL of *N,N-*dimethylformamide, and *N,N*-diisopropylethylamine (898 mg, 6.95 mmol) and 2-(7-azabenzotriazole)-*N,N,N',N'*-tetramethyluronium hexafluorophosphate (1.05 g, 2.78 mmol) were added under an ice bath. The mixture was stirred for 1 h. The reaction mixture was concentrated under reduced pressure, then diluted with ethyl acetate, and washed with water, saturated sodium carbonate solution, and saturated sodium chloride solution in sequence. The organic phase was dried over anhydrous sodium sulfate and filtered. The filtrate was concentrated under reduced pressure, and the residue was purified by silica gel column chromatography with eluent system A to give the title compound **1f** (600 mg, yield: 54.9%).

MS m/z (ESI): 415.2 [M-55].

### Step 4

### tert-Butyl (S)-3-bromo-2-fluoro-14-oxo-7,8,8a,9,11,12-hexahydro-10H,14H-pyrazino[1',2':5,6][1, 5]diazocino[3,2,1-hi]indazole-10-carboxylate 1g

Compound **1f** (600 mg, 1.27 mmol) was dissolved in 36 mL of tetrahydrofuran in a nitrogen atmosphere, and triphenylphosphine (667 mg, 2.54 mmol) and diethyl azodicarboxylate (514 mg, 2.54 mmol) were sequentially added under an ice bath. The mixture was stirred for 30 min with the temperature maintained. The reaction mixture was quenched with saturated ammonium chloride solution and extracted with ethyl acetate (15 mL × 2). The organic phases were combined, washed with saturated sodium chloride solution, dried over anhydrous sodium sulfate, and filtered. The filtrate was concentrated under reduced pressure, and the residue was purified by silica gel column chromatography with eluent system A to give the title compound **1g** (577 mg, yield: 99.9%).

MS m/z (ESI): 397.2 [M-55].

### Step 5

### tert-Butyl (8aS)-3-(2-((tert-butoxycarbonyl)amino)-3-cyano-7-fluorobenzo[b]thiophen-4-yl)-2-flu oro-14-oxo-7,8,8a,9,11,12-hexahydro-10H,14H-pyrazino[1',2':5,6][1,5]diazocino[3,2,1-hi]indazole-10-carboxylate 1i

Compound **1g** (80 mg, 0.17 mmol) and tert-butyl (3-cyano-4-(5,5-dimethyl-1,3,2-dioxaborinan-2-yl)-7-fluorobenzo[*b*]thiophen-2-yl)carb amate **1h** (107 mg, 0.26 mmol, prepared using the method disclosed in preparation 15 on page 50 of the specification of the patent application "WO2021118877A1") were dissolved in 2 mL of toluene, and dichloro[bis(diphenylphosphinophenyl)ether]palladium(II) (18 mg, 0.025 mmol, Shanghai Titan) and cesium carbonate (115 mg, 0.35 mmol) were added. The system was purged with nitrogen gas, and the mixture was stirred at 105 °C for 6 h. The reaction mixture was cooled to room temperature and then filtered. The filtrate was concentrated under reduced pressure, and the residue was purified by silica gel column chromatography with eluent system A to give the title compound **1i** (50 mg, yield: 42.6%).

MS m/z (ESI): 665.0 [M+1].

### Step 6

### 2-Amino-7-fluoro-4-((S)-2-fluoro-14-oxo-8,8a,9,10,11,12-hexahydro-7H,14H-pyrazino [1',2':5,6][1,5]diazocino[3,2,1-hi]indazol-3-yl)benzo[b]thiophene-3-carbonitrile bis(2,2,2-trifluoroacetate) 1j

Compound **1i** (50 mg, 0.075 mmol) was dissolved in 1 mL of dichloromethane, and 1 mL of trifluoroacetic acid was added at 0 °C. The mixture was stirred for 2 h. The reaction mixture was concentrated under reduced pressure to give a crude product of the title compound **1j** (52 mg). The product was directly used in the next step without purification.

MS m/z (ESI): 465.4 [M+1].

### Step 7

### (R)-4-((S)-10-Acryloyl-2-fluoro-14-oxo-8,8a,9,10,11,12-hexahydro-7H,14H-pyrazino[1' ,2':5,6][1,5]diazocino[3,2,1-hi]indazol-3-yl)-2-amino-7-fluorobenzo[b]thiophene-3-carb onitrile 1-P1

### (S)-4-((S)-10-Acryloyl-2-fluoro-14-oxo-8,8a,9,10,11,12-hexahydro-7H,14H-pyrazino[1' ,2':5,6][1,5]diazocino[3,2,1-hi]indazol-3-yl)-2-amino-7-fluorobenzo[b]thiophene-3-carb onitrile 1-P2

Compound **1j** (52 mg, 0.075 mmol) was suspended in 2 mL of ethyl acetate, 1 mL of tetrahydrofuran, and 2 mL of water, and anhydrous potassium carbonate (52 mg, 0.37 mmol) was added. Acryloyl chloride (7 mg, 0.077 mmol) was added under an ice bath. After 5 min of reaction, extraction was performed with ethyl acetate (5 mL × 2). The organic phases were combined and concentrated under reduced pressure to give a crude product of compound **1,** i.e., 4-((*S*)-10-acryloyl-2-fluoro-14-oxo-8,8*a*,9,10,11,12-hexahydro-7*H*,14*H*-pyrazino[1',2':5,6][1,5]diazocino[3,2,1-*hi*]indazol-3-yl)-2-amino-7-f luorobenzo[b]thiophene-3-carbonitrile (39 mg). The crude product was purified by preparative high performance liquid chromatography (Waters-2545; column: YMC Triart-Exrs, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compounds **1-P2** (5 mg, yield: 12.7%) and **1-P1** (3 mg, yield: 7.6%).
Single-configuration compound (shorter retention time) **1-P2** (5 mg, yield: 12.7%)

MS m/z (ESI): 519.3 [M+1].

HPLC analysis: retention time: 1.12 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD3OD): δ 7.70 (s, 1H), 7.66 (d, 1H), 7.30 (s, 1H), 7.08 (dd, 1H), 6.82 (dt, 1H), 6.34-6.25 (m, 1H), 5.83 (t, 1H), 4.75 (d, 3H), 4.49-4.19 (m, 2H), 4.16-3.93 (m, 1H), 3.57-3.44 (m, 1H), 3.22-3.06 (m, 2H), 2.25 (dd, 1H), 2.06-1.92 (m, 1H).
Single-configuration compound (longer retention time) **1-P1** (3 mg, yield: 7.6%)

MS m/z (ESI): 519.5 [M+1].

LCMS analysis: retention time: 1.13 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD3OD): δ 7.65 (s, 1H), 7.61 (d, 1H), 7.38 (dd, 1H), 7.13-6.99 (m, 1H), 6.95-6.68 (m, 1H), 6.35-6.28 (m, 1H), 5.84 (d, 1H), 4.75 (d, 4H), 4.39-4.23 (m, 1H), 4.16-3.98 (m, 1H), 3.25-2.95 (m, 3H), 2.24 (d, 1H), 1.94 (d, 1H).

### Example 2

### 4-((S)-10-Acryloyl-2-fluoro-14-oxo-8,8a,9,10,11,12-hexahydro-7H,14H-pyrazino[1',2': 5,6][1,5]diazocino[3,2,1-hi]indol-3-yl)-2-amino-7-fluorobenzo[b]thiophene-3-carbonitri le2

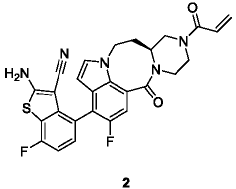

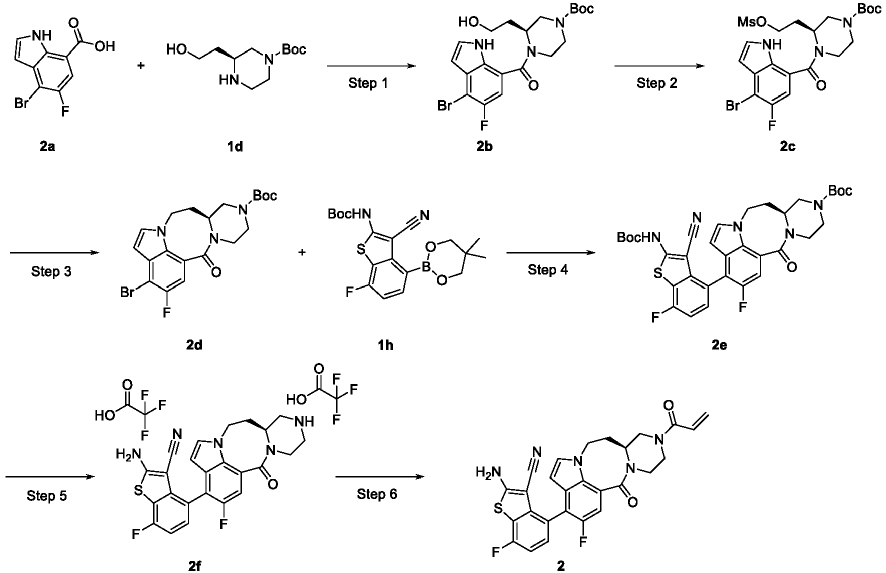

### Step 1

### tert-Butyl (S)-4-(4-bromo-5-fluoro-1H-indole-7-carbonyl)-3-(2-hydroxyethyl)piperazine-I-carbox ylate 2b

4-Bromo-5-fluoro-1*H*-indole-7-carboxylic acid **2a** (180 mg, 0.69 mmol, prepared using the method disclosed in paragraph [0152] on page 22 of the specification of the patent application "US20190300526A1") and compound **1d** (160 mg, 0.69 mmol) were dissolved in 3 mL of *N,N-*dimethylformamide, and *N*,*N*-diisopropylethylamine (270 mg, 2.0 mmol) and 2-(7-azabenzotriazole)-*N*,*N*,*N'*,*N'*-tetramethyluronium hexafluorophosphate (318 mg, 0.83 mmol) were added under an ice bath. The mixture was stirred for 1 h. The reaction mixture was concentrated under reduced pressure, and the residue was purified by silica gel column chromatography with eluent system A to give the title compound **2b** (328 mg, yield: 99.9%).

MS m/z (ESI): 469.9 [M+1].

### Step 2

### tert-Butyl (S)-4-(4-bromo-5-fluoro-1H-indole-7-carbonyl)-3-(2-((methylsulfonyl)oxy)ethyl)pipera zine-1-carboxylate 2c

Compound **2b** (328 mg, 0.69 mmol) was dissolved in 5 mL of dichloromethane, and *N*,*N*-diisopropylethylamine (216 mg, 1.67 mmol) was added. Methanesulfonyl chloride (119 mg, 1.04 mmol) was added under an ice bath, and the mixture was stirred for 2 h. Water was added to the reaction mixture, and extraction was performed with dichloromethane (5 mL × 3). The organic phases were combined, washed with saturated sodium chloride solution, and dried over anhydrous sodium sulfate, and the drying agent was removed by filtration. The filtrate was concentrated under reduced pressure to give a crude product of the title compound **2c** (382 mg). The product was directly used in the next step without purification.

MS m/z (ESI): 392.3 [M-55].

### Step 3

### tert-Butyl (S)-3-bromo-2-fluoro-14-oxo-7,8,8a,9,11,12-hexahydro-10H,14H-pyrazino[1',2':5,6][1, 5]diazocino[3,2,1-hi]indole-10-carboxylate 2d

The crude product of compound **2c** (382 mg, 0.69 mmol) was dissolved in 5 mL of *N*,*N*-dimethylformamide, and sodium hydride (53 mg, 1.38 mmol, 60% pure) was added. The mixture was stirred for 1 h. The reaction mixture was quenched with saturated ammonium chloride solution and extracted with ethyl acetate (5 mL × 3). The organic phases were combined, washed with saturated sodium chloride solution, and dried over anhydrous sodium sulfate, and the drying agent was removed by filtration. The liquid was concentrated under reduced pressure, and the residue was purified by silica gel column chromatography with eluent system A to give the title compound **2d** (160 mg, yield: 50.7%).

MS m/z (ESI): 452.0 [M+1].

### Step 4

### tert-Butyl

### (8aS)-3-(2-((tert-butoxycarbonyl)amino)-3-cyano-7-fluorobenzo[b]thiophen-4-yl)-2-flu oro-14-oxo-7,8,8a,9,11,12-hexahydro-10H,14H-pyrazino[1',2":5,6][1,5]diazocino[3,2,1-hi]indole-10-carboxylate 2e

Compound **2d** (80 mg, 0.17 mmol) and compound **1h** (105 mg, 0.26 mmol) were dissolved in 2 mL of toluene, and dichloro[bis(diphenylphosphinophenyl)ether]palladium(II) (20 mg, 0.027 mmol) and cesium carbonate (115 mg, 0.35 mmol, Shanghai Accela ChemBio) were added. The system was purged with nitrogen gas, and the mixture was heated to 105 °C and stirred for 6 h. The reaction mixture was filtered. The filtrate was concentrated under reduced pressure, and the residue was purified by silica gel column chromatography with eluent system A to give the title compound **2e** (100 mg, yield: 85%).

MS m/z (ESI): 664.0 [M+1].

### Step 5

### 2-Amino-7-fluoro-4-((S)-2-fluoro-14-oxo-8,8a,9,10,11,12-hexahydro-7H,14H-pyrazino [1',2':5,6][1,5]diazocino[3,2,1-hi]indol-3-yl)benzo[b]thiophene-3-carbonitrile bis(2,2,2-trifluoroacetate) 2f

Compound **2e** (100 mg, 0.15 mmol) was dissolved in 1 mL of dichloromethane, and 1 mL of trifluoroacetic acid was added at 0 °C. The mixture was stirred for 2 h. The reaction mixture was concentrated under reduced pressure to give a crude product of the title compound **2f** (104 mg). The product was directly used in the next step without purification.

MS m/z (ESI): 464.0 [M+1].

### Step 6

### 4-((S)-10-Acryloyl-2-fluoro-14-oxo-8,8a,9,10,11,12-hexahydro-7H,14H-pyrazino[1',2': 5,6][1,5]diazocino[3,2,1-hi]indol-3-yl)-2-amino-7-fluorobenzo[b]thiophene-3-carbonitri le 2

Compound **2f** (104 mg, 0.15 mmol) was suspended in a mixed solvent of 2 mL of ethyl acetate, 1 mL of tetrahydrofuran, and 2 mL of water, and anhydrous potassium carbonate (85 mg, 0.65 mmol) was added. Acryloyl chloride (13 mg, 0.14 mmol) was added under an ice bath. The mixture was left to react for 5 min with the temperature maintained. The reaction mixture was extracted with ethyl acetate (5 mL × 3). The organic phase was concentrated under reduced pressure, and the residue was purified by high performance liquid chromatography (Waters-2545, column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compound **2** (30 mg, yield: 38.5%).

MS m/z (ESI): 518.0 [M+1].

¹H NMR (500 MHz, CD3OD): δ 7.37-7.24 (m, 2H), 7.17 (d, 1H), 7.01 (t, 1H), 6.80 (ddd, 1H), 6.29 (dd, 1H), 6.11 (d, 1H), 5.82 (dd,1H), 4.72 (d, 2H), 4.36 (d, 2H), 4.15-4.00 (m, 2H), 3.46 (d, 1H), 3.06 (dd, 2H), 2.26-2.13 (m, 1H), 1.86 (d, 1H).

### Example 3

### (8aS)-10-Acryloyl-3-(-2-amino-7-fluorobenzo[d]thiazol-4-yl)-2-fluoro-8,8a,9,10,11,12-hexahydro-7H,14H-pyrazino[1',2':5,6][1,5]diazocino[3,2,1-hi]indol-14-one 3

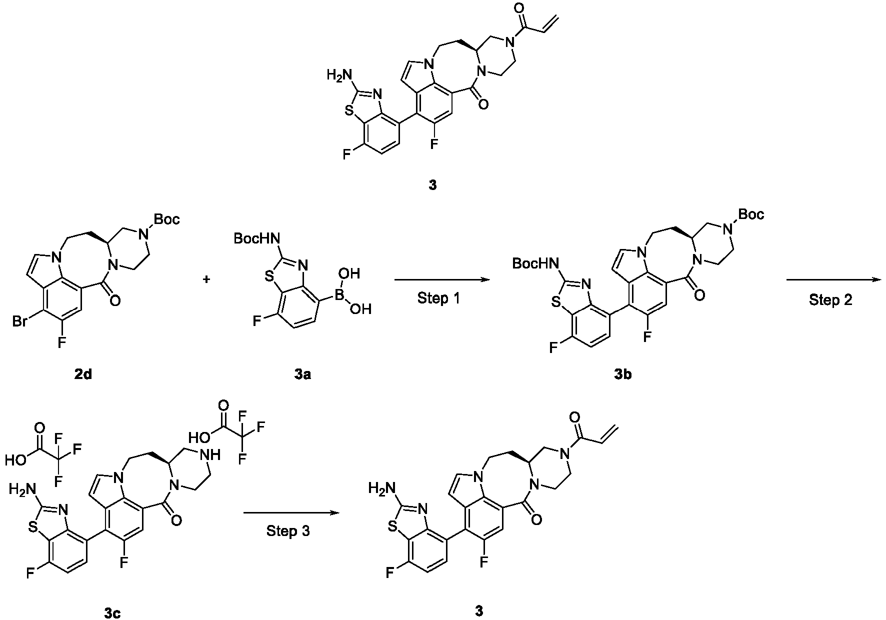

### Step 1

### tert-Butyl (8aS)-3-(2-((tert-butoxycarbonyl)amino)-7-fluorobenzo[d]thiazol-4-yl)-2-fluoro-14-oxo -7,8,8a,9,11,12-hexahydro-10H,14H-pyrazino[1',2':5,6][1,5]diazocino[3,2,1-hi]indole-1 0-carboxylate 3b

Compound **2d** (30 mg, 0.07 mmol) and (2-((tert-butoxycarbonyl)amino)-7-fluorobenzo[*d*]thiazol-4-yl)boronic acid **3a** (31 mg, 0.1 mmol, prepared using the method disclosed in preparation 25 on pages 56-57 of the specification of the patent application "WO2021118877A1") were dissolved in 1 mL of dioxane and 0.3 mL of water, and dichloro[1,1'-bis(di-tert-butylphosphino)ferrocene]palladium(II) (4 mg, 0.006 mmol, Shanghai Titan) and potassium carbonate (14 mg, 0.1 mmol) were added. The system was purged with nitrogen gas, and the mixture was heated to 80 °C and stirred for 1 h. The reaction mixture was cooled to room temperature and then filtered. The filtrate was concentrated under reduced pressure, and the residue was purified by silica gel column chromatography with eluent system A to give the title compound **3b** (42 mg, yield: 98.9%).

MS m/z (ESI): 640.0 [M+1].

### Step 2

### (8aS)-3-(2-Amino-7-fluorobenzo[d]thiazol-4-yl)-2-fluoro-8,8a,9,10,11,12-hexahydro-7 H,14H-pyrazino[1',2':5,6][1,5]diazocino[3,2,1-hi]indol-14-one bis(2,2,2-trifluoroacetate) 3c

Compound **3b** (42 mg, 0.065 mmol) was dissolved in 1 mL of dichloromethane, and 1 mL of trifluoroacetic acid was added at 0 °C. The mixture was stirred for 2 h. The reaction mixture was concentrated under reduced pressure to give a crude product of the title compound **3c** (50 mg). The product was directly used in the next step without purification.

MS m/z (ESI): 440.4 [M+1].

### Step 3

### (8aS)-10-Acryloyl-3-(-2-amino-7-fluorobenzo[d]thiazol-4-yl)-2-fluoro-8,8a,9,10,11,12-hexahydro-7H,14H-pyrazino[1',2':5,6][1,5]diazocino[3,2,1-hi]indol-14-one 3

The crude product of compound **3c** (50 mg, 0.075 mmol) was dissolved in 1 mL of dichloromethane, and triethylamine (36 mg, 0.35 mmol) was added at -78 °C. Acryloyl chloride (6 mg, 0.07 mmol) was added, and the mixture was left to react for 1 h. Extraction was performed with dichloromethane (5 mL × 2). The organic phase was concentrated under reduced pressure, and the residue was purified by high performance liquid chromatography (Waters-2545, column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compound **3** (10 mg, yield: 30.7%).

MS m/z (ESI): 494.3 [M+1].

¹H NMR (500 MHz, CD3OD): δ 7.39-7.23 (m, 3H), 7.00 (q, 1H), 6.97-6.68 (m, 1H), 6.30 (d, 1H), 6.21-6.10 (m, 1H), 5.83 (t, 1H), 4.73 (d, 2H), 4.38 (q, 2H), 4.24-3.97 (m, 2H), 3.49 (d, 1H), 3.03 (d, 2H), 2.21 (t, 1H), 1.88 (s, 1H).

### Example 4

### 4-((S)-10-Acryloyl-2-fluoro-5-methyl-14-oxo-8,8a,9,10,11,12-hexahydro-7H,14H-pyra zino[1',2':5,6][1,5]diazocino[3,2,1-hi]indol-3-yl)-2-amino-7-fluorobenzo[b]thiophene-3-carbonitrile 4

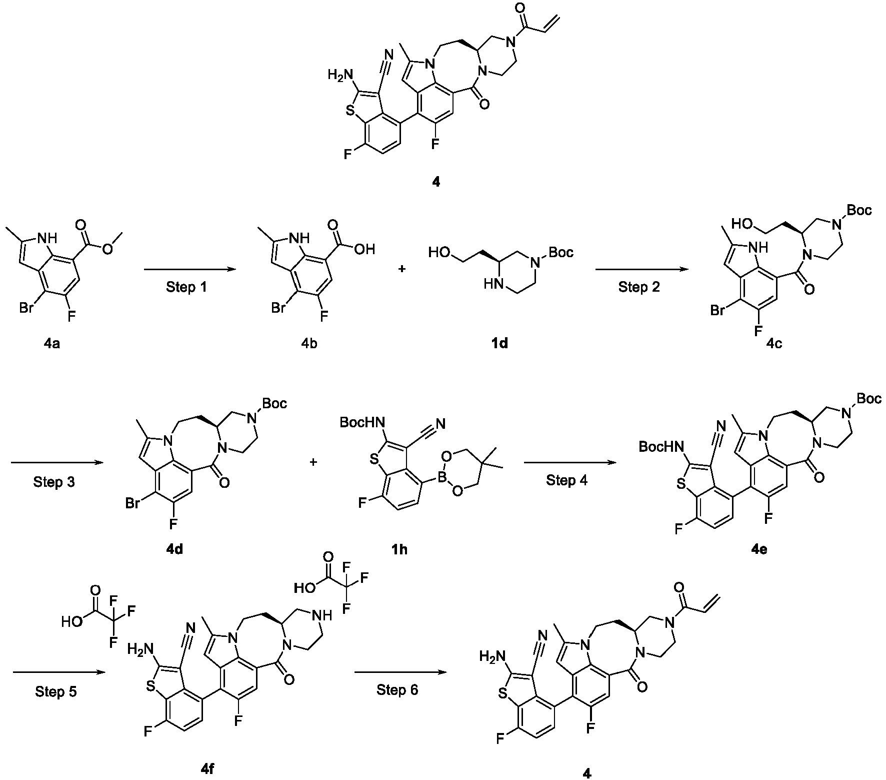

### Step 1

### 4-Bromo-5-fluoro-2-methyl-1H-indole-7-carboxylic acid 4b

Methyl 4-bromo-5-fluoro-2-methyl-1*H*-indole-7-carboxylate **4a** (350 mg, 1.22 mmol, prepared using the method disclosed in example 9.1 on pages 93-95 of the specification of the patent application "WO2018203302A1") was dissolved in a mixed solvent of 4 mL of tetrahydrofuran, 2 mL of methanol, and 2 mL of water, and lithium hydroxide (260 mg, 6.19 mmol) was added. The mixture was stirred at 40 °C for 1 h. The reaction mixture was concentrated under reduced pressure to remove most of the solvent, made slightly acidic with 6 M hydrochloric acid, and then extracted with ethyl acetate (10 mL × 3). The organic phases were combined, washed with saturated sodium chloride solution, dried over anhydrous sodium sulfate, and filtered. The filtrate was concentrated under reduced pressure to give a crude product of the title compound **4b** (300 mg).

MS m/z (ESI): 272.0 [M+1].

### Step 2

### tert-Butyl (S)-4-(4-bromo-5-fluoro-2-methyl-1H-indole-7-carbonyl)-3-(2-hydroxyethyl)piperazine -1-carboxylate 4c

The crude product of compound **4b** (330 mg, 1.21 mmol) and compound **1d** (307 mg, 1.33 mmol) were dissolved in 6 mL of *N*,*N*-dimethylformamide, and *N*,*N*-diisopropylethylamine (470 mg, 3.64 mmol) and 2-(7-azabenzotriazole)-*N*,*N*,*N'*,*N'*-tetramethyluronium hexafluorophosphate (553 g, 1.45 mmol) were added under an ice bath. The reaction mixture was stirred for 1 h. The reaction mixture was concentrated under reduced pressure, and the residue was diluted with ethyl acetate and washed with water, saturated sodium carbonate solution, and saturated sodium chloride solution in sequence. The organic phase was dried over anhydrous sodium sulfate, and the drying agent was removed by filtration. The filtrate was concentrated under reduced pressure, and the residue was purified by silica gel column chromatography with eluent system A to give the title compound **4c** (250 mg, yield: 42.5%).

MS m/z (ESI): 484.2 [M+1].

### Step 3

### tert-Butyl (S)-3-bromo-2-fluoro-5-methyl-14-oxo-7,8,8a,9,11,12-hexahydro-10H,14H-pyrazino[1', 2':5,6][1,5]diazocino[3,2,1-hi]indole-10-carboxylate 4d

Compound **4c** (249 mg, 0.51 mmol) was dissolved in 5 mL of tetrahydrofuran in a nitrogen atmosphere, and triphenylphosphine (269 mg, 1.02 mmol) and diethyl azodicarboxylate (207 mg, 1.02 mmol) were sequentially added under an ice bath. The mixture was stirred for 1 h. The reaction mixture was quenched with saturated ammonium chloride and extracted with ethyl acetate (10 mL × 3). The organic phases were combined, washed with saturated sodium chloride solution, and dried over anhydrous sodium sulfate, and the drying agent was removed by filtration. The filtrate was concentrated under reduced pressure, and the residue was purified by silica gel column chromatography with eluent system A to give the title compound **4d** (80 mg, yield: 33.3%).

MS m/z (ESI): 410.2 [M-55].

### Step 4

### tert-Butyl (8aS)-3-(2-((tert-butoxycarbonyl)amino)-3-cyano-7-fluorobenzo[b]thiophen-4-yl)-2-flu oro-5-methyl-14-oxo-7,8,8a,9,11,12-hexahydro-10H,14H-pyrazino[1',2':5,6][1,5]diazoc ino[3,2,1-hi]indole-10-carboxylate 4e

Compound **4d** (32 mg, 0.07 mmol) and compound **1h** (41 mg, 0.1 mmol) were dissolved in 1 mL of toluene, and dichloro[bis(diphenylphosphinophenyl)ether]palladium(II) (8 mg, 0.011 mmol) and cesium carbonate (44 mg, 0.0.13 mmol, Shanghai Accela ChemBio) were added. The system was purged with nitrogen gas, and the mixture was heated to 105 °C and stirred for 10 h. The reaction mixture was cooled to room temperature and then filtered. The filtrate was concentrated under reduced pressure to give a crude product of the title compound **4e** (40 mg). The product was directly used in the next step without purification.

MS m/z (ESI): 678.6 [M+1].

### Step 5

### 2-Amino-7-fluoro-4-((S)-2-fluoro-5-methyl-14-oxo-8,8a,9,10,11,12-hexahydro-7H,14H -pyrazino[1',2':5,6][1,5]diazocino[3,2,1-hi]indol-3-yl)benzo[b]thiophene-3-carbonitrile bis(2,2,2-trifluoroacetate) 4f

Compound **4e** (40 mg, 0.059 mmol) was dissolved in 0.5 mL of dichloromethane, and 0.5 mL of trifluoroacetic acid was added at 0 °C. The mixture was stirred for 2 h. The reaction mixture was concentrated under reduced pressure to give a crude product of the title compound **4f** (41.5 mg). The product was directly used in the next step without purification.

MS m/z (ESI): 478.4 [M+1].

### Step 6

### 4-((S)-10-Acryloyl-2-fluoro-5-methyl-14-oxo-8,8a,9,10,11,12-hexahydro-7H,14H-pyra zino[1',2':5,6][1,5]diazocino[3,2,1-hi]indol-3-yl)-2-amino-7-fluorobenzo[b]thiophene-3-carbonitrile 4

Compound **4f** (41.5 mg, 0.059 mmol) was suspended in a mixed solvent of 2 mL of ethyl acetate, 1 mL of tetrahydrofuran, and 2 mL of water, and anhydrous potassium carbonate (40 mg, 0.29 mmol) was added. Acryloyl chloride (6 mg, 0.066 mmol) was added under an ice bath. The mixture was left to react for 5 min. The reaction mixture was extracted with ethyl acetate (5 mL × 2). The organic phase was concentrated under reduced pressure, and the residue was purified by high performance liquid chromatography (Waters-2545, column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compound **4** (5 mg, yield: 15.8%).

MS m/z (ESI): 532.3 [M+1].

¹H NMR (500 MHz, CD3OD): δ 7.22 (d, 2H), 7.02 (t, 1H), 6.93-6.69 (m, 1H), 6.30 (d,1H), 5.92 (s, 1H), 5.87-5.75 (m, 1H), 4.71 (d, 2H), 4.37 (q, 2H), 4.17-3.83 (m, 2H), 3.47 (s, 1H), 3.20-2.91 (m, 2H), 2.41 (d, 3H), 2.05 (d, 1H), 1.79 (s, 1H).

### Example 5

### 2-Amino-7-fluoro-4-((S)-2-fluoro-10-(2-fluoroacryloyl)-14-oxo-8,8a,9,10,11,12-hexahy dro-7H,14H-pyrazino[1',2':5,6][1,5]diazocino[3,2,1-hi]indol-3-yl)benzo[b]thiophene-3-carbonitrile 5

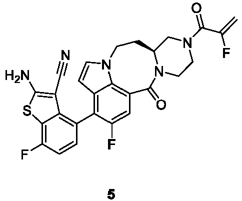

The title compound **5** (15 mg, yield: 40.4%) was obtained by using the synthesis scheme of Example **2** and replacing the starting material of step 6, acryloyl chloride, with 2-fluoroacryloyl chloride (prepared using the method disclosed in "Tetrahedron, 2016, vol. 72, 32, p. 4845-4853").

MS m/z (ESI): 536.1 [M+1].

¹H NMR (500 MHz, CD3OD): δ 7.36-7.26 (m, 2H), 7.21 (dd, 1H), 7.03 (t,1H), 6.12 (d,1H), 5.38-5.25 (m, 2H), 4.75 (d,2H), 4.45-4.20 (m, 2H), 4.20-4.03 (m, 2H), 3.47 (d,1H), 3.12 (t, 2H), 2.24 (d,1H), 1.88 (t, 1H).

### Example 6-P1, 6-P2

### (R)-2-Amino-7-fluoro-4-((S)-2-fluoro-10-(2-fluoroacryloyl)-14-oxo-8,8a,9,10,11,12-he xahydro-7H,14H-pyrazino[1',2':5,6][1,5]diazocino[3,2,1-hi]indazol-3-yl)benzo[b]thioph ene-3-carbonitrile 6-P1

### (S)-2-Amino-7-fluoro-4-((S)-2-fluoro-10-(2-fluoroacryloyl)-14-oxo-8,8a,9,10,11,12-hex ahydro-7H,14H-pyrazino[1',2':5,6][1,5]diazocino[3,2,1-hi]indazol-3-yl)benzo[b]thiophe ne-3-carbonitrile 6-P2

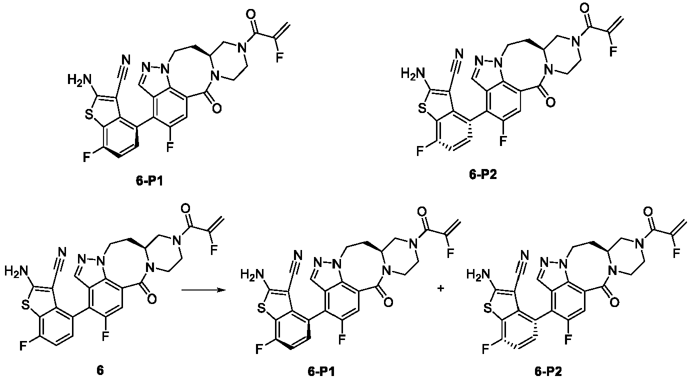

A crude product of compound 6, i.e., 2-amino-7-fluoro-4-((*S*)-2-fluoro-10-(2-fluoroacryloyl)-14-oxo-8,8*a*,9,10,11,12-hexahydro-7*H*,14*H*-pyrazino[1',2':5,6][1,5]diaz ocino[3,2,1-*hi*]indazol-3-yl)benzo[*b*]thiophene-3-carbonitrile (50 mg), was obtained by using the synthesis scheme of Example **1-P1** and **1-P2** and replacing the starting material of step 7, acryloyl chloride, with 2-fluoroacryloyl chloride. The crude product was purified by preparative high performance liquid chromatography (Waters-2545, column: YMC Triart-Exrs, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compounds **6-P2** (10 mg, yield: 15.4%) and **6-P1** (2 mg, yield: 3.8%).
Single-configuration compound (shorter retention time) **6-P2** (10 mg, yield: 15.4%)

(MS m/z (ESI): 537.2M+1].

Preparative HPLC analysis: retention time: 11.7 min; purity: 99% (Waters-2545, column: YMC Triart-Exrs, Prep 30 × 150 mm, 5 µm; C18, mobile phase: aqueous phase (10 mmol/L ammonium bicarbonate) and acetonitrile; gradient ratio: acetonitrile 34%-45%; flow rate: 30 mL/min)

¹H NMR (500 MHz, CD3OD): δ 7.70 (d, 1H), 7.66 (dd,1H), 7.29 (dd, 1H), 7.12-7.04 (m, 1H), 5.38-5.26 (m, 2H), 4.80-4.66 (m, 3H), 4.40-4.18 (m, 2H), 4.10 (s, 1H), 3.41 (s, 1H), 3.19 (td,2H), 2.33 (s, 1H), 1.96 (ddd,1H).
Single-configuration compound (longer retention time) **6-P1** (2 mg, yield: 3.8%)

MS m/z (ESI): 537.2 [M+1].

Preparative HPLC analysis: retention time: 12.1 min; purity: 99% (Waters-2545, column: YMC Triart-Exrs, Prep 30 × 150 mm, 5 µm; C18, mobile phase: aqueous phase (10 mmol/L ammonium bicarbonate) and acetonitrile; gradient ratio: acetonitrile 34%-45%; flow rate: 30 mL/min)
¹H NMR (500 MHz, CD3OD): δ 7.65 (d,1H), 7.61 (d,1H), 7.38 (dd, 1H), 7.14-7.04 (m, 1H), 5.32 (s, 2H), 4.76 (s, 3H), 4.33 (t, 2H), 4.11 (s, 1H), 3.47 (s, 1H), 3.20 (d, 2H), 2.33 (s, 1H), 1.99-1.87 (m, 1H).

### Example 7-P1, 7-P2

### (R)-4-((S)-10-Acryloyl-4-chloro-2-fluoro-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 7-P1

### (S)-4-((S)-10-Acryloyl-4-chloro-2-fluoro-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 7-P2

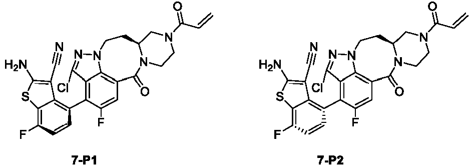

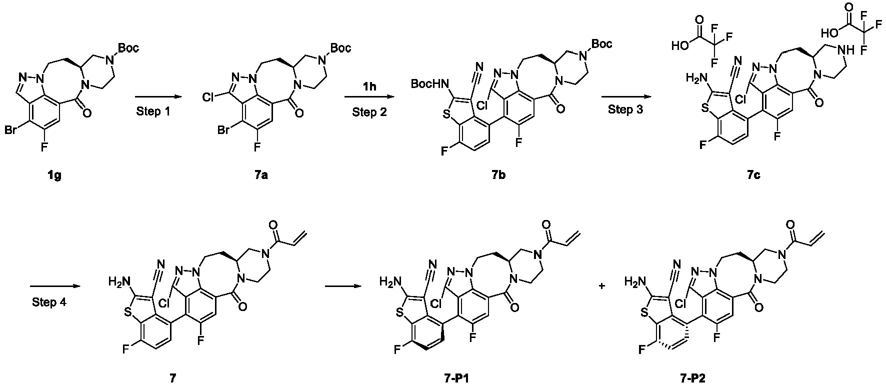

### Step 1

### tert-Butyl (S)-3-bromo-4-chloro-2-fluoro-14-oxo-7,8,8a,9,11,12-hexahydro-10H,14H-pyrazino[1', 2':5,6][1,5]diazocino[3,2,1-hi]indazole-10-carboxylate 7a

Compound **1g** (630 mg, 1.38 mmol) was dissolved in acetonitrile (6 mL), and *N*-chlorosuccinimide (278 mg, 2.08 mmol) was added. The mixture was heated to 60 °C and left to react for 0.5 h, and a solid precipitated. An additional amount of *N*-Chlorosuccinimide (100 mg, 0.75 mmol) was added, and the mixture was left to react for another 20 min. The reaction mixture was cooled to room temperature. Saturated sodium bicarbonate solution was added, and extraction was performed with ethyl acetate (10 mL × 3). The organic phases were combined and dried over anhydrous sodium sulfate, and the drying agent was removed by filtration. The filtrate was concentrated under reduced pressure, and the residue was purified by silica gel column chromatography with eluent system A to give the title compound **7a** (380 mg, yield: 56%).

MS m/z (ESI): 487.1 [M+1].

### Step 2

### tert-Butyl (8aS)-3-(2-((tert-butoxycarbonyl)amino)-3-cyano-7-fluorobenzo[b]thiophen-4-yl)-4-chl oro-2-fluoro-14-oxo-7,8,8a,9,11,12-hexahydro-10H,14H-pyrazino[1',2':5,6][1,5]diazoci no[3,2,1-hi]indazole-10-carboxylate 7b

Compound **7a** (150 mg, 0.31 mmol) and compound **1h** (186 mg, 0.46 mmol) were dissolved in 5 mL of toluene, and dichloro[bis(diphenylphosphinophenyl)ether]palladium(II) (43 mg, 0.06 mmol) and cesium carbonate (300 mg, 0.92 mmol) were added. The system was purged with nitrogen gas, and the mixture was stirred at 105 °C for 6 h. The reaction mixture was cooled to room temperature and then filtered. The filtrate was concentrated under reduced pressure, and the residue was purified by silica gel column chromatography with eluent system A to give the title compound **7b** (40 mg, yield: 18.6%).

MS m/z (ESI): 699.2 [M+1].

### Step 3

### 2-Amino-4-((S)-4-chloro-2-fluoro-14-oxo-8,8a,9,10,11,12-hexahydro-7H,14H-pyrazino [1',2':5,6][1,5]diazocino[3,2,1-hi]indazol-3-yl)-7-fluorobenzo[b]thiophene-3-carbonitril e bis(2,2,2-trifluoroacetate) 7c

Compound **7b** (40 mg, 57.21 µmol) was dissolved in 1 mL of dichloromethane, and 1 mL of trifluoroacetic acid was added at 0 °C. The mixture was stirred for 2 h. The reaction mixture was concentrated under reduced pressure to give a crude product of the title compound **7c** (41 mg). The product was directly used in the next step without purification.

MS m/z (ESI): 499.2 [M+1].

### Step 4

### (R)-4-((S)-10-Acryloyl-4-chloro-2-fluoro-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 7-P1

### (S)-4-((S)-10-Acryloyl-4-chloro-2-fluoro-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 7-P2

The crude product of compound **7c** (41 mg, 56.4 µmol) was suspended in 2 mL of ethyl acetate, 1 mL of tetrahydrofuran, and 2 mL of water, and anhydrous potassium carbonate (24 mg, 173.6 µmol) was added. Acryloyl chloride (5.4 mg, 59.6 µmol) was added under an ice bath. After 5 min of reaction, extraction was performed with ethyl acetate (5 mL × 2). The organic phases were combined and concentrated under reduced pressure to give a crude product of compound 7, i.e., 4-((*S*)-10-acryloyl-4-chloro-2-fluoro-14-oxo-8,8*a*,9,10,11,12-hexahydro-7*H*,14*H*-pyrazi no[1',2':5][1,5]diazocino[3,2,1-*hi*]indazol-3-yl)-2-amino-7-fluorobenzo[*b*]thiophene-3-c arbonitrile (39 mg). The crude product was purified by preparative high performance liquid chromatography (Waters-2545; column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compounds **7-P2** (5 mg, yield: 15.1%) and **7-P1** (2 mg, yield: 6%).
Single-configuration compound (shorter retention time) **7-P2** (5 mg, yield: 15.1%)

MS m/z (ESI): 553.2 [M+1].

HPLC analysis: retention time: 2.21 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.67 (d, 1H), 7.20 (t, 1H), 7.02 (dd, 1H), 6.87 (dd, 1H), 6.72-6.28 (m, 1H), 5.81 (t, 1H), 4.74-4.67 (m, 1H), 4.66-4.56 (m, 1H), 4.41 (d, 1H), 4.30 (d, -1H), 4.12-4.00 (m, 1H), 3.96 (d, 1H), 3.49 (d, 1H), 3.09 (dd, 2H), 2.26-2.16 (m, 1H), 1.94 (s, 1H).
Single-configuration compound (longer retention time) **7-P1** (2 mg, yield: 6%)

MS m/z (ESI): 553.2 [M+1].

HPLC analysis: retention time: 2.29 min; purity: 95% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.65 (d, 1H), 7.20 (t, 1H), 7.00 (dd, 1H), 6.85 (dd, 1H), 6.70-6.26 (m, 1H), 5.81 (t, 1H), 4.73-4.65 (m, 1H), 4.66-4.56 (m, 1H), 4.39 (d, 1H), 4.30 (d, 1H), 4.10-4.00 (m, 1H), 3.92 (d, 1H), 3.49 (d, 1H), 3.12 (dd, 2H), 2.24-2.16 (m, 1H), 1.92 (s, 1H).

### Example 8-P1, 8-P2

### (R)-4-((S)-10-Acryloyl-2,4-difluoro-14-oxo-8,8a,9,10,11,12-hexahydro-7H,14H-pyrazin o[1',2':5][1,5]diazocino[3,2,1-hi]indazol-3-yl)-2-amino-7-fluorobenzo[b]thiophene-3-ca rbonitrile 8-P1

### (S)-4-((S)-10-Acryloyl-2,4-difluoro-14-oxo-8,8a,9,10,11,12-hexahydro-7H,14H-pyrazin o[1',2':5][1,5]diazocino[3,2,1-hi]indazol-3-yl)-2-amino-7-fluorobenzo[b]thiophene-3-ca rbonitrile 8-P2

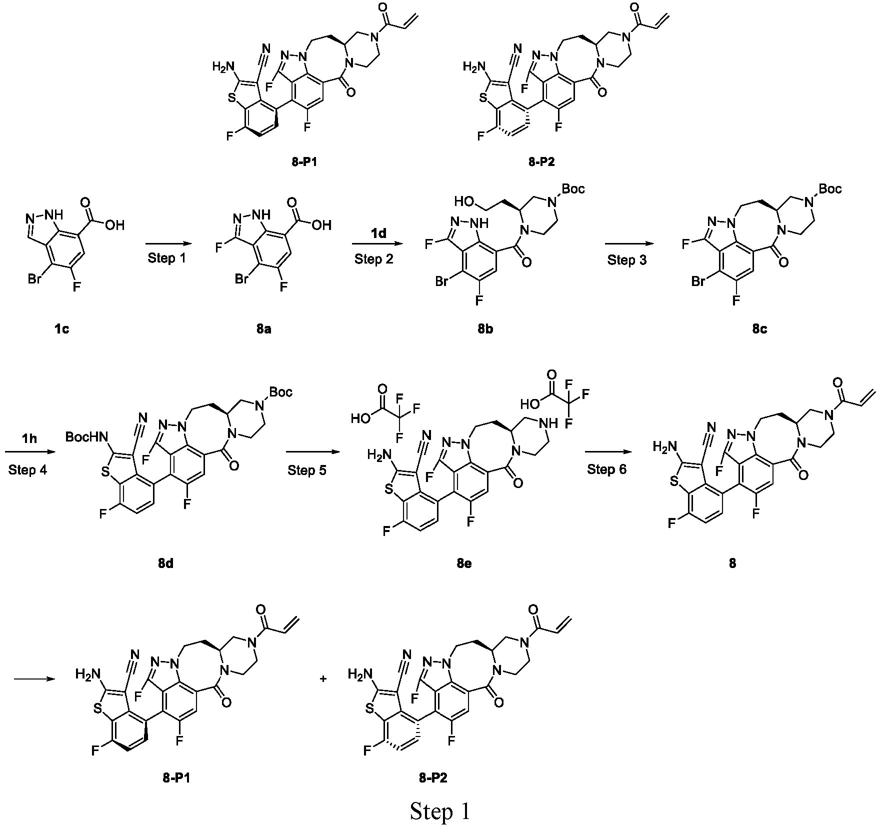

### 4-Bromo-3,5-difluoro-1H-indazole-7-carboxylic acid 8a

Compound **1c** (1 g, 3.86 mmol) was dissolved in *N*,*N*-dimethylformamide (15 mL), and 1-chloromethyl-4-fluoro-1,4-diazabicyclo[2.2.2]octane bis(tetrafluoroborate) (6.83 g, 19.3 mmol, Shanghai Bide) was added. The mixture was microwaved at 100 °C for 6 h. The reaction mixture was cooled to room temperature and poured into ice water, and a solid precipitated. The solid was collected by filtration and dried to give a crude product of the title compound **8a** (950 mg, yield: 88.8%). The product was directly used in the next step without purification.

MS m/z (ESI): 275.1 [M-1].

### Step 2

### tert-Butyl (S)-4-(4-bromo-3,5-difluoro-1H-indazole-7-carbonyl)-3-(2-hydroxyethyl)piperazine-1-c arboxylate 8b

The crude product of compound **8a** (900 mg, 3.24 mmol) and compound **1d** (897 mg, 3.89 mmol) were dissolved in 30 mL of *N*,*N*-dimethylformamide, and N,N-diisopropylethylamine (1.62 g, 12.5 mmol) and 2-(7-azabenzotriazole)-*N*,*N*,*N'*,*N'*-tetramethyluronium hexafluorophosphate (1.48 g, 3.89 mmol) were added under an ice bath. The mixture was stirred for 3 h. The reaction mixture was concentrated under reduced pressure, then diluted with ethyl acetate, and washed with water, saturated sodium carbonate solution, and saturated sodium chloride solution in sequence. The organic phase was dried over anhydrous sodium sulfate and filtered. The filtrate was concentrated under reduced pressure to give a crude product of the title compound **8b** (1.5 g). The product was directly used in the next step without purification.

MS m/z (ESI): 433.2 [M-55].

### Step 3

### tert-Butyl (S)-3-bromo-2,4-difluoro-14-oxo-7,8,8a,9,11,12-hexahydro-10H,14H-pyrazino[1',2':5,6 ][1,5]diazocino[3,2,1-hi]indazole-10-carboxylate 8c

The crude product of compound **8b** (1.3 g, 2.65 mmol) was dissolved in 20 mL of tetrahydrofuran in a nitrogen atmosphere, and triphenylphosphine (1.39 g, 5.3 mmol) and diethyl azodicarboxylate (1.07 g, 5.3 mmol) were sequentially added under an ice bath. The mixture was stirred for 30 min with the temperature maintained. The reaction mixture was quenched with saturated ammonium chloride solution and extracted with ethyl acetate (15 mL × 2). The organic phases were combined, washed with saturated sodium chloride solution, dried over anhydrous sodium sulfate, and filtered. The filtrate was concentrated under reduced pressure, and the residue was purified by silica gel column chromatography with eluent system A to give the title compound **8c** (200 mg, yield: 15.9%).

MS m/z (ESI): 415.2 [M-55].

### Step 4

### tert-Butyl (8aS)-3-(2-((tert-butoxycarbonyl)amino)-3-cyano-7-fluorobenzo[b]thiophen-4-yl)-2,4-d ifluoro-14-oxo-7,8,8a,9,11,12-hexahydro-10H,14H-pyrazino[1,2:5,6][1,5]diazocino[3,2, 1-hi]indazole-10-carboxylate 8d

Compound **8c** (80 mg, 169.7451 µmol) and compound **1h** (137 mg, 338.8 µmol) were dissolved in 2 mL of toluene, and dichloro[bis(diphenylphosphinophenyl)ether]palladium(II) (36 mg, 50.34 µmol) and cesium carbonate (165 mg, 506.4 µmol) were added. The system was purged with nitrogen gas, and the mixture was stirred at 105 °C for 6 h. The reaction mixture was cooled to room temperature and then filtered. The filtrate was concentrated under reduced pressure, and the residue was purified by silica gel column chromatography with eluent system B to give the title compound **8d** (100 mg, yield: 86.2%).

MS m/z (ESI): 683.2 [M+1].

### Step 5

### 2-Amino-4-((S)-2,4-difluoro-14-oxo-8,8a,9,10,11,12-hexahydro-7H,14H-pyrazino[1',2': 5,6][1,5]diazocino[3,2,1-hi]indazol-3-yl)-7-fluorobenzo[b]thiophene-3-carbonitrile bis(2,2,2-trifluoroacetate) 8e

Compound **8d** (100 mg, 146.4 µmol) was dissolved in 1 mL of dichloromethane, and 1 mL of trifluoroacetic acid was added at 0 °C. The mixture was stirred for 2 h. The reaction mixture was concentrated under reduced pressure to give a crude product of the title compound **8e** (100 mg). The product was directly used in the next step without purification.

MS m/z (ESI): 483.2 [M+1].

### Step 6

### (R)-4-((S)-10-Acryloyl-2,4-difluoro-14-oxo-8,8a,9,10,11,12-hexahydro-7H,14H-pyrazin o[1',2':5][1,5]diazocino[3,2,1-hi]indazol-3-yl)-2-amino-7-fluorobenzo[b]thiophene-3-ca rbonitrile 8-P1

### (S)-4-((S)-10-Acryloyl-2,4-difluoro-14-oxo-8,8a,9,10,11,12-hexahydro-7H,14H-pyrazin o[1',2':5][1,5]diazocino[3,2,1-hi]indazol-3-yl)-2-amino-7-fluorobenzo[b]thiophene-3-ca rbonitrile 8-P2

The crude product of compound **8e** (100 mg, 140 µmol) was suspended in 2 mL of ethyl acetate, 1 mL of tetrahydrofuran, and 2 mL of water, and anhydrous potassium carbonate (100 mg, 723 µmol) was added. Acryloyl chloride (14 mg, 154 µmol) was added under an ice bath. After 5 min of reaction, extraction was performed with ethyl acetate (5 mL × 2). The organic phases were combined and concentrated under reduced pressure to give a crude product of compound **8,** i.e., 4-((*S*)-10-acryloyl-2,4-difluoro-14-oxo-8,8a,9,10,11,12-hexahydro-7*H*,14*H*-pyrazino[1', 2':5,6][1,5]diazocino[3,2,1-*hi*]indazol-3-yl)-2-amino-7-fluorobenzo[*b*]thiophene-3-carb onitrile (70 mg). The crude product was purified by preparative high performance liquid chromatography (Waters-2545; column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compounds **8-P2** (30 mg, yield: 38.3%) and **8-P1** (13 mg, yield: 16.6%).
Single-configuration compound (shorter retention time) **8-P2** (30 mg, yield: 38.3%)

MS m/z (ESI): 537.2 [M+1].

HPLC analysis: retention time: 1.20 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.69 (d, 1H), 7.27 (d, 1H), 7.04 (dd, 1H), 6.96-6.70 (m, 1H), 6.30 (dd, 1H), 5.83 (t, 1H), 4.72 (d, 2H), 4.51-4.25 (m, 3H), 4.11 (s, 1H), 3.57-3.39 (m, 1H), 3.13 (dt, 2H), 2.20 (dd, 1H), 1.96 (s, 1H).
Single-configuration compound (longer retention time) **8-P1** (13 mg, yield: 16.6%)

MS m/z (ESI): 537.2 [M+1].

HPLC analysis: retention time: 1.23 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.64 (d, 1H), 7.36 (dt, 1H), 7.06 (t, 1H), 6.81 (dt, 1H), 6.30 (dd, 1H), 5.84 (d, 1H), 4.72 (d, 2H), 4.49-4.23 (m, 3H), 4.12 (s, 1H), 3.51 (d, 1H), 3.08 (dd, 2H), 2.26-2.18 (m, 1H), 1.92 (s, 1H).

### Example 9-P1, 9-P2

### (R)-4-((S)-10-Acryloyl-2-fluoro-4-methyl-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 9-P1

### (S)-4-((S)-10-Acryloyl-2-fluoro-4-methyl-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 9-P2

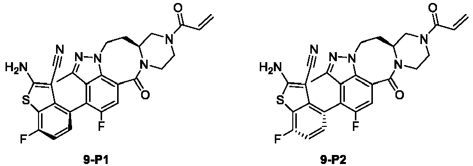

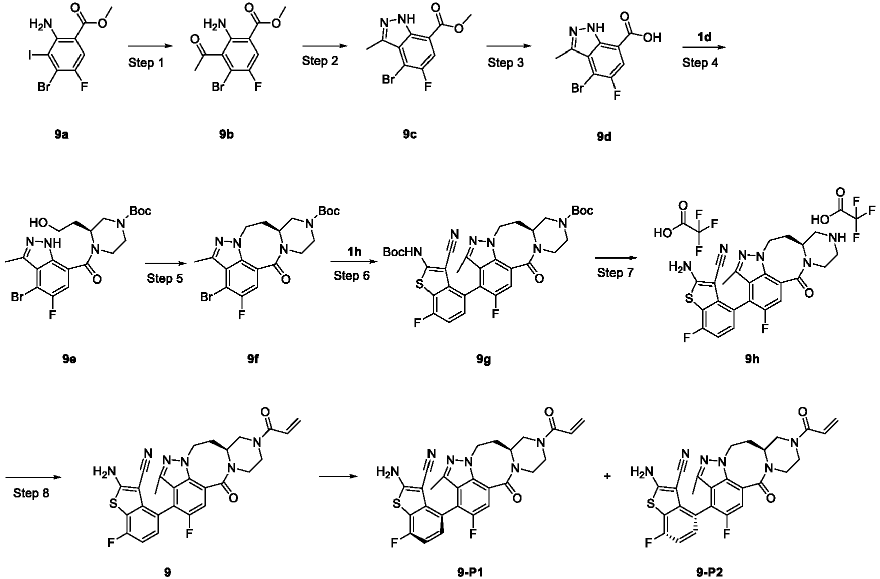

### Step 1

### Methyl 3-acetyl-2-amino-4-bromo-5-fluorobenzoate 9b

Methyl 2-amino-4-bromo-5-fluoro-3-iodobenzoate **9a** (5 g, 13.37 mmol, prepared using the method disclosed in Intermediate 15 on page 103 of the specification of the patent application "WO2016020836A1") was dissolved in *N,N-*dimethylformamide (100 mL), and bis(triphenylphosphine)palladium(II) dichloride (1.87 g, 2.67 mmol) and tributyl(1-ethoxyvinyl)tin (5.79 g, 16.04 mmol, Shanghai Accela ChemBio) were added. The system was purged with nitrogen gas, and the mixture was left to react at 90 °C for 12 h. The reaction mixture was cooled to room temperature, and 10% aqueous potassium fluoride was added. The mixture was stirred for 30 min and filtered, and the filtrate was separated. The organic phase was stirred with 10 mL of concentrated hydrochloric acid for 16 h, made neutral with saturated sodium carbonate solution, and extracted with ethyl acetate (50 mL × 3). The organic phases were combined and dried over anhydrous sodium sulfate, and the drying agent was removed by filtration. The filtrate was concentrated under reduced pressure, and the residue was purified by silica gel column chromatography with eluent system A to give the title compound **9b** (1.8 g, yield: 46.4%).

MS m/z (ESI): 289.9 [M+1].

### Step 2

### Methyl 4-bromo-5-fluoro-3-methyl-1H-indazole-7-carboxylate 9c

Compound **9b** (1 g, 3.44 mmol) was dissolved in concentrated hydrochloric acid (5 mL), and a solution of sodium nitrite (261 mg, 3.78 mmol) in water (2 mL) was added dropwise under an ice bath. After the mixture was stirred for 1 h with the temperature maintained, a solution of stannous chloride dihydrate (1.71 g, 7.58 mmol) in hydrochloric acid (2 mL) was added dropwise. The mixture was stirred for another 30 min, and the pH was adjusted to 8 with saturated potassium carbonate solution. Extraction was performed with ethyl acetate (30 mL × 3). The organic phases were combined and dried over anhydrous sodium sulfate, and the drying agent was removed by filtration. The filtrate was concentrated under reduced pressure, and the residue was purified by silica gel column chromatography with eluent system A to give the title compound **9c** (760 mg, yield: 76.7%).

MS m/z (ESI): 286.9 [M+1].

### Step 3

### 4-Bromo-5-fluoro-3-methyl-1H-indazole-7-carboxylic acid 9d

The crude product of compound **9c** (760 mg, 2.6 mmol) was dissolved in a mixed solvent of tetrahydrofuran (5 mL), methanol (5 mL), and water (5 mL), and lithium hydroxide (555 mg, 13.2 mmol) was added. The mixture was stirred at 40 °C for 1 h. The reaction mixture was concentrated under reduced pressure to remove most of the solvent. Water was added, and extraction was performed with ethyl acetate (150 mL × 6). The organic phases were combined, washed with dilute hydrochloric acid and saturated sodium chloride solution in sequence, and dried over anhydrous sodium sulfate, and the drying agent was removed by filtration. The filtrate was concentrated under reduced pressure to give a crude product of compound **9d** (722 mg). The product was directly used in the next step without purification.

MS m/z (ESI): 270.9 [M-1].

### Step 4

### tert-Butyl (S)-4-(4-bromo-5-fluoro-3-methyl-1H-indazole-7-carbonyl)-3-(2-hydroxyethyl)piperazi ne-1-carboxylate 9e

The crude product of compound **9d** (722 mg, 2.64 mmol) and compound **1d** (730 mg, 3.16 mmol) were dissolved in 10 mL of *N,N*-dimethylformamide, and *N,N*-diisopropylethylamine (720 mg, 5.5 mmol) and 2-(7-azabenzotriazole)-*N*,*N*,*N'*,*N'*-tetramethyluronium hexafluorophosphate (1.2 g, 3.17 mmol) were added under an ice bath. The mixture was stirred for 1 h. The reaction mixture was concentrated under reduced pressure, then diluted with ethyl acetate, and washed with water, saturated sodium carbonate solution, and saturated sodium chloride solution in sequence. The organic phase was dried over anhydrous sodium sulfate and filtered. The filtrate was concentrated under reduced pressure to give a crude product of the title compound **9e** (1.2 g). The product was directly used in the next step without purification.

MS m/z (ESI): 429.2 [M-55].

### Step 5

### tert-Butyl (S)-3-bromo-2-fluoro-4-methyl-14-oxo-7,8,8a,9,11,12-hexahydro-10H,14H-pyrazino[1', 2':5,6][1,5]diazocino[3,2,1-hi]indazole-10-carboxylate 9f

The crude product of compound **9e** (1.28 g, 2.63 mmol) was dissolved in 50 mL of tetrahydrofuran in a nitrogen atmosphere, and triphenylphosphine (1.38 g, 5.26 mmol) and diethyl azodicarboxylate (1.06 g, 5.26 mmol) were sequentially added under an ice bath. The mixture was stirred for 30 min with the temperature maintained. The reaction mixture was quenched with saturated ammonium chloride solution and extracted with ethyl acetate (20 mL × 2). The organic phases were combined, washed with saturated sodium chloride solution, dried over anhydrous sodium sulfate, and filtered. The filtrate was concentrated under reduced pressure, and the residue was purified by silica gel column chromatography with eluent system A to give the title compound **9f** (650 mg, yield: 52.7%).

MS m/z (ESI): 411.2 [M-55].

### Step 6

### tert-Butyl (8aS)-3-(2-((tert-butoxycarbonyl)amino)-3-cyano-7-fluorobenzo[b]thiophen-4-yl)-2-flu oro-4-methyl-14-oxo-7,8,8a,9,11,12-hexahydro-10H,14H-pyrazino[1',2':5,6][1,5]diazoc ino[3,2,1-hi]indazole-10-carboxylate 9g

The crude product of compound **9f** (210 mg, 449.36 µmol) and compound **1h** (363 mg, 897 µmol) were dissolved in 10 mL of toluene, and dichloro[bis(diphenylphosphinophenyl)ether]palladium(II) (96 mg, 134.2 µmol) and cesium carbonate (439 mg, 1.34 mmol) were added. The system was purged with nitrogen gas, and the mixture was stirred at 105 °C for 6 h. The reaction mixture was cooled to room temperature and then filtered. The filtrate was concentrated under reduced pressure, and the residue was purified by silica gel column chromatography with eluent system A to give the title compound **9g** (200 mg, yield: 65.6%).

MS m/z (ESI): 679.2 [M+1].

### Step 7

### 2-Amino-7-fluoro-4-((S)-2-fluoro-4-methyl-14-oxo-8,8a,9,10,11,12-hexahydro-7H,14H -pyrazino[1',2':5,6][1,5]diazocino[3,2,1-hi]indazol-3-yl)benzo[b]thiophene-3-carbonitril e bis(2,2,2-trifluoroacetate) 9h

Compound **9g** (200 mg, 294.6 µmol) was dissolved in 2 mL of dichloromethane, and 2 mL of trifluoroacetic acid was added at 0 °C. The mixture was stirred for 2 h. The reaction mixture was concentrated under reduced pressure to give a crude product of the title compound **9h** (200 mg). The product was directly used in the next step without purification.

MS m/z (ESI): 479.2 [M+1].

### Step 8

### (R)-4-((S)-10-Acryloyl-2-fluoro-4-methyl-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 9-P1

### (S)-4-((S)-10-Acryloyl-2-fluoro-4-methyl-14-oxo-8,8a,9,10,11,12-hexahydro-7H,14H-p yrazino[1',2':5,6][1,5]diazocino[3,2,1-hi]indazol-3-yl)-2-amino-7-fluorobenzo[b]thiophe ne-3-carbonitrile 9-P2

Compound **9h** (200 mg, 283.2 µmol) was suspended in 2 mL of ethyl acetate, 1 mL of tetrahydrofuran, and 2 mL of water, and anhydrous potassium carbonate (202 mg, 1.46 mmol) was added. Acryloyl chloride (29 mg, 320.4 µmol) was added under an ice bath. After 5 min of reaction, extraction was performed with ethyl acetate (5 mL × 2). The organic phases were combined and concentrated under reduced pressure to give a crude product of compound **9**, i.e., 4-((*S*)-10-acryloyl-2-fluoro-4-methyl-14-oxo-8,8*a*,9,10,11,12-hexahydro-7*H*,14*H*-pyrazino[1',2':5,6][1,5]diazocino[3,2,1-*hi*]indazol-3-yl)-2-amino-7-f luorobenzo[b]thiophene-3-carbonitrile (160 mg). The crude product was purified by preparative high performance liquid chromatography (Waters-2545; column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compounds **9-P2** (50 mg, yield: 31.9%) and **9-P1** (50 mg, yield: 31.9%).
Single-configuration compound (shorter retention time) **9-P2** (50 mg, yield: 31.9%)

MS m/z (ESI): 533.2 [M+1].

HPLC analysis: retention time: 1.17 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.61 (d, 1H), 7.21 (t, 1H), 7.06 (dd, 1H), 6.81 (ddd, 1H), 6.30 (d, 1H), 5.83 (t, 1H), 4.80-4.51 (m, 3H), 4.47-4.18 (m, 2H), 4.05 (t, 1H), 3.44 (dd, 1H), 3.19-2.87 (m, 2H), 2.32-2.15 (m, 1H), 1.85 (s, 4H).
Single-configuration compound (longer retention time) **9-P1** (50 mg, yield: 31.9%)

MS m/z (ESI): 533.2 [M+1].

HPLC analysis: retention time: 1.19 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.56 (d, 1H), 7.34 (dd, 1H), 7.08 (dd, 1H), 6.96-6.65 (m, 1H), 6.29 (dd, 1H), 5.82 (t, 1H), 4.67 (dd, 3H), 4.40-4.27 (m, 1H), 4.26-4.01 (m, 2H), 3.48 (d, 1H), 3.18-2.90 (m, 2H), 2.22 (d, 1H), 1.81 (s, 4H).

### Example 10

### 4-((8aS,11R)-10-Acryloyl-2-fluoro-11-methyl-14-oxo-8,8a,9,10,11,12-hexahydro-7H,1 4H-pyrazino[1',2':5,6][1,5]diazocino[3,2,1-hi]indazol-3-yl)-2-amino-7-fluorobenzo[b]th iophene-3-carbonitrile 10

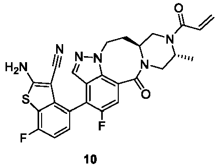

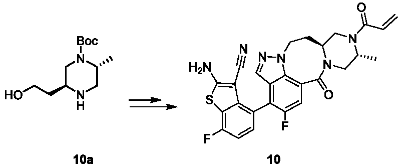

The title compound **10** (72 mg, yield: 62.2%) was obtained by using the synthesis scheme of Example **1-P1** and **1-P2** and replacing the starting material of step 3, compound **1d**, with tert-butyl (2*R*,5*S*)-5-(2-hydroxyethyl)-2-methylpiperazine-1-carboxylate **10a** (prepared using the method disclosed in preparation 67 on page 80 of the specification of the patent application "WO2021118877A1").

MS m/z (ESI): 533.2 [M+1].

¹H NMR (500 MHz, CD₃OD): δ 7.73-7.64 (m, 2H), 7.30 (td, 1H), 7.08 (ddd, 1H), 6.80 (ddd, 1H), 6.27 (ddd, 1H), 5.81 (ddd, 1H), 4.74 (dt, 1H), 4.62-4.55 (m, 2H), 4.31 (td, 1H), 4.15-4.00 (m, 1H), 3.82-3.61 (m, 1H), 3.24 (ddd, 2H), 2.34 (dt, 1H), 2.02-1.85 (m, 1H), 1.37 (dd, 3H).

### Example 11-P1, 11-P2

### (R)-4-((S)-10-Acryloyl-1,2-difluoro-14-oxo-8,8a,9,10,11,12-hexahydro-7H,14H-pyrazin o[1',2':5.6][1,5]diazocino[3,2,1-hi]indazol-3-yl)-2-amino-7-fluorobenzo[b]thiophene-3-carbonitrile 11-P1

### (S)-4-((S)-10-Acryloyl-1,2-difluoro-14-oxo-8,8a,9,10,11,12-hexahydro-7H,14H-pyrazin o[1',2':5.6][1,5]diazocino[3,2,1-hi]indazol-3-yl)-2-amino-7-fluorobenzo[b]thiophene-3-carbonitrile 11-P2

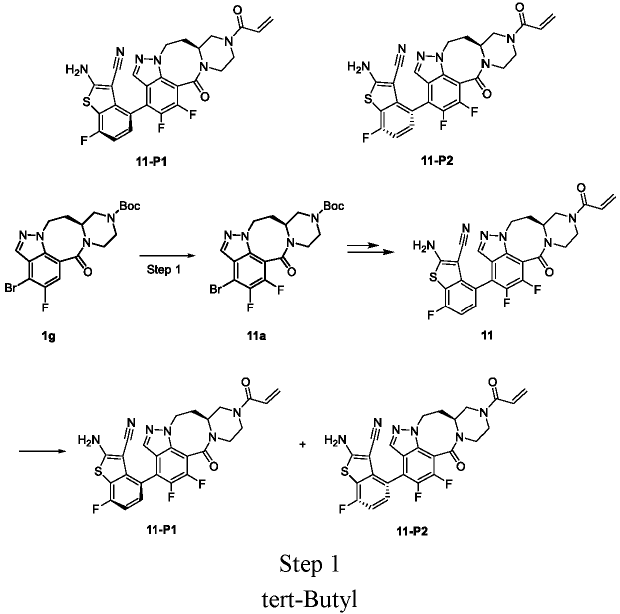

### (S)-3-bromo-1,2-difluoro-14-oxo-7,8,8a,9,11,12-hexahydro-10H,14H-pyrazino[1',2':5,6 ][1,5]diazocino[3,2,1-hi]indazole-10-carboxylate 11a

Compound **1g** (500 mg, 1.10 mmol) was dissolved in tetrahydrofuran (6 mL), and a 2 M solution of lithium diisopropylamide in tetrahydrofuran (896 µL) was added at -78 °C. The mixture was left to react for 40 min with the temperature maintained, and N-fluorobenzenesulfonimide (417 mg, 1.32 mmol, Shanghai Accela ChemBio) was added. The mixture was stirred for 20 min. Then the reaction mixture was quenched with saturated ammonium chloride solution and extracted with ethyl acetate (15 mL × 3). The organic phases were combined and dried over anhydrous sodium sulfate, and the drying agent was removed by filtration. The filtrate was concentrated under reduced pressure, and the residue was purified by silica gel column chromatography with eluent system A to give the title compound **11a** (400 mg, yield: 76.9%).

MS m/z (ESI): 471.1 [M+1].

Subsequently, the title compounds **11-P2** (30 mg, yield: 33.3%) and **11-P1** (8 mg, yield: 8.9%) were obtained by using the synthesis scheme of Example **1-P1** and **1-P2** and replacing the starting material of step 5, compound **1g,** with compound **11a.**
Single-configuration compound (shorter retention time) **11-P2** (30 mg, yield: 33.3%)

MS m/z (ESI): 537.2 [M+1].

HPLC analysis: retention time: 2.05 min; purity: 96% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.70 (d, 1H), 7.48-7.26 (m, 1H), 7.10 (q, 1H), 6.99-6.63 (m, 1H), 6.30 (d, 1H), 5.84 (d, 1H), 4.72 (d, 2H), 4.42 (dd, 1H), 4.29-3.90 (m, 2H), 3.45 (d, 2H), 3.04 (d, 2H), 2.42-2.15 (m, 1H), 1.93 (s, 1H).
Single-configuration compound (longer retention time) **11-P1** (8 mg, yield: 8.9%)

MS m/z (ESI): 537.2 [M+1].

HPLC analysis: retention time: 2.10 min; purity: 97% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.66 (s, 1H), 7.41 (dd, 1H), 7.11 (t, 1H), 6.94-6.65 (m, 1H), 6.30 (dd, 1H), 5.84 (d, 1H), 4.73-4.70 (m, 2H), 4.37 (d, 1H), 4.17 (d, 2H), 3.36 (d, 2H), 3.16-3.03 (m, 2H), 2.30-2.21 (m, 2H).

### Example 12-P1, 12-P2

### (R)-4-((R)-10-Acryloyl-2-fluoro-14-oxo-8,8a,9,10,11,12-hexahydro-7H,14H-pyrazino[1 ',2':5,6][1,5]diazocino[3,2,1-hi]indazol-3-yl)-2-amino-7-fluorobenzo[b]thiophene-3-carb onitrile 12-P1

### (S)-4-((R)-10-Acryloyl-2-fluoro-14-oxo-8,8a,9,10,11,12-hexahydro-7H,14H-pyrazino[1' ,2':5,6][1,5]diazocino[3,2,1-hi]indazol-3-yl)-2-amino-7-fluorobenzo[b]thiophene-3-carb onitrile 12-P2

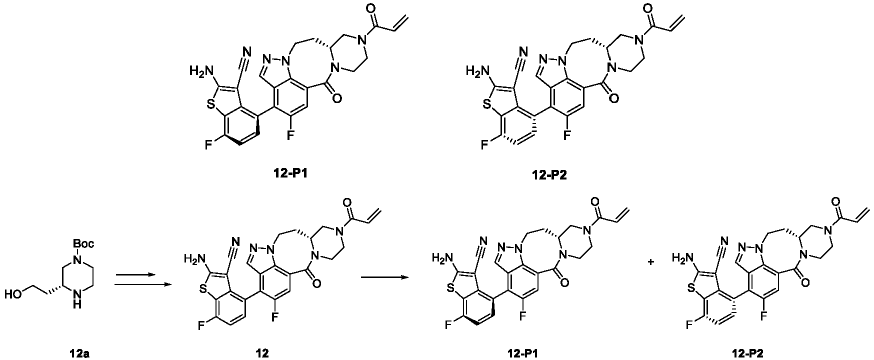

The title compounds **12-P2** (5 mg, yield: 9.7%) and **12-P1** (1.5 mg, yield: 2.9%) were obtained by using the synthesis scheme of Example **1-P1** and **1-P2** and replacing the starting material of step 3, compound **1d**, with tert-butyl (R)-3-(2-hydroxyethyl)piperazine-1-carboxylate **12a** (prepared using the method disclosed in "Tetrahedron Letters, 2018, 59(21), 2030-2033").
Single-configuration compound (shorter retention time) **12-P2** (5 mg, yield: 9.7%)

MS m/z (ESI): 519.2 [M+1].

HPLC analysis: retention time: 2.42 min; purity: 99% (column: HALO^{®} 90 Å, C18, 2.7 µm, 3.0 × 30 mm; mobile phase: water (1 ‰ trifluoroacetic acid), acetonitrile; gradient ratio: acetonitrile 10%-95%).

¹H NMR (500 MHz, CD3OD): δ 7.70 (s, 1H), 7.66 (d, 1H), 7.31 (s, 1H), 7.08 (d, 1H), 6.82 (d, 1H), 6.30 (dd, 1H), 5.83 (t, 1H), 4.75 (d, 3H), 4.33 (d, 2H), 4.04 (d, 1H), 3.51 (d, 1H), 3.12 (dd, 2H), 2.29 (s, 1H), 2.05 (t, 1H).
Single-configuration compound (longer retention time) **12-P1** (1.5 mg, yield: 2.9%)

MS m/z (ESI): 519.2 [M+1].

HPLC analysis: retention time: 2.45 min; purity: 92% (column: HALO^{®} 90 Å, C18, 2.7 µm, 3.0 × 30 mm; mobile phase: water (1 ‰ trifluoroacetic acid), acetonitrile; gradient ratio: acetonitrile 10%-95%).

¹H NMR (500 MHz, CD3OD): δ 7.65 (s, 1H), 7.60 (d, 1H), 7.38 (ddd, 1H), 7.13-7.05 (m, 1H), 6.92-6.75 (m, 1H), 6.30 (dt, 1H), 5.83 (t, 1H), 4.82-4.69 (m, 3H), 4.37 (d, 2H), 4.10 (s, 1H), 3.51 (d, 1H), 3.10 (dd, 2H), 2.26 (d, 1H), 2.05 (d, 1H).

### Example 13-P1, 13-P2

### (R)-4-((S)-10-Acryloyl-2,4-difluoro-14-oxo-8,8a,9,10,11,12-hexahydro-7H,14H-pyrazin o[1',2':5.6][1,5]diazocino[3,2,1-hi]indol-3-yl)-2-amino-7-fluorobenzo[b]thiophene-3-car bonitrile 13-P1

### (S)-4-((S)-10-Acryloyl-2,4-difluoro-14-oxo-8,8a,9,10,11,12-hexahydro-7H,14H-pyrazin o[1',2':5.6][1,5]diazocino[3,2,1-hi]indol-3-yl)-2-amino-7-fluorobenzo[b]thiophene-3-car bonitrile 13-P2

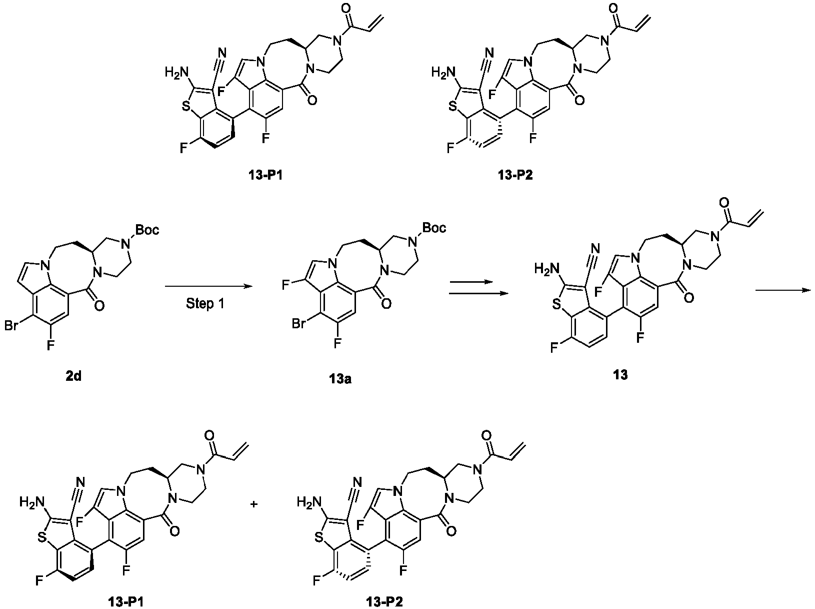

### Step 1

### (S)-3-Bromo-2,4-difluoro-14-oxo-7,8,8a,9,11,12-hexahydro-10H,14H-pyrazino[1',2':5,6 ][1,5]diazocino[3,2,1-hi]indole-10-carboxylic acid tert-butyl 13a

Compound **2d** (300 mg, 0.66 mmol) was dissolved in acetonitrile (6 mL), and 1-chloromethyl-4-fluoro-1,4-diazabicyclo[2.2.2]octane bis(tetrafluoroborate) (211 mg, 0.59 mmol) was added under an ice bath. The mixture was stirred for 6 h. Saturated sodium bicarbonate solution was added to the reaction mixture, and extraction was performed with ethyl acetate (10 mL × 3). The organic phases were combined and dried over anhydrous sodium sulfate, and the drying agent was removed by filtration. The filtrate was concentrated under reduced pressure, and the residue was purified by preparative high performance liquid chromatography (Waters-2545, column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compound **13a** (68 mg, yield: 21.8%).

MS m/z (ESI): 470.1 [M+1].

Subsequently, the title compounds **13-P2** (5 mg, yield: 6.4%) and **13-P1** (5 mg, yield: 6.4%) were obtained by using the synthesis scheme of Example **1-P1** and **1-P2** and replacing the starting material of step 5, compound 1g, with compound 13a.
Single-configuration compound (shorter retention time) **13-P2** (5 mg, yield: 6.4%)

MS m/z (ESI): 536.2 [M+1].

Preparative HPLC analysis: retention time: 15.17 min; purity: 99% (SharpSil-T C18, 50 × 250 mm, 5 µm; mobile phase: aqueous phase (10 mmol/L ammonium bicarbonate) and acetonitrile; gradient ratio: acetonitrile 35%-42%; flow rate: 80 mL/min).

¹H NMR (500 MHz, CD₃OD): δ 7.35 (d, 2H), 7.22 (s, 1H 7.00 (dd, 1H), 6.87 (d, 1H 6.29 (d, 1H), 5.82 (s, 1H), 4.70 (d, 2H), 4.37 (d, 1H), 4.23 (d, 1H), 4.06 (s, 2H), 3.51 (s, 1H), 3.07 (s, 2H), 2.21 (t, 1H), 2.05 (d, 1H).
Single-configuration compound (longer retention time) **13-P1** (5 mg, yield: 6.4%)

MS m/z (ESI): 536.2 [M+1].

Preparative HPLC analysis: retention time: 16.02 min; purity: 99% (SharpSil-T C18, 50 × 250 mm, 5 µm; mobile phase: aqueous phase (10 mmol/L ammonium bicarbonate) and acetonitrile; gradient ratio: acetonitrile 35%-42%; flow rate: 80 mL/min).

¹H NMR (500 MHz, CD₃OD): δ 7.34-7.26 (m, 2H), 7.20 (d, 1H), 7.01 (dd, 1H), 6.80 (dd, 1H), 6.29 (dd, 1H), 5.82 (t, 1H), 4.70 (t, 2H), 4.35 (dd, 1H), 4.25 (s, 1H), 4.22-4.13 (m, 1H), 4.01 (s, 1H), 3.50 (s, 1H), 3.06 (dt, 2H), 2.24-2.15 (m, 1H), 2.05 (d, 1H).

### Example 14

### 4-((R)-10-Acryloyl-4-fluoro-6-oxo-8,9,10,11,11a,12-hexahydro-6H-pyrazino[2',1':3,4][ 1,4]diazepino[6,7,1-hi]indazol-3-yl)-2-amino-7-fluorobenzo[b]thiophene-3-carbonitrile 14

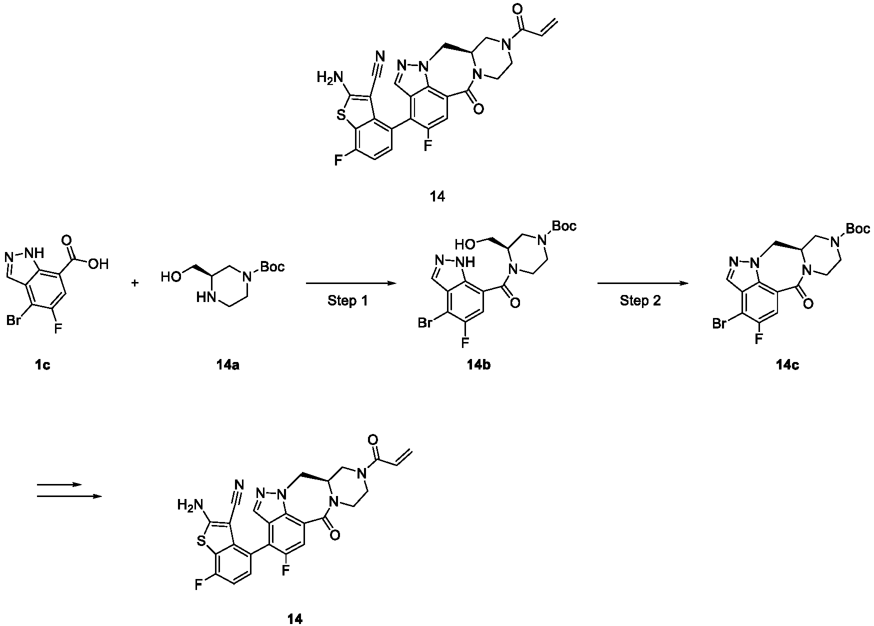

### Step 1

### tert-Butyl (R)-4-(4-bromo-5-fluoro-1H-indazole-7-carbonyl)-3-(hydroxymethyl)piperazine-1-carb oxylate 14b

The crude product of compound **1c** (560 mg, 2.16 mmol) and tert-butyl (*R*)-3-(hydroxymethyl)piperazine-1-carboxylate **14a** (514 mg, 2.37 mmol, Shanghai Accela ChemBio) were dissolved in 10 mL of *N*,*N*-dimethylformamide, and *N*,*N*-diisopropylethylamine (838 mg, 6.48 mmol) and 2-(7-azabenzotriazole)-*N*,*N*,*N*',*N*'-tetramethyluronium hexafluorophosphate (986 mg, 2.59 mmol) were added under an ice bath. The mixture was stirred for 1 h. The reaction mixture was concentrated under reduced pressure, then diluted with ethyl acetate, and washed with water, saturated sodium carbonate solution, and saturated sodium chloride solution in sequence. The organic phase was dried over anhydrous sodium sulfate and filtered. The filtrate was concentrated under reduced pressure, and the residue was purified by preparative high performance liquid chromatography (Waters-2545, column: SharpSil-T C18, 30 × 150 mm, 5 µm; mobile phase: aqueous phase (10 mmol/L ammonium bicarbonate) and acetonitrile; gradient ratio: acetonitrile 35%-45%; flow rate: 30 mL/min) to give the title compound **14b** (100 mg, yield: 10.1%).

MS m/z (ESI): 457.2 [M+1].

### Step 2

### tert-Butyl (R)-3-bromo-4-fluoro-6-oxo-8,9,11a,12-tetrahydro-6H-pyrazino[2',1':3,4][1,4]diazepino [6,7,1-hi]indazole-10(11H)-carboxylate 14c

Compound **14b** (250 mg, 546 µmol) and triethylamine (166 mg, 1.6 mmol) were dissolved in 5 mL of dichloromethane, and methanesulfonyl chloride (187 mg, 1.6 mmol) was added under an ice bath. The mixture was stirred for 2 h. The reaction mixture was quenched with a small amount of water and concentrated under reduced pressure, and the residue was dissolved in 5 mL of *N,N-*dimethylformamide. Potassium carbonate (226 mg, 1.6 mmol) was added, and the mixture was stirred at 80 °C for 1 h. The reaction mixture was cooled to room temperature and then filtered. The filtrate was concentrated under reduced pressure, and the residue was purified by silica gel column chromatography with eluent system B to give the title compound **14c** (30 mg, yield: 12.4%).

MS m/z (ESI): 439.29 [M+1].

Subsequently, the title compound **14** (5 mg, yield: 17.8%) was obtained by using the synthesis scheme of Example **1-P1** and **1-P2** and replacing the starting material of step 5, compound **1g**, with compound **14c**.

MS m/z (ESI): 505.2 [M+1].

¹H NMR (500 MHz, CD₃OD): δ 8.05 (d, 1H), 7.75 (s, 1H), 7.31 (d, 1H), 7.07 (t, 1H), 6.78 (ddd, 1H), 6.35-6.25 (m, 1H), 5.82 (dd, 1H), 4.79 (s, 1H), 4.77-4.73 (m, 1H), 4.73-4.65 (m, 1H), 4.51 (t, 1H), 4.41 (ddd, 1H), 4.14 (ddd, 1H), 4.02-3.96 (m, 1H), 2.19 (t, 1H), 2.03 (d, 1H).

### Example 15-P1, 15-P2

### (R)-2-Amino-7-fluoro-4-((S)-2-fluoro-10-(2-fluoroacryloyl)-4-methyl-14-oxo-8,8a,9,10, 11,12-hexahydro-7H,14H-pyrazino[1',2':5,6][1,5]diazocino[3,2,1-hi]indazol-3-yl)benzo [b]thiophene-3-carbonitrile 15-P1

### (S)-2-Amino-7-fluoro-4-((S)-2-fluoro-10-(2-fluoroacryloyl)-4-methyl-14-oxo-8,8a,9,10, 11,12-hexahydro-7H,14H-pyrazino[1',2':5,6][1,5]diazocino[3,2,1-hi]indazol-3-yl)benzo [b]thiophene-3-carbonitrile 15-P2

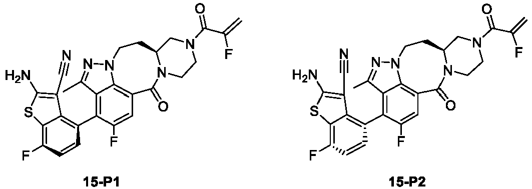

The title compounds **15-P2** (50 mg, yield: 24.9%) and **15-P1** (50 mg, yield: 24.9%) were obtained by using the synthesis scheme of Example **9-P1** and **9-P2** and replacing the starting material of step 8, acryloyl chloride, with 2-fluoroacryloyl chloride. Single-configuration compound (shorter retention time) **15-P2** (50 mg, yield: 24.9%) MS m/z (ESI): 551.3 [M+1].

HPLC analysis: retention time: 1.27 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.62 (d, 1H), 7.21 (dd, 1H), 7.06 (dd, 1H), 5.39-5.26 (m, 2H), 4.76 (d, 1H), 4.65-4.59 (m, 1H), 4.38-3.96 (m, 3H), 3.47 (s, 1H), 3.21-2.99 (m, 2H), 2.35-2.17 (m, 1H), 2.05 (d, 1H), 1.85 (s, 4H).
Single-configuration compound (longer retention time) **15-P1** (50 mg, yield: 24.9%)

MS m/z (ESI): 551.3 [M+1].

HPLC analysis: retention time: 1.28 min; purity: 99% (column: ACQUITY UPLC^{®}BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.56 (d, 1H), 7.35 (dd, 1H), 7.08 (dd, 1H), 5.42-5.22 (m, 2H), 4.80-4.71 (m, 1H), 4.69-4.59 (m, 1H), 4.37-3.98 (m, 3H), 3.55-3.36 (m, 1H), 3.15 (td, 2H), 2.32-2.02 (m, 2H), 1.95-1.76 (m, 4H).

### Example 16-P1, 16-P2

### (R)-2-Amino-4-((S)-2,4-difluoro-10-(2-fluoroacryloyl)-14-oxo-8,8a,9,10,11,12-hexahyd ro-7H,14H-pyrazino[1',2':5,6][1,5]diazocino[3,2,1-hi]indazol-3-yl)-7-fluorobenzo[b]thi ophene-3-carbonitrile 16-P1

### (S)-2-Amino-4-((S)-2,4-difluoro-10-(2-fluoroacryloyl)-14-oxo-8,8a,9,10,11,12-hexahyd ro-7H,14H-pyrazino[1',2':5,6][1,5]diazocino[3,2,1-hi]indazol-3-yl)-7-fluorobenzo[b]thi ophene-3-carbonitrile 16-P2

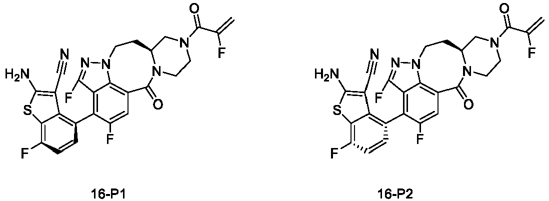

The title compounds **16-P2** (40 mg, yield: 24.7%) and **16-P1** (10 mg, yield: 6.1%) were obtained by using the synthesis scheme of Example **8-P1** and **8-P2** and replacing the starting material of step 6, acryloyl chloride, with 2-fluoroacryloyl chloride. Single-configuration compound (shorter retention time) **16-P2** (40 mg, yield: 24.7%) MS m/z (ESI): 555.3 [M+1].

HPLC analysis: retention time: 1.31 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.69 (d, 1H), 7.27 (dd, 1H), 7.04 (dd, 1H), 5.42-5.22 (m, 2H), 4.79-4.70 (m, 1H), 4.60 (s, 1H), 4.50 (ddd, 1H), 4.39-3.88 (m, 3H), 3.64-3.35 (m, 1H), 3.29-2.87 (m, 2H), 2.35-2.18 (m, 1H), 2.02-1.89 (m, 1H).
Single-configuration compound (longer retention time) **16-P1** (10 mg, yield: 6.1%)

MS m/z (ESI): 555.3 [M+1].

HPLC analysis: retention time: 1.33 min; purity: 96% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.64 (d, 1H), 7.36 (dd, 1H), 7.06 (dd, 1H), 5.41-5.27 (m, 2H), 4.74 (d, 1H), 4.68-4.43 (m, 2H), 4.37-3.97 (m, 3H), 3.55-3.35 (m, 1H), 3.24-2.87 (m, 2H), 2.41-2.13 (m, 1H), 2.00-1.78 (m, 1H).

### Example 17-P1, 17-P2

### (R)-2-Amino-4-((S)-4-chloro-2-fluoro-10-(2-fluoroacryloyl)-14-oxo-8,8a,9,10,11,12-he xahydro-7H,14H-pyrazino[1',2':5,6][1,5]diazocino[3,2,1-hi]indazol-3-yl)-7-fluorobenzo [b]thiophene-3-carbonitrile 17-P1

### (S)-2-Amino-4-((S)-4-chloro-2-fluoro-10-(2-fluoroacryloyl)-14-oxo-8,8a,9,10,11,12-he xahydro-7H,14H-pyrazino[1',2':5,6][1,5]diazocino[3,2,1-hi]indazol-3-yl)-7-fluorobenzo [b]thiophene-3-carbonitrile 17-P2

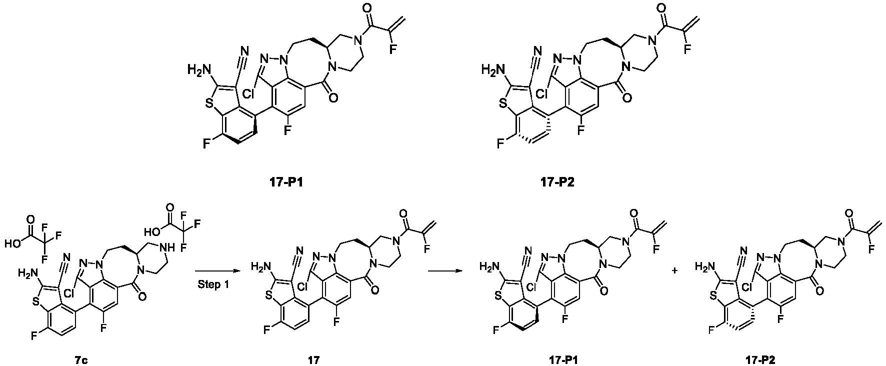

Compound **7c** (299 mg, 599.2 µmol) was suspended in 2 mL of ethyl acetate, 1 mL of tetrahydrofuran, and 2 mL of water, and anhydrous potassium carbonate (414 mg, 2.99 mmol) was added. 2-Fluoroacryloyl chloride (322 mg, 2.98 mmol) was added under an ice bath. After 5 min of reaction, extraction was performed with ethyl acetate (5 mL × 2). The organic phases were combined and concentrated under reduced pressure to give a crude product of compound **17,** i.e., 2-amino-4-((*S*)-4-chloro-2-fluoro-10-(2-fluoroacryloyl)-14-oxo-8,8*a*,9,10,11,12-hexahy dro-7*H*,14*H*-pyrazino[1',2':5,6][1,5]diazocino[3,2,1-*hi*]indazol-3-yl)-7-fluorobenzo[*b*]th iophene-3-carbonitrile (340 mg). The crude product was purified by preparative high performance liquid chromatography (Waters-2545; column: YMC Triart-Exrs, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compounds **17-P2** (50 mg, yield: 14.6%) and **17-P1** (50 mg, yield: 14.6%). Single-configuration compound (shorter retention time) **17-P2** (50 mg, yield: 14.6%) MS m/z (ESI): 571.2 [M+1].

HPLC analysis: retention time: 2.42 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.67 (d, 1H), 7.19 (dd, 1H), 7.02 (dd, 1H), 5.35 (t, 1H), 5.26 (s, 1H), 4.73 (dt, 2H), 4.64 (ddd, 2H), 4.30 (ddd, 2H), 4.09 (s, 1H), 3.20-3.10 (m, 2H), 2.07-1.89 (m, 2H).
Single-configuration compound (longer retention time) **17-P1** (50 mg, yield: 14.6%)

MS m/z (ESI): 571.2 [M+1].

HPLC analysis: retention time: 2.47 min; purity: 99% (column: ACQUITY UPLC^{®} BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.63 (d, 1H), 7.31 (dd, 1H), 7.04 (dd, 1H), 5.35 (d, 1H), 5.25 (d, 1H), 4.72 (dt, 2H), 4.64 (ddd, 2H), 4.27 (ddd, 2H), 4.10 (s, 1H), 3.19-3.10 (m, 2H), 1.95-1.83 (m, 2H).

### Example 18-P1, 18-P2

### (R)-4-((S)-10-Acryloyl-2-fluoro-4-methyl-14-oxo-8,8a,9,10,11,12-hexahydro-7H,14H-p yrazino[1',2":5,6][1,5]diazocino[3,2,1-hi]indol-3-yl)-2-amino-7-fluorobenzo[b]thiophen e-3-carbonitrile 18-P1

### (S)-4-((S)-10-Acryloyl-2-fluoro-4-methyl-14-oxo-8,8a,9,10,11,12-hexahydro-7H,14H-p yrazino[1',2':5,6][1,5]diazocino[3,2,1-hi]indol-3-yl)-2-amino-7-fluorobenzo[b]thiophen e-3-carbonitrile 18-P2

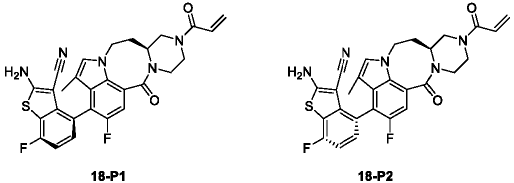

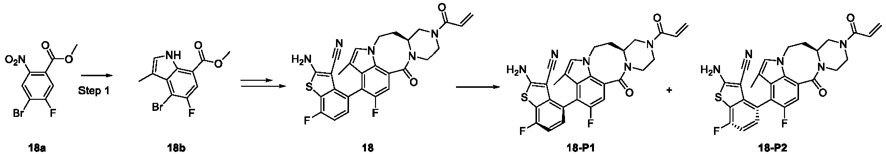

### Step 1

### Methyl 4-bromo-5-fluoro-3-methyl-1H-indole-7-carboxylate 18b

Compound **18a** (2.1 g, 7.95 mmol, Shanghai Bide) was dissolved in anhydrous tetrahydrofuran (15 mL), and a 0.5 M solution of 1-propenylmagnesium bromide in tetrahydrofuran (40 mL, Sigma-Aldrich) was added in a nitrogen atmosphere at -40 °C. The mixture was warmed to room temperature and left to react for 5 h. The reaction mixture was quenched with saturated ammonium chloride solution and extracted with ethyl acetate (30 mL × 3). The organic phases were combined and dried over anhydrous sodium sulfate, and the drying agent was removed by filtration. The filtrate was concentrated under reduced pressure, and the residue was purified by silica gel column chromatography with eluent system A to give the title compound **18b** (200 mg, yield: 8.7%).

MS m/z (ESI): 285.9 [M+1].

Subsequently, the title compounds **18-P2** (3 mg, yield: 5.5%) and **18-P1** (10 mg, yield: 18.3%) were obtained by using the synthesis scheme of Example **1-P1** and **1-P2** and replacing the starting material of step 2, **1b**, with compound **18b**.
Single-configuration compound (shorter retention time) **18-P2** (3 mg, yield: 5.5%)

MS m/z (ESI): 532.3 [M+1].

Preparative HPLC analysis: retention time: 12.30 min; purity: 99% (SharpSil-T C18, 30 × 150 mm, 5 µm; mobile phase: aqueous phase (10 mmol/L ammonium bicarbonate) and acetonitrile; gradient ratio: acetonitrile 35%-45%; flow rate: 30 mL/min).

¹H NMR (500 MHz, CD₃OD): δ 7.27 (d, 1H), 7.13 (s, 1H), 7.05-6.96 (m, 2H), 6.81 (ddd, 1H), 6.29 (d, 1H), 5.86-5.78 (m, 1H), 4.74-4.68 (m, 1H), 4.45-4.22 (m, 2H), 4.02 (dq, 3H), 3.14-2.90 (m, 2H), 2.24-2.02 (m, 2H), 1.83 (s, 1H), 1.58-1.55 (m, 3H).
Single-configuration compound (longer retention time) **18-P1** (10 mg, yield: 18.3%)

MS m/z (ESI): 532.3 [M+1].

Preparative LCMS analysis: retention time: 12.87 min; purity: 99% (SharpSil-T C18, 30 × 150 mm, 5 µm; mobile phase: aqueous phase (10 mmol/L ammonium bicarbonate) and acetonitrile; gradient ratio: acetonitrile 35%-45%; flow rate: 30 mL/min).

¹H NMR (500 MHz, CD₃OD): δ 7.29 (dd, 1H), 7.20 (d, 1H), 7.07-7.00 (m, 2H), 6.93-6.67 (m, 1H), 6.29 (dd, 1H), 5.82 (dd, 1H), 4.70 (d, 1H), 4.38-4.13 (m, 3H), 4.0-3.86 (m, 2H), 3.12-2.90 (m, 2H), 2.18-2.12 (m, 1H), 2.07-2.04 (m, 1H), 1.79 (d, 1H), 1.52 (s, 3H).

### Example 19-P1, 19-P2

### (R)-4-((S)-10-Acryloyl-4-chloro-2-fluoro-14-oxo-8,8a,9,10,11,12-hexahydro-7H,14H-p yrazino[1',2':5,6][1,5]diazocino[3,2,1-hi]indol-3-yl)-2-amino-7-fluorobenzo[b]thiophen e-3-carbonitrile 19-P1

### (S)-4-((S)-10-Acryloyl-4-chloro-2-fluoro-14-oxo-8,8a,9,10,11,12-hexahydro-7H,14H-p yrazino[1',2':5,6][1,5]diazocino[3,2,1-hi]indol-3-yl)-2-amino-7-fluorobenzo[b]thiophen e-3-carbonitrile 19-P2

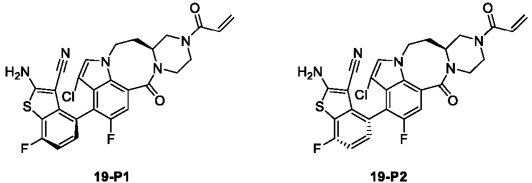

The title compounds **19-P2** (5 mg, yield: 50%) and **19-P1** (5 mg, yield: 50%) (5 mg, 5 mg, yield: 50%, 50%) were obtained by using the synthesis scheme of Example **7-P1** and **7-P2** and replacing the starting material of step 1, **1g,** with compound **2d.** Single-configuration compound (shorter retention time) **19-P2** (5 mg, yield: 50%)

MS m/z (ESI): 552.3 [M+1].

Preparative HPLC analysis: retention time: 12.30 min; purity: 99% (column: Welch Xtimate Phenyl-hexyl, 30 × 150 mm, 5 µm; mobile phase: aqueous phase (10 mmol/L ammonium bicarbonate) and acetonitrile; gradient ratio: acetonitrile 40%-55%; flow rate: 30 mL/min).

¹H NMR (500 MHz, CD₃OD): δ 7.38-7.35 (m, 2H), 7.16 (s, 1H), 6.99 (dd, 1H), 6.92-6.70 (m, 1H), 6.29 (d, 1H), 5.88-5.78 (m, 1H), 4.70 (d, 1H), 4.45-4.30 (m, 2H), 4.12-3.94 (m, 2H), 3.52-3.46 (m, 1H), 3.15-2.92 (m, 2H), 2.27-2.15 (m, 1H), 1.93-1.83 (m, 1H), 0.96-0.88 (m, 1H).
Single-configuration compound (longer retention time) **19-P1** (5 mg, yield: 50%)

MS m/z (ESI): 552.3 [M+1].

Preparative HPLC analysis: retention time: 12.83 min; purity: 99% (column: Welch Xtimate Phenyl-hexyl, 30 × 150 mm, 5 µm; mobile phase: aqueous phase (10 mmol/L ammonium bicarbonate) and acetonitrile; gradient ratio: acetonitrile 40%-55%; flow rate: 30 mL/min).

¹H NMR (500 MHz, CD₃OD): δ 7.35 (s, 1H), 7.34-7.25 (m, 2H), 7.01 (dd, 1H), 6.93-6.67 (m, 1H), 6.29 (dd, 1H), 5.82 (t, 1H), 4.69 (dd, 1H), 4.40-4.25 (m, 2H), 4.16 (d, 1H), 4.02 (s, 1H), 3.56-3.43 (m, 1H), 3.16-2.91 (m, 2H), 2.26-2.02 (m, 1H), 1.93-1.83 (m, 1H), 0.92 (t, 1H).

### Example 20-P1, 20-P2

### (R)-4-((S)-9-Acryloyl-2-fluoro-12-oxo-7,7a,8,9,10,11-hexahydro-6H,12H-4,5,5a,9,11a-pentaazabenzo[5,6]cycloocta[1,2,3-cd]inden-3-yl)-2-amino-7-fluorobenzo[b]thiophene-3-carbonitrile 20-P1

### (S)-4-((S)-9-Acryloyl-2-fluoro-12-oxo-7,7a,8,9,10,11-hexahydro-6H,12H-4,5,5a,9,11a-pentaazabenzo[5,6]cycloocta[1,2,3-cd]inden-3-yl)-2-amino-7-fluorobenzo[b]thiophene-3-carbonitrile 20-P2

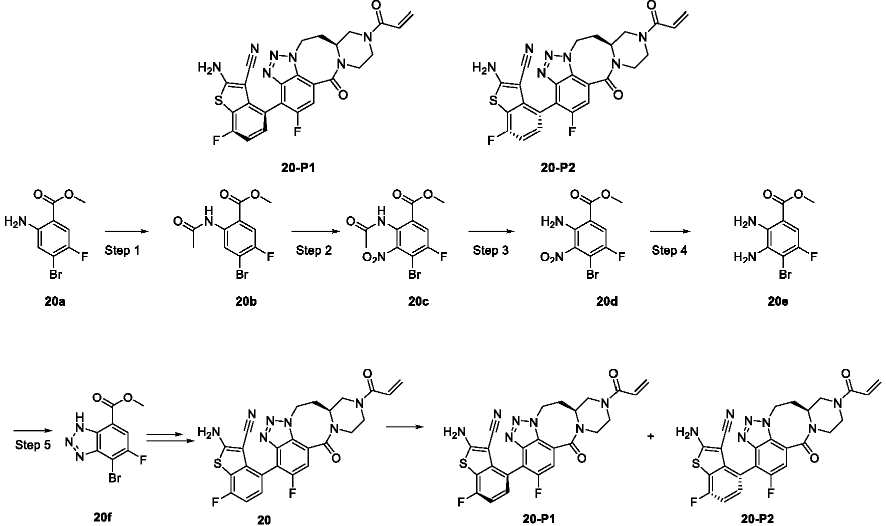

### Step 1

### Methyl 2-acetamido-4-bromo-5-fluorobenzoate 20b

Methyl 2-amino-4-bromo-5-fluorobenzoate **20a** (4 g, 16.12 mmol, Shanghai Bide) was dissolved in acetic anhydride (20 mL), and the solution was left to react at 90 °C for 1 h. The reaction mixture was concentrated under reduced pressure, and the residue was dissolved in dichloromethane. The solution was washed with saturated sodium carbonate and dried over anhydrous sodium sulfate, and the drying agent was removed by filtration. The filtrate was concentrated under reduced pressure to give a crude product of the title compound **20b** (6.5 g). The product was directly used in the next step without purification.

MS m/z (ESI): 289.9 [M+1].

### Step 2

### Methyl 2-acetamido-4-bromo-5-fluoro-3-nitrobenzoate 20c

The crude product of compound **20b** (6 g, 20.68 mmol) was dissolved in concentrated sulfuric acid (30 mL), and nitric acid (15 mL) was added dropwise under an ice bath. The mixture was left to react for 1 h with the temperature maintained. The reaction mixture was poured into ice water and extracted with dichloromethane (10 mL × 3). The organic phases were combined, washed with saturated sodium bicarbonate solution, and dried over anhydrous sodium sulfate, and the drying agent was removed by filtration. The filtrate was concentrated under reduced pressure to give a crude product of the title compound **20c** (1.5 g). The product was directly used in the next step without purification.

MS m/z (ESI): 334.9 [M+1].

### Step 3

### Methyl 2-amino-4-bromo-5-fluoro-3-nitrobenzoate 20d

The crude product of compound **20c** (3.2 g, 9.5 mmol) was dissolved in ethanol (32 mL), and hydrochloric acid (32 mL) was added. The mixture was left to react at 80 °C for 3 h. The reaction mixture was concentrated under reduced pressure. Saturated sodium bicarbonate solution (20 mL) was added, and extraction was performed with dichloromethane (20 mL × 3). The organic phases were combined and dried over anhydrous sodium sulfate, and the drying agent was removed by filtration. The filtrate was concentrated under reduced pressure to give a crude product of the title compound **20d** (6.5 g). The product was directly used in the next step without purification.

MS m/z (ESI): 292.9 [M+1].

### Step 4

### Methyl 2,3-diamino-4-bromo-5-fluorobenzoate 20e

The crude product of compound **20d** (500 mg, 1.7 mmol) was dissolved in ethanol (25 mL) and water (5 mL), and ammonium chloride (910 mg, 17.01 mmol) and iron powder (500 mg, 8.9 mmol) were added. The mixture was left to react at 60 °C for 1 h. The reaction mixture was concentrated under reduced pressure, and the residue was purified by silica gel column chromatography with eluent system B to give the title compound **20e** (400 mg, yield: 89.1%).

MS m/z (ESI): 262.9 [M+1].

### Step 5

### Methyl 4-bromo-5-fluoro-1H-benzo[d][1,2,3]triazole-7-carboxylate 20f

The crude product of compound **20e** (600 mg, 2.28 mmol) was dissolved in glacial acetic acid (6 mL), and sodium nitrite (188 mg, 2.72 mmol) was added. The mixture was left to react for 30 min. The reaction mixture was filtered, and the filter cake was washed with water and dried to give a crude product of the title compound **20f** (600 mg). The product was directly used in the next step without purification.

MS m/z (ESI): 273.9 [M+1].

Subsequently, the title compounds **20-P2** (6 mg, yield: 27.4%) and **20-P1** (5 mg, yield: 22.8%) were obtained by using the synthesis scheme of Example **1-P1** and **1-P2** and replacing the starting material of step 2, compound **1b,** with compound **20f.**
Single-configuration compound (shorter retention time) **20-P2** (6 mg, yield: 27.4%)

MS m/z (ESI): 519.2 [M+1].

HPLC analysis: retention time: 1.531 min; purity: 99% (column: HALO^{®} 90 Å, C18, 2.7 µm, 3.0 × 30 mm; mobile phase: water (5 mM ammonium acetate), acetonitrile; gradient ratio: acetonitrile 10%-95%).

¹H NMR (500 MHz, CDCl₃): δ 7.92-7.83 (m, 1H), 7.45 (s, 1H), 7.09 (br, 1H), 6.43 (d, 1H), 5.84 (br, 1H), 5.32-5.28 (m, 3H), 4.91 (br, 1H), 4.47 (br, 1H), 4.10 (br, 1H), 3.06 (br, 1H), 2.24 (br, 1H), 2.04 (br, 2H), 1.65 (br, 1H), 0.90 (t, 1H).
Single-configuration compound (longer retention time) **20-P1** (5 mg, yield: 22.8%)

MS m/z (ESI): 519.2 [M+1].

HPLC analysis: retention time: 1.545 min; purity: 99% (column: HALO^{®} 90 Å, C18, 2.7 µm, 3.0 × 30 mm; mobile phase: water (5 mM ammonium acetate), acetonitrile; gradient ratio: acetonitrile 10%-95%).

¹H NMR (500 MHz, CDCl₃): δ 7.85 (d, 1H), 7.53-7.48 (m, 1H), 7.19-7.04 (m, 1H), 6.73-6.35 (m, 2H), 5.82 (br, 1H), 5.33-5.11 (m, 3H), 4.87 (br, 1H), 4.56-4.34 (m, 1H), 4.22-3.98 (m, 1H), 3.52-2.87 (m, 2H), 2.39 (br, 1H), 2.10-1.95 (m, 1H), 0.90 (t, 1H).

### Example 21-P1, 21-P2

### (R)-4-((S)-9-Acryloyl-2-fluoro-12-oxo-7,7a,8,9,10,11-hexahydro-6H,12H-4,5a,9,11a-te traazabenzo[5,6]cycloocta[1,2,3-cd]inden-3-yl)-2-amino-7-fluorobenzo[b]thiophene-3-c arbonitrile 21-P1

### (S)-4-((S)-9-Acryloyl-2-fluoro-12-oxo-7,7a,8,9,10,11-hexahydro-6H,12H-4,5a,9,11a-tet raazabenzo[5,6]cycloocta[1,2,3-cd]inden-3-yl)-2-amino-7-fluorobenzo[b]thiophene-3-c arbonitrile 21-P2

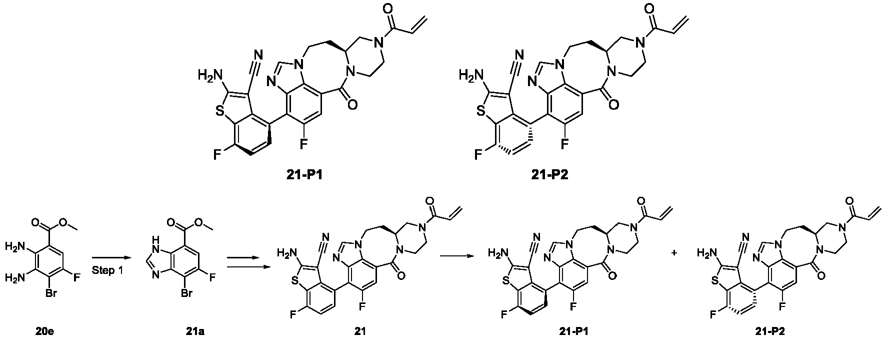

### Step 1

### Methyl 4-bromo-5-fluoro-1H-benzo[d]imidazole-7-carboxylate 21a

Compound **20e** (500 mg, 1.9 mmol) was dissolved in triethyl orthoformate (5 mL), and 4-p-toluenesulfonic acid (98.1 mg, 569.6 µmol) was added. The mixture was stirred for 5 min, and a large amount of solid precipitated. Saturated aqueous sodium bicarbonate was added to the reaction mixture, and extraction was performed with ethyl acetate (10 mL × 3). The organic phases were combined and dried over anhydrous sodium sulfate, and the drying agent was removed by filtration. The filtrate was concentrated under reduced pressure to give a crude product of the title compound **21a** (500 mg). The product was directly used in the next step without purification.

MS m/z (ESI): 272.9 [M+1].

Subsequently, the title compounds **21-P2** (3 mg, yield: 38.3%) and **21-P1** (2 mg, yield: 25.5%) were obtained by using the synthesis scheme of Example **1-P1** and **1-P2** and replacing the starting material of step 2, compound **1b,** with compound **21a.**
Single-configuration compound (shorter retention time) **21-P2** (3 mg, yield: 38.3%)

MS m/z (ESI): 519.2 [M+1].

HPLC analysis: retention time: 1.455 min; purity: 99% (column: HALO^{®} 90 Å, C18, 2.7 µm, 3.0 × 30 mm; mobile phase: water (5 mM ammonium acetate), acetonitrile; gradient ratio: acetonitrile 10%-95%).

¹H NMR (500 MHz, DMSO-*d₆*): δ 8.26 (s, 1H), 7.36 (d, 1H), 7.14 (t, 1H), 6.87 (s, 1H), 6.66 (s, 1H), 6.18 (d, 1H), 6.05 (s, 2H), 5.74 (d, 1H), 4.58 (d, 2H), 4.24 (s, 1H), 3.92 (d, 2H), 3.63 (s, 2H), 2.86 (s, 2H), 2.15 (s, 1H), 2.01 (s, 1H).
Single-configuration compound (longer retention time) **21-P1** (2 mg, yield: 25.5%)

MS m/z (ESI): 519.2 [M+1].

HPLC analysis: retention time: 1.465 min; purity: 99% (column: HALO^{®} 90 Å, C18, 2.7 µm, 3.0 × 30 mm; mobile phase: water (5 mM ammonium acetate), acetonitrile; gradient ratio: acetonitrile 10%-95%).

¹H NMR (500 MHz, DMSO-*d₆*): δ 8.27 (s, 1H), 7.94 (s, 2H), 7.38 (d, 1H), 7.27 (dd, 1H), 7.12 (t, 1H), 6.80 (d, 1H), 6.18 (t, 1H), 5.75 (s, 1H), 4.56 (s, 2H), 4.24 (d, 1H), 4.03-3.89 (m, 2H), 3.47 (s, 2H), 3.09-2.93 (m, 2H), 2.14 (s, 1H), 1.79 (s, 1H).

### Biological Evaluations

The present disclosure is further described and explained below with reference to test examples. However, these test examples are not intended to limit the scope of the present disclosure.

### Test Example 1: H358 Proliferation Assay

The inhibitory activity of the compounds of the present disclosure against H358 cell proliferation was measured. Below is a brief description of the experimental method: H358 cells (ATCC, CRL-5807) were cultured in RPMI1640 medium (Hyclone, SH30809.01) containing 10% fetal bovine serum (Corning, 35-076-CV) (i.e., complete medium). On the first day of the experiment, H358 cells were seeded into a 96-well plate at a density of 1200 cells/well using complete medium, with 100 µL of cell suspension per well, and the plate was incubated overnight in a 37 °C, 5% CO₂ cell incubator. On the second day, the test compounds, prepared in complete medium and serially diluted, were added at 10 µL/well. The final concentrations of the compounds were 9 concentration points obtained by a 5-fold serial dilution from 10 µM. A blank control containing 0.5% DMSO was set up. The plate was incubated for 120 h in a 37 °C, 5% CO₂ cell incubator. On the seventh day, the 96-well cell plate was removed from the incubator, and CellTiter-Glo^{®} Luminescent Cell Viability Assay (Promega, G7573) was added at 50 µL/well. After 10 min of room temperature incubation, luminescence signals were measured using a multifunctional microplate reader (PerkinElmer, EnVision^{®} 2105). The IC₅₀ values for the inhibitory activity of the compounds were calculated using Graphpad Prism software.

**Table 1. The inhibitory activity of the compounds of the present disclosure against H358 cell proliferation**

| Compound No. | IC₅₀ (nM) |
|---|---|
| 1-P2 | 0.93 |
| 1-P1 | 11.98 |
| 2 | 10.49 |
| 4 | 13.36 |
| 6-P2 | 5.73 |
| 6-P1 | 9.75 |
| 7-P2 | 0.74 |
| 8-P2 | 0.84 |
| 9-P2 | 0.52 |
| 10 | 0.8 |
| 11-P2 | 0.32 |
| 11-P1 | 2.25 |
| 13-P2 | 11.44 |
| 15-P2 | 4.21 |
| 15-P1 | 41.59 |
| 16-P2 | 8.00 |
| 17-P2 | 1.87 |
| 18-P2 | 9.57 |
| 19-P2 | 5.65 |
| 20-P2 | 7.90 |
| 20-P1 | 4.94 |

| | |
|---|---|
| Conclusion: The compounds of the present disclosure have inhibitory effects on H358 cell proliferation. | |

### Test Example 2: H358 Cell ERK Phosphorylation Inhibition Assay

The inhibitory effects of the compounds of the present disclosure on ERK phosphorylation in H358 cells were measured. Below is a brief description of the experimental method:
H358 cells (ATCC, CRL-5807) were cultured in RPMI1640 medium (Hyclone, SH30809.01) containing 10% fetal bovine serum (Corning, 35-076-CV) (i.e., complete medium). On the first day of the experiment, H358 cells were seeded into a 96-well plate at a density of 25,000 cells/well using complete medium, with 190 µL of cell suspension per well, and the plate was incubated overnight in a 37 °C, 5% CO₂ cell incubator. On the second day, the test compounds, prepared in complete medium and serially diluted, were added at 10 µL/well. The final concentrations of the compounds were 9 concentration points obtained by a 6-fold serial dilution from 10 µM. A blank control containing 0.5% DMSO was set up. The plate was incubated for 3 h in a 37 °C, 5% CO₂ cell incubator. After 3 h, the 96-well cell culture plate was removed from the incubator, and the culture medium was pipetted off. PBS (Shanghai BasalMedia Technologies Co., Ltd., B320) was added at 200 µL/well for a single wash. PBS was pipetted off, and lysis buffer (Cisbio, 64KL1FDF) containing a blocking agent (Cisbio, 64KB1AAC) was added at 50 µL/well. The plate was incubated on a shaker for 30 min at room temperature to lyse the cells. After lysis, the lysates were well mixed by pipetting, and 16 µL of lysate was transferred from each well to two HTRF 96-well assay plates (Cisbio, 66PL96100). Subsequently, 4 µL of a premixed phospho-ERK1/2 antibody solution (Cisbio, 64AERPEG) or 4 µL of a premixed total-ERK1/2 antibody solution (Cisbio, 64NRKPEG) was added to each of the plates. The plates were sealed with a plate sealer film, centrifuged for 1 min in a microplate centrifuge, and incubated overnight at room temperature in the dark. On the third day, fluorescence values at an excitation wavelength of 337 nm and emission wavelengths of 665 nm and 620 nm were measured using a PHERAstar multifunctional microplate reader (BMG Labtech, PHERAstar FS). The IC₅₀ values for the inhibitory activity of the compounds were calculated using Graphpad Prism software, based on the compound concentration and the ratio of pERK/total ERK.

**Table 2. The inhibitory effects of the compounds of the present disclosure on ERK phosphorylation in H358 cells**

| Compound No. | IC₅₀ (nM) |
|---|---|
| 1-P2 | 0.07 |
| 1-P1 | 1.33 |
| 2 | 0.28 |
| 3 | 23.74 |
| 4 | 1.16 |
| 5 | 36.21 |
| 6-P2 | 0.35 |
| 6-P1 | 1.45 |
| 7-P2 | 0.13 |
| 8-P2 | 0.16 |
| 9-P2 | 0.06 |
| 9-P1 | 32.55 |
| 10 | 0.15 |
| 11-P2 | 0.09 |
| 11-P1 | 7.67 |
| 12-P2 | 11.32 |
| 13-P2 | 0.76 |
| 14 | 1.35 |
| 15-P2 | 3.94 |
| 15-P1 | 19.71 |
| 16-P2 | 1.06 |
| 17-P2 | 2.0 |
| 18-P2 | 1.09 |
| 19-P2 | 0.97 |
| 20-P2 | 0.88 |
| 20-P1 | 3.67 |

| | |
|---|---|
| Conclusion: The compounds of the present disclosure have inhibitory effects on ERK phosphorylation in H358 cells. | |

### Test Example 3: MIA PaCa-2 Cell Proliferation Assay

The inhibitory activity of the compounds of the present disclosure against MIA PaCa-2 cell proliferation was measured. Below is a brief description of the experimental method:
MIA PaCa-2 cells (ATCC, CRL-1420) were cultured in DMEM/HIGH GLUCOSE medium (GE, SH30243.01) containing 10% fetal bovine serum (Corning, 35-076-CV) and 2.5% horse serum (Beyotime Biological Tech., C0262) (i.e., complete medium). On the first day of the experiment, MIA PaCa-2 cells were seeded into a 96-well plate at a density of 500 cells/well using complete medium, with 90 µL of cell suspension per well, and the plate was incubated overnight in a 37 °C, 5% CO₂ cell incubator. On the second day, the test compounds, prepared in complete medium and serially diluted, were added at 10 µL/well. The final concentrations of the compounds were 9 concentration points obtained by a 5-fold serial dilution from 10 µM. A blank control containing 0.5% DMSO was set up. The plate was incubated for 72 h in a 37 °C, 5% CO₂ cell incubator. On the fifth day, the 96-well cell plate was removed from the incubator, and CellTiter-Glo^{®} Luminescent Cell Viability Assay (Promega, G7573) was added at 50 µL/well. After 10 min of room temperature incubation, luminescence signals were measured using a multifunctional microplate reader (PerkinElmer, EnVision2015). The IC₅₀ values for the inhibitory activity of the compounds were calculated using Graphpad Prism software.

**Table 3. The inhibitory activity of the compounds of the present disclosure against MIA PaCa-2 cell proliferation**

| Compound No. | IC₅₀ (nM) |
|---|---|
| 1-P2 | 0.40 |
| 1-P1 | 2.79 |
| 2 | 5.87 |
| 4 | 11.15 |
| 6-P2 | 5.39 |
| 6-P1 | 13.87 |
| 7-P2 | 0.28 |
| 8-P2 | 0.19 |
| 9-P2 | 0.37 |
| 10 | 0.94 |
| 11-P2 | 0.46 |
| 11-P1 | 2.71 |
| 12-P2 | 35.29 |
| 13-P2 | 20.54 |
| 14 | 39.58 |
| 15-P2 | 7.75 |
| 15-P1 | 56.84 |
| 16-P2 | 4.56 |
| 17-P2 | 1.88 |
| 18-P2 | 9.16 |
| 19-P2 | 4.90 |
| 20-P2 | 1.21 |
| 20-P1 | 1.66 |

| | |
|---|---|
| Conclusion: The compounds of the present disclosure have inhibitory effects on MIA PaCa-2 cell proliferation. | |

## Claims

1. A compound represented by general formula (IM) or a pharmaceutically acceptable salt thereof: 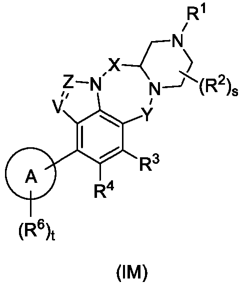 wherein:
X is C(R^{a}R^{b}) or C(R^{a}R^{b})-C(R^{c}R^{d});
Y is C(O) or CH₂;
Z is CR^{5a} or N;
V is CR⁵ or N;
ring A is aryl or heteroaryl;
R^{a}, R^{b}, R^{c}, and R^{d} are identical or different at each occurrence and are each independently selected from the group consisting of a hydrogen atom, halogen, alkyl, haloalkyl, hydroxyalkyl, alkoxy, haloalkoxy, alkenyl, alkynyl, hydroxy, and cyano;
R¹ is selected from the group consisting of cyano, 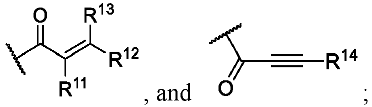
each R² is identical or different and is independently selected from the group consisting of a hydrogen atom, halogen, cyano, alkyl, alkoxy, hydroxy, and amino, wherein the alkyl and alkoxy are each independently optionally substituted with one or more substituents selected from the group consisting of halogen, cyano, amino, and hydroxy;
R³, R⁴, R⁵, and R^{5a} are identical or different and are each independently selected from the group consisting of a hydrogen atom, halogen, cyano, alkyl, alkenyl, alkynyl, -NR^{7a}R^{7b}, -C(O)R⁸, -OR⁸, -S(O)ₚR⁸, cycloalkyl, heterocyclyl, aryl and heteroaryl, wherein the alkyl, cycloalkyl, heterocyclyl, aryl, and heteroaryl are each independently optionally substituted with one or more substituents selected from the group consisting of halogen, alkyl, haloalkyl, cyano, -NR^{7c}R^{7d}, -OR^{8a}, cycloalkyl, heterocyclyl, aryl, and heteroaryl;
each R⁶ is identical or different and is independently selected from the group consisting of a hydrogen atom, halogen, cyano, alkyl, alkenyl, alkynyl, -NR^{9a}R^{9b}, -C(O)NR^{9a}R^{9b}, -C(O)R¹⁰, -C(O)OR¹⁰, -OC(O)R¹⁰, -OR¹⁰, -S(O)ₚR¹⁰, -S(O)ₚNR^{9a}R^{9b}, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, haloalkyl, cyano, -NR^{9c}R^{9d}, -OR^{10a}, cycloalkyl, heterocyclyl, aryl, and heteroaryl;
R¹¹, R¹², R¹³, and R¹⁴ are identical or different and are each independently selected from the group consisting of a hydrogen atom, halogen, alkyl, -NR^{15a}R^{15b}, -OR¹⁶, cyano, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, oxo, alkyl, haloalkyl, alkoxy, haloalkoxy, cyano, -NR^{15c}R^{15d}, hydroxy, hydroxyalkyl, cycloalkyl, heterocyclyl, aryl, and heteroaryl;
R⁸, R^{8a}, R¹⁰, R^{10a}, and R¹⁶ are identical or different at each occurrence and are each independently selected from the group consisting of a hydrogen atom, alkyl, hydroxyalkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkenyl, alkynyl, oxo, alkoxy, haloalkyl, haloalkoxy, cyano, -NR^{17a}R^{17b}, hydroxy, cycloalkyl, heterocyclyl, aryl, and heteroaryl;
R^{7a}, R^{7b}, R^{7c}, R^{7d}, R^{9a}, R^{9b}, R^{9c}, R^{9d}, R^{15a}, R^{15b}, R^{15c}, R^{15d}, R^{17a}, and R^{17b} are identical or different at each occurrence and are each independently selected from the group consisting of a hydrogen atom, alkyl, hydroxyalkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, oxo, hydroxy, cyano, alkyl, alkoxy, haloalkyl, and haloalkoxy;
or R^{7a} and R^{7b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{7c} and R^{7d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{9a} and R^{9b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{9c} and R^{9d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{15a} and R^{15b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{15c} and R^{15d}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
or R^{17a} and R^{17b}, together with the nitrogen atom to which they are attached, form heterocyclyl, wherein the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl, and heteroaryl;
s is 0, 1, 2, 3, 4, 5, or 6;
t is 0, 1, 2, 3, 4, or 5; and
p is 0, 1, or 2.

2. The compound represented by general formula (IM) or the pharmaceutically acceptable salt thereof according to claim 1, being a compound represented by general formula (I) or a pharmaceutically acceptable salt thereof: 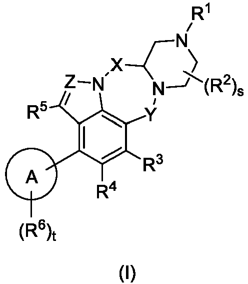 wherein:
ring A, X, Y, Z, R¹, R², R³, R⁴, R⁵, R⁶, s, and t are as defined in claim 1.

3. The compound represented by general formula (IM) or the pharmaceutically acceptable salt thereof according to claim 1 or 2, wherein Y is C(O); and/or s is 0 or 1.

4. The compound represented by general formula (IM) or the pharmaceutically acceptable salt thereof according to any of claims 1 to 3, being a compound represented by general formula (II) or a pharmaceutically acceptable salt thereof: 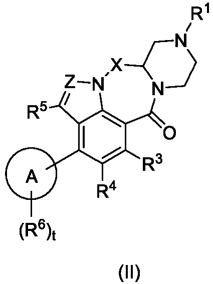 wherein:
ring A, X, Z, R¹, R³, R⁴, R⁵, R⁶, and t are as defined in claim 1.

5. The compound represented by general formula (IM) or the pharmaceutically acceptable salt thereof according to any of claims 1 to 4, wherein ring A is 8- to 10-membered bicyclic heteroaryl containing in the ring 1, 2, or 3 heteroatoms selected from the group consisting of nitrogen, oxygen, and sulfur, and/or
R¹ is 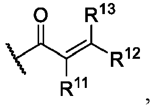 wherein R¹¹, R¹², and R¹³ are as defined in claim 1, and/or X is CH₂ or CH₂-CH₂; preferably, X is CH₂-CH₂.

6. The compound represented by general formula (IM) or the pharmaceutically acceptable salt thereof according to any of claims 1 to 5, being a compound represented by general formula (III) or a pharmaceutically acceptable salt thereof: 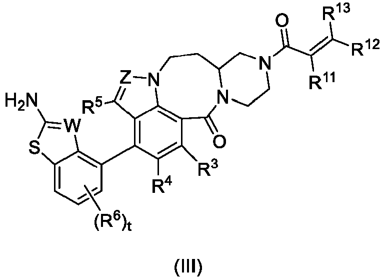 wherein:
W is C(CN) or N;
t is 0, 1, 2, or 3;
Z, R³, R⁴, R⁵, R⁶, R¹¹, R¹², and R¹³ are as defined in claim 1.

7. The compound represented by general formula (IM) or the pharmaceutically acceptable salt thereof according to any of claims 1 to 6, wherein R³, R⁴, R⁵, and R^{5a} are identical or different and are each independently selected from the group consisting of a hydrogen atom, halogen, C₁₋₆ alkyl, and C₁₋₆ haloalkyl, and/or R¹¹ is a hydrogen atom or halogen, and/or R¹² is a hydrogen atom, and/or R¹³ is a hydrogen atom.

8. The compound represented by general formula (IM) or the pharmaceutically acceptable salt thereof according to any of claims 1 to 7, wherein each R⁶ is identical or different and is independently selected from the group consisting of halogen, cyano, -NH₂, C₁₋₆ alkyl, and C₁₋₆ haloalkyl, and t is 0, 1, 2, or 3.

9. The compound represented by general formula (IM) or the pharmaceutically acceptable salt thereof according to any of claims 1 to 8, being selected from the group consisting of the following compounds:

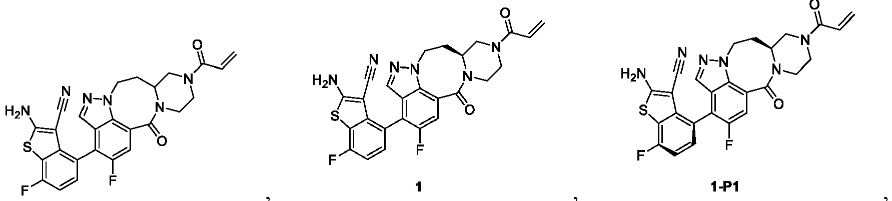

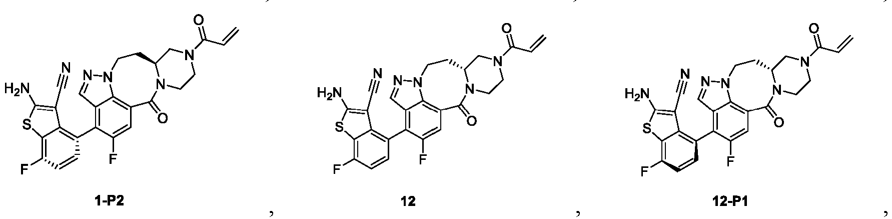

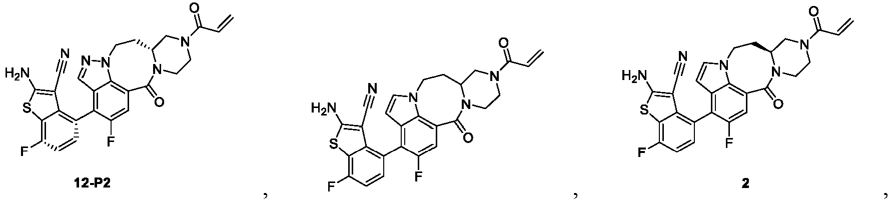

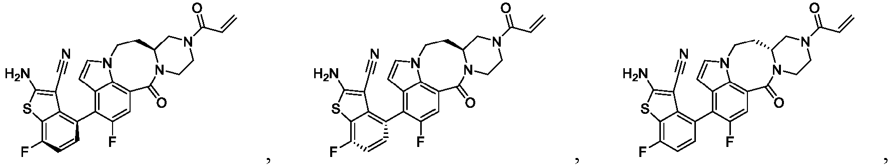

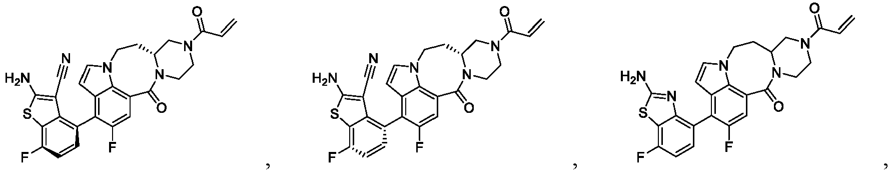

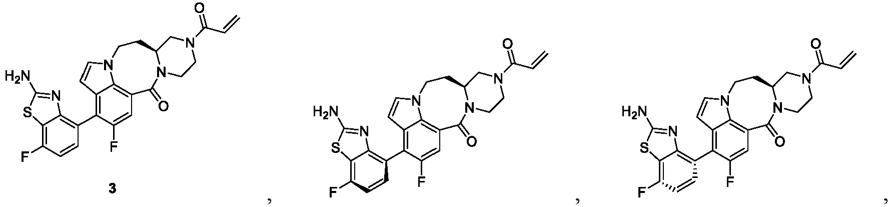

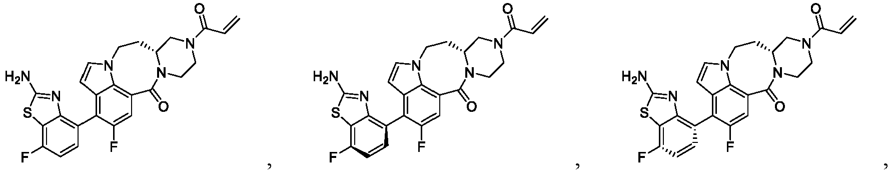

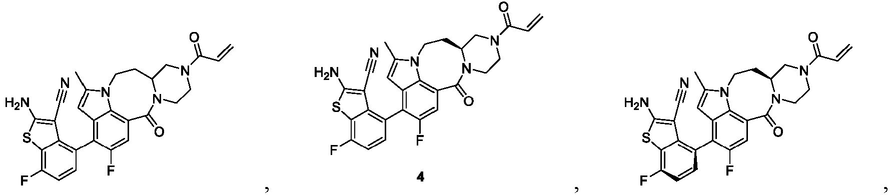

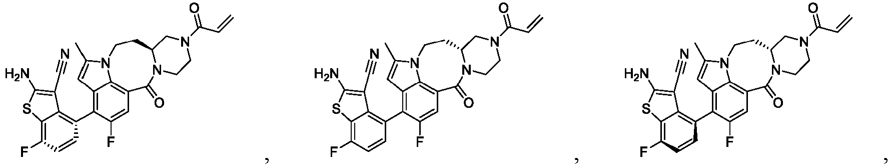

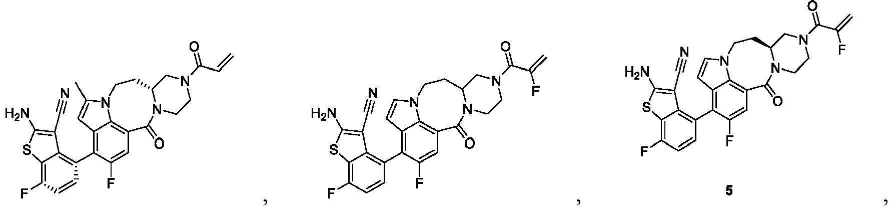

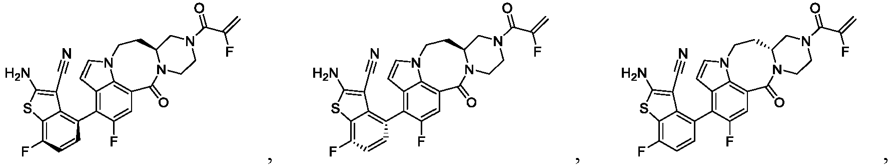

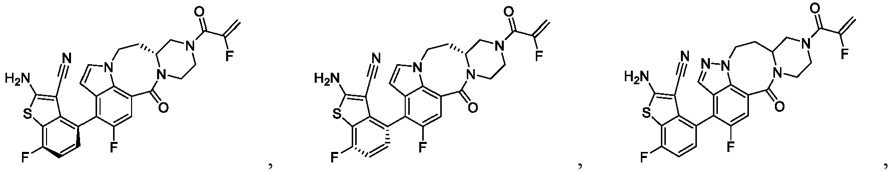

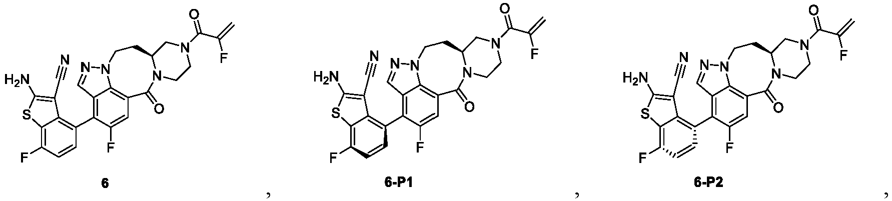

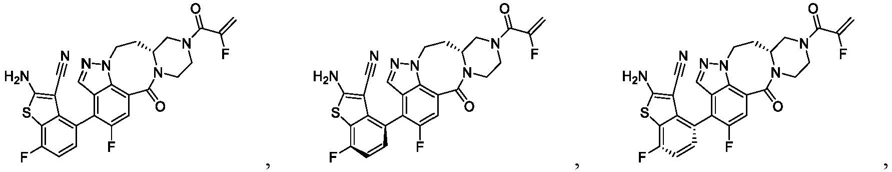

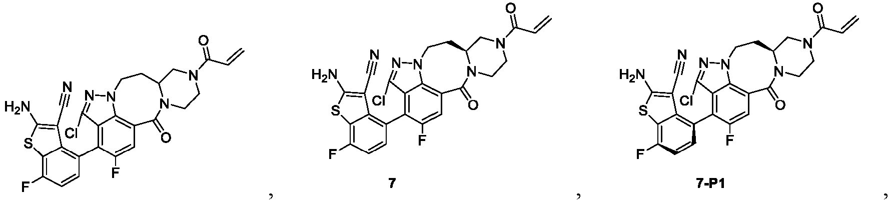

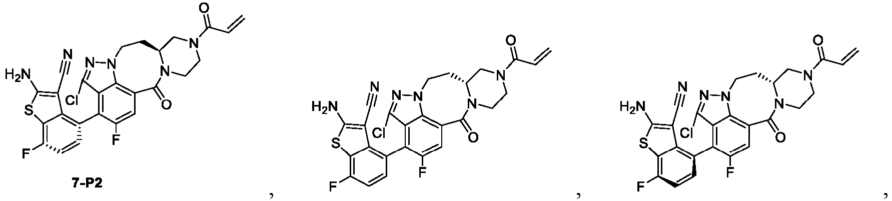

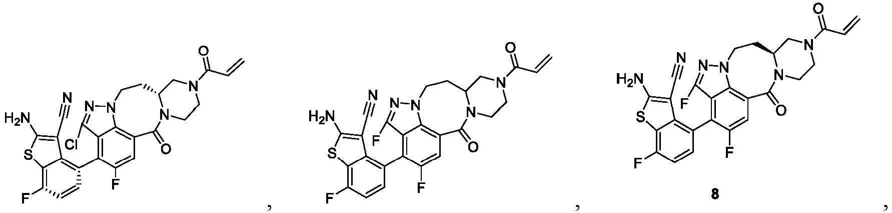

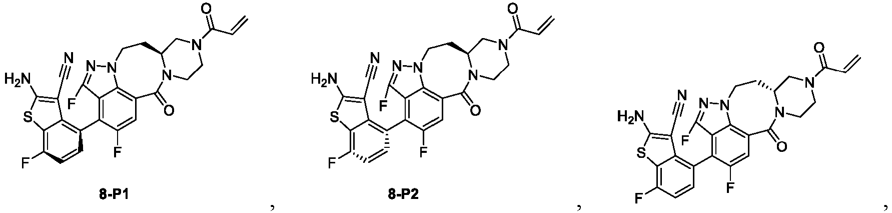

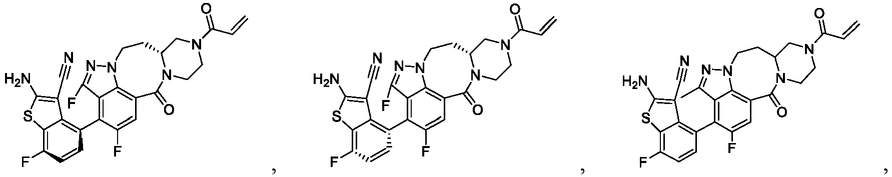

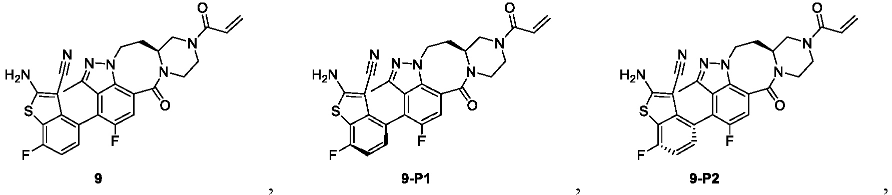

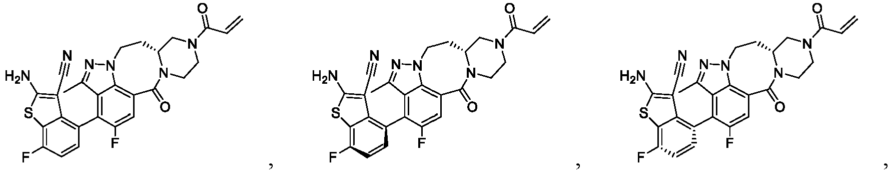

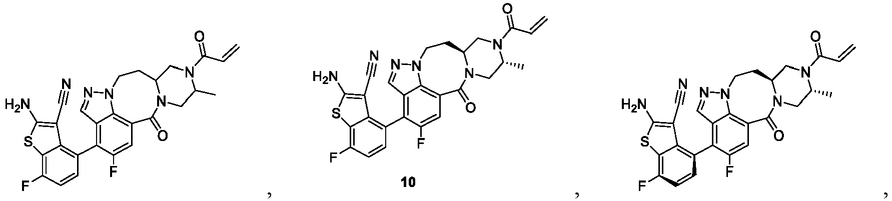

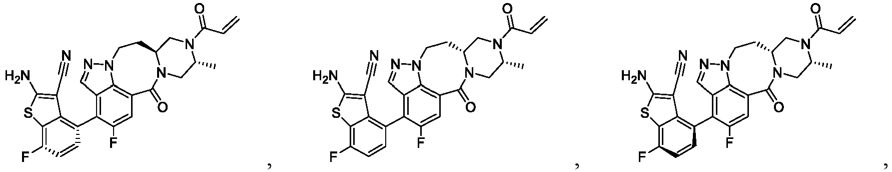

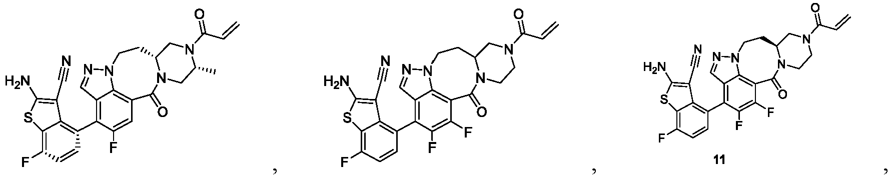

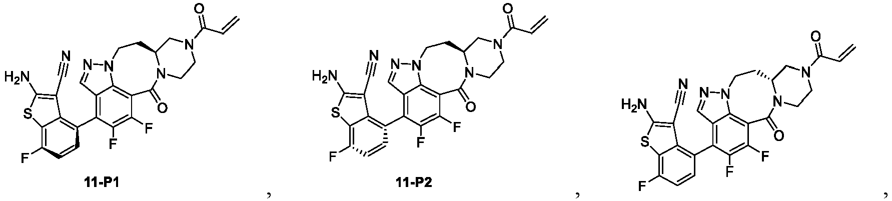

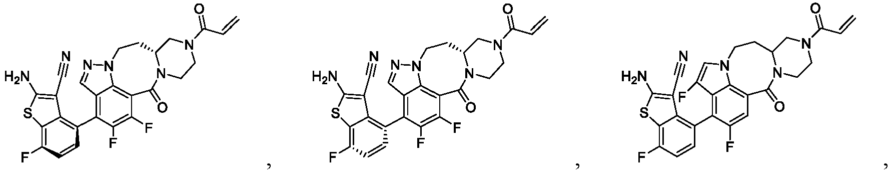

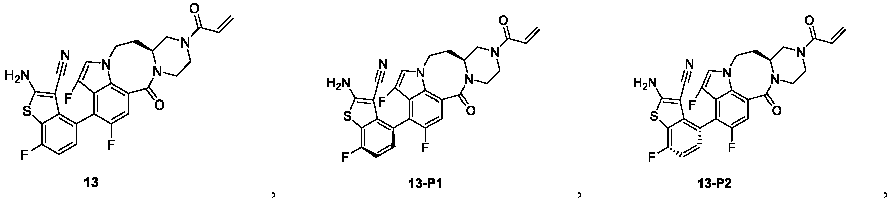

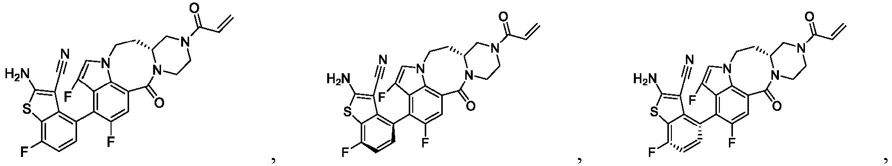

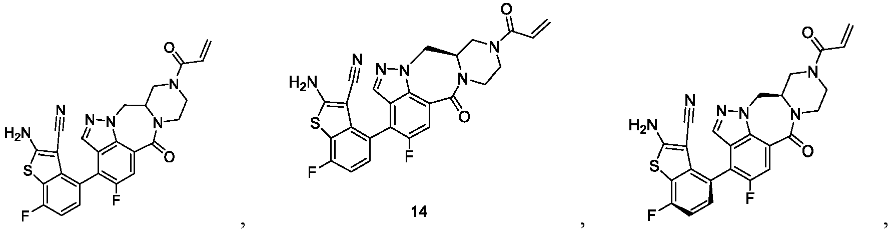

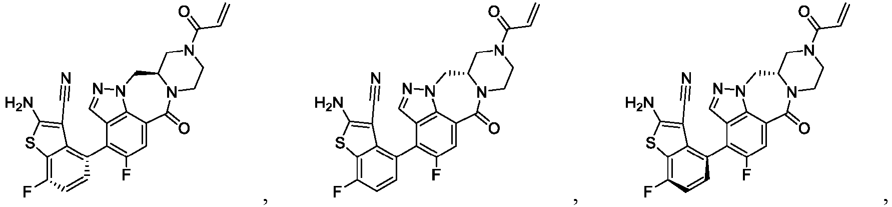

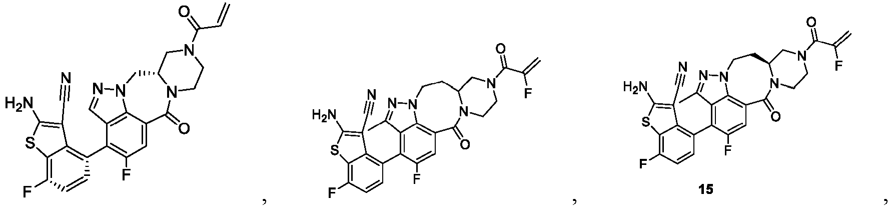

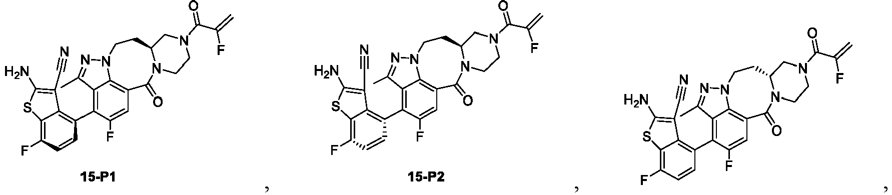

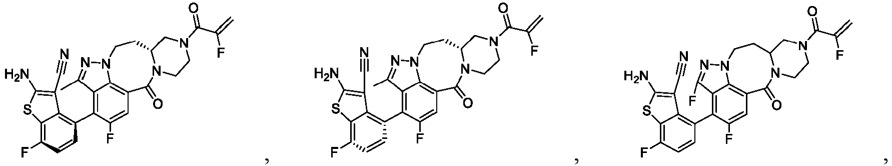

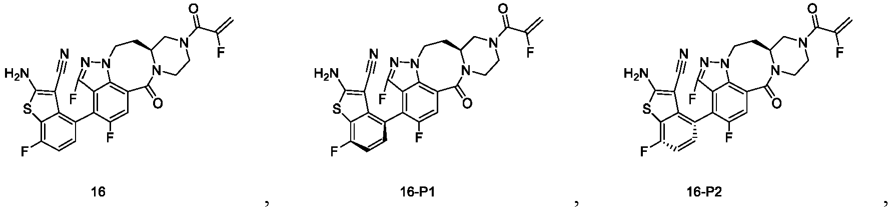

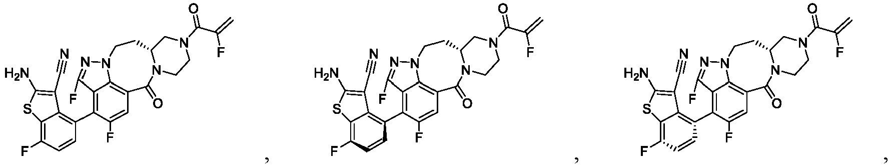

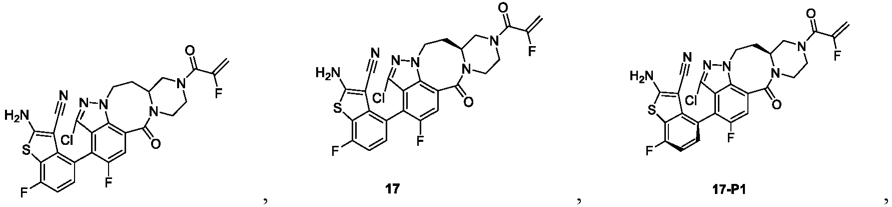

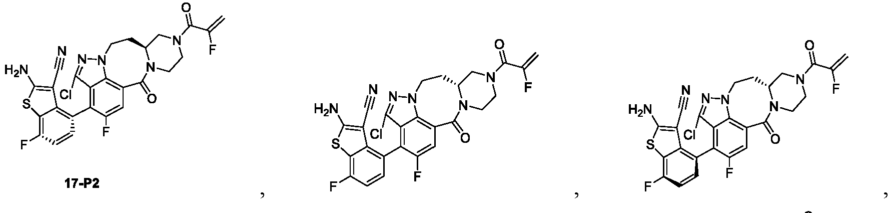

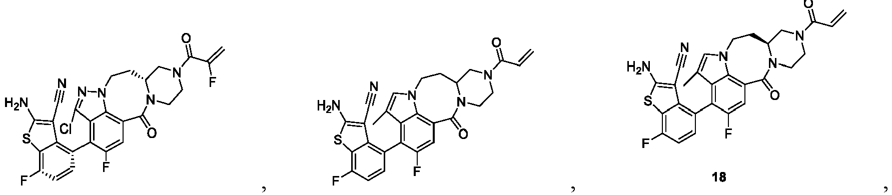

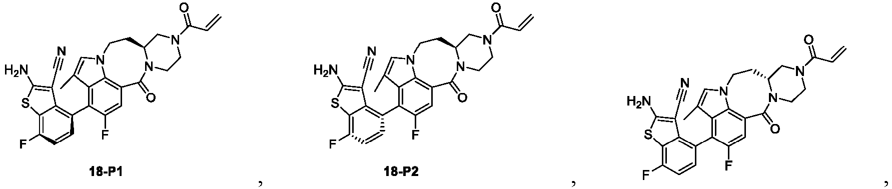

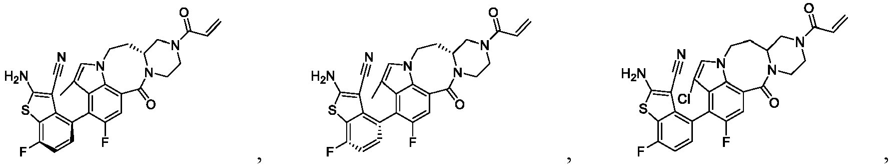

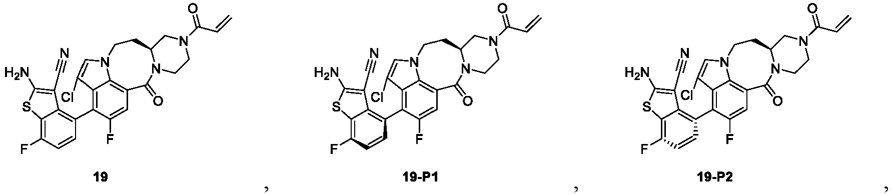

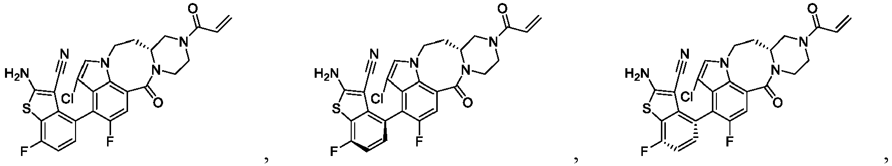

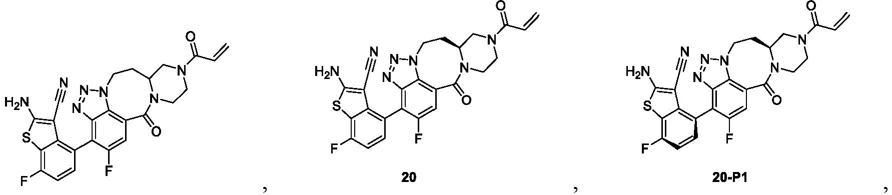

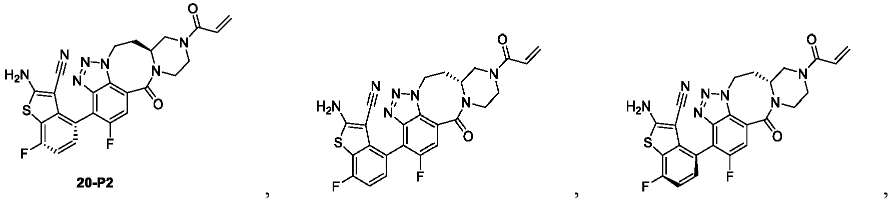

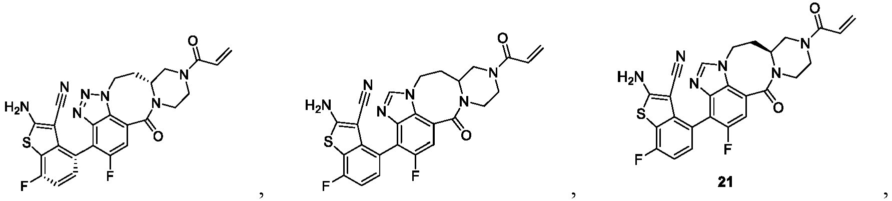

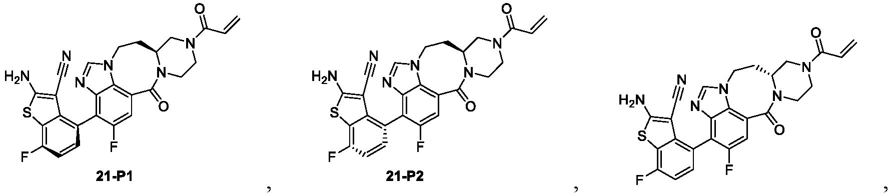

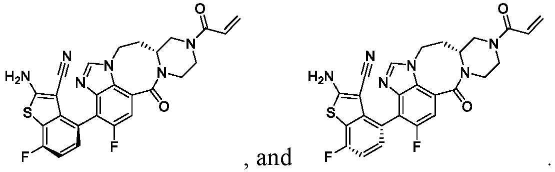

10. A compound represented by general formula (IMa) or a salt thereof: 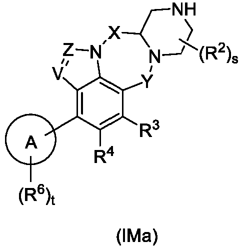 wherein:
ring A, V, X, Y, Z, R², R³, R⁴, R⁶, s, and t are as defined in claim 1.

11. A compound or a salt thereof, being selected from the group consisting of the following compounds:

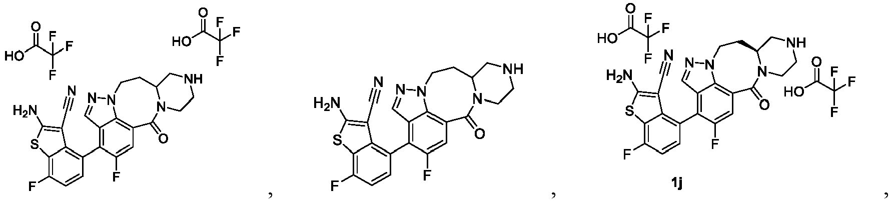

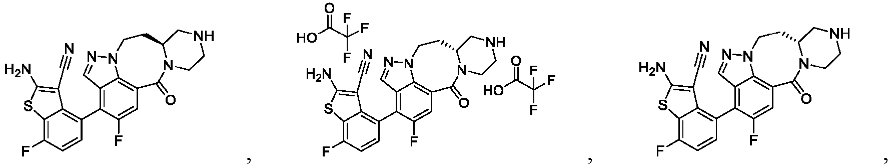

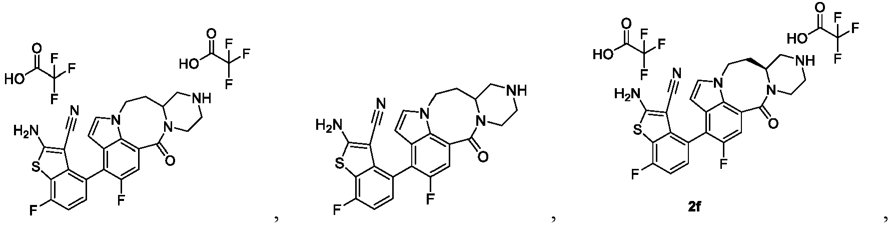

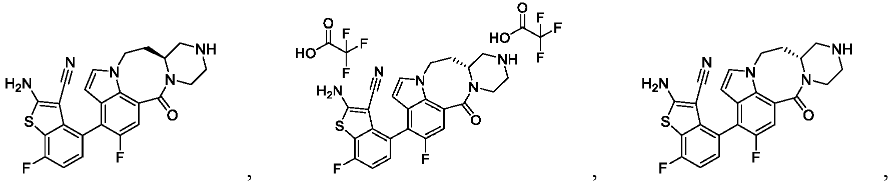

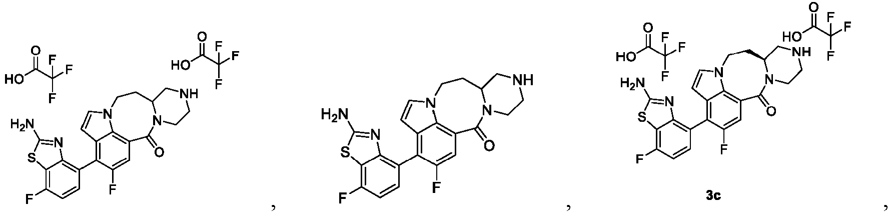

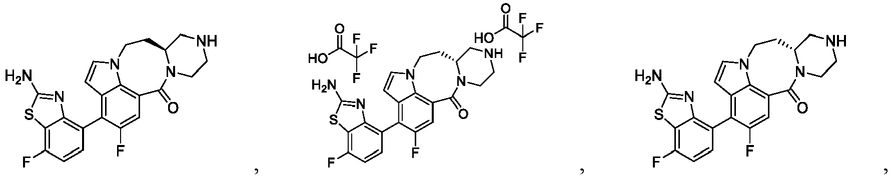

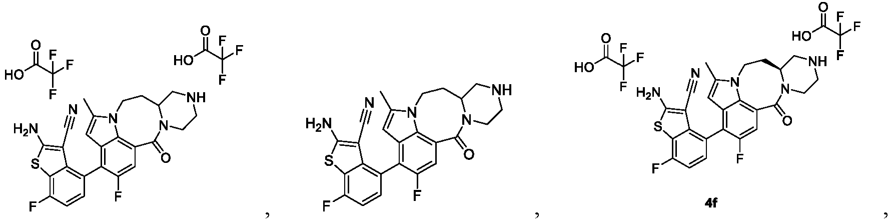

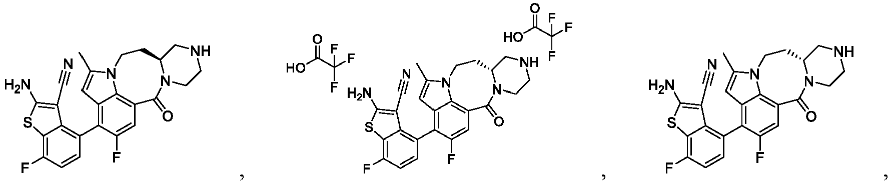

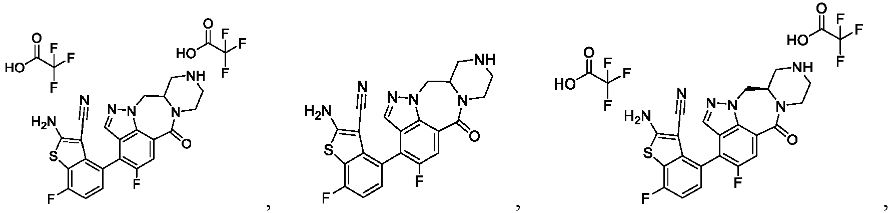

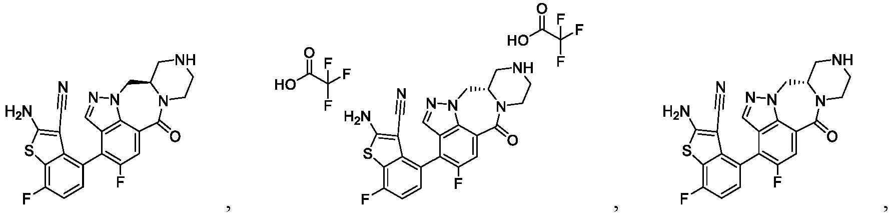

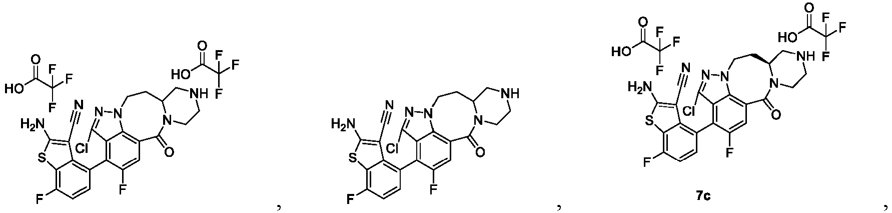

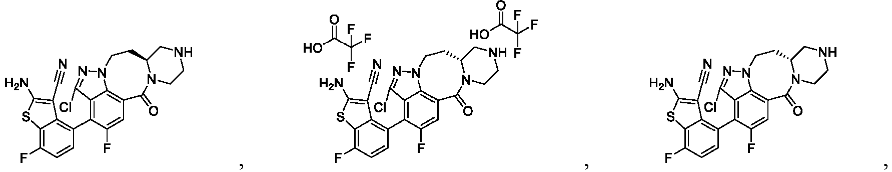

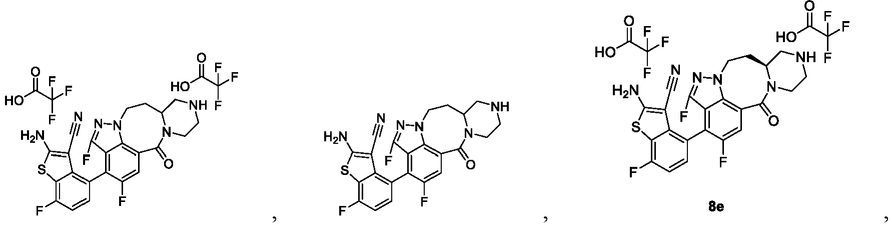

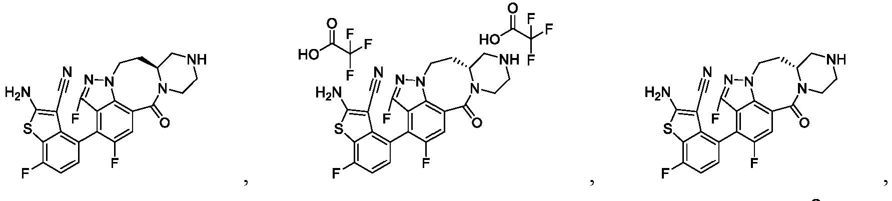

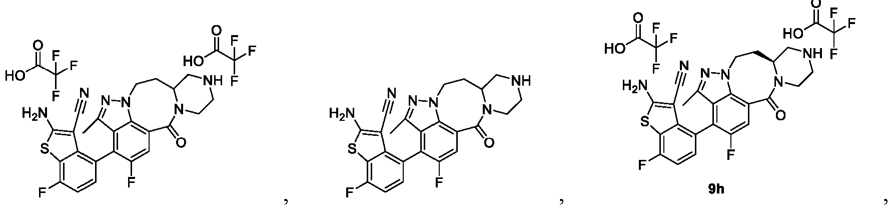

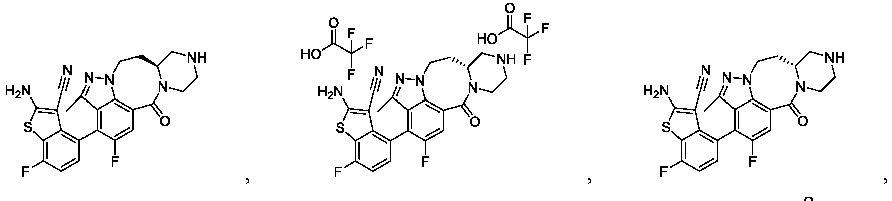

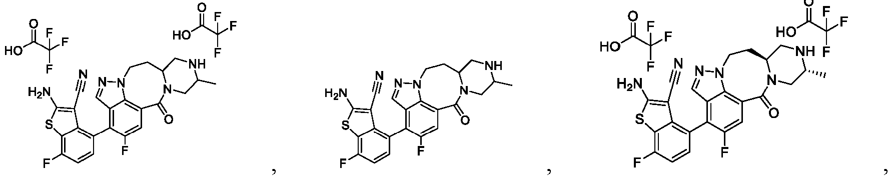

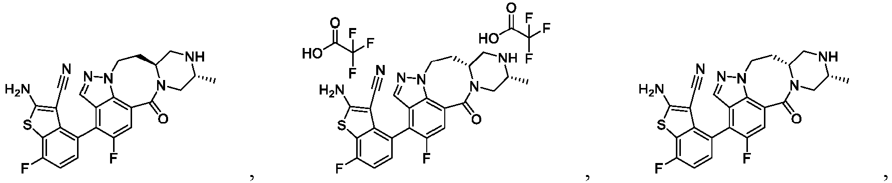

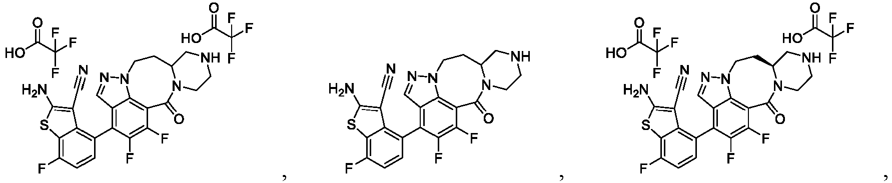

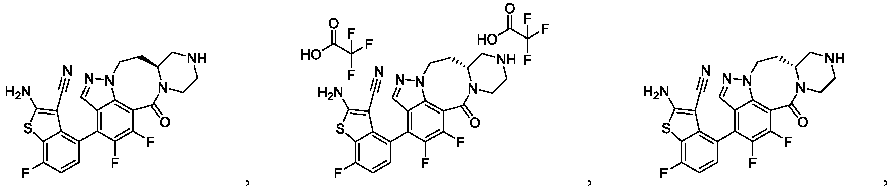

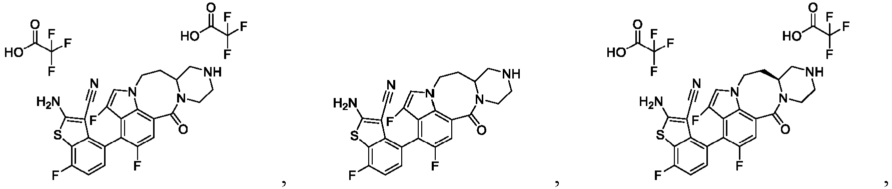

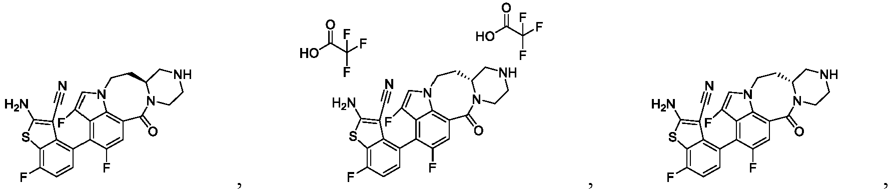

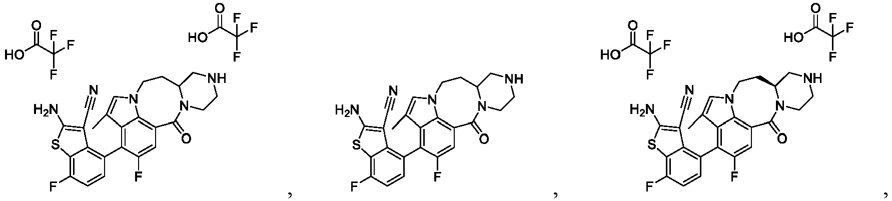

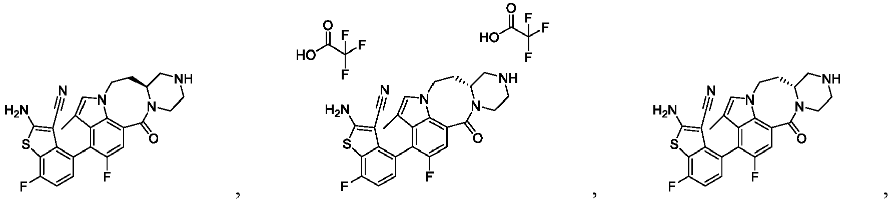

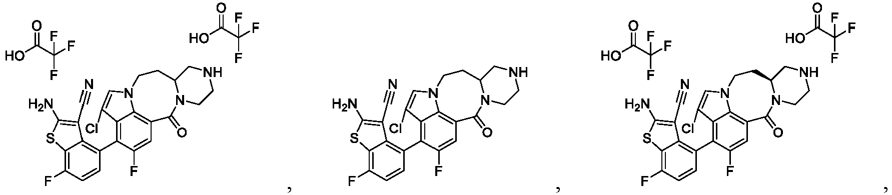

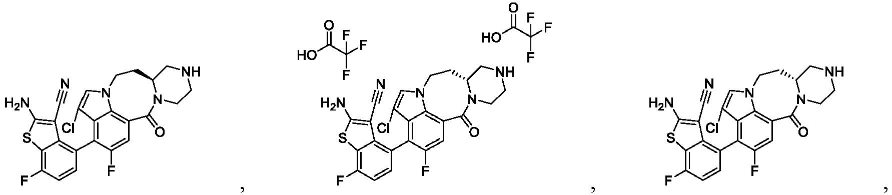

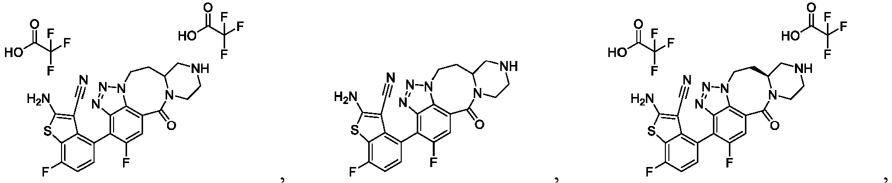

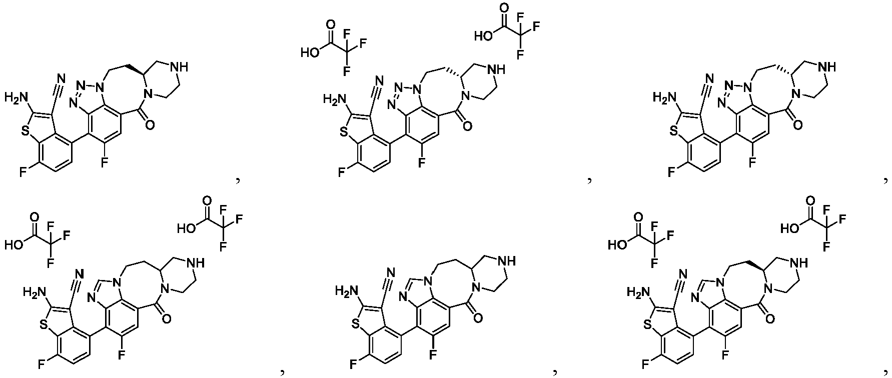

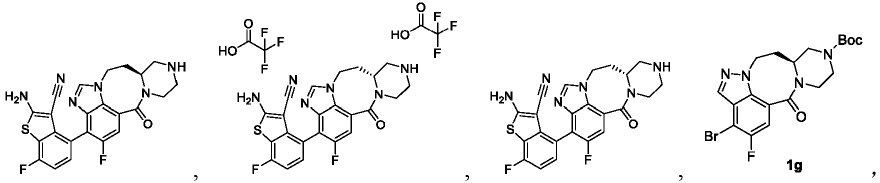

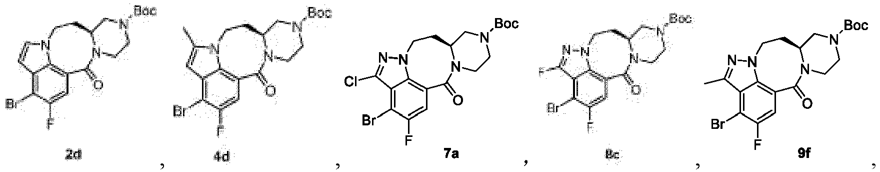

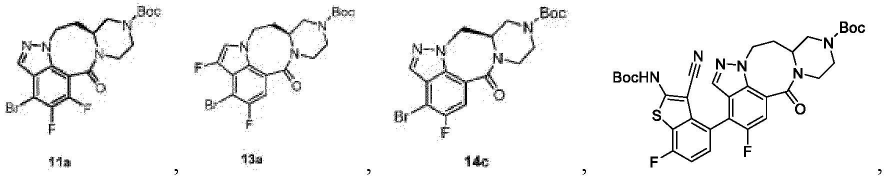

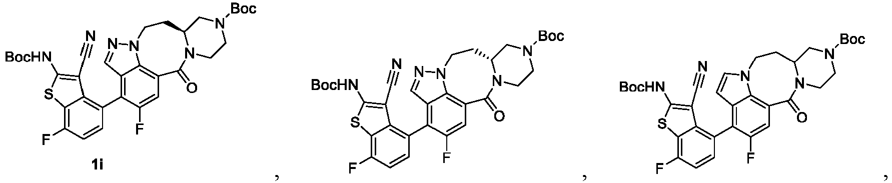

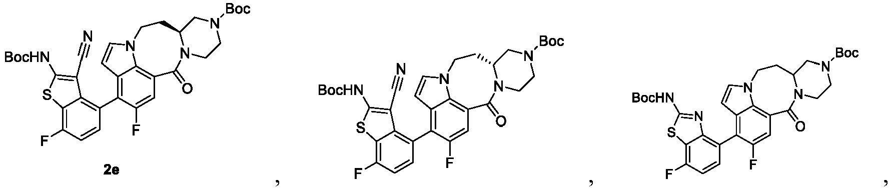

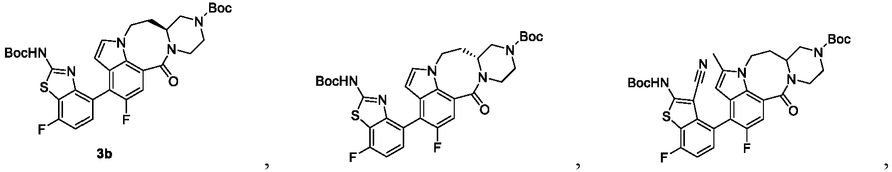

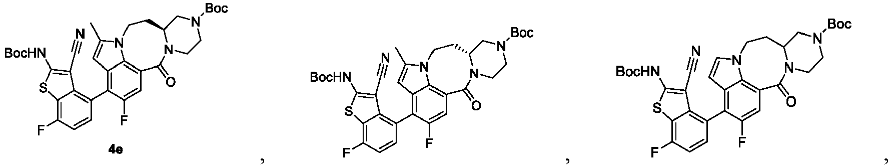

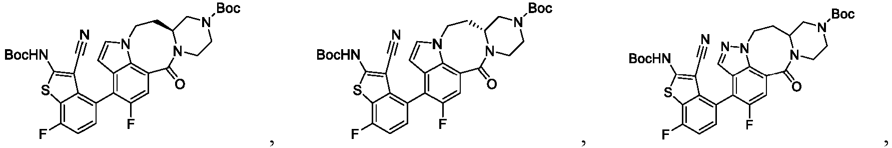

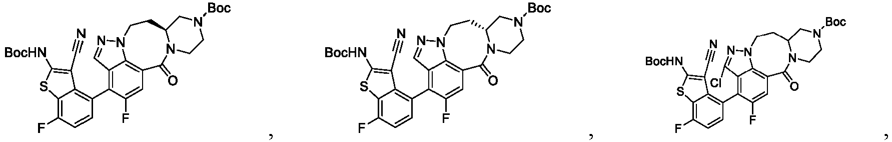

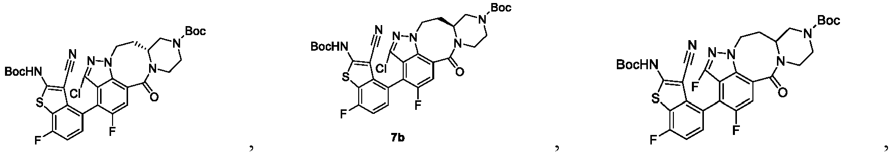

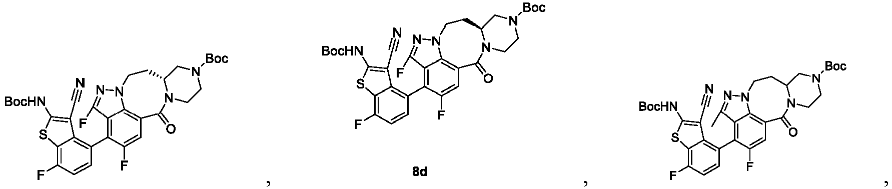

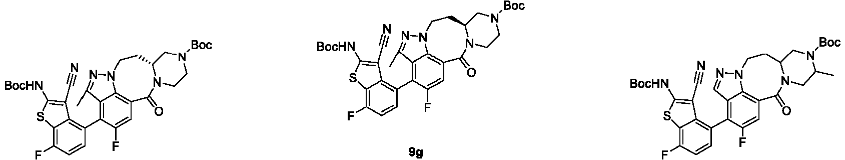

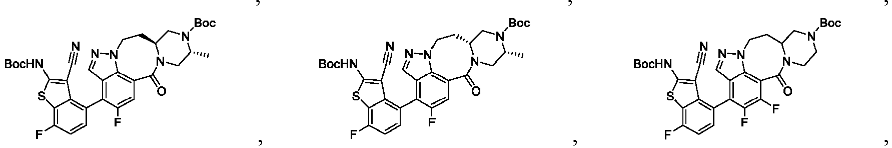

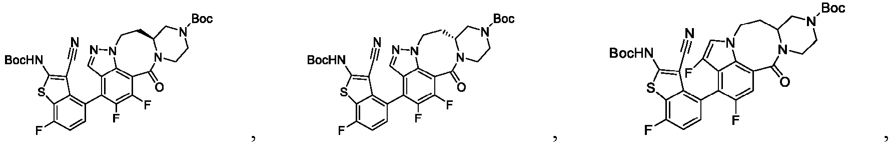

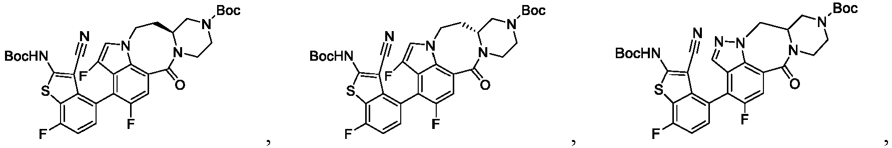

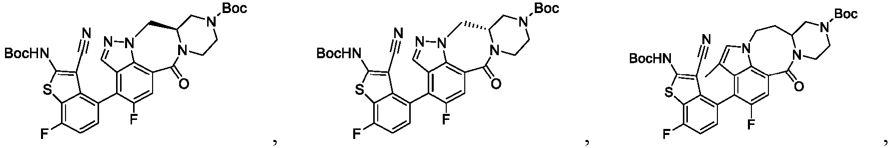

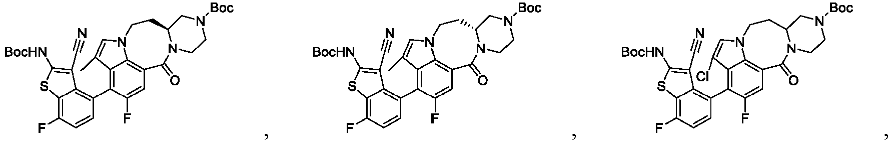

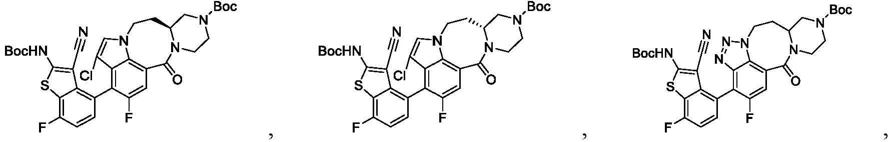

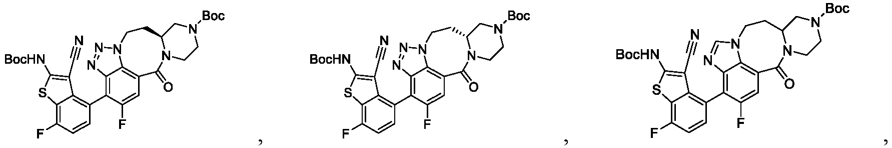

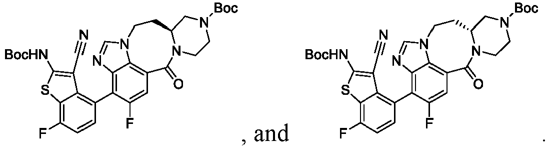

12. A method for preparing a compound represented by general formula (IM) or a pharmaceutically acceptable salt thereof according to claim 1, comprising: 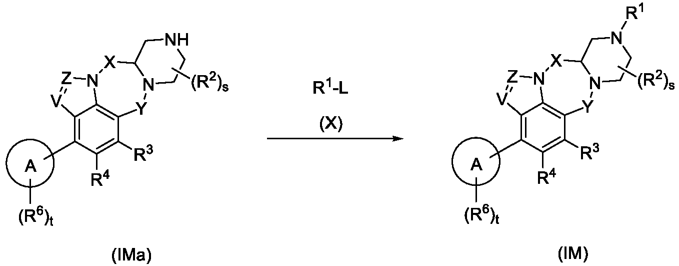 conducting a reaction of a compound represented by general formula (IMa) or a salt thereof with a compound of general formula (X) or a salt thereof to give the compound represented by general formula (IM) or the pharmaceutically acceptable salt thereof;
wherein:
L is halogen; preferably, L is Cl;
R¹ is 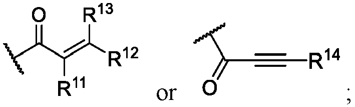
ring A, V, X, Y, Z, R² , R³, R⁴, R⁶, R¹¹, R¹², R¹³, R¹⁴, s, and t are as defined in claim 1.

13. A pharmaceutical composition, wherein the pharmaceutical composition comprises the compound represented by general formula (IM) or the pharmaceutically acceptable salt thereof according to any of claims 1 to 9, and one or more pharmaceutically acceptable carriers, diluents, or excipients.

14. The compound represented by general formula (IM) or the pharmaceutically acceptable salt thereof according to any of claims 1 to 9 or the pharmaceutical composition according to claim 13 for use as a medicament, preferably for use as a KRAS G12C inhibitor.

15. The compound represented by general formula (IM) or the pharmaceutically acceptable salt thereof according to any of claims 1 to 9 or the pharmaceutical composition according to claim 13 for use in treating and/or preventing a tumor; preferably, the tumor is a cancer; more preferably, the cancer is selected from the group consisting of lung cancer, pancreatic cancer, cervical cancer, esophageal cancer, endometrial cancer, ovarian cancer, cholangiocarcinoma, colorectal cancer, liver cancer, breast cancer, prostate cancer, thyroid cancer, stomach cancer, urothelial cancer, testicular cancer, leukemia, skin cancer, squamous cell cancer, basal cell cancer, bladder cancer, head and neck cancer, kidney cancer, nasopharyngeal cancer, bone cancer, lymphoma, melanoma, sarcoma, peripheral neuroepithelioma, glioma, brain tumor, and myeloma; most preferably, the cancer is selected from the group consisting of lung cancer, pancreatic cancer, cervical cancer, esophageal cancer, endometrial cancer, ovarian cancer, cholangiocarcinoma, and colorectal cancer.

## Patentansprüche

1. Verbindung der allgemeinen Formel (IM) oder ein pharmazeutisch verträgliches Salz davon: 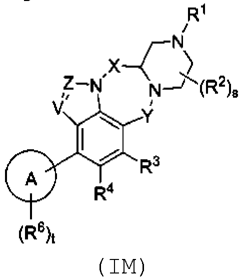 wobei:
X C(R^{a}R^{b}) oder C(R^{a}R^{b})-C(R^{c}R^{d}) ist;
Y C(O) oder CH₂ ist;
Z CR^{5a} oder N ist;
V CR⁵ oder N ist;
Ring A eine Aryl- oder Heteroarylgruppe ist;
R^{a}, R^{b}, R^{c} und R^{d} bei jedem Vorkommen identisch oder unterschiedlich sind und jeweils unabhängig voneinander aus der Gruppe ausgewählt werden, die aus einem Wasserstoffatom, einem Halogenatom, einer Alkylgruppe, einer Halogenalkylgruppe, einer Hydroxyalkylgruppe, einer Alkoxygruppe, einer Halogenalkoxygruppe, einer Alkenylgruppe, einer Alkinylgruppe, einer Hydroxygruppe und einer Cyanogruppe besteht;
R¹ ausgewählt ist aus der Gruppe bestehend aus Cyano, 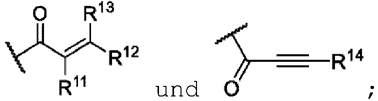
jedes R² gleich oder unterschiedlich ist und unabhängig aus der Gruppe ausgewählt wird, die aus einem Wasserstoffatom, einem Halogenatom, einer Cyanogruppe, einer Alkylgruppe, einer Alkoxygruppe, Hydroxy- und Aminogruppen besteht, wobei die Alkyl- und Alkoxygruppen jeweils unabhängig voneinander gegebenenfalls mit einem oder mehreren Substituenten substituiert sind, die aus der Gruppe ausgewählt sind, bestehend aus Halogen, Cyano, Amino und Hydroxy;
R³, R⁴, R⁵ und R^{5a} gleich oder unterschiedlich sind und jeweils unabhängig voneinander aus der Gruppe ausgewählt werden, die aus einem Wasserstoffatom, Halogen, Cyano, Alkyl, Alkenyl, Alkinyl, -NR^{7a}R^{7b}, -C(O)R⁸, -OR⁸, -S(O)ₚR⁸, Cycloalkyl, Heterocyclyl, Aryl und Heteroaryl besteht, wobei die Alkyl-, Cycloalkyl-, Heterocyclyl-, Aryl- und Heteroaryl-Gruppen jeweils unabhängig voneinander gegebenenfalls mit einem oder mehreren Substituenten substituiert sind, die aus der Gruppe ausgewählt sind, bestehend aus Halogen, Alkyl, Halogenalkyl, Cyano, - NR^{7c}R^{7d}, -OR^{8a}, Cycloalkyl, Heterocyclyl, Aryl und Heteroaryl;
jedes R⁶ gleich oder unterschiedlich ist und unabhängig aus der Gruppe ausgewählt wird, die aus einem Wasserstoffatom, einem Halogenatom, einer Cyanogruppe, einer Alkylgruppe, einer Alkenylgruppe, einer Alkinylgruppe,-NR^{9a}R^{9b}, -C(O)NR^{9a}R^{9b}, -C(O)R¹⁰, -C(O)OR¹⁰, - OC(O)R¹⁰, -OR¹⁰, -S(O)ₚR¹⁰, -S(O)ₚNR^{9a}R^{9b}, Cycloalkyl, Heterocyclyl, Aryl und Heteroaryl besteht, wobei die Alkyl-, Cycloalkyl-, Heterocyclyl-, Aryl- und Heteroarylresten jeweils unabhängig voneinander gegebenenfalls mit einem oder mehreren Substituenten substituiert sind, die aus der Gruppe ausgewählt sind, bestehend aus Halogen, Alkyl, Halogenalkyl, Cyano, - NR^{9c}R^{9d}, -OR^{10a}, Cycloalkyl, Heterocyclyl, Aryl und Heteroaryl;
R¹¹, R¹², R¹³ und R¹⁴ gleich oder unterschiedlich sind und jeweils unabhängig voneinander aus der Gruppe ausgewählt werden, die aus einem Wasserstoffatom, einem Halogenatom, einer Alkylgruppe, -NR^{15a}R^{15b}, -OR¹⁶, Cyano, Cycloalkyl, Heterocyclyl, Aryl und Heteroaryl besteht, wobei die Alkyl-, Cycloalkyl-, Heterocyclyl-, Aryl- und Heteroarylreste jeweils unabhängig voneinander gegebenenfalls mit einem oder mehreren Substituenten substituiert sind, die aus der Gruppe ausgewählt sind, bestehend aus Halogen, Oxo, Alkyl, Halogenalkyl, Alkoxy, Halogenalkoxy, Cyano, NR^{15c}R^{15d}, Hydroxy, Hydroxyalkyl, Cycloalkyl, Heterocyclyl, Aryl und Heteroaryl;
R⁸, R^{8a}, R¹⁰, R^{10a} und R¹⁶ bei jedem Vorkommen gleich oder unterschiedlich sind und jeweils unabhängig voneinander aus der Gruppe ausgewählt werden, die aus einem Wasserstoffatom, Alkyl, Hydroxyalkyl, Cycloalkyl, Heterocyclyl, Aryl und Heteroaryl besteht, wobei das Alkyl, Cycloalkyl, Heterocyclyl-, Aryl- und Heteroarylgruppen jeweils unabhängig voneinander gegebenenfalls mit einem oder mehreren Substituenten substituiert sind, die aus der Gruppe ausgewählt sind, bestehend aus Halogen, Alkyl, Alkenyl, Alkinyl, Oxo, Alkoxy, Halogenalkyl, Halogenalkoxy, Cyano, -NR^{17a}R^{17b}, Hydroxy, Cycloalkyl, Heterocyclyl, Aryl und Heteroaryl; R^{7a}, R^{7b}, R^{7c}, R^{7d}, R^{9a}, R^{9b}, R^{9c}, R^{9d}, R^{15a}, R^{15b}, R^{15c}, R^{15d}, R^{17a} und R^{17b} bei jedem Vorkommen identisch oder unterschiedlich sind und jeweils unabhängig voneinander aus der Gruppe ausgewählt werden, bestehend aus einem Wasserstoffatom, Alkyl, Hydroxyalkyl, Cycloalkyl, Heterocyclyl, Aryl und Heteroaryl, wobei das Alkyl, Cycloalkyl, Heterocyclyl-, Aryl- und Heteroaryl-Gruppen jeweils unabhängig voneinander gegebenenfalls mit einem oder mehreren Substituenten substituiert sind, die aus der Gruppe ausgewählt sind, bestehend aus Halogen, Oxo, Hydroxy, Cyano, Alkyl, Alkoxy, Halogenalkyl und Halogenalkoxy;
oder R^{7a} und R^{7b} zusammen mit dem Stickstoffatom, an das sie gebunden sind, eine Heterocyclylgruppe bilden, wobei die Heterocyclylgruppe gegebenenfalls mit einem oder mehreren Substituenten substituiert ist, die aus der Gruppe ausgewählt sind, bestehend aus Halogen, Oxo, Alkyl, Alkoxy, Halogenalkyl, Halogenalkoxy, Cyano, Amino, Nitro, Hydroxy, Hydroxyalkyl, Cycloalkyl, Heterocyclyl, Aryl und Heteroaryl;
oder R^{7c} und R^{7d} zusammen mit dem Stickstoffatom, an das sie gebunden sind, eine Heterocyclylgruppe bilden, wobei die Heterocyclylgruppe gegebenenfalls mit einem oder mehreren Substituenten substituiert ist, die aus der Gruppe ausgewählt sind, bestehend aus Halogen, Oxo, Alkyl, Alkoxy, Halogenalkyl, Halogenalkoxy, Cyano, Amino, Nitro, Hydroxy, Hydroxyalkyl, Cycloalkyl, Heterocyclyl, Aryl und Heteroaryl;
oder R^{9a} und R^{9b} zusammen mit dem Stickstoffatom, an das sie gebunden sind, eine Heterocyclylgruppe bilden, wobei die Heterocyclylgruppe gegebenenfalls mit einem oder mehreren Substituenten substituiert ist, die aus der Gruppe ausgewählt sind, bestehend aus Halogen, Oxo, Alkyl, Alkoxy, Halogenalkyl, Halogenalkoxy, Cyano, Amino, Nitro, Hydroxy, Hydroxyalkyl, Cycloalkyl, Heterocyclyl, Aryl und Heteroaryl;
oder R^{9c} und R^{9d} zusammen mit dem Stickstoffatom, an das sie gebunden sind, eine Heterocyclylgruppe bilden, wobei die Heterocyclylgruppe gegebenenfalls mit einem oder mehreren Substituenten substituiert ist, die aus der Gruppe ausgewählt sind, bestehend aus Halogen, Oxo, Alkyl, Alkoxy, Halogenalkyl, Halogenalkoxy, Cyano, Amino, Nitro, Hydroxy, Hydroxyalkyl, Cycloalkyl, Heterocyclyl, Aryl und Heteroaryl;
oder R^{15a} und R^{15b} zusammen mit dem Stickstoffatom, an das sie gebunden sind, eine Heterocyclylgruppe bilden, wobei die Heterocyclylgruppe gegebenenfalls mit einem oder mehreren Substituenten substituiert ist, die aus der Gruppe ausgewählt sind, bestehend aus Halogen, Oxo, Alkyl, Alkoxy, Halogenalkyl, Halogenalkoxy, Cyano, Amino, Nitro, Hydroxy, Hydroxyalkyl, Cycloalkyl, Heterocyclyl, Aryl und Heteroaryl;
oder R^{15c} und R^{15d} zusammen mit dem Stickstoffatom, an das sie gebunden sind, eine Heterocyclylgruppe bilden, wobei die Heterocyclylgruppe gegebenenfalls mit einem oder mehreren Substituenten substituiert ist, die aus der Gruppe ausgewählt sind, bestehend aus Halogen, Oxo, Alkyl, Alkoxy, Halogenalkyl, Halogenalkoxy, Cyano, Amino, Nitro, Hydroxy, Hydroxyalkyl, Cycloalkyl, Heterocyclyl, Aryl und Heteroaryl;
oder R^{17a} und R^{17b} zusammen mit dem Stickstoffatom, an das sie gebunden sind, eine Heterocyclylgruppe bilden, wobei die Heterocyclylgruppe gegebenenfalls mit einem oder mehreren Substituenten substituiert ist, die aus der Gruppe ausgewählt sind, bestehend aus Halogen, Oxo, Alkyl, Alkoxy, Halogenalkyl, Halogenalkoxy, Cyano, Amino, Nitro, Hydroxy, Hydroxyalkyl, Cycloalkyl, Heterocyclyl, Aryl und Heteroaryl;
s 0, 1, 2, 3, 4, 5 oder 6 ist;
t 0, 1, 2, 3, 4 oder 5 ist; und
p 0, 1 oder 2 ist.

2. Verbindung der allgemeinen Formel (IM) oder ein pharmazeutisch verträgliches Salz davon nach Anspruch 1, wobei es sich um eine Verbindung der allgemeinen Formel (I) oder ein pharmazeutisch verträgliches Salz davon handelt: 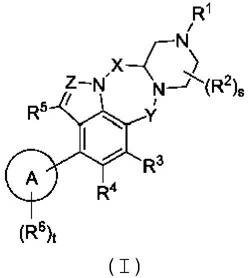 wobei:
Ring A, X, Y, Z, R¹, R², R³, R⁴, R⁵, R⁶, s und t nach Anspruch 1 definiert sind.

3. Verbindung der allgemeinen Formel (IM) oder ein pharmazeutisch verträgliches Salz davon nach Anspruch 1 oder 2, wobei Y C(O) ist; und/oder s 0 oder 1 ist.

4. Verbindung der allgemeinen Formel (IM) oder ein pharmazeutisch verträgliches Salz davon nach einem der Ansprüche 1 bis 3, wobei es sich um eine Verbindung der allgemeinen Formel (II) oder ein pharmazeutisch verträgliches Salz davon handelt: 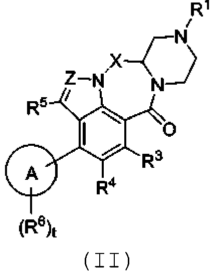 wobei:
Ring A, X, Z, R¹, R³, R⁴, R⁵, R⁶ und t nach Anspruch 1 definiert sind.

5. Verbindung der allgemeinen Formel (IM) oder ein pharmazeutisch verträgliches Salz davon nach einem der Ansprüche 1 bis 4, wobei Ring A ein 8- bis 10-gliedriger bicyclischer Heteroarylring ist, der im Ring 1, 2 oder 3 Heteroatome enthält, die aus der Gruppe ausgewählt sind, bestehend aus Stickstoff, Sauerstoff und Schwefel, und/oder
R¹ 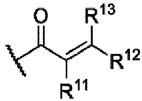 ist, wobei R¹¹, R¹² und R¹³ nach Anspruch 1 definiert sind und/oder X CH₂ oder CH₂-CH₂ ist; vorzugsweise ist X CH₂-CH₂.

6. Verbindung der allgemeinen Formel (IM) oder ein pharmazeutisch verträgliches Salz davon nach einem der Ansprüche 1 bis 5, wobei es sich um eine Verbindung der allgemeinen Formel (III) oder ein pharmazeutisch verträgliches Salz davon handelt: 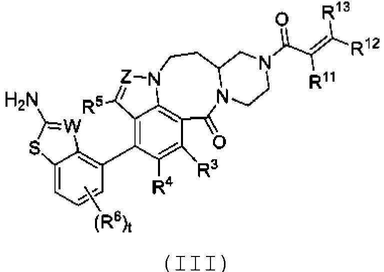 wobei:
W C(CN) oder N ist;
t 0, 1, 2 oder 3 ist;
Z, R³, R⁴, R⁵, R⁶, R¹¹, R¹² und R¹³ nach Anspruch 1 definiert sind.

7. Verbindung der allgemeinen Formel (IM) oder ein pharmazeutisch verträgliches Salz davon nach einem der Ansprüche 1 bis 6, wobei R³, R⁴, R⁵ und R^{5a} gleich oder unterschiedlich sind und jeweils unabhängig voneinander aus der Gruppe ausgewählt sind, bestehend aus einem Wasserstoffatom, einem Halogenatom, C₁₋₆-Alkyl und C₁₋₆-Halogenalkyl ausgewählt sind, und/oder R¹¹ ein Wasserstoffatom oder ein Halogen ist, und/oder R¹² ein Wasserstoffatom ist, und/oder R¹³ ein Wasserstoffatom ist.

8. Verbindung der allgemeinen Formel (IM) oder ein pharmazeutisch verträgliches Salz davon nach einem der Ansprüche 1 bis 7, wobei jedes R⁶ gleich oder unterschiedlich ist und unabhängig aus der Gruppe ausgewählt ist, die aus Halogen, Cyano, -NH₂, C₁₋₆-Alkyl und C₁₋₆-Halogenalkyl ausgewählt ist und t 0, 1, 2 oder 3 ist.

9. Verbindung der allgemeinen Formel (IM) oder ein pharmazeutisch verträgliches Salz davon nach einem der Ansprüche 1 bis 8, ausgewählt aus der Gruppe, bestehend aus den folgenden Verbindungen:

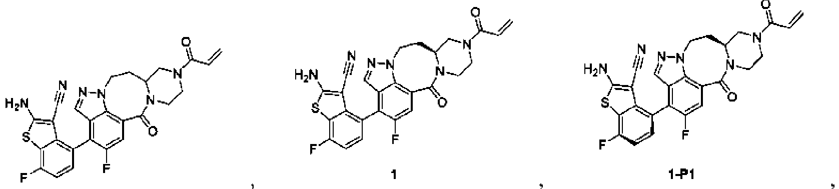

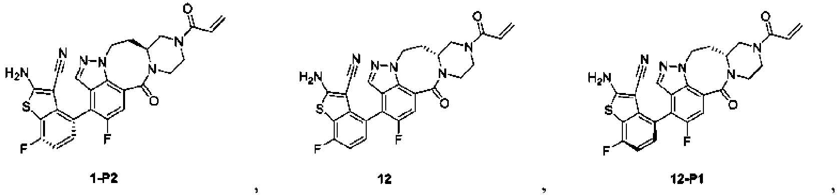

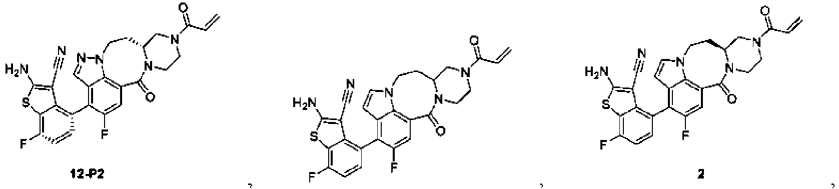

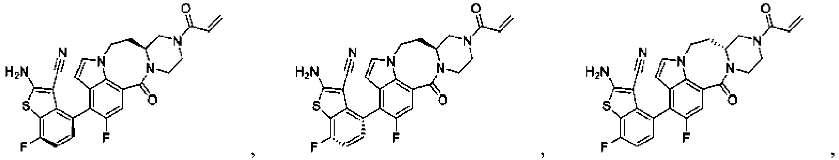

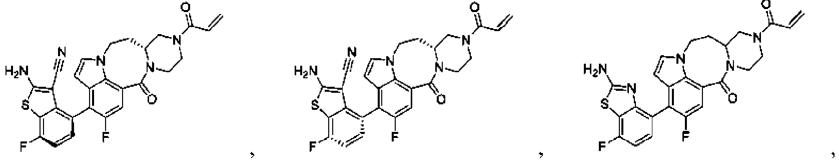

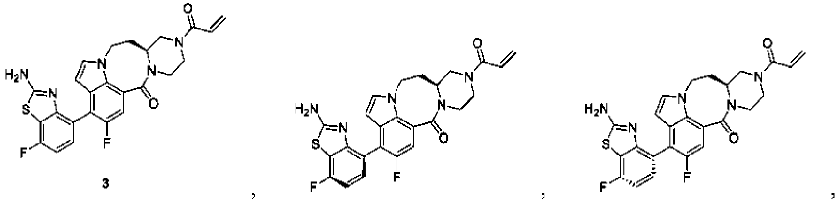

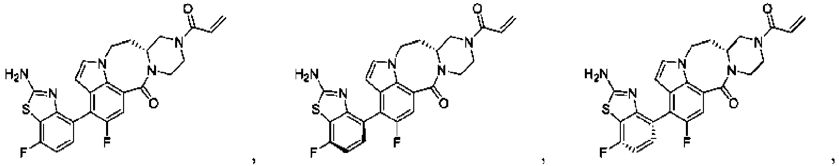

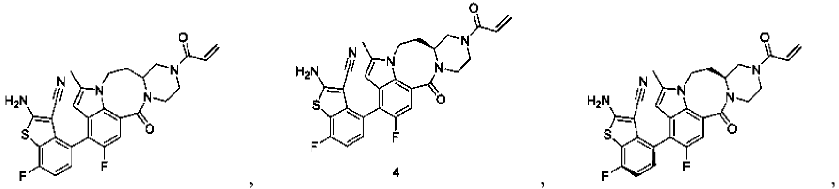

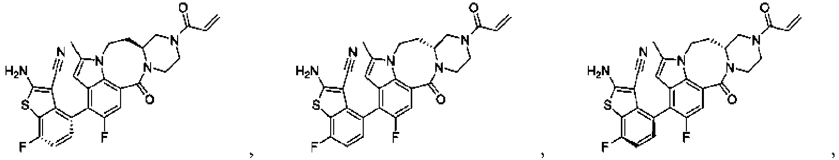

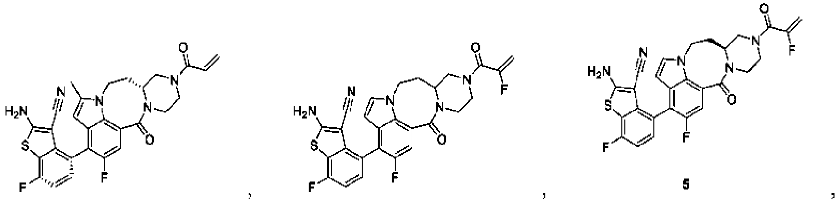

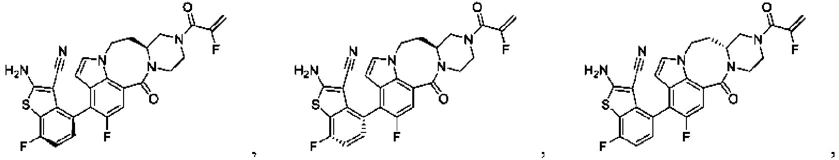

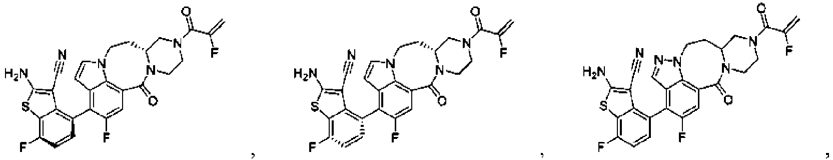

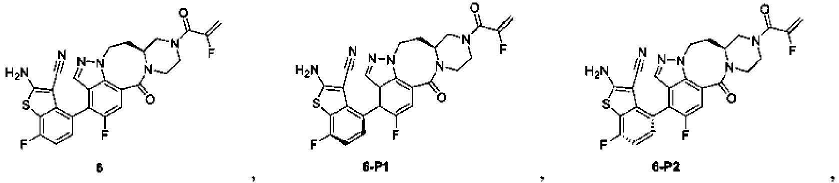

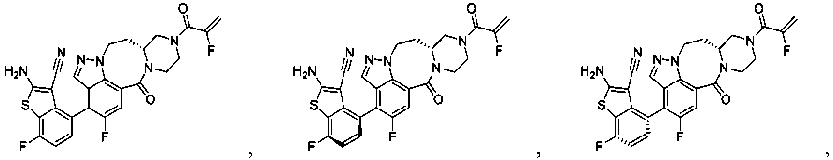

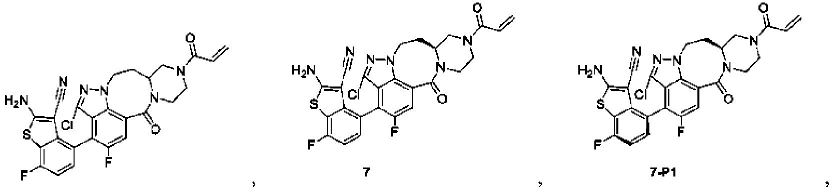

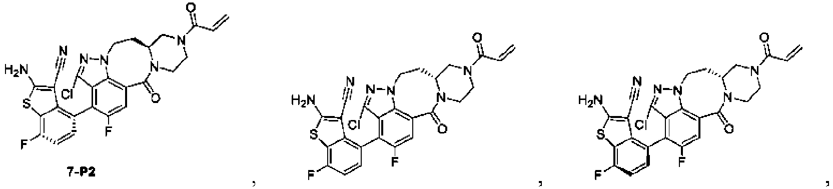

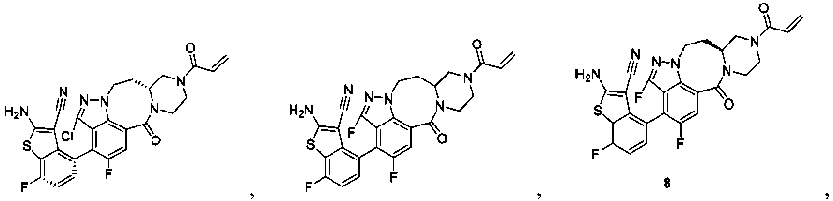

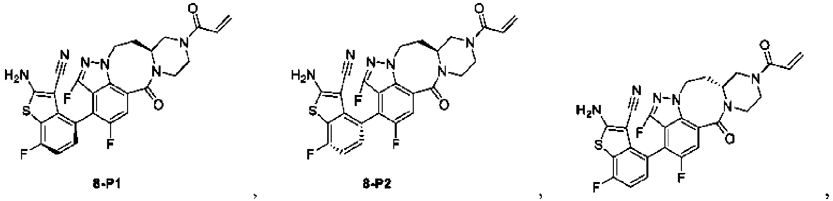

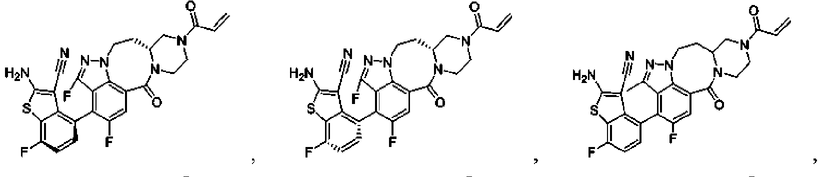

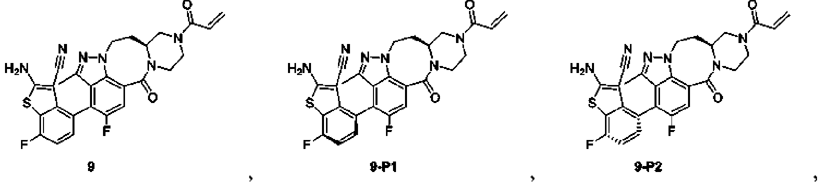

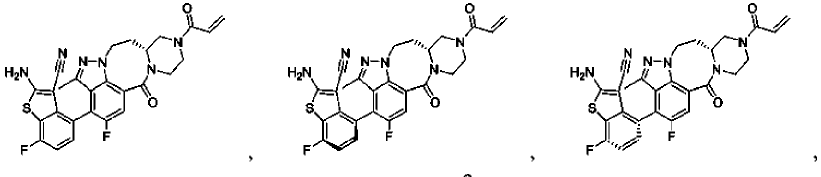

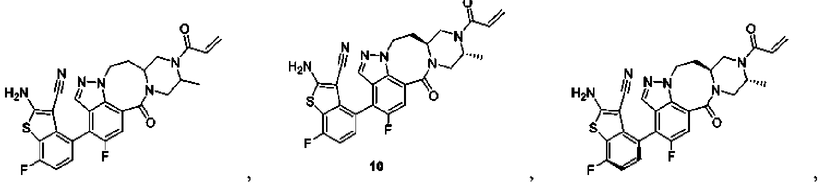

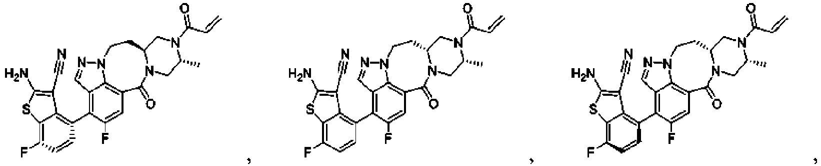

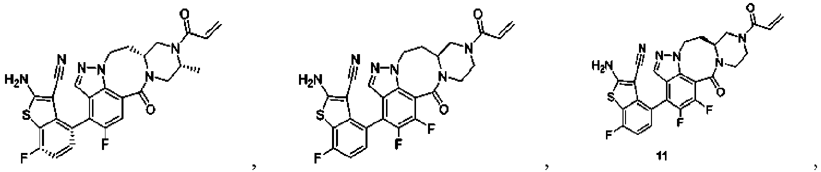

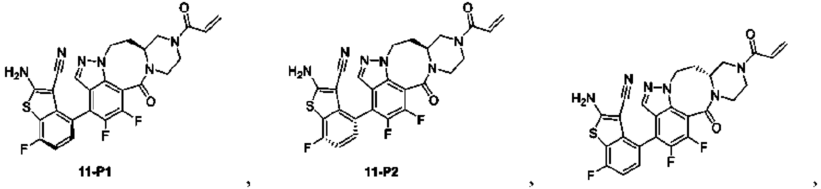

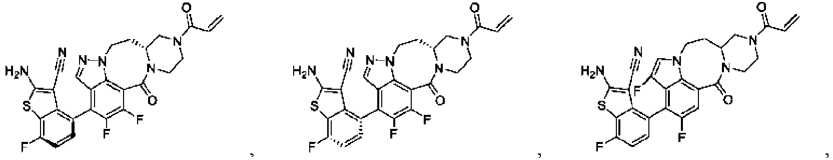

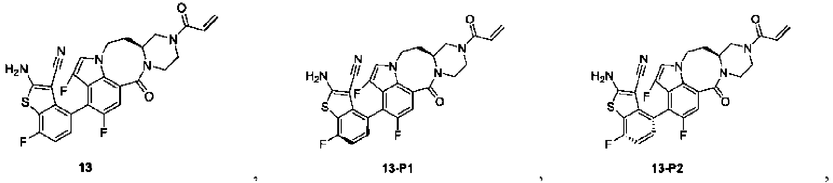

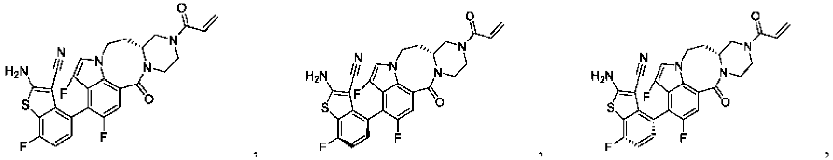

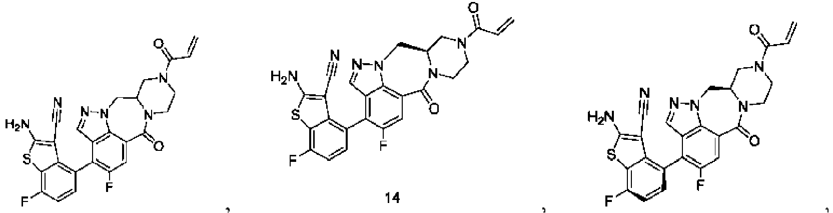

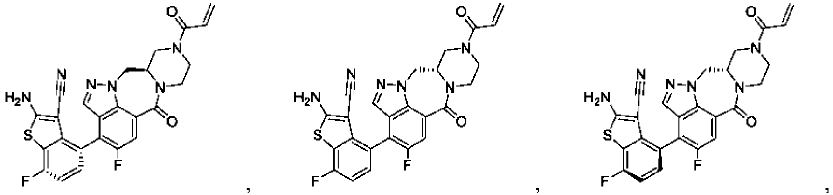

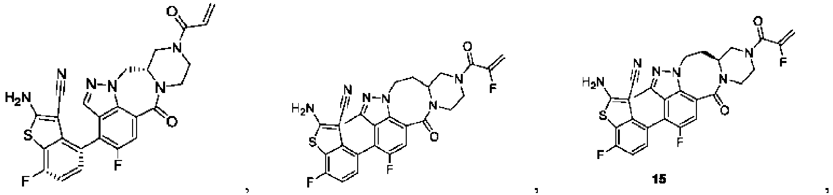

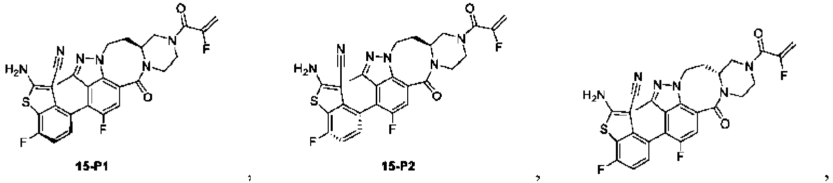

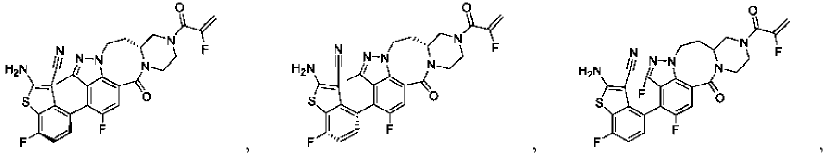

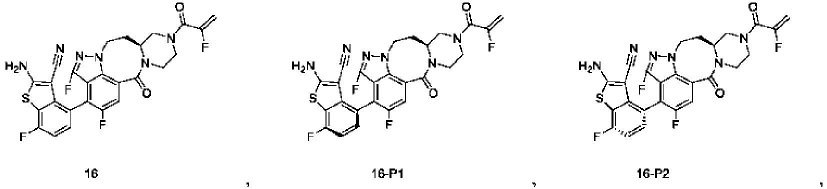

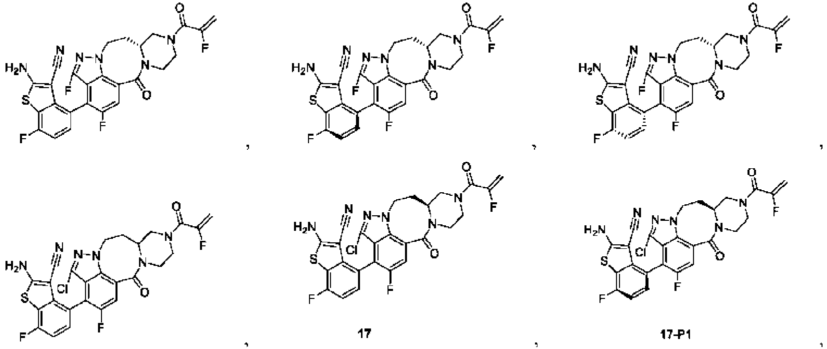

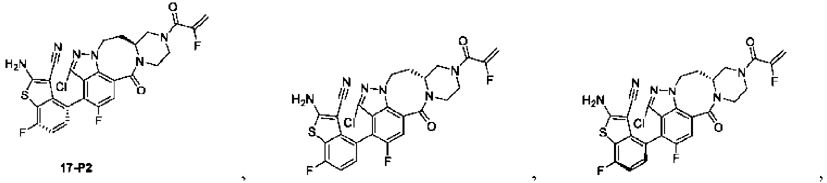

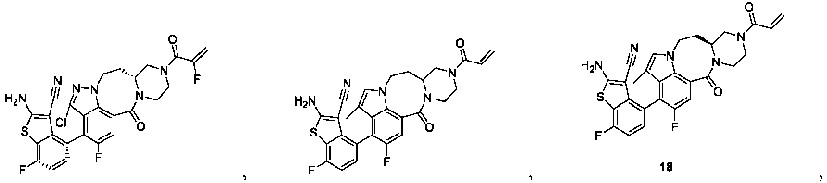

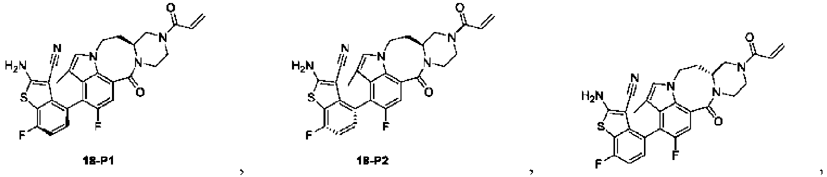

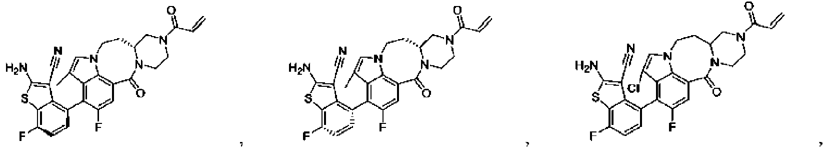

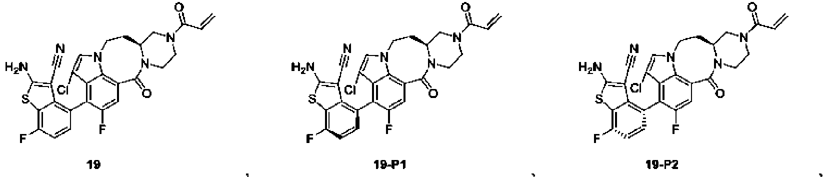

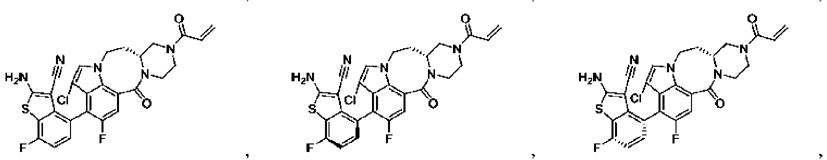

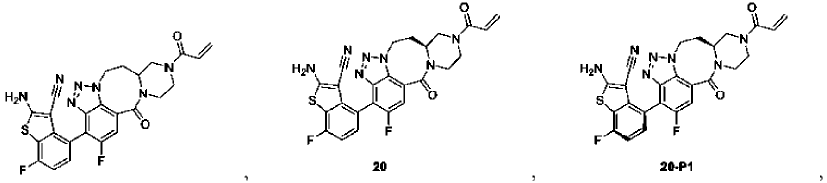

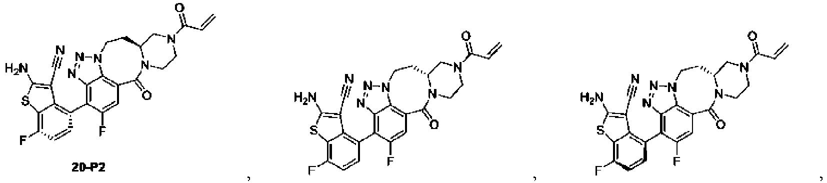

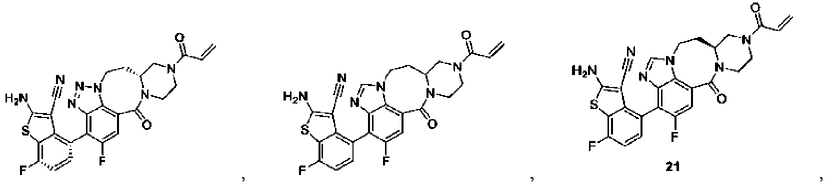

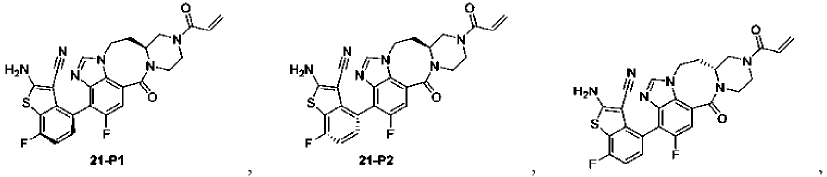

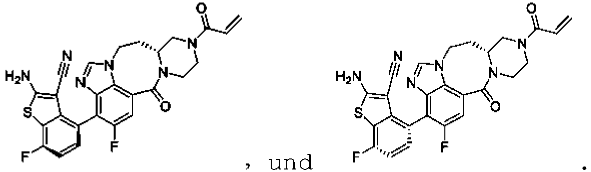

10. Verbindung der allgemeinen Formel (IMa) oder ein Salz davon: 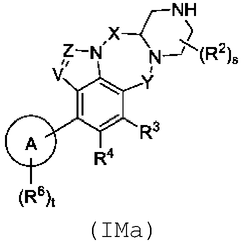 wobei:
Ring A, V, X, Y, Z, R², R³, R⁴, R⁶, s und t nach Anspruch 1 definiert sind.

11. Verbindung oder ein Salz davon, ausgewählt aus der Gruppe, bestehend aus den folgenden Verbindungen:

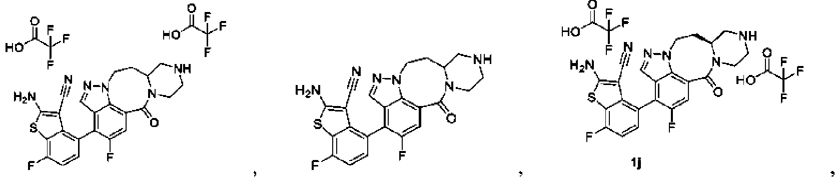

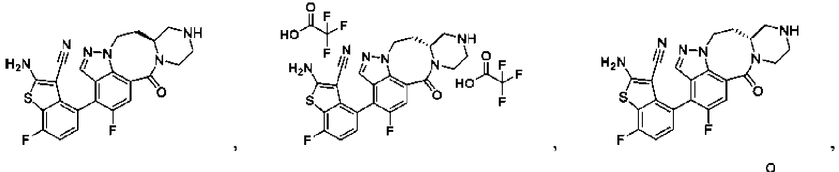

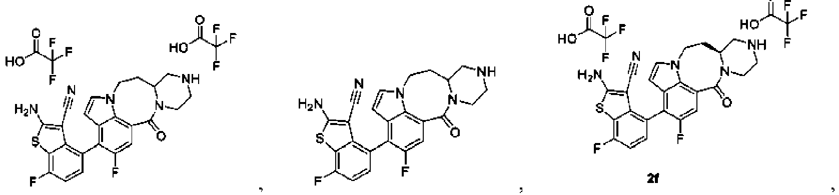

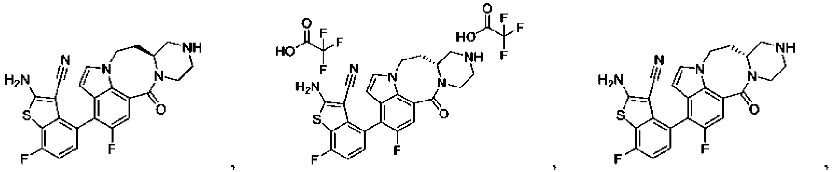

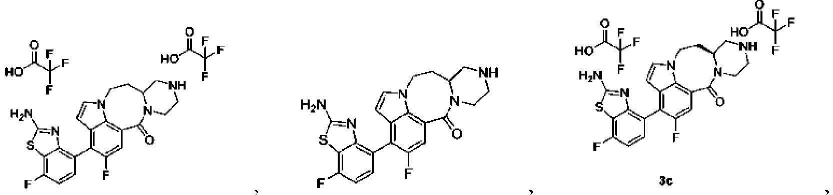

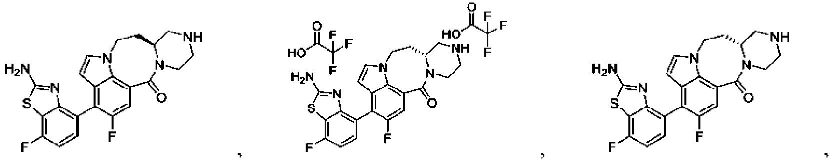

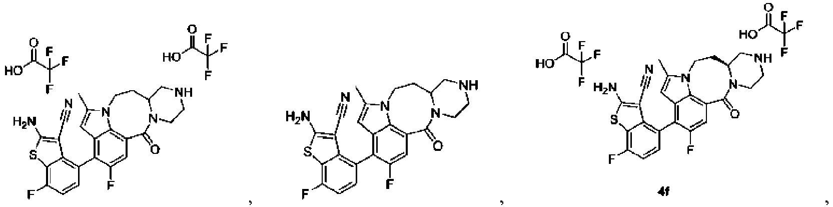

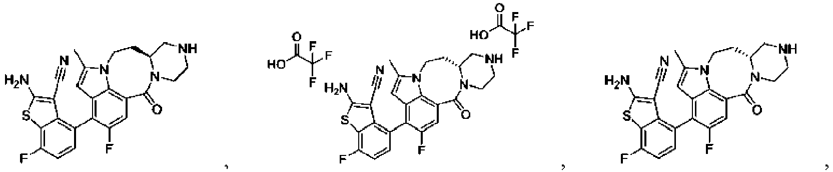

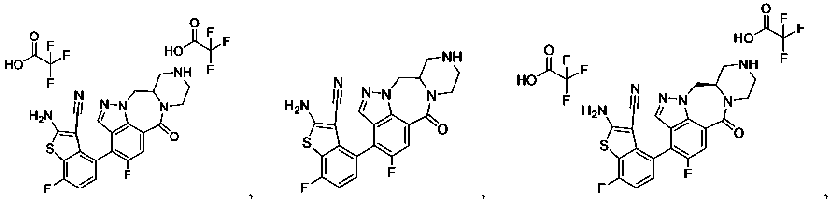

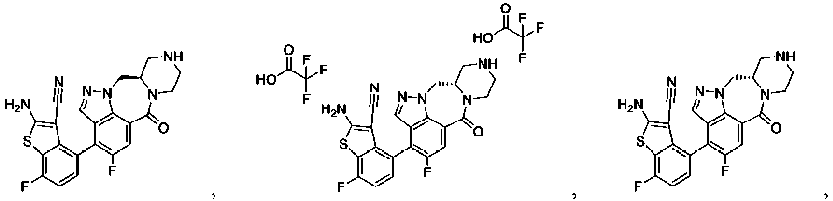

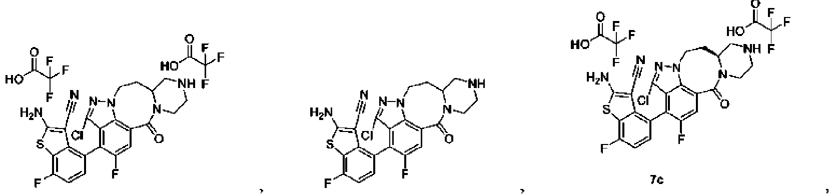

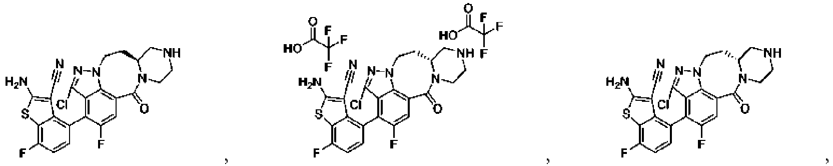

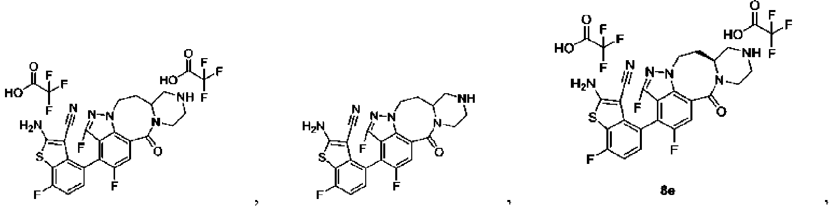

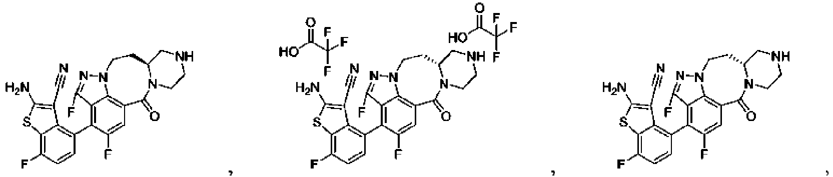

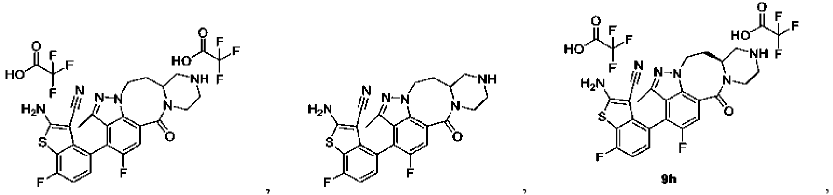

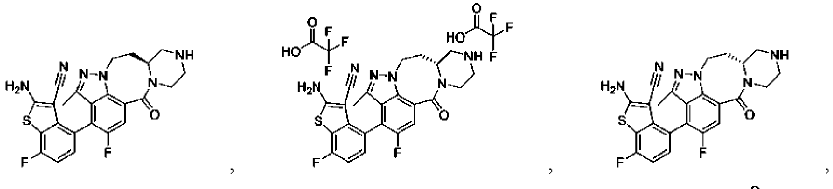

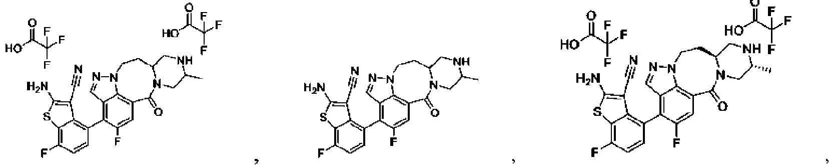

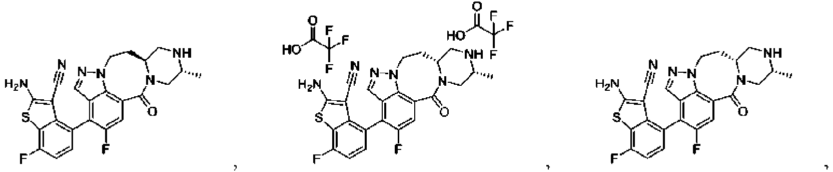

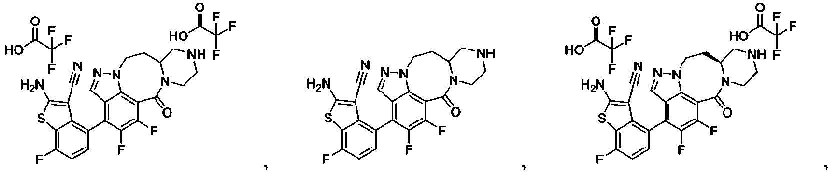

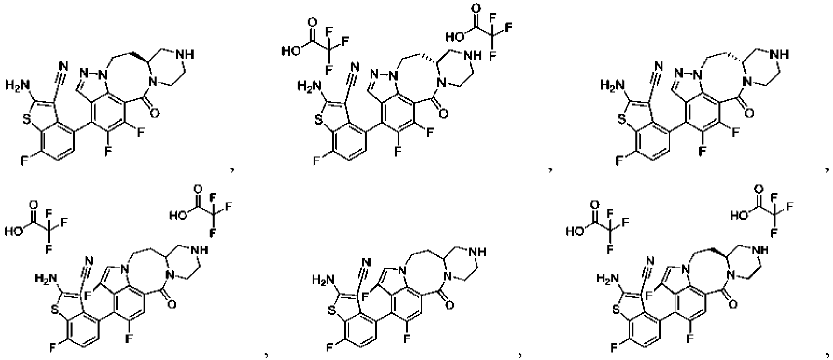

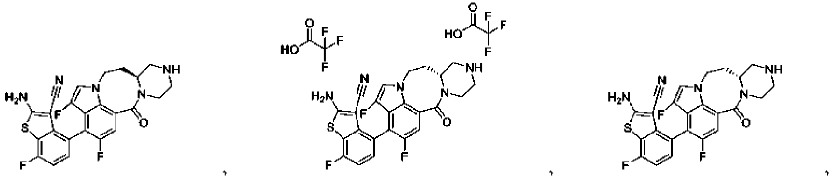

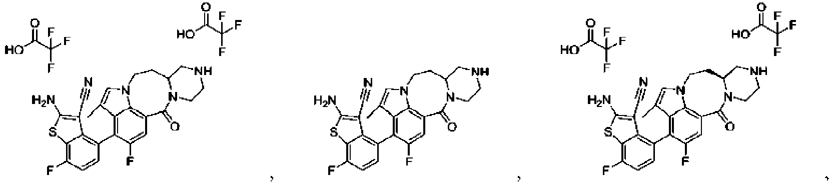

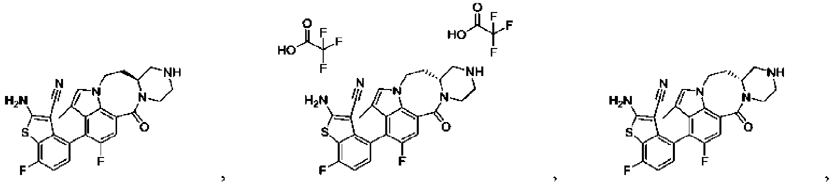

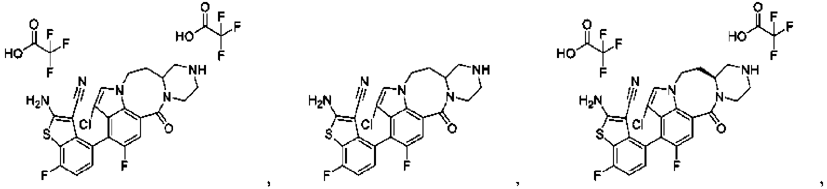

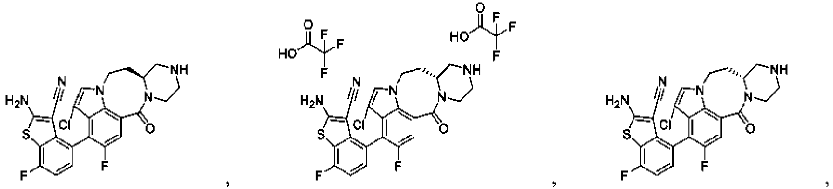

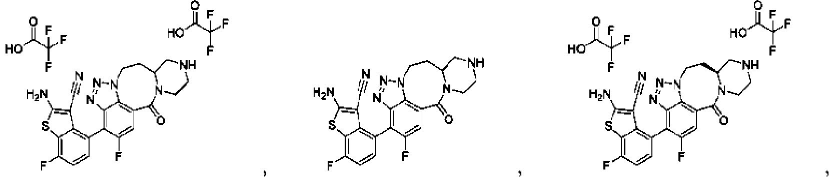

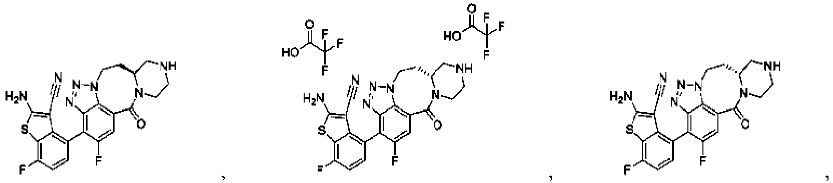

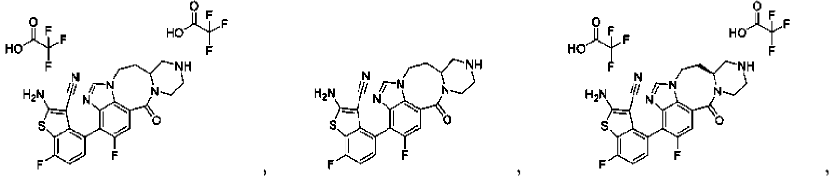

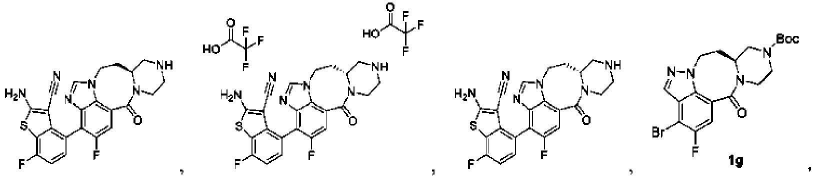

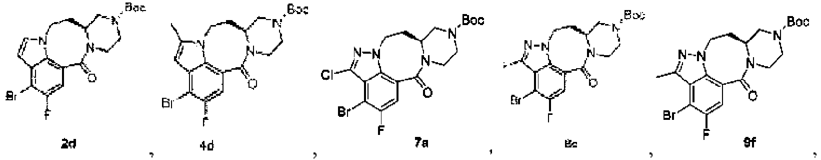

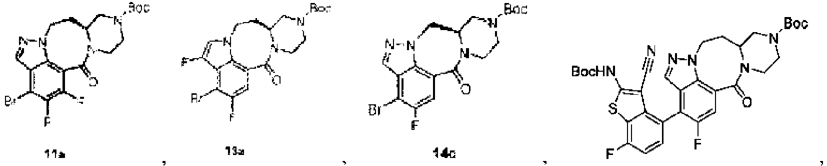

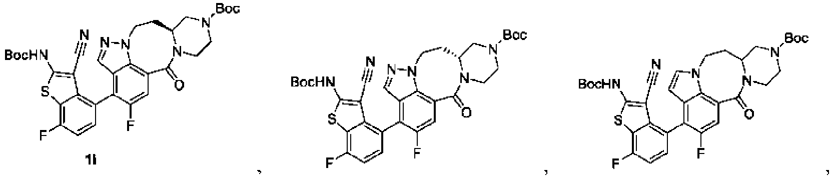

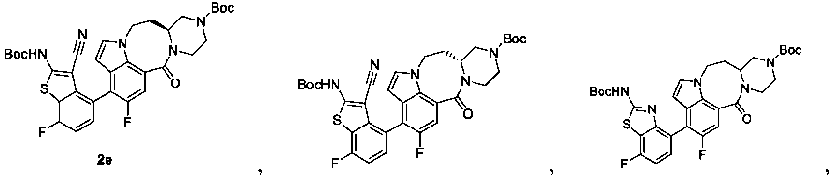

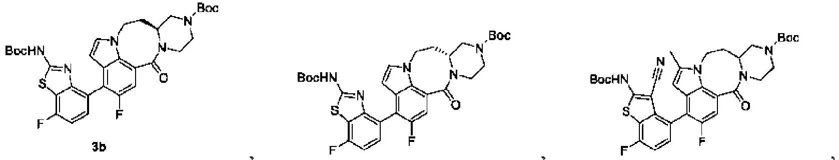

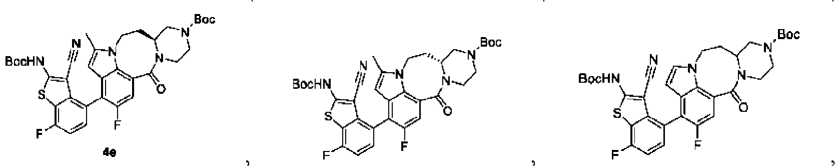

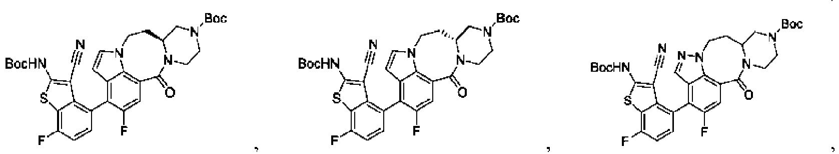

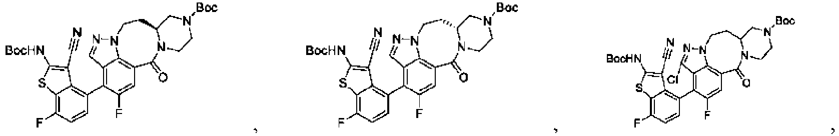

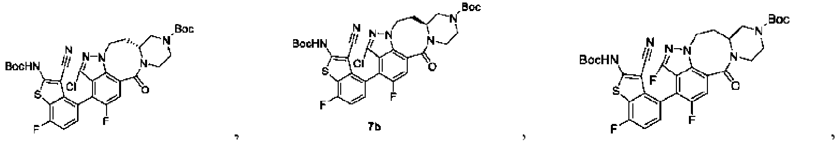

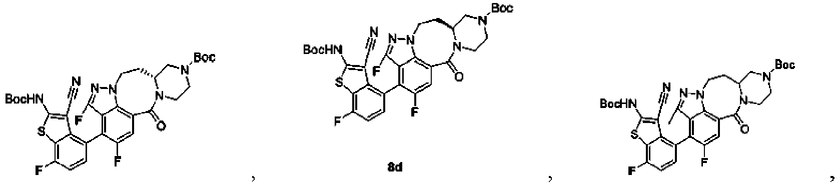

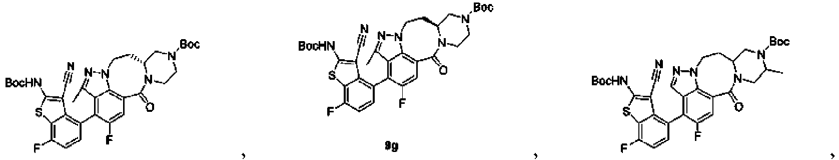

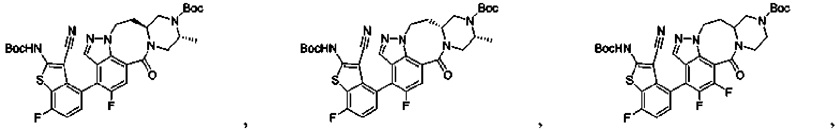

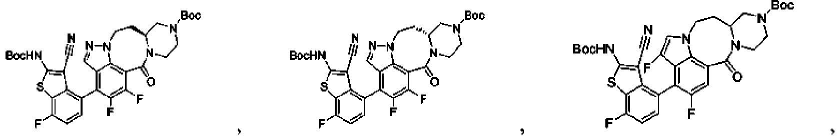

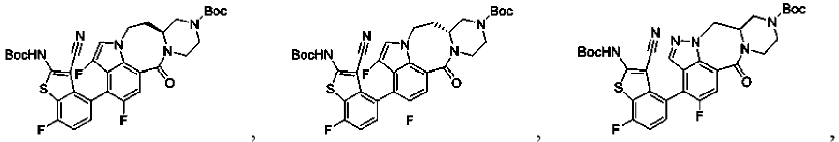

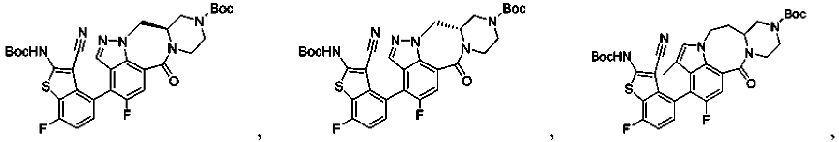

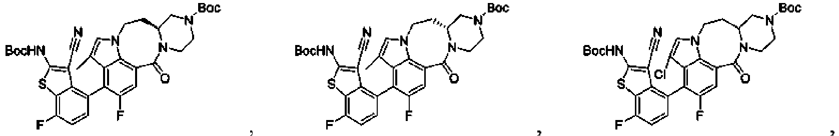

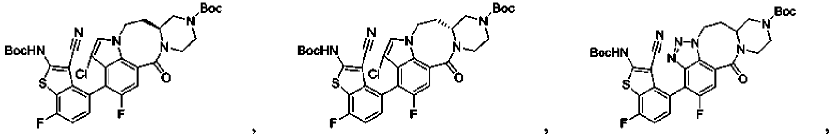

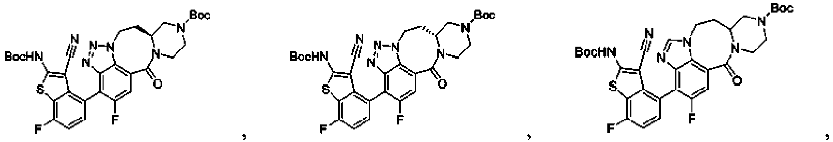

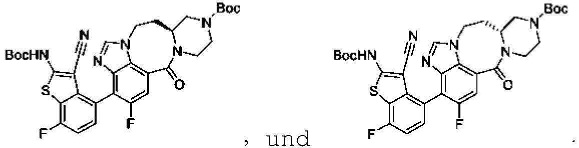

12. Verfahren zur Herstellung einer Verbindung der allgemeinen Formel (IM) oder eines pharmazeutisch verträglichen Salzes davon nach Anspruch 1, umfassend: 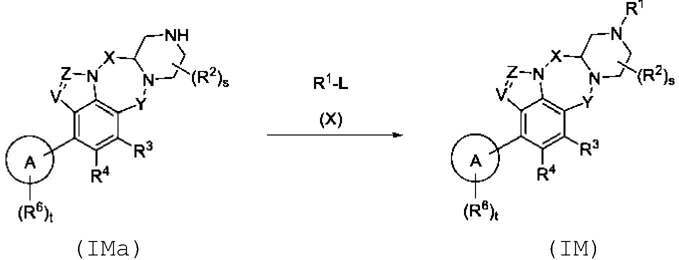 Durchführen einer Reaktion einer Verbindung der allgemeinen Formel (IMa) oder eines Salzes davon mit einer Verbindung der allgemeinen Formel (X) oder einem Salz davon, um die Verbindung der allgemeinen Formel (IM) oder ein pharmazeutisch verträgliches Salz davon zu erhalten;
wobei:
L ein Halogen ist; vorzugsweise L Cl ist;
R¹ 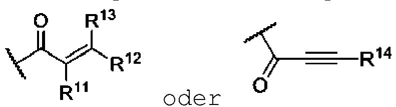 ist;
Ring A, V, X, Y, Z, R², R³, R⁴, R⁶, R¹¹, R¹², R¹³, R¹⁴, s und t nach Anspruch 1 definiert sind.

13. Pharmazeutische Zusammensetzung, wobei die pharmazeutische Zusammensetzung die Verbindung der allgemeinen Formel (IM) oder ein pharmazeutisch verträgliches Salz davon nach einem der Ansprüche 1 bis 9 sowie einen oder mehrere pharmazeutisch verträgliche Trägerstoffe, Verdünnungsmittel oder Hilfsstoffe umfasst.

14. Verbindung der allgemeinen Formel (IM) oder ein pharmazeutisch verträgliches Salz davon nach einem der Ansprüche 1 bis 9 oder die pharmazeutische Zusammensetzung nach Anspruch 13 zur Verwendung als Arzneimittel, vorzugsweise zur Verwendung als KRAS-G12C-Inhibitor.

15. Verbindung der allgemeinen Formel (IM) oder ein pharmazeutisch verträgliches Salz davon nach einem der Ansprüche 1 bis 9 oder die pharmazeutische Zusammensetzung nach Anspruch 13 zur Verwendung bei der Behandlung und/oder Vorbeugung eines Tumors; vorzugsweise handelt es sich bei dem Tumor um eine Krebserkrankung; noch bevorzugter wird die Krebserkrankung aus der Gruppe ausgewählt, die aus Lungenkrebs, Bauchspeicheldrüsenkrebs, Gebärmutterhalskrebs, Speiseröhrenkrebs, Gebärmutterschleimhautkrebs, Eierstockkrebs, Gallengangskarzinom, Darmkrebs, Leberkrebs, Brustkrebs, Prostatakrebs, Schilddrüsenkrebs, Magenkrebs, Urothelkarzinom, Hodenkrebs, Leukämie, Hautkrebs, Plattenepithelkarzinom, Basalzellkarzinom, Blasenkrebs, Kopf-Hals-Krebs, Nierenkrebs, Nasopharynxkarzinom, Knochenkrebs, Lymphom, Melanom, Sarkom, peripherem Neuroepitheliom, Gliom, Hirntumor und Myelom besteht; am bevorzugtesten wird die Krebsart aus der Gruppe ausgewählt, die aus Lungenkrebs, Bauchspeicheldrüsenkrebs, Gebärmutterhalskrebs, Speiseröhrenkrebs, Gebärmutterschleimhautkrebs, Eierstockkrebs, Cholangiokarzinom und Darmkrebs besteht.

## Revendications

1. Composé représenté par la formule générale (IM) ou sel pharmaceutiquement acceptable de celui-ci : 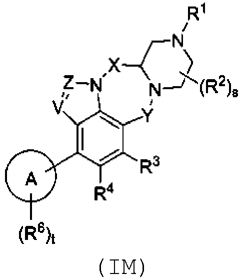 où :
X représente C(R^{a}R^{b}) ou C(R^{a}R^{b})-C(R^{c}R^{d}) ;
Y représente C(O) ou CH₂ ;
Z représente CR^{5a} ou N ;
V représente CR⁵ ou N ;
le cycle A représente un groupe aryle ou hétéroaryle ;
R^{a}, R^{b}, R^{c} et R^{d} sont identiques ou différents à chaque occurrence et sont chacun indépendamment sélectionnés parmi le groupe constitué d'un atome d'hydrogène, d'un halogène, d'un groupe alkyle, haloalkyle, hydroxyalkyle, alcoxy, haloalcoxy, alcényle, alcynyle, hydroxy et cyano ;
R¹ est sélectionné parmi le groupe constitué de cynao, 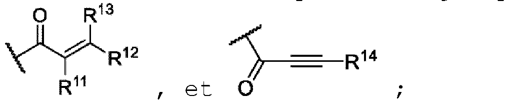
chaque groupe R² est identique ou différent et est indépendamment sélectionné parmi le groupe constitué d'un atome d'hydrogène, d'un halogène, d'un groupe cyano, alkyle, alcoxy, hydroxy et amino, dans lequel les groupes alkyle et alcoxy sont chacun indépendamment éventuellement substitués par un ou plusieurs substituants sélectionnés parmi le groupe constitué d'un halogène, d'un groupe cyano, amino et hydroxy ;
R³, R⁴, R⁵ et R^{5a} sont identiques ou différents et sont chacun indépendamment sélectionnés parmi le groupe constitué d'un atome d'hydrogène, d'un halogène, d'un groupe cyano, alkyle, alcényle, alcynyle, -NR^{7a}R^{7b}, - C(O)R⁸, -OR⁸, -S(O)ₚR⁸, cycloalkyle, hétérocyclyle, aryle et hétéroaryle, dans lequel les groupes alkyle, cycloalkyle, hétérocyclyle, aryle et hétéroaryle sont chacun indépendamment éventuellement substitués par un ou plusieurs substituants sélectionnés parmi le groupe constitué d'un halogène, d'un groupe alkyle, haloalkyle, cyano, -NR^{7c}R^{7d}, -OR^{8a}, cycloalkyle, hétérocyclyle, aryle et hétéroaryle ;
chaque R⁶ est identique ou différent et est indépendamment sélectionné parmi le groupe constitué d'un atome d'hydrogène, d'un halogène, d'un groupe cyano, alkyle, alcényle, alcynyle, -NR^{9a}R^{9b}, -C(O)NR^{9a}R^{9b}, -C(O)R¹⁰, - C(O)OR¹⁰, -OC(O)R¹⁰, -OR¹⁰, -S(O)ₚR¹⁰, -S(O)ₚNR^{9a}R^{9b}, cycloalkyle, hétérocyclyle, aryle et hétéroaryle, dans lequel les groupes alkyle, cycloalkyle, hétérocyclyle, aryle et hétéroaryle sont chacun indépendamment éventuellement substitués par un ou plusieurs substituants sélectionnés parmi le groupe constitué d'un halogène, d'un groupe alkyle, haloalkyle, cyano, -NR^{9c}R^{9d}, -OR^{10a}, cycloalkyle, hétérocyclyle, aryle et hétéroaryle ;
R¹¹, R¹², R¹³ et R¹⁴ sont identiques ou différents et sont indépendamment sélectionnés parmi le groupe constitué d'un atome d'hydrogène, d'un halogène, d'un groupe alkyle, -NR^{15a}R^{15b}, -OR¹⁶, cyano, cycloalkyle, hétérocyclyle, aryle et hétéroaryle, dans lequel les groupes alkyle, cycloalkyle, hétérocyclyle, aryle et hétéroaryle sont chacun indépendamment éventuellement substitués par un ou plusieurs substituants sélectionnés parmi le groupe constitué d'un halogène, d'un groupe oxo, alkyle, haloalkyle, alcoxy, haloalcoxy, cyano, -NR^{15c}R^{15d}, hydroxy, hydroxyalkyle, cycloalkyle, hétérocyclyle, aryle et hétéroaryle ;
R⁸, R^{8a}, R¹⁰, R^{10a} et R¹⁶ sont identiques ou différents à chaque occurrence et sont chacun indépendamment sélectionnés parmi le groupe constitué d'un atome d'hydrogène, d'un groupe alkyle, hydroxyalkyle, cycloalkyle, hétérocyclyle, aryle et hétéroaryle, dans lequel les groupes alkyle, cycloalkyle, hétérocyclyle, aryle et hétéroaryle sont chacun indépendamment éventuellement substitués par un ou plusieurs substituants sélectionnés parmi le groupe constitué d'un halogène, d'un groupe alkyle, alcényle, alcynyle, oxo, alcoxy, haloalkyle, haloalcoxy, cyano, -NR^{17a}R^{17b}, hydroxy, cycloalkyle, hétérocyclyle, aryle et hétéroaryle ;
R^{7a}, R^{7b}, R^{7c}, R^{7d}, R^{9a}, R^{9b}, R^{9c}, R^{9d}, R^{15a}, R^{15b}, R^{15c}, R^{15d}, R^{17a} et R^{17b} sont identiques ou différents à chaque occurrence et sont chacun indépendamment sélectionnés parmi le groupe constitué d'un atome d'hydrogène, d'un groupe alkyle, hydroxyalkyle, cycloalkyle, hétérocyclyle, aryle et hétéroaryle, dans lequel les groupes alkyle, cycloalkyle, hétérocyclyle, aryle et hétéroaryle sont chacun indépendamment éventuellement substitués par un ou plusieurs substituants sélectionnés parmi le groupe constitué d'un atome d'halogène, d'un groupe oxo, hydroxy, cyano, alkyle, alcoxy, haloalkyle et haloalcoxy ;
ou R^{7a} et R^{7b}, conjointement avec l'atome d'azote auquel ils sont liés, forment un groupe hétérocyclyle, dans lequel le groupe hétérocyclyle est éventuellement substitué par un ou plusieurs substituants sélectionnés parmi le groupe constitué d'un atome d'halogène, d'un groupe oxo, alkyle, alcoxy, haloalkyle, haloalcoxy, cyano, amino, nitro, hydroxy, hydroxyalkyle, cycloalkyle, hétérocyclyle, aryle et hétéroaryle ;
ou R^{7c} et R^{7d}, conjointement avec l'atome d'azote auquel ils sont liés, forment un hétérocyclyle, dans lequel le groupe hétérocyclyle est éventuellement substitué par un ou plusieurs substituants sélectionnés parmi le groupe constitué d'un atome d'halogène, d'un groupe oxo, alkyle, alcoxy, haloalkyle, haloalcoxy, cyano, amino, nitro, hydroxy, hydroxyalkyle, cycloalkyle, hétérocyclyle, aryle et hétéroaryle ;
ou R^{9a} et R^{9b}, conjointement avec l'atome d'azote auquel ils sont liés, forment un groupe hétérocyclyle, dans lequel le groupe hétérocyclyle est éventuellement substitué par un ou plusieurs substituants sélectionnés parmi le groupe constitué d'un atome d'halogène, d'un groupe oxo, alkyle, alcoxy, haloalkyle, haloalcoxy, cyano, amino, nitro, hydroxy, hydroxyalkyle, cycloalkyle, hétérocyclyle, aryle et hétéroaryle ;
ou R^{9c} et R^{9d}, conjointement avec l'atome d'azote auquel ils sont liés, forment un hétérocyclyle, dans lequel le groupe hétérocyclyle est éventuellement substitué par un ou plusieurs substituants sélectionnés parmi le groupe constitué d'un atome d'halogène, d'un groupe oxo, alkyle, alcoxy, haloalkyle, haloalcoxy, cyano, amino, nitro, hydroxy, hydroxyalkyle, cycloalkyle, hétérocyclyle, aryle et hétéroaryle ;
ou R^{15a} et R^{15b}, conjointement avec l'atome d'azote auquel ils sont liés, forment un groupe hétérocyclyle, dans lequel le groupe hétérocyclyle est éventuellement substitué par un ou plusieurs substituants sélectionnés parmi le groupe constitué d'un atome d'halogène, d'un groupe oxo, alkyle, alcoxy, haloalkyle, haloalcoxy, cyano, amino, nitro, hydroxy, hydroxyalkyle, cycloalkyle, hétérocyclyle, aryle et hétéroaryle ;
ou R^{15c} et R^{15d}, conjointement avec l'atome d'azote auquel ils sont liés, forment un hétérocyclyle, dans lequel le groupe hétérocyclyle est éventuellement substitué par un ou plusieurs substituants sélectionnés parmi le groupe constitué d'un atome d'halogène, d'un groupe oxo, alkyle, alcoxy, haloalkyle, haloalcoxy, cyano, amino, nitro, hydroxy, hydroxyalkyle, cycloalkyle, hétérocyclyle, aryle et hétéroaryle ;
ou R^{17a} et R^{17b}, conjointement avec l'atome d'azote auquel ils sont liés, forment un groupe hétérocyclyle, dans lequel le groupe hétérocyclyle est éventuellement substitué par un ou plusieurs substituants sélectionnés parmi le groupe constitué d'un atome d'halogène, d'un groupe oxo, alkyle, alcoxy, haloalkyle, haloalcoxy, cyano, amino, nitro, hydroxy, hydroxyalkyle, cycloalkyle, hétérocyclyle, aryle et hétéroaryle ;
s représente 0, 1, 2, 3, 4, 5 ou 6 ;
t représente 0, 1, 2, 3, 4 ou 5 ; et
p représente 0, 1 ou 2.

2. Composé représenté par la formule générale (IM) ou sel pharmaceutiquement acceptable de celui-ci selon la revendication 1, étant un composé représenté par la formule générale (I) ou un sel pharmaceutiquement acceptable de celui-ci : 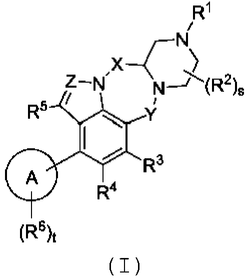 où :
le cycle A, X, Y, Z, R¹, R², R³, R⁴, R⁵, R⁶, s et t sont tels que définis dans la revendication 1.

3. Composé représenté par la formule générale (IM) ou sel pharmaceutiquement acceptable de celui-ci selon la revendication 1 ou 2, dans lequel Y représente C(O) ; et/ou s représente 0 ou 1.

4. Composé représenté par la formule générale (IM) ou sel pharmaceutiquement acceptable de celui-ci selon l'une quelconque des revendications 1 à 3, étant un composé représenté par la formule générale (II) ou un sel pharmaceutiquement acceptable de celui-ci : 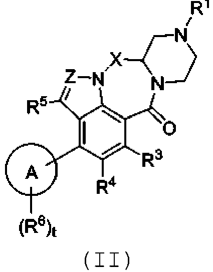 où :
le cycle A, X, Z, R¹, R³, R⁴, R⁵, R⁶ et t sont tels que définis dans la revendication 1.

5. Composé représenté par la formule générale (IM) ou sel pharmaceutiquement acceptable de celui-ci selon l'une quelconque des revendications 1 à 4, dans lequel le cycle A représente un groupe hétéroaryle bicyclique de 8 à 10 chaînons contenant dans le cycle 1, 2 ou 3 hétéroatomes sélectionnés parmi le groupe constitué d'azote, d'oxygène et de soufre, et/ou
R¹ représente 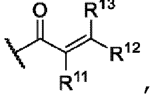 où R¹¹, R¹² et R¹³ sont tels que définis dans la revendication 1, et/ou X représente CH₂ ou CH₂-CH₂ ; de préférence, X représente CH₂-CH₂.

6. Composé représenté par la formule générale (IM) ou sel pharmaceutiquement acceptable de celui-ci selon l'une quelconque des revendications 1 à 5, étant un composé représenté par la formule générale (III) ou un sel pharmaceutiquement acceptable de celui-ci : 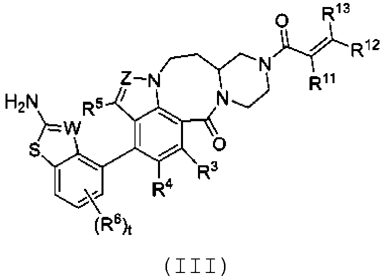 où :
W représente C(CN) ou N ;
t représente 0, 1, 2 ou 3 ;
Z, R³, R⁴, R⁵, R⁶, R¹¹, R¹² et R¹³ sont tels que définis dans la revendication 1.

7. Composé représenté par la formule générale (IM) ou sel pharmaceutiquement acceptable de celui-ci selon l'une quelconque des revendications 1 à 6, dans lequel R³, R⁴, R⁵ et R^{5a} sont identiques ou différents et sont chacun indépendamment sélectionnés parmi le groupe constitué d'un atome d'hydrogène, d'un halogène, un groupe alkyle en C₁₋₆ et un groupe haloalkyle C₁₋₆, et/ou R¹¹ représente un atome d'hydrogène ou un halogène, et/ou R¹² représente un atome d'hydrogène, et/ou R¹³ représente un atome d'hydrogène.

8. Composé représenté par la formule générale (IM) ou sel pharmaceutiquement acceptable de celui-ci selon l'une quelconque des revendications 1 à 7, dans lequel chaque R⁶ est identique ou différent et est indépendamment sélectionné parmi le groupe constitué d'un halogène, d'un groupe cyano, -NH₂, alkyle en C₁₋₆ et haloalkyle en C₁₋₆, et t représente 0, 1, 2 ou 3.

9. Composé représenté par la formule générale (IM) ou sel pharmaceutiquement acceptable de celui-ci selon l'une quelconque des revendications 1 à 8, étant sélectionné parmi le groupe constitué des composés suivants :

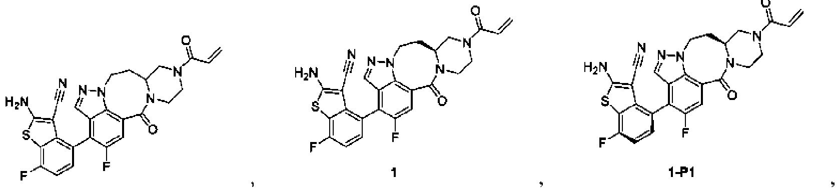

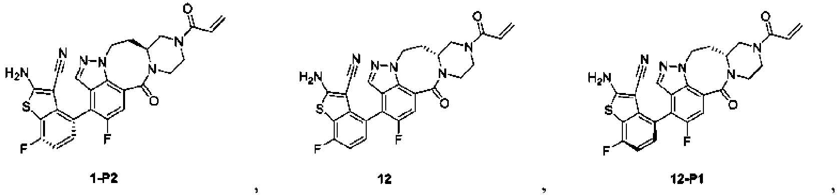

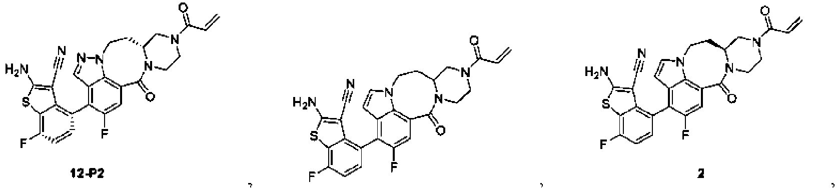

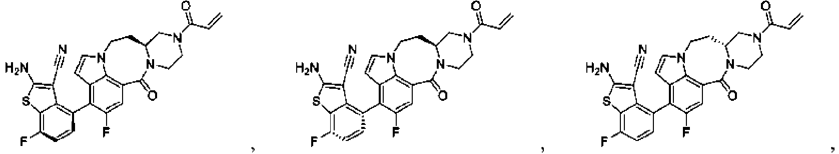

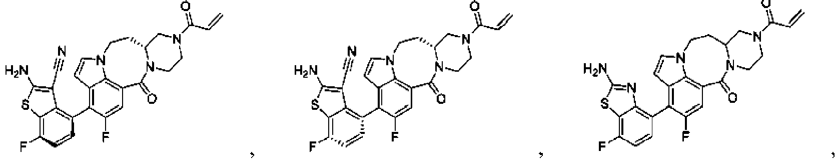

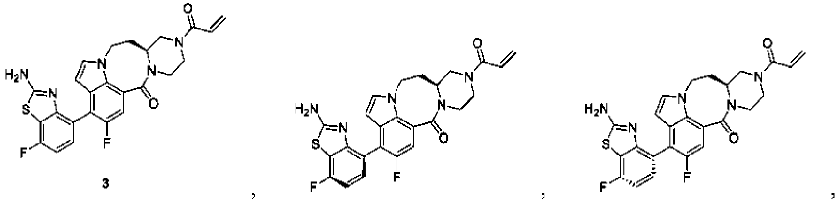

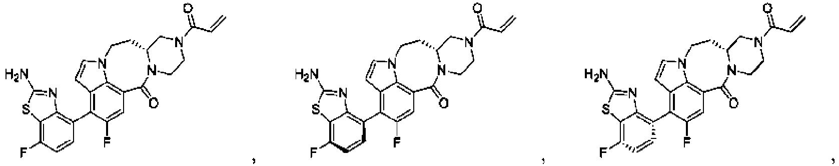

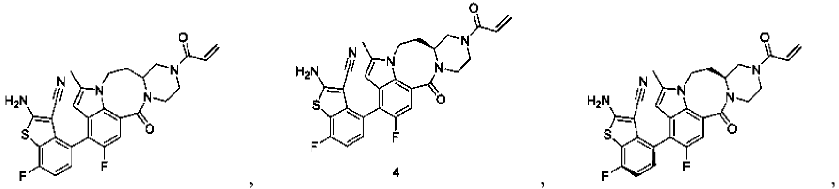

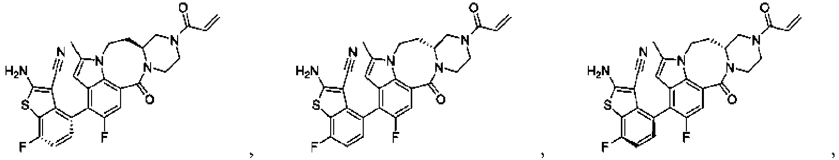

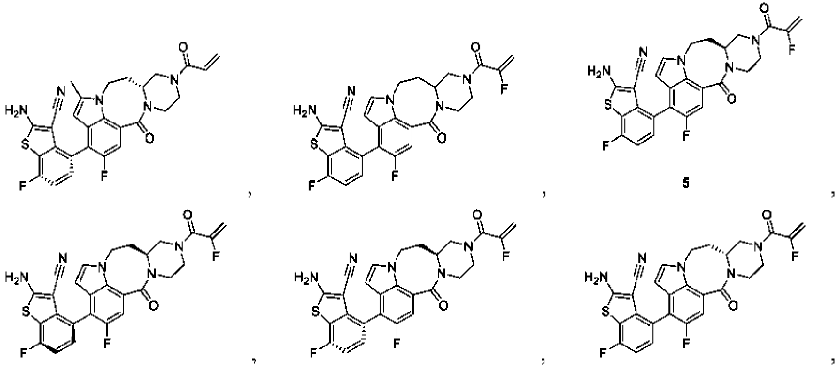

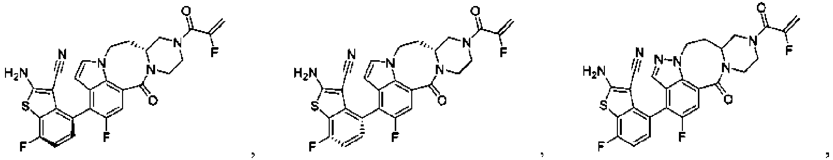

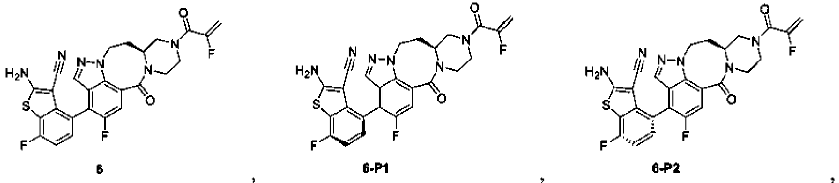

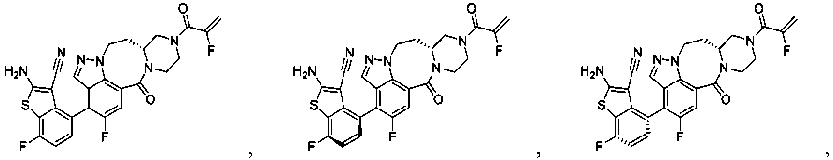

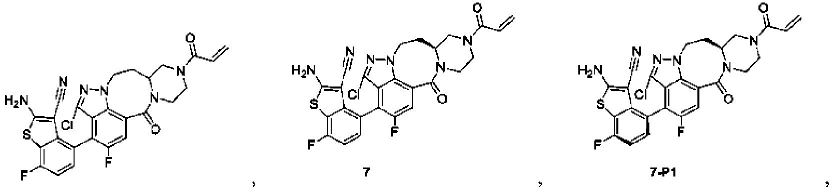

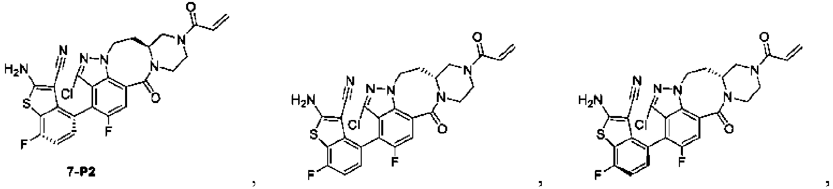

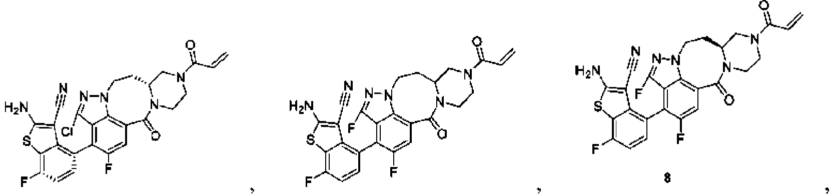

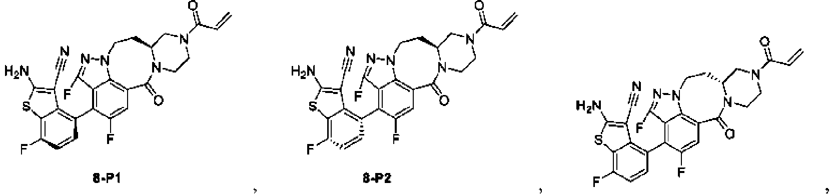

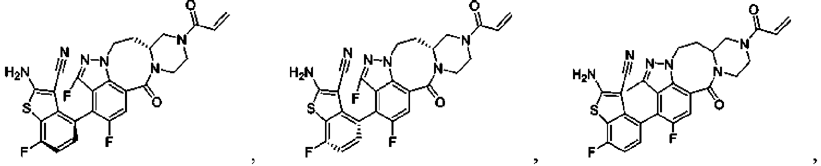

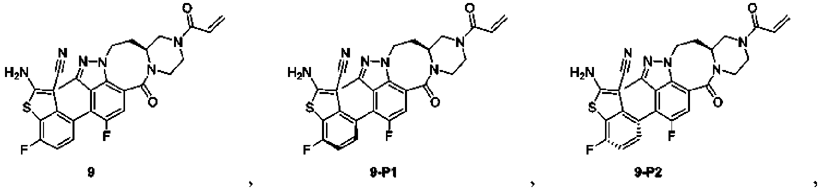

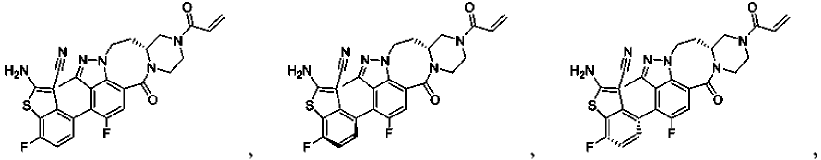

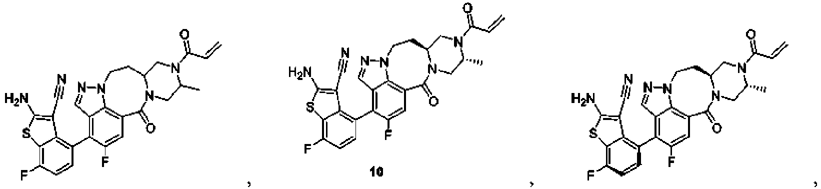

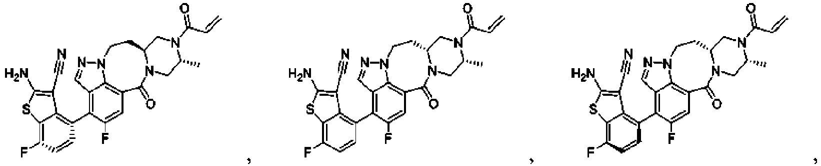

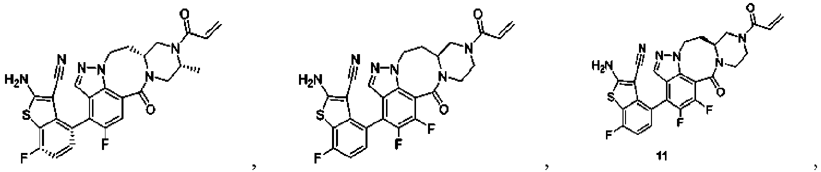

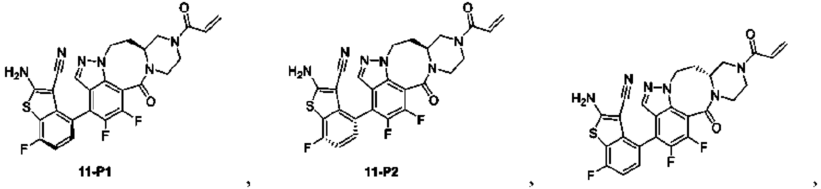

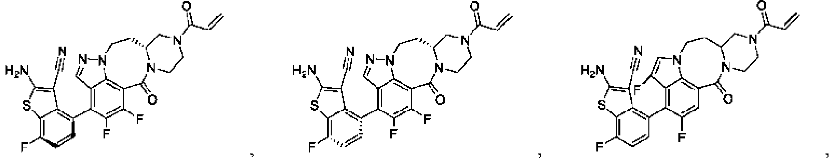

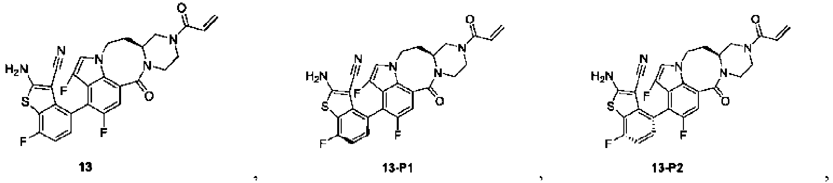

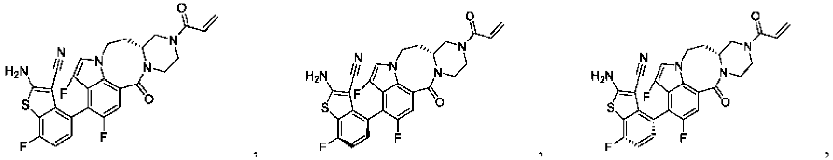

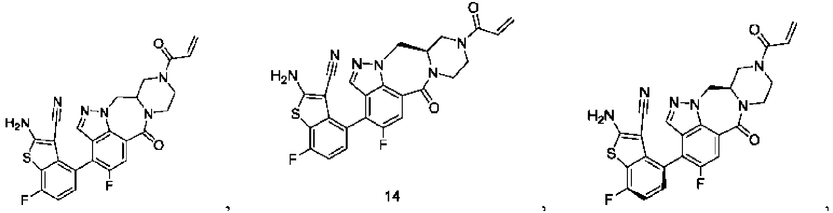

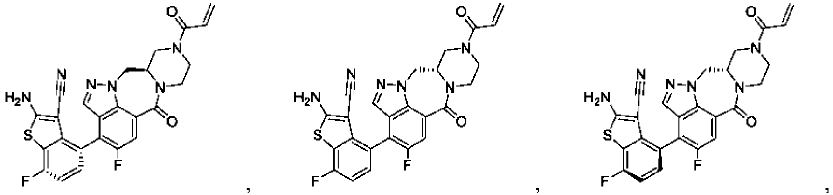

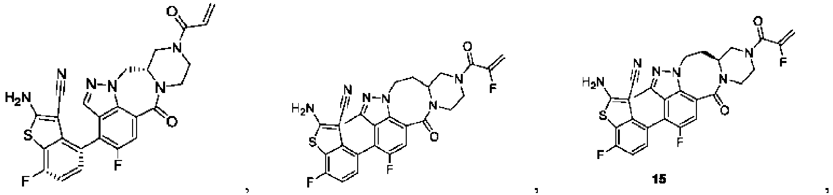

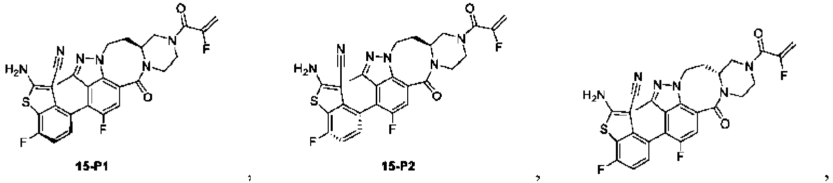

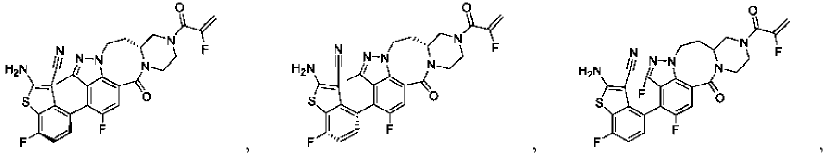

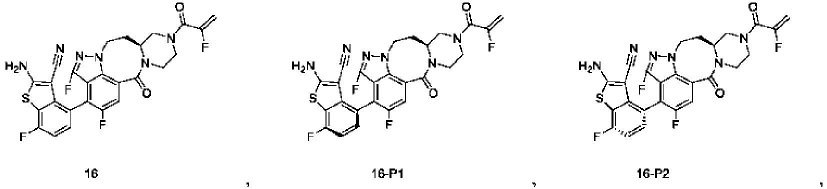

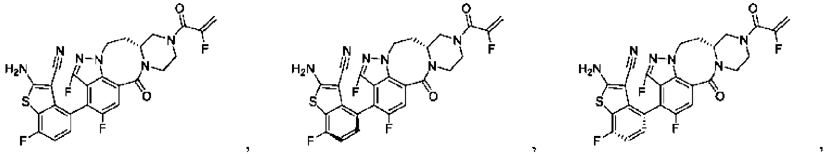

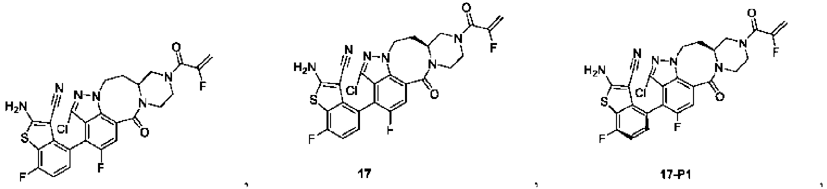

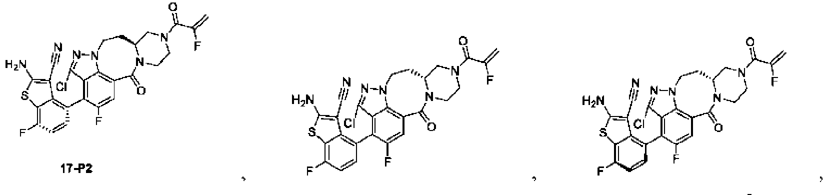

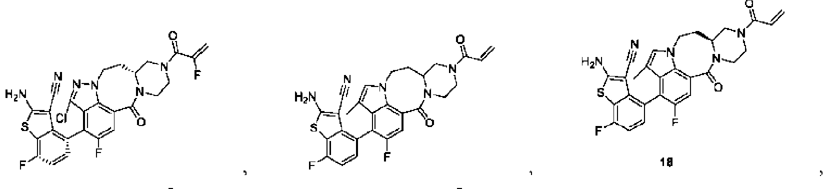

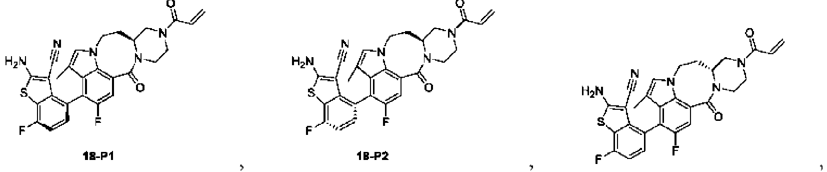

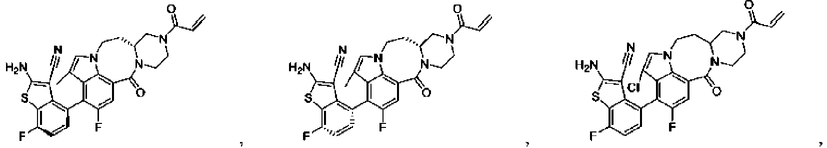

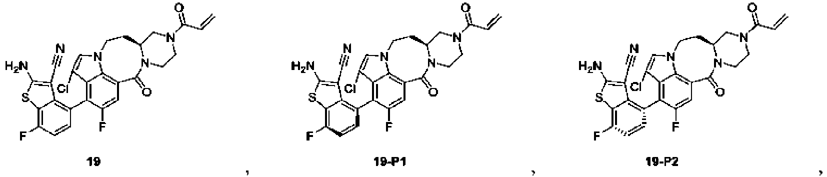

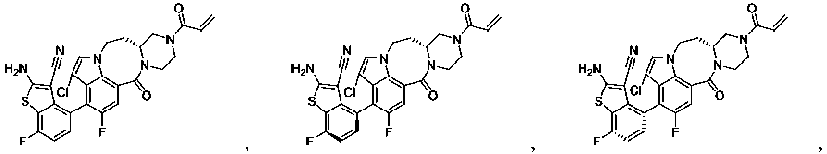

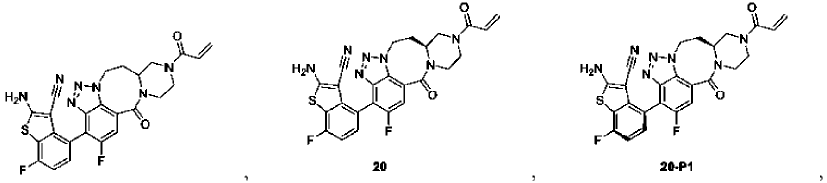

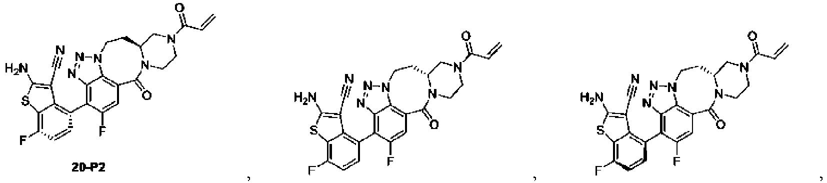

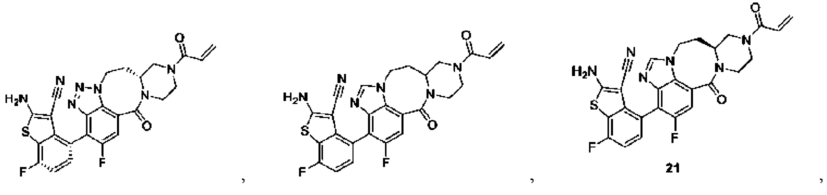

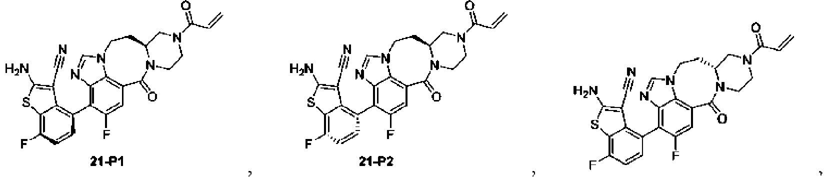

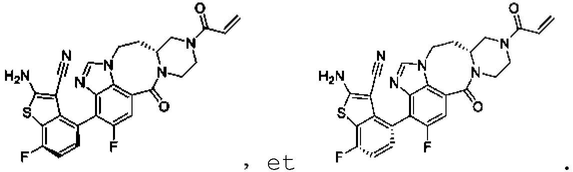

10. Composé représenté par la formule générale (IMa) ou sel de celui-ci : 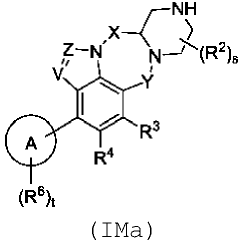 où :
les cycles A, V, X, Y, Z, R², R³, R⁴, R⁶, s et t sont tels que définis dans la revendication 1.

11. Composé ou sel de celui-ci, étant sélectionné parmi le groupe constitué des composés suivants :

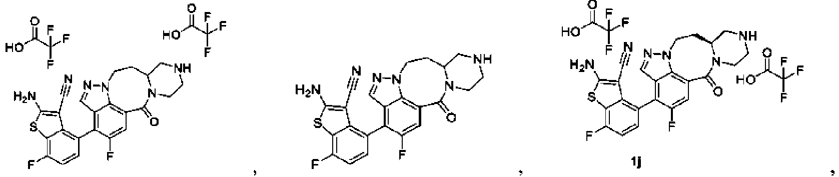

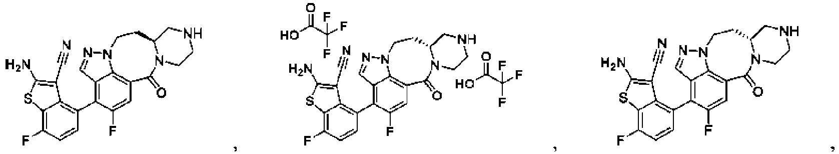

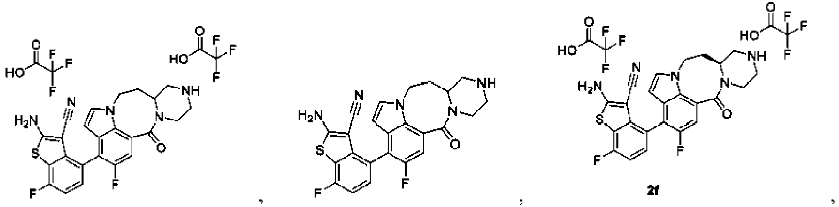

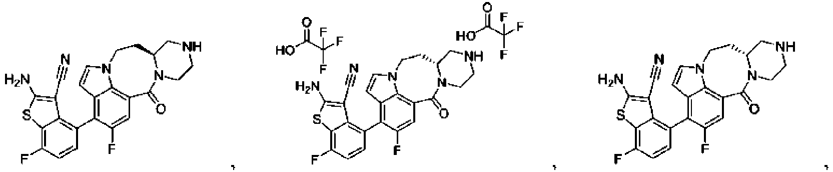

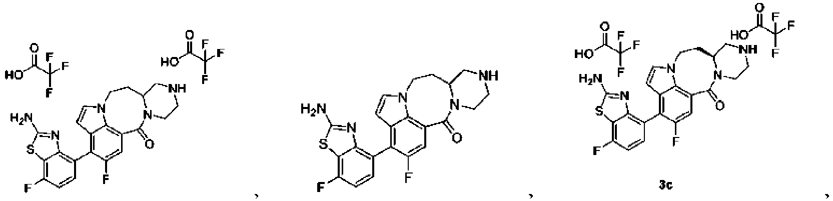

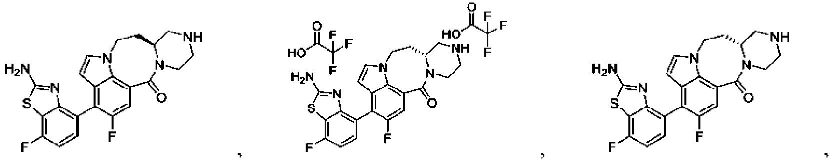

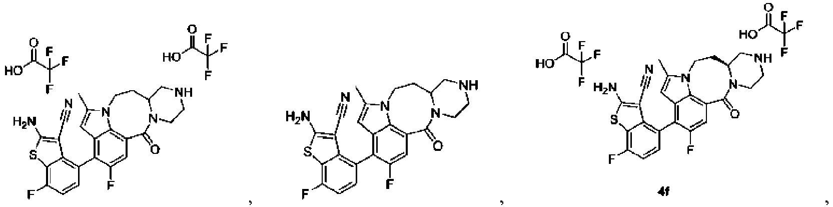

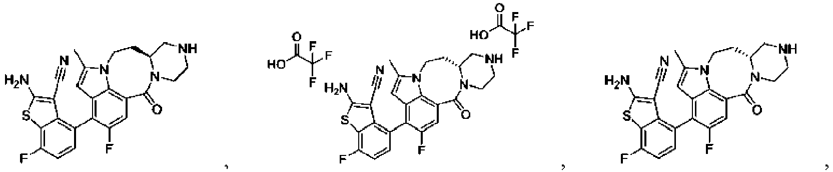

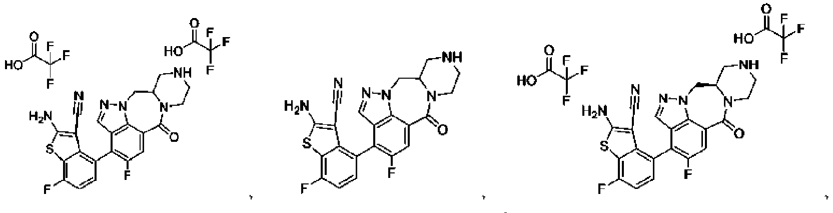

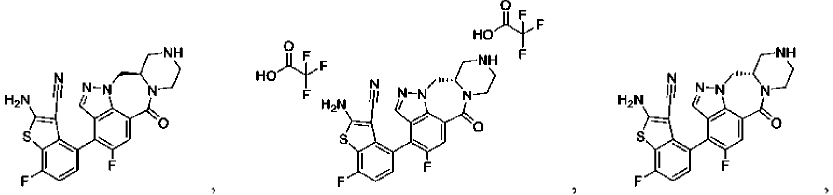

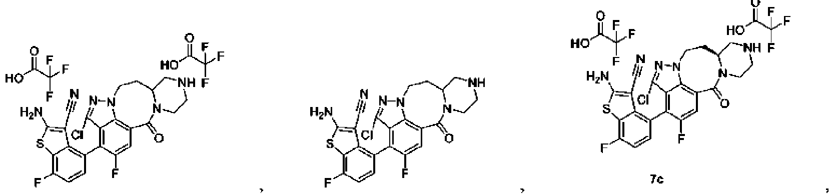

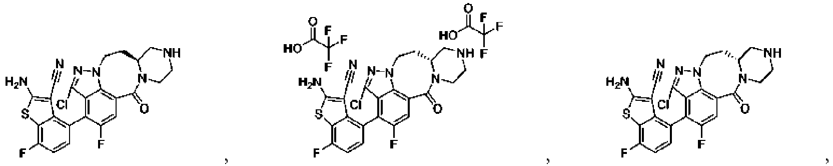

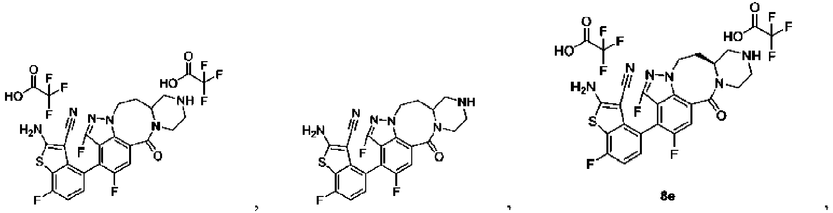

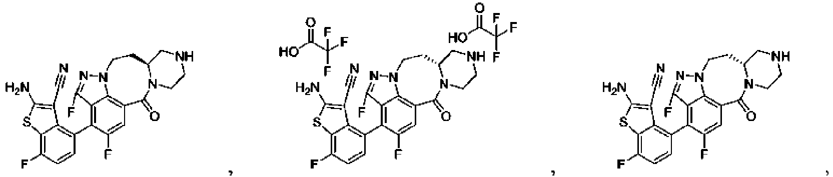

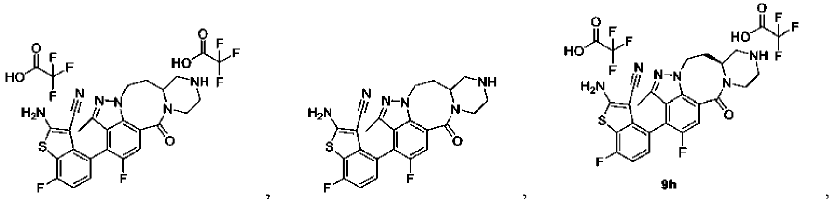

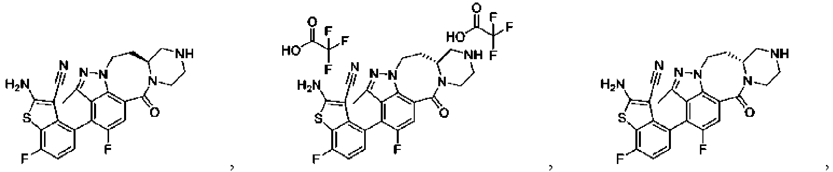

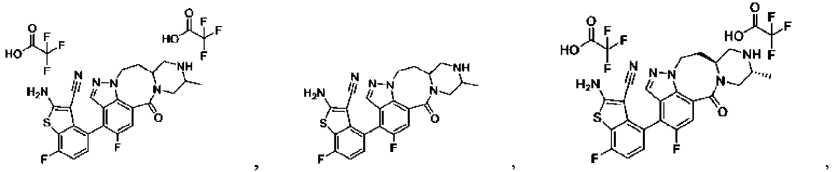

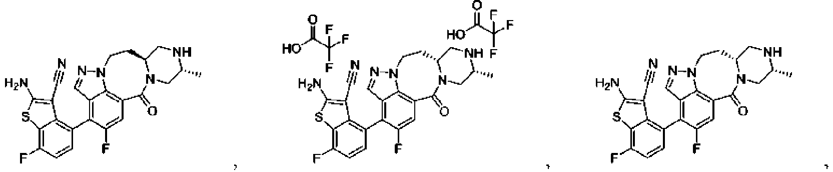

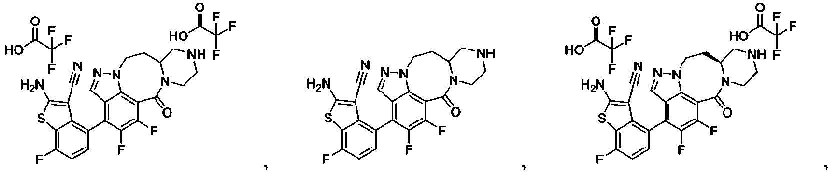

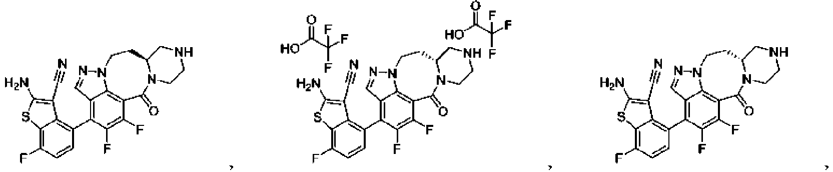

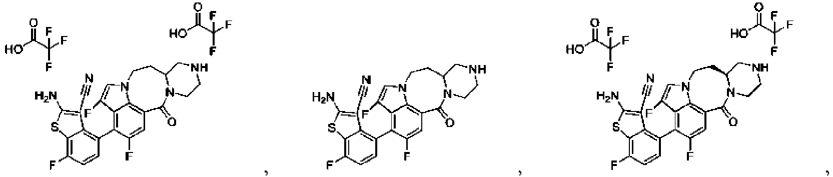

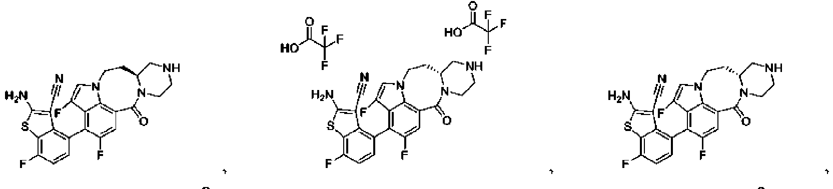

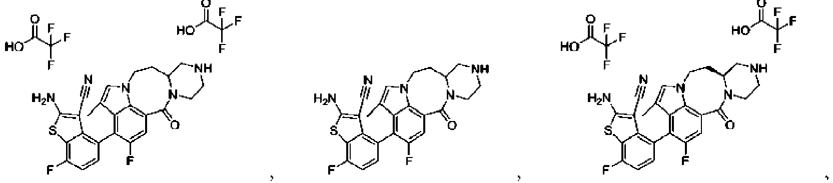

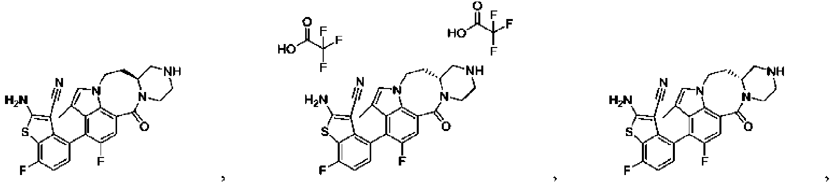

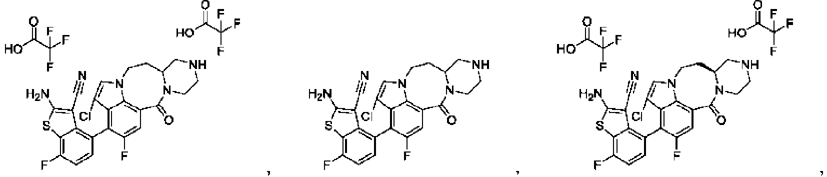

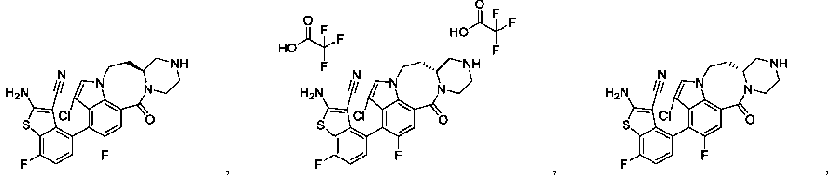

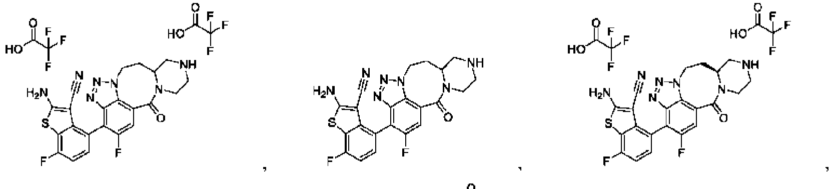

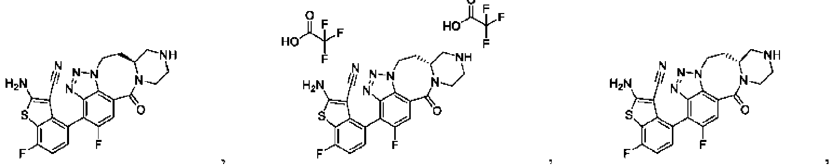

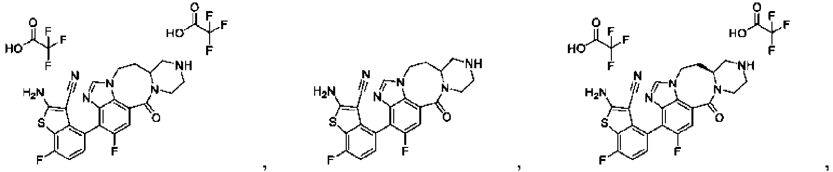

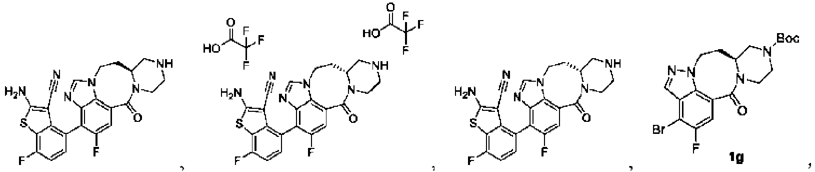

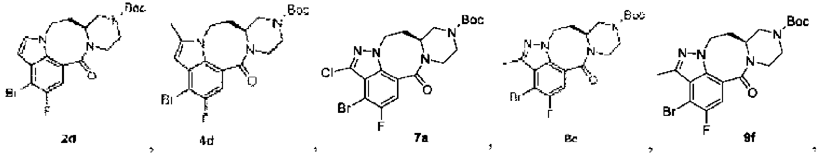

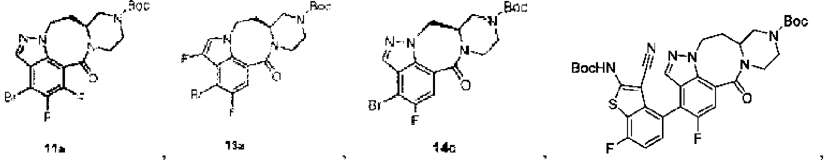

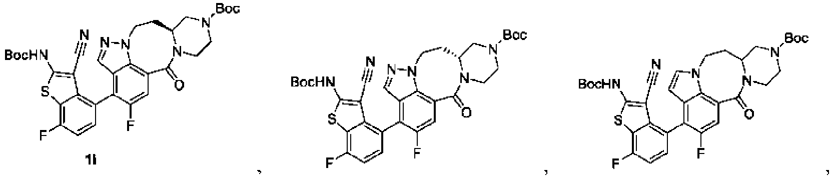

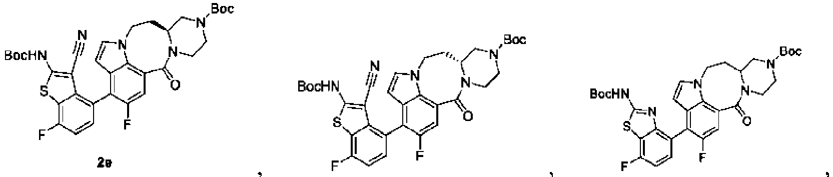

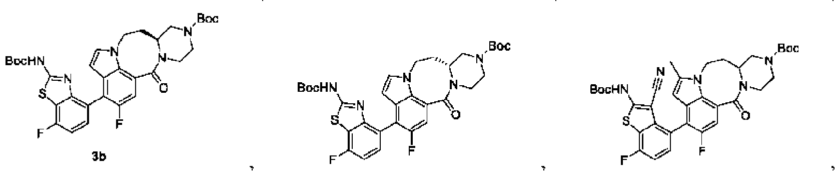

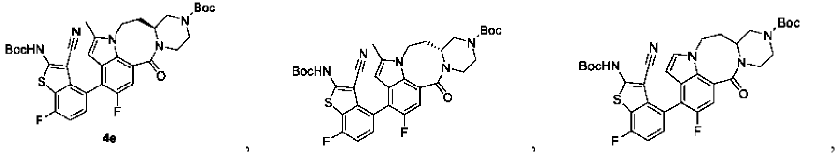

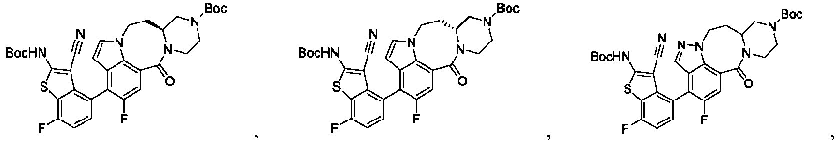

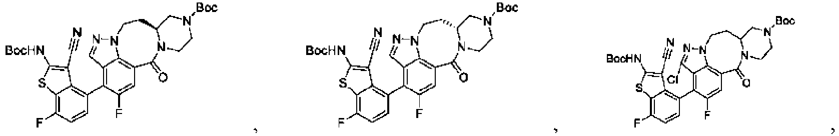

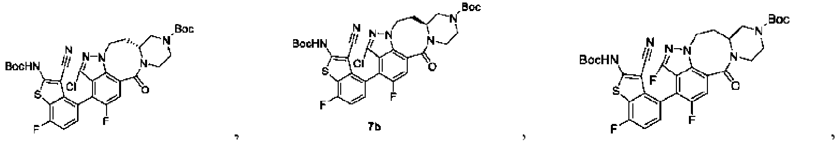

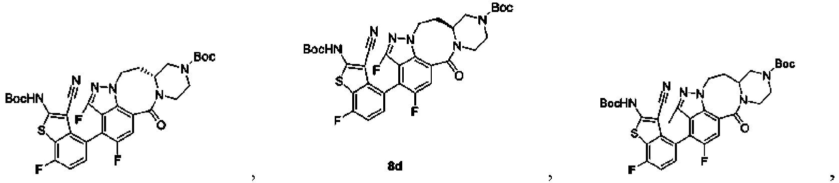

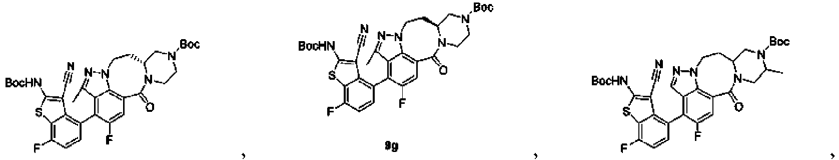

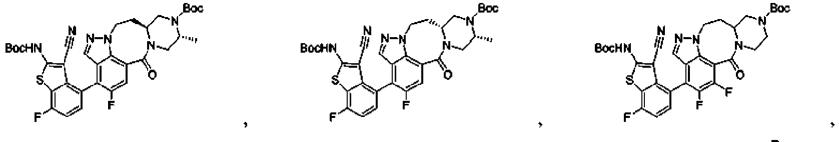

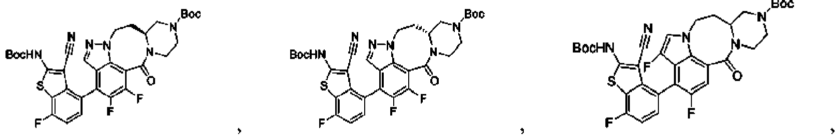

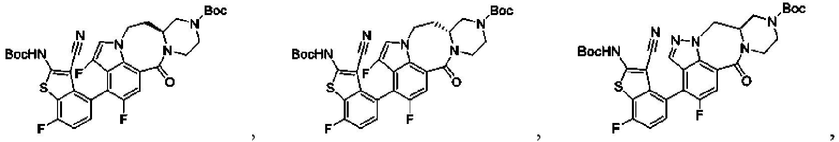

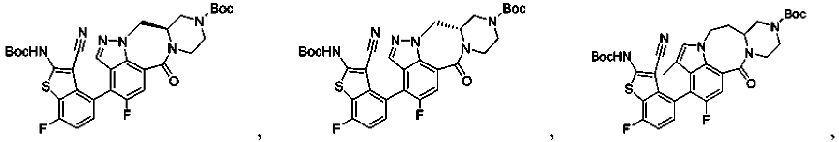

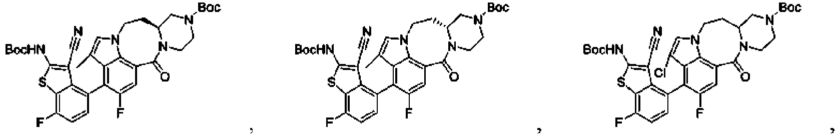

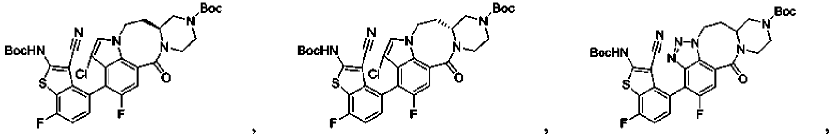

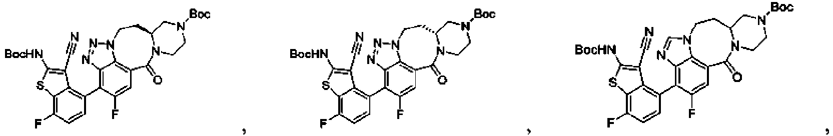

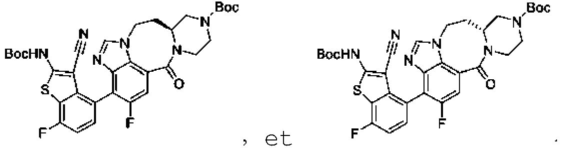

12. Procédé permettant de préparer un composé représenté par la formule générale (IM) ou sel pharmaceutiquement acceptable de celui-ci selon la revendication 1, comprenant : 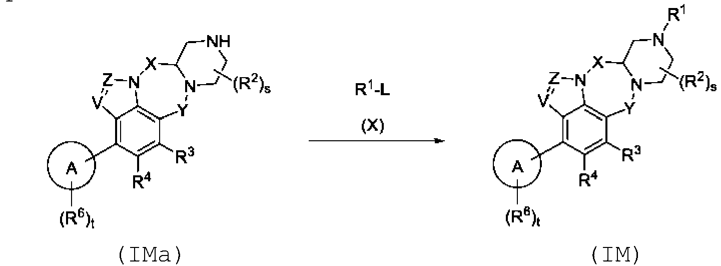 la réalisation d'une réaction d'un composé représenté par la formule générale (IMa) ou d'un sel de celui-ci avec un composé de formule générale (X) ou un sel de celui-ci pour obtenir le composé représenté par la formule générale (IM) ou un sel pharmaceutiquement acceptable de celui-ci ;
dans lequel :
L représente un halogène ; de préférence, L représente Cl ;
R¹ représente 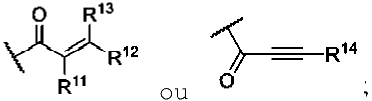
les cycles A, V, X, Y, Z, R², R³, R⁴, R⁶, R¹¹, R¹², R¹³, R¹⁴, s et t sont tels que définis dans la revendication 1.

13. Composition pharmaceutique, dans laquelle la composition pharmaceutique comprend le composé représenté par la formule générale (IM) ou le sel pharmaceutiquement acceptable de celui-ci selon l'une quelconque des revendications 1 à 9, et un ou plusieurs supports, diluants ou excipients pharmaceutiquement acceptables.

14. Composé représenté par la formule générale (IM) ou sel pharmaceutiquement acceptable de celui-ci selon l'une quelconque des revendications 1 à 9, ou composition pharmaceutique selon la revendication 13 destinée à être utilisée comme médicament, de préférence destinée à être utilisée comme inhibiteur de KRAS G12C.

15. Composé représenté par la formule générale (IM) ou sel pharmaceutiquement acceptable de celui-ci selon l'une quelconque des revendications 1 à 9, ou composition pharmaceutique selon la revendication 13 destinée à être utilisée dans le traitement et/ou la prévention d'une tumeur ; de préférence, la tumeur est un cancer ; plus préférablement, le cancer est sélectionné parmi le groupe constitué de cancer du poumon, cancer du pancréas, cancer du col de l'utérus, cancer de l'œsophage, cancer de l'endomètre, cancer de l'ovaire, cholangiocarcinome, cancer colorectal, cancer du foie, cancer du sein, cancer de la prostate, cancer de la thyroïde, cancer de l'estomac, cancer urothélial, cancer des testicules, leucémie, cancer de la peau, cancer épidermoïde, cancer basocellulaire, cancer de la vessie, cancer de la tête et du cou, cancer du rein, cancer du nasopharynx, cancer des os, lymphome, mélanome, sarcome, neuroépithéliome périphérique, gliome, tumeur cérébrale et myélome ; le plus préférablement, le cancer est sélectionné parmi le groupe constitué de cancer du poumon, cancer du pancréas, cancer du col de l'utérus, cancer de l'œsophage, cancer de l'endomètre, cancer de l'ovaire, cholangiocarcinome et cancer colorectal.
